# EUROPEAN PATENT APPLICATION

(11) **EP 1 498 483 A1**
(43) Date of publication of application: **19.01.2005**
(21) Application number: 03719119.4
(22) Date of filing: 16.04.2003
(51) Int. Cl.: C12N 15/09, C12N 5/10, C07K 14/705, C12Q 1/02

(54) **ENDOCRINE CELL LINES AND METHOD OF USING THE SAME**

(30) Priority: 16.04.2002 JP 2002113030
(71) Applicant: KYOWA HAKKO KOGYO CO., LTD., Chiyoda-ku, Tokyo 100-8185 (JP)
(72) Inventor: SASAKI, Katsutoshi, c/o Kyowa Hakko Kogyo Co. Ltd., Machida, Tokyo 194-8533 (JP); MIURA, Kazumi, c/o Kyowa Hakko Kogyo Co. Ltd., Machida-shi, Tokyo 194-8533 (JP); SAEKI, Satoshi, c/o Kyowa Hakko Kogyo Co. Ltd., Machida-shi, Tokyo 194-8533 (JP); YOSHIZAWA, Misako, c/o Kyowa Hakko Kogyo Co. Ltd., Machida-shi, Tokyo 194-8533 (JP); KISHIMOTO, Kazuya, c/o Kyowa Hakko Kogyo Co. Ltd., Sunto-gun, Shizuoka 411 (JP); KUNITOMO, Hirofumi, Mitaka-shi, Tokyo 181-0015 (JP); NISHI, Tatsunari, Ota-ku, Tokyo 145-0074 (JP); OBINATA, Masuo, Sendai-shi, Miyagi 980-0871 (JP)
(74) Representative: Williams, Aylsa
(86) International application number: PCT/JP2003/004840
(87) International publication number: WO 2003/087366

(57) **Abstract**

In accordance with the present invention, there are provided (1) various cell lines derived from hypothalamus and Langerhans islets of mammals, (2) process for producing an active peptide and expression cloning system of active peptide precursor gene using the cell line as a host, (3) a method of screening or evaluating a substance capable of acting on the cells using the cell line, (4) a method of screening or isolating a useful gene or useful peptide using the cell line and (5) a highly-sensitive and simple assay system for GPCR ligand used in the above expression cloning system.

SEQUENCE LISTING

<110>  Kyowa Hakko Kogyo Co., Ltd.

<120>  Endocrine cell lines and use thereof

<130>  1480

<150>  JP 2002-113030
<151>  2002-04-16

<160>  198

<170>  PatentIn version 3.1

<210>  1
<211>  24
<212>  DNA
<213>  Artificial

<220>
<223>  Inventor: Sasaki, Katsutoshi; Miura, Kazumi; Saeki, Satoshi;
       Inventor: Yoshizawa, Misako; Kishimoto, Kazuya;
       Inventor: Kunitomo, Hirofumi; Nishi, Tatsunari; Obinata, Masuo

<220>
<223>  synthetic DNA

<400>  1
atgaagtggc ttagaatccc ttcg                                              24

<210>  2
<211>  21
<212>  DNA
<213>  Artificial

<220>
<223>  synthetic DNA

<400> 2
atatcactga ttctgcatgc t 21

<210> 3
<211> 23
<212> DNA
<213> Artificial

<220>
<223> synthetic DNA

<400> 3
gttgattccc agcctcaggc atc 23

<210> 4
<211> 23
<212> DNA
<213> Artificial

<220>
<223> synthetic DNA

<400> 4
tgaggaacga gctgcagcaa cgg 23

<210> 5
<211> 23
<212> DNA
<213> Artificial

<220>
<223> synthetic DNA

<400> 5
gatgccccat tttcacagca aag 23

<210> 6
<211> 23
<212> DNA
<213> Artificial

<220>
<223> synthetic DNA

<400> 6
gttctcccaa acctttgggg cag 23


<210> 7
<211> 25
<212> DNA
<213> Artificial

<220>
<223> synthetic DNA

<400> 7
ctgagctcca gacaccatga acctt 25


<210> 8
<211> 23
<212> DNA
<213> Artificial

<220>
<223> synthetic DNA

<400> 8
ttaccgttgg cctgaaggag gcg 23


<210> 9
<211> 23
<212> DNA
<213> Artificial

<220>
<223>  synthetic DNA

<400>  9
caggacctca ccacggaaag caa                                              23


<210>  10
<211>  21
<212>  DNA
<213>  Artificial

<220>
<223>  synthetic DNA

<400>  10
ggggcgtctg gctcttctcg g                                                21


<210>  11
<211>  22
<212>  DNA
<213>  Artificial

<220>
<223>  synthetic DNA

<400>  11
ggtacccctc caagccggac aa                                               22


<210>  12
<211>  26
<212>  DNA
<213>  Artificial

<220>
<223>  synthetic DNA

<400> 12
gcaagtttca tttcccatca ccacat 26

<210> 13
<211> 22
<212> DNA
<213> Artificial

<220>
<223> synthetic DNA

<400> 13
ctgctgtgct gctgctactg ct 22

<210> 14
<211> 23
<212> DNA
<213> Artificial

<220>
<223> synthetic DNA

<400> 14
   caaacctctg gggctgaaac aag 23

<210> 15
<211> 22
<212> DNA
<213> Artificial

<220>
<223> synthetic DNA

<400> 15
ctccgcctgg ggaacctcaa ca 22

<210> 16
<211> 24
<212> DNA
<213> Artificial

<220>
<223> synthetic DNA

<400> 16
tttcctgttg ctgtgagctt gctg 24


<210> 17
<211> 25
<212> DNA
<213> Artificial

<220>
<223> synthetic DNA

<400> 17
aaaagggagg aggaggaaaa agaca 25


<210> 18
<211> 24
<212> DNA
<213> Artificial

<220>
<223> synthetic DNA

<400> 18
agggaacagg atagggaata cagg 24


<210> 19
<211> 22
<212> DNA
<213> Artificial

<220>
<223>  synthetic DNA

<400>  19
cccagagcac cagaaagccc ag                                                22

<210>  20
<211>  22
<212>  DNA
<213>  Artificial

<220>
<223>  synthetic DNA

<400>  20
ggcgcctctt tgacctcttc cc                                                22

<210>  21
<211>  24
<212>  DNA
<213>  Artificial

<220>
<223>  synthetic DNA

<400>  21
gaccacaaag aacacaggct ccaa                                              24

<210>  22
<211>  24
<212>  DNA
<213>  Artificial

<220>
<223>  synthetic DNA

<400>  22
caggtatcag acttgccaac aggg                                              24

<210>  23
<211>  24
<212>  DNA
<213>  Artificial

<220>
<223>  synthetic DNA

<400>  23
catctactct gccgtggatg atgc                                              24

<210>  24
<211>  23
<212>  DNA
<213>  Artificial

<220>
<223>  synthetic DNA

<400>  24
gttcctcggg gacagtcaca cag                                               23

<210>  25
<211>  24
<212>  DNA
<213>  Artificial

<220>
<223>  synthetic DNA

<400>  25
ccagcatcca tggcatcaag ttcc                                              24

<210>  26
<211>  25
<212>  DNA
<213>  Artificial

<220>
<223>  synthetic DNA

<400>  26
gaggaccaag acaaacagca tcttg                                                25


<210>  27
<211>  23
<212>  DNA
<213>  Artificial

<220>
<223>  synthetic DNA

<400>  27
tgacggcgat ggtgacgagc gcc                                                  23


<210>  28
<211>  23
<212>  DNA
<213>  Artificial

<220>
<223>  synthetic DNA

<400>  28
cagcctccca acagcagttg gcc                                                  23


<210>  29
<211>  24
<212>  DNA
<213>  Artificial

<220>
<223>  synthetic DNA

<400>  29
ggtgtttgtg actctgggtg tcat                                              24

<210>  30
<211>  23
<212>  DNA
<213>  Artificial

<220>
<223>  synthetic DNA

<400>  30
gtgcaagctg cccagataca act                                               23

<210>  31
<211>  27
<212>  DNA
<213>  Artificial

<220>
<223>  synthetic DNA

<400>  31
gaaaaggaga aacaacatga tggacaa                                           27

<210>  32
<211>  25
<212>  DNA
<213>  Artificial

<220>
<223>  synthetic DNA

<400>  32
agaagaactg caagtctctc tggaa                                          25

<210>  33
<211>  23
<212>  DNA
<213>  Artificial

<220>
<223>  synthetic DNA

<400>  33
ggctttgcca tctgttctcc cct                                            23

<210>  34
<211>  27
<212>  DNA
<213>  Artificial

<220>
<223>  synthetic DNA

<400>  34
cattttcttc gagtctgttt tctttgt                                        27

<210>  35
<211>  24
<212>  DNA
<213>  Artificial

<220>
<223>  synthetic DNA

<400>  35
gaagcagcat gtgcaagatc agtg                                           24

<210>  36
<211>  28
<212>  DNA
<213>  Artificial

<220>
<223>  synthetic DNA

<400>  36
caatccttgc atacatgata acaatgag                                          28


<210>  37
<211>  20
<212>  DNA
<213>  Artificial

<220>
<223>  synthetic DNA

<400>  37
caatggctac cgggagtgcc                                                   20


<210>  38
<211>  20
<212>  DNA
<213>  Artificial

<220>
<223>  synthetic DNA

<400>  38
gtacgtgagc acaatggctg                                                   20


<210>  39
<211>  23
<212>  DNA
<213>  Artificial

<220>
<223>  synthetic DNA

<400>  39
ggatgacaag cagaggaagt atg                                              23

<210>  40
<211>  23
<212>  DNA
<213>  Artificial

<220>
<223>  synthetic DNA

<400>  40
gcaggtagca gccttccaca aac                                              23

<210>  41
<211>  22
<212>  DNA
<213>  Artificial

<220>
<223>  synthetic DNA

<400>  41
cttccgcgag ctgcgcacca cc                                               22

<210>  42
<211>  22
<212>  DNA
<213>  Artificial

<220>
<223>  synthetic DNA

<400>  42
gctggacacc cacaccatga cg                                                22

<210>  43
<211>  22
<212>  DNA
<213>  Artificial

<220>
<223>  synthetic DNA

<400>  43
gcctttcatg atccaccagc tc                                                22

<210>  44
<211>  23
<212>  DNA
<213>  Artificial

<220>
<223>  synthetic DNA

<400>  44
gtgaaccagt agaggtcagt gtc                                               23

<210>  45
<211>  22
<212>  DNA
<213>  Artificial

<220>
<223>  synthetic DNA

<400>  45
accgacctgc ctgcaagatc cc                                                22

<210> 46
<211> 24
<212> DNA
<213> Artificial

<220>
<223> synthetic DNA

<400> 46
gctcccagtc tgctgcatag aagg 24


<210> 47
<211> 23
<212> DNA
<213> Artificial

<220>
<223> synthetic DNA

<400> 47
gaccttcagc tccaagagcg agt 23


<210> 48
<211> 22
<212> DNA
<213> Artificial

<220>
<223> synthetic DNA

<400> 48
gtgaagaagt ggcgctggtc cg 22


<210> 49
<211> 31
<212> DNA
<213> Artificial

<220>
<223>  synthetic DNA

<400>  49
aataagcttg tgcacaggat gcctgacacc a                                    31


<210>  50
<211>  29
<212>  DNA
<213>  Artificial

<220>
<223>  synthetic DNA

<400>  50
aaatgcggcc gctcagtagg cgtcgggct                                       29


<210>  51
<211>  32
<212>  DNA
<213>  Artificial

<220>
<223>  synthetic DNA

<400>  51
gtataagctt gagtgcccct aacatgcggc tg                                   32


<210>  52
<211>  37
<212>  DNA
<213>  Artificial

<220>
<223>  synthetic DNA

<400>  52
aaatgcggcc gcttggccaa acgcaccgtt ttatttc                                37

<210>  53
<211>  28
<212>  DNA
<213>  Artificial

<220>
<223>  synthetic DNA

<400>  53
tgcccaggct tttgtcaaac agcacctt                                          28

<210>  54
<211>  20
<212>  DNA
<213>  Artificial

<220>
<223>  synthetic DNA

<400>  54
ctccagtgcc aaggtctgaa                                                   20

<210>  55
<211>  24
<212>  DNA
<213>  Artificial

<220>
<223>  synthetic DNA

<400>  55
gtggctggat tgtttgtaat gctg                                              24

<210> 56
<211> 24
<212> DNA
<213> Artificial

<220>
<223> synthetic DNA

<400> 56
cggttcctct tggtgttcat caac 24


<210> 57
<211> 21
<212> DNA
<213> Artificial

<220>
<223> synthetic DNA

<400> 57
ccctcggacc ccagactccg t 21


<210> 58
<211> 23
<212> DNA
<213> Artificial

<220>
<223> synthetic DNA

<400> 58
ttcttgcagc cagctttgcg ttc 23


<210> 59
<211> 24
<212> DNA
<213> Artificial

<220>
<223>  synthetic DNA

<400>  59
tgaacagagg gctcaatacg aaac                                              24


<210>  60
<211>  24
<212>  DNA
<213>  Artificial

<220>
<223>  synthetic DNA

<400>  60
agacagaagg gaggctacaa atcc                                              24


<210>  61
<211>  26
<212>  DNA
<213>  Artificial

<220>
<223>  synthetic DNA

<400>  61
taatgatgat ggaaaaactg tggagg                                            26


<210>  62
<211>  26
<212>  DNA
<213>  Artificial

<220>
<223>  synthetic DNA

<400>  62
tgctgataga gatggtgtaa atgctg                                          26

<210>  63
<211>  24
<212>  DNA
<213>  Artificial

<220>
<223>  synthetic DNA

<400>  63
actctgaggc attctgggac atct                                            24

<210>  64
<211>  25
<212>  DNA
<213>  Artificial

<220>
<223>  synthetic DNA

<400>  64
gtcatgttga tgttcaggtc tcctc                                           25

<210>  65
<211>  21
<212>  DNA
<213>  Artificial

<220>
<223>  synthetic DNA

<400>  65
gcagactcgc gaagtccact c                                               21

<210> 66
<211> 20
<212> DNA
<213> Artificial

<220>
<223> synthetic DNA

<400> 66
gcaggtggct gcatacacgc 20


<210> 67
<211> 20
<212> DNA
<213> Artificial

<220>
<223> synthetic DNA

<400> 67
gccgggtgga gctggcagtg 20


<210> 68
<211> 20
<212> DNA
<213> Artificial

<220>
<223> synthetic DNA

<400> 68
cggtcgcagt ggcgcgatgc 20


<210> 69
<211> 23
<212> DNA
<213> Artificial

<220>
<223>  synthetic DNA

<400>  69
gcgataagag ggatgcgacc ctg                                              23


<210>  70
<211>  24
<212>  DNA
<213>  Artificial

<220>
<223>  synthetic DNA

<400>  70
ccactgaatc aggatactgc ccac                                             24


<210>  71
<211>  24
<212>  DNA
<213>  Artificial

<220>
<223>  synthetic DNA

<400>  71
gagtgcccgt ccatctttgc ttgg                                             24


<210>  72
<211>  24
<212>  DNA
<213>  Artificial

<220>
<223>  synthetic DNA

<400> 72
tttcttctct tctccacttg gccc 24

<210> 73
<211> 24
<212> DNA
<213> Artificial

<220>
<223> synthetic DNA

<400> 73
cccggagcct cggaccacga agtg 24

<210> 74
<211> 24
<212> DNA
<213> Artificial

<220>
<223> synthetic DNA

<400> 74
aagctgtggt ccgctatgcg cagc 24

<210> 75
<211> 24
<212> DNA
<213> Artificial

<220>
<223> synthetic DNA

<400> 75
ggacagacga gtgcctcagt tctc 24

<210> 76
<211> 24
<212> DNA
<213> Artificial

<220>
<223> synthetic DNA

<400> 76
gggcttttgc ctgagcgcag gatc 24


<210> 77
<211> 29
<212> DNA
<213> Artificial

<220>
<223> synthetic DNA

<400> 77
gaaccatgtc gctgaccaac acaaagacg 29


<210> 78
<211> 25
<212> DNA
<213> Artificial

<220>
<223> synthetic DNA

<400> 78
cgccctgggt ctccttgtca ttgtc 25


<210> 79
<211> 24
<212> DNA
<213> Artificial

<220>
<223>  synthetic DNA

<400>  79
atcttctggc ctggggtgat gcag                                              24


<210>  80
<211>  24
<212>  DNA
<213>  Artificial

<220>
<223>  synthetic DNA

<400>  80
gtctccgagt cctgcttctt cttg                                              24


<210>  81
<211>  20
<212>  DNA
<213>  Artificial

<220>
<223>  synthetic DNA

<400>  81
aagggaacaa catcgtagga                                                   20


<210>  82
<211>  20
<212>  DNA
<213>  Artificial

<220>
<223>  synthetic DNA

<400>  82
cattggcggt cttcatagta                                                    20

<210>  83
<211>  20
<212>  DNA
<213>  Artificial

<220>
<223>  synthetic DNA

<400>  83
ttagcaactg ggtctgcaat                                                    20

<210>  84
<211>  20
<212>  DNA
<213>  Artificial

<220>
<223>  synthetic DNA

<400>  84
ggtgtagtcc tacactcatg                                                    20

<210>  85
<211>  21
<212>  DNA
<213>  Artificial

<220>
<223>  synthetic DNA

<400>  85
ggactcgact gccccaggca g                                                  21

<210> 86
<211> 20
<212> DNA
<213> Artificial

<220>
<223> synthetic DNA

<400> 86
ccacctgctc acaccgggcc 20


<210> 87
<211> 54
<212> DNA
<213> Artificial

<220>
<223> synthetic DNA

<400> 87
tcgacaaata aagcaatagc atcacaaatt tcacaaataa agcatttttt tcaa 54


<210> 88
<211> 54
<212> DNA
<213> Artificial

<220>
<223> synthetic DNA

<400> 88
tgcattgaaa aaaatgcttt atttgtgaaa tttgtgatgc tattgcttta tttg 54


<210> 89
<211> 39
<212> DNA
<213> Artificial

<220>
<223>  synthetic DNA

<400>  89
tgcattctag ttgtggtttg tccaaactcg agcccgggg                                39

<210>  90
<211>  39
<212>  DNA
<213>  Artificial

<220>
<223>  synthetic DNA

<400>  90
gtacccccgg gctcgagttt ggacaaacca caactagaa                               39

<210>  91
<211>  40
<212>  DNA
<213>  Artificial

<220>
<223>  synthetic DNA

<400>  91
tcgacggtat cgattcgact gacgtcatac ttgacgtcac                               40

<210>  92
<211>  40
<212>  DNA
<213>  Artificial

<220>
<223>  synthetic DNA

<400>  92
tcgagtgacg tcaagtatga cgtcagtcga atcgataccg                                40

<210>  93
<211>  29
<212>  DNA
<213>  Artificial

<220>
<223>  synthetic DNA

<400>  93
tataagcttg ccgccgccat gggctgcct                                            29

<210>  94
<211>  34
<212>  DNA
<213>  Artificial

<220>
<223>  synthetic DNA

<400>  94
attgttacct ctcttagagc agctcgtact gacg                                      34

<210>  95
<211>  28
<212>  DNA
<213>  Artificial

<220>
<223>  synthetic DNA

<400>  95
caccttcgtg agtacaatct ggtctaac                                             28

<210>  96
<211>  36
<212>  DNA
<213>  Artificial

<220>
<223>  synthetic DNA

<400>  96
tcgagttaga ccagattgta ctcacgaagg tgcatg                                36


<210>  97
<211>  26
<212>  DNA
<213>  Artificial

<220>
<223>  synthetic DNA

<400>  97
caccttcgtg attgtggtct ctttta                                          26


<210>  98
<211>  35
<212>  DNA
<213>  Artificial

<220>
<223>  synthetic DNA

<400>  98
aagcttaaaa gagaccacaa tcacgaaggt gcatg                                35


<210>  99
<211>  30
<212>  DNA
<213>  Artificial

<220>
<223>  synthetic DNA

<400>  99
tatggatcca gccccaccat gctcatggcg                                        30


<210>  100
<211>  32
<212>  DNA
<213>  Artificial

<220>
<223>  synthetic DNA

<400>  100
aatggtacct cctcacgatg aagtgtcctt gg                                     32


<210>  101
<211>  28
<212>  DNA
<213>  Artificial

<220>
<223>  synthetic DNA

<400>  101
agccaagctt gcccgaggat gggagggc                                          28


<210>  102
<211>  39
<212>  DNA
<213>  Artificial

<220>
<223>  synthetic DNA

<400>  102
ctcgaggcgg ccgctcagac tgctgtggac tgcttgatg                              39


<210>  103
<211>  49
<212>  DNA
<213>  Artificial

<220>
<223>  synthetic DNA

<400>  103
gttcatttca aagcttccgc catggcatca tcatcctggc cccctctag                   49


<210>  104
<211>  41
<212>  DNA
<213>  Artificial

<220>
<223>  synthetic DNA

<400>  104
ttggttcaat ggtaccttaa ttccgccaga aaagttggaa g                           41


<210>  105
<211>  34
<212>  DNA
<213>  Artificial

<220>
<223>  synthetic DNA

<400>  105
agtcaagctt cctttctcct gcaggtacca tgat                                   34

<210> 106
<211> 37
<212> DNA
<213> Artificial

<220>
<223> synthetic DNA

<400> 106
tttatagcgg ccgctcagct agacatcact gggggag 37


<210> 107
<211> 29
<212> DNA
<213> Artificial

<220>
<223> synthetic DNA

<400> 107
gccccagaag cttaagtgcc caccatggg 29


<210> 108
<211> 33
<212> DNA
<213> Artificial

<220>
<223> synthetic DNA

<400> 108
gttcattgtg gcggccgcag catcttcagc tgc 33


<210> 109
<211> 32
<212> DNA
<213> Artificial

<220>
<223>  synthetic DNA

<400>  109
agtcaagctt acgcctgcac tccctcccta tg                                    32


<210>  110
<211>  35
<212>  DNA
<213>  Artificial

<220>
<223>  synthetic DNA

<400>  110
tttatagcgg ccgcttcaga cctcgctggg agacc                                 35


<210>  111
<211>  35
<212>  DNA
<213>  Artificial

<220>
<223>  synthetic DNA

<400>  111
agtcaagctt gttgaagagg acaggggtta aaatg                                 35


<210>  112
<211>  37
<212>  DNA
<213>  Artificial

<220>
<223>  synthetic DNA

<400> 112
tttatagcgg ccgcaagggt gctacacatc actgggc 37

<210> 113
<211> 32
<212> DNA
<213> Artificial

<220>
<223> synthetic DNA

<400> 113
agtcaagctt caggcctcat agccagccat gg 32

<210> 114
<211> 33
<212> DNA
<213> Artificial

<220>
<223> synthetic DNA

<400> 114
ttatgcggcc gcgctaccag gaggccgagg cag 33

<210> 115
<211> 39
<212> DNA
<213> Artificial

<220>
<223> synthetic DNA

<400> 115
ctgagcgccg aagcttggcg cgcaccatga actcgtggg 39

<210> 116
<211> 38
<212> DNA
<213> Artificial

<220>
<223> synthetic DNA

<400> 116
ctaaactctt cagcggccgc gcggtccttc actcagac 38


<210> 117
<211> 33
<212> DNA
<213> Artificial

<220>
<223> synthetic DNA

<400> 117
tactaagctt ggcgcagaga catggatgtg act 33


<210> 118
<211> 35
<212> DNA
<213> Artificial

<220>
<223> synthetic DNA

<400> 118
aatagcggcc gcaaggctgt ctacacggca ctgct 35


<210> 119
<211> 38
<212> DNA
<213> Artificial

<220>
<223>  synthetic DNA

<400>  119
acgtaagctt ccaccatgaa tggcacctac aacacctg                                38

<210>  120
<211>  37
<212>  DNA
<213>  Artificial

<220>
<223>  synthetic DNA

<400>  120
ctcgaggcgg ccgcttaggc gagggtcacg cacagag                                 37

<210>  121
<211>  34
<212>  DNA
<213>  Artificial

<220>
<223>  synthetic DNA

<400>  121
gcatctgctt gctgtagcca agcttgcagg tatc                                    34

<210>  122
<211>  35
<212>  DNA
<213>  Artificial

<220>
<223>  synthetic DNA

<400> 122
gatccaaaac cgcggccgca ttacatttga tttac 35

<210> 123
<211> 35
<212> DNA
<213> Artificial

<220>
<223> synthetic DNA

<400> 123
agtcaagctt gttgaagagg acaggggtta aaatg 35

<210> 124
<211> 37
<212> DNA
<213> Artificial

<220>
<223> synthetic DNA

<400> 124
tttatagcgg ccgcaagggt gctacacatc actgggc 37

<210> 125
<211> 42
<212> DNA
<213> Artificial

<220>
<223> synthetic DNA

<400> 125
agtcaagctt gccgccacca tggccaactc cacagggctg aa 42

<210> 126
<211> 23
<212> DNA
<213> Artificial

<220>
<223> synthetic DNA

<400> 126
tgtgacctgt gcaggggttg gat 23


<210> 127
<211> 36
<212> DNA
<213> Artificial

<220>
<223> synthetic DNA

<400> 127
ggaagcttcc accatgacca actcctcctc cacatc 36


<210> 128
<211> 28
<212> DNA
<213> Artificial

<220>
<223> synthetic DNA

<400> 128
cgctcgagtt accagtgctg gcccgcgg 28


<210> 129
<211> 40
<212> DNA
<213> Artificial

<220>
<223>  synthetic DNA

<400>  129
cagtccaagc ttccaccatg ttagccaaca gctcctcaac                                40


<210>  130
<211>  35
<212>  DNA
<213>  Artificial

<220>
<223>  synthetic DNA

<400>  130
gttatagcgg ccgctcagag ggcggaatcc tgggg                                     35


<210>  131
<211>  35
<212>  DNA
<213>  Artificial

<220>
<223>  synthetic DNA

<400>  131
gaactaatat aattgcaagc ttaaaaagga aaaaa                                     35


<210>  132
<211>  33
<212>  DNA
<213>  Artificial

<220>
<223>  synthetic DNA

<400>  132
cttaaacttc gcggccgctc aaaacatcct tgg                                    33


<210>  133
<211>  33
<212>  DNA
<213>  Artificial

<220>
<223>  synthetic DNA

<400>  133
gcccacccca agcttaggtg cactgaccat gag                                    33


<210>  134
<211>  37
<212>  DNA
<213>  Artificial

<220>
<223>  synthetic DNA

<400>  134
gggaaaacgc ggccgctgag aggcttataa agcacgc                                37


<210>  135
<211>  38
<212>  DNA
<213>  Artificial

<220>
<223>  synthetic DNA

<400>  135
agtcaagctt ccaccatgca ggcgcttaac attacccc                               38

<210> 136
<211> 40
<212> DNA
<213> Artificial

<220>
<223> synthetic DNA

<400> 136
gtttatagcg gccgcttaat gcccactgtc taaaggagaa 40

<210> 137
<211> 35
<212> DNA
<213> Artificial

<220>
<223> synthetic DNA

<400> 137
gggaagatga gaagcttctg ccgacggatg ctggc 35

<210> 138
<211> 34
<212> DNA
<213> Artificial

<220>
<223> synthetic DNA

<400> 138
gaaccacatt ggcggccgca ggacccccaa cctg 34

<210> 139
<211> 32
<212> DNA
<213> Artificial

<220>
<223>  synthetic DNA

<400>  139
gagcccatga gaagcttggc cccttcaggc cc                                    32


<210>  140
<211>  33
<212>  DNA
<213>  Artificial

<220>
<223>  synthetic DNA

<400>  140
ctgaaggctg cggccgcacg tggagccacc cgc                                   33


<210>  141
<211>  48
<212>  DNA
<213>  Artificial

<220>
<223>  synthetic DNA

<400>  141
agtcaagctt ccaccatggc taaccttgac aaatacactg aaacattc                   48


<210>  142
<211>  41
<212>  DNA
<213>  Artificial

<220>
<223>  synthetic DNA

<400> 142
tttatagcgg ccgcttagcc aatcattgat gaaccactct c 41

<210> 143
<211> 37
<212> DNA
<213> Artificial

<220>
<223> synthetic DNA

<400> 143
cagtcaagct tccaccatga cgtccacctg caccaac 37

<210> 144
<211> 40
<212> DNA
<213> Artificial

<220>
<223> synthetic DNA

<400> 144
gttatagcgg ccgcggacag ttcaaggttt gccttagaac 40

<210> 145
<211> 38
<212> DNA
<213> Artificial

<220>
<223> synthetic DNA

<400> 145
cagtcaagct tccaccatgc aggtcccgaa cagcaccg 38

<210> 146
<211> 36
<212> DNA
<213> Artificial

<220>
<223> synthetic DNA

<400> 146
gttatagcgg ccgctcagaa cacactctcc tgcctc 36


<210> 147
<211> 41
<212> DNA
<213> Artificial

<220>
<223> synthetic DNA

<400> 147
gaccgctcga gccaccatga accagacttt gaatagcagt g 41


<210> 148
<211> 31
<212> DNA
<213> Artificial

<220>
<223> synthetic DNA

<400> 148
ctcgaggtac cacctgtggg cggctctcaa g 31


<210> 149
<211> 25
<212> DNA
<213> Artificial

<220>
<223>  synthetic DNA

<400>  149
cggagactct agagggtata taatg                                          25


<210>  150
<211>  21
<212>  DNA
<213>  Artificial

<220>
<223>  synthetic DNA

<400>  150
ctaatacgac tcactatagg g                                              21


<210>  151
<211>  24
<212>  DNA
<213>  Artificial

<220>
<223>  synthetic DNA

<220>
<221>  misc_feature
<222>  (8)..(9)
<223>  n is a, g, c or t


<400>  151
ggccgcgnns agcctggagc gcat                                           24


<210>  152
<211>  24

<212>  DNA
<213>  Artificial

<220>
<223>  synthetic DNA

<220>
<221>  misc_feature
<222>  (11)..(12)
<223>  n is a, g c or t


<400>  152
ggccgcggtc nnsctggagc gcat                                              24


<210>  153
<211>  24
<212>  DNA
<213>  Artificial

<220>
<223>  synthetic DNA

<220>
<221>  misc_feature
<222>  (11)..(12)
<223>  n is a, g, c or t


<400>  153
cgcggtcagc nnsgagcgca tggt                                              24


<210>  154
<211>  24
<212>  DNA
<213>  Artificial

<220>

<223>  synthetic DNA

<220>
<221>  misc_feature
<222>  (11)..(12)
<223>  n is a, g, c or t


<400>  154
ggtcagcctg nnscgcatgg tgtg                                              24


<210>  155
<211>  25
<212>  DNA
<213>  Artificial

<220>
<223>  synthetic DNA

<220>
<221>  misc_feature
<222>  (11)..(12)
<223>  n is a, g, c or t


<400>  155
cagcctggag nnsatggtgt gcatc                                             25


<210>  156
<211>  25
<212>  DNA
<213>  Artificial

<220>
<223>  synthetic DNA

<220>
<221>  misc_feature

<222>  (11)..(12)
<223>  n is a, g, c or t


<400>  156
cctggagcgc nnsgtgtgca tcgtg                                                25


<210>  157
<211>  25
<212>  DNA
<213>  Artificial

<220>
<223>  synthetic DNA

<220>
<221>  misc_feature
<222>  (11)..(12)
<223>  n is a, g, c or t


<400>  157
ggagcgcatg nnstgcatcg tgcac                                                25


<210>  158
<211>  25
<212>  DNA
<213>  Artificial

<220>
<223>  synthetic DNA

<220>
<221>  misc_feature
<222>  (11)..(12)
<223>  n is a, g, c or t

<400>  158
gcgcatggtg nnsatcgtgc acctg                                          25


<210>  159
<211>  25
<212>  DNA
<213>  Artificial

<220>
<223>  synthetic DNA

<220>
<221>  misc_feature
<222>  (11)..(12)
<223>  n is a, g, c or t


<400>  159
catggtgtgc nnsgtgcacc tgcag                                          25


<210>  160
<211>  24
<212>  DNA
<213>  Artificial

<220>
<223>  synthetic DNA

<220>
<221>  misc_feature
<222>  (11)..(12)
<223>  n is a, g, c or t


<400>  160
ggtgtgcatc nnscacctgc agcg                                           24

<210>  161
<211>  25
<212>  DNA
<213>  Artificial

<220>
<223>  synthetic DNA

<220>
<221>  misc_feature
<222>  (12)..(13)
<223>  n is a, g, c or t


<400>  161
agtcctgctg snncacgcgg atctg                                          25


<210>  162
<211>  25
<212>  DNA
<213>  Artificial

<220>
<223>  synthetic DNA

<220>
<221>  misc_feature
<222>  (12)..(13)
<223>  n is a, g, c or t


<400>  162
ggaagtcctg snnggacacg cggat                                          25


<210>  163
<211>  24
<212>  DNA
<213>  Artificial

<220>
<223>  synthetic DNA

<220>
<221>  misc_feature
<222>  (11)..(12)
<223>  n is a, g, c or t


<400>  163
ccggaagtcs nnctgggaca cgcg                                              24


<210>  164
<211>  24
<212>  DNA
<213>  Artificial

<220>
<223>  synthetic DNA

<220>
<221>  misc_feature
<222>  (11)..(12)
<223>  n is a, g, c or t


<400>  164
gagccggaas nnctgctggg acac                                              24


<210>  165
<211>  25
<212>  DNA
<213>  Artificial

<220>
<223>  synthetic DNA

<220>
<221> misc_feature
<222> (12)..(13)
<223> n is a, g, c or t

<400> 165
ggaagagccg snngtcctgc tggga 25

<210> 166
<211> 25
<212> DNA
<213> Artificial

<220>
<223> synthetic DNA

<220>
<221> misc_feature
<222> (11)..(12)
<223> n is a, g, c or t

<400> 166
gcggaagags nngaagtcct gctgg 25

<210> 167
<211> 24
<212> DNA
<213> Artificial

<220>
<223> synthetic DNA

<220>
<221> misc_feature
<222> (11)..(12)
<223> n is a, g, c or t

<400> 167
ggtgcggaas nnccggaagt cctg 24

<210> 168
<211> 24
<212> DNA
<213> Artificial

<220>
<223> synthetic DNA

<220>
<221> misc_feature
<222> (11)..(12)
<223> n is a, g, c or t

<400> 168
gagggtgcgs nngagccgga agtc 24

<210> 169
<211> 25
<212> DNA
<213> Artificial

<220>
<223> synthetic DNA

<220>
<221> misc_feature
<222> (12)..(13)
<223> n is a, g, c or t

<400> 169
ggaagagggt snngaagagc cggaa 25

<210>  170
<211>  24
<212>  DNA
<213>  Artificial

<220>
<223>  synthetic DNA

<220>
<221>  misc_feature
<222>  (12)..(13)
<223>  n is a, g, c or t


<400>  170
ggaggaagag snngcggaag agcc                                              24


<210>  171
<211>  25
<212>  DNA
<213>  Artificial

<220>
<223>  synthetic DNA

<400>  171
cggagactct agagggtata taatg                                             25


<210>  172
<211>  21
<212>  DNA
<213>  Artificial

<220>
<223>  synthetic DNA

<400> 172
ctaatacgac tcactatagg g 21

<210> 173
<211> 35
<212> DNA
<213> Artificial

<220>
<223> synthetic DNA

<400> 173
ctgctgcatc tccgtggcca gatacctggc tgtgg 35

<210> 174
<211> 35
<212> DNA
<213> Artificial

<220>
<223> synthetic DNA

<400> 174
ccacagccag gtatctggcc acggagatgc agcag 35

<210> 175
<211> 33
<212> DNA
<213> Artificial

<220>
<223> synthetic DNA

<400> 175
ggggtcattg ccatcgcgcg ctactatgct gtc 33

<210>  176
<211>  34
<212>  DNA
<213>  Artificial

<220>
<223>  synthetic DNA

<400>  176
gacagcatag taggcgcgcg atggcaatga cccc                                    34


<210>  177
<211>  34
<212>  DNA
<213>  Artificial

<220>
<223>  synthetic DNA

<400>  177
cacggccatc gccgtggcgc gctatgtggc cgtg                                    34


<210>  178
<211>  34
<212>  DNA
<213>  Artificial

<220>
<223>  synthetic DNA

<400>  178
cacggccaca tagcgcgcca cggcgatggc cgtg                                    34


<210>  179
<211>  35
<212>  DNA
<213>  Artificial

<220>
<223> synthetic DNA

<400> 179
cctgcattgc cgttgcgcgc tatttggctg ttgtc 35


<210> 180
<211> 36
<212> DNA
<213> Artificial

<220>
<223> synthetic DNA

<400> 180
gacaacagcc aaatagcgcg caacggcaat gcaagg 36


<210> 181
<211> 38
<212> DNA
<213> Artificial

<220>
<223> synthetic DNA

<400> 181
acgtaagctt ccaccatgct gccggactgg aagagctc 38


<210> 182
<211> 39
<212> DNA
<213> Artificial

<220>
<223> synthetic DNA

<400> 182
ctcgaggcgg ccggctactc tgtagtgaag tccgaactt 39

<210> 183
<211> 38
<212> DNA
<213> Artificial

<220>
<223> synthetic DNA

<400> 183
acgtaagctt ccaccatgga tacaggcccc gaccagtc 38

<210> 184
<211> 35
<212> DNA
<213> Artificial

<220>
<223> synthetic DNA

<400> 184
ctcgaggcgg ccgctagctt tcagcacagg ccacc 35

<210> 185
<211> 40
<212> DNA
<213> Artificial

<220>
<223> synthetic DNA

<400> 185
agctaagctt gtcccaaatg tcagtgaaac ccagctgggg 40

<210> 186
<211> 40
<212> DNA
<213> Artificial

<220>
<223> synthetic DNA

<400> 186
ctttatagtg cggccgctac ctcagctggg tgtaagaggc 40

<210> 187
<211> 365
<212> PRT
<213> Artificial

<220>
<223> OGR1S221N

<400> 187

Met Gly Asn Ile Thr Ala Asp Asn Ser Ser Met Ser Cys Thr Ile Asp
1               5                   10                  15

His Thr Ile His Gln Thr Leu Ala Pro Val Val Tyr Val Thr Val Leu
            20                  25                  30

Val Val Gly Phe Pro Ala Asn Cys Leu Ser Leu Tyr Phe Gly Tyr Leu
        35                  40                  45

Gln Ile Lys Ala Arg Asn Glu Leu Gly Val Tyr Leu Cys Asn Leu Thr
    50                  55                  60

Val Ala Asp Leu Phe Tyr Ile Cys Ser Leu Pro Phe Trp Leu Gln Tyr
65                  70                  75                  80

Val Leu Gln His Asp Asn Trp Ser His Gly Asp Leu Ser Cys Gln Val
                85                  90                  95

Cys Gly Ile Leu Leu Tyr Glu Asn Ile Tyr Ile Ser Val Gly Phe Leu
            100                 105                 110

Cys Cys Ile Ser Val Asp Arg Tyr Leu Ala Val Ala His Pro Phe Arg
        115                 120                 125

Phe His Gln Phe Arg Thr Leu Lys Ala Ala Val Gly Val Ser Val Val
    130                 135                 140

Ile Trp Ala Lys Glu Leu Leu Thr Ser Ile Tyr Phe Leu Met His Glu
145                 150                 155                 160

Glu Val Ile Glu Asp Glu Asn Gln His Arg Val Cys Phe Glu His Tyr
                165                 170                 175

Pro Ile Gln Ala Trp Gln Arg Ala Ile Asn Tyr Tyr Arg Phe Leu Val
            180                 185                 190

Gly Phe Leu Phe Pro Ile Cys Leu Leu Leu Ala Ser Tyr Gln Gly Ile
        195                 200                 205

Leu Arg Ala Val Arg Arg Ser His Gly Thr Gln Lys Asn Arg Lys Asp
    210                 215                 220

Gln Ile Gln Arg Leu Val Leu Ser Thr Val Val Ile Phe Leu Ala Cys
225                 230                 235                 240

Phe Leu Pro Tyr His Val Leu Leu Leu Val Arg Ser Val Trp Glu Ala
                245                 250                 255

Ser Cys Asp Phe Ala Lys Gly Val Phe Asn Ala Tyr His Phe Ser Leu
            260                 265                 270

Leu Leu Thr Ser Phe Asn Cys Val Ala Asp Pro Val Leu Tyr Cys Phe
        275                 280                 285

Val Ser Glu Thr Thr His Arg Asp Leu Ala Arg Leu Arg Gly Ala Cys
    290                 295                 300

Leu Ala Phe Leu Thr Cys Ser Arg Thr Gly Arg Ala Arg Glu Ala Tyr
305                 310                 315                 320

Pro Leu Gly Ala Pro Glu Ala Ser Gly Lys Ser Gly Ala Gln Gly Glu
                325                 330                 335

Glu Pro Glu Leu Leu Thr Lys Leu His Pro Ala Phe Gln Thr Pro Asn
            340                 345                 350

Ser Pro Gly Ser Gly Gly Phe Pro Thr Gly Arg Leu Ala
        355                 360                 365

<210>  188
<211>  365
<212>  PRT
<213>  Artificial

<220>

<223>  OGR1D118A

<400>  188

Met Gly Asn Ile Thr Ala Asp Asn Ser Ser Met Ser Cys Thr Ile Asp
1               5                   10                  15

His Thr Ile His Gln Thr Leu Ala Pro Val Val Tyr Val Thr Val Leu
            20                  25                  30

Val Val Gly Phe Pro Ala Asn Cys Leu Ser Leu Tyr Phe Gly Tyr Leu
        35                  40                  45

Gln Ile Lys Ala Arg Asn Glu Leu Gly Val Tyr Leu Cys Asn Leu Thr
    50                  55                  60

Val Ala Asp Leu Phe Tyr Ile Cys Ser Leu Pro Phe Trp Leu Gln Tyr
65                  70                  75                  80

Val Leu Gln His Asp Asn Trp Ser His Gly Asp Leu Ser Cys Gln Val
                85                  90                  95

Cys Gly Ile Leu Leu Tyr Glu Asn Ile Tyr Ile Ser Val Gly Phe Leu
            100                 105                 110

Cys Cys Ile Ser Val Ala Arg Tyr Leu Ala Val Ala His Pro Phe Arg
        115                 120                 125

Phe His Gln Phe Arg Thr Leu Lys Ala Ala Val Gly Val Ser Val Val
    130                 135                 140

Ile Trp Ala Lys Glu Leu Leu Thr Ser Ile Tyr Phe Leu Met His Glu
145                 150                 155                 160

Glu Val Ile Glu Asp Glu Asn Gln His Arg Val Cys Phe Glu His Tyr
                165                 170                 175

Pro Ile Gln Ala Trp Gln Arg Ala Ile Asn Tyr Tyr Arg Phe Leu Val
            180                 185                 190

Gly Phe Leu Phe Pro Ile Cys Leu Leu Leu Ala Ser Tyr Gln Gly Ile
        195                 200                 205

Leu Arg Ala Val Arg Arg Ser His Gly Thr Gln Lys Ser Arg Lys Asp
    210                 215                 220

Gln Ile Gln Arg Leu Val Leu Ser Thr Val Val Ile Phe Leu Ala Cys
225                 230                 235                 240

Phe Leu Pro Tyr His Val Leu Leu Leu Val Arg Ser Val Trp Glu Ala
                245                 250                 255

Ser Cys Asp Phe Ala Lys Gly Val Phe Asn Ala Tyr His Phe Ser Leu
            260                 265                 270

Leu Leu Thr Ser Phe Asn Cys Val Ala Asp Pro Val Leu Tyr Cys Phe
        275                 280                 285

Val Ser Glu Thr Thr His Arg Asp Leu Ala Arg Leu Arg Gly Ala Cys
    290                 295                 300

Leu Ala Phe Leu Thr Cys Ser Arg Thr Gly Arg Ala Arg Glu Ala Tyr
305                 310                 315                 320

Pro Leu Gly Ala Pro Glu Ala Ser Gly Lys Ser Gly Ala Gln Gly Glu
                325                 330                 335

Glu Pro Glu Leu Leu Thr Lys Leu His Pro Ala Phe Gln Thr Pro Asn
            340                 345                 350

Ser Pro Gly Ser Gly Gly Phe Pro Thr Gly Arg Leu Ala
        355                 360                 365

<210>  189
<211>  365
<212>  PRT
<213>  Artificial

<220>
<223>  OGR1S221N/D118A

<400>  189

Met Gly Asn Ile Thr Ala Asp Asn Ser Ser Met Ser Cys Thr Ile Asp
1               5                   10                  15

His Thr Ile His Gln Thr Leu Ala Pro Val Val Tyr Val Thr Val Leu
            20                  25                  30

Val Val Gly Phe Pro Ala Asn Cys Leu Ser Leu Tyr Phe Gly Tyr Leu
        35                  40                  45

Gln Ile Lys Ala Arg Asn Glu Leu Gly Val Tyr Leu Cys Asn Leu Thr
    50                  55                  60

Val Ala Asp Leu Phe Tyr Ile Cys Ser Leu Pro Phe Trp Leu Gln Tyr
65                  70                  75                  80

Val Leu Gln His Asp Asn Trp Ser His Gly Asp Leu Ser Cys Gln Val
                85                  90                  95

Cys Gly Ile Leu Leu Tyr Glu Asn Ile Tyr Ile Ser Val Gly Phe Leu
            100                 105                 110

Cys Cys Ile Ser Val Ala Arg Tyr Leu Ala Val Ala His Pro Phe Arg
        115                 120                 125

Phe His Gln Phe Arg Thr Leu Lys Ala Ala Val Gly Val Ser Val Val
    130                 135                 140

Ile Trp Ala Lys Glu Leu Leu Thr Ser Ile Tyr Phe Leu Met His Glu
145                 150                 155                 160

Glu Val Ile Glu Asp Glu Asn Gln His Arg Val Cys Phe Glu His Tyr
                165                 170                 175

Pro Ile Gln Ala Trp Gln Arg Ala Ile Asn Tyr Tyr Arg Phe Leu Val
            180                 185                 190

Gly Phe Leu Phe Pro Ile Cys Leu Leu Leu Ala Ser Tyr Gln Gly Ile
        195                 200                 205

Leu Arg Ala Val Arg Arg Ser His Gly Thr Gln Lys Asn Arg Lys Asp
    210                 215                 220

Gln Ile Gln Arg Leu Val Leu Ser Thr Val Val Ile Phe Leu Ala Cys
225                 230                 235                 240

Phe Leu Pro Tyr His Val Leu Leu Leu Val Arg Ser Val Trp Glu Ala
                245                 250                 255

Ser Cys Asp Phe Ala Lys Gly Val Phe Asn Ala Tyr His Phe Ser Leu
            260                 265                 270

Leu Leu Thr Ser Phe Asn Cys Val Ala Asp Pro Val Leu Tyr Cys Phe
        275                 280                 285

Val Ser Glu Thr Thr His Arg Asp Leu Ala Arg Leu Arg Gly Ala Cys
    290                 295                 300

Leu Ala Phe Leu Thr Cys Ser Arg Thr Gly Arg Ala Arg Glu Ala Tyr
305                 310                 315                 320

Pro Leu Gly Ala Pro Glu Ala Ser Gly Lys Ser Gly Ala Gln Gly Glu
                325                 330                 335

Glu Pro Glu Leu Leu Thr Lys Leu His Pro Ala Phe Gln Thr Pro Asn
            340                 345                 350

Ser Pro Gly Ser Gly Gly Phe Pro Thr Gly Arg Leu Ala
        355                 360                 365

<210>  190
<211>  407

<212>  PRT
<213>  Artificial

<220>
<223>  RE2D124A

<400>  190

Met Ser Leu Asn Ser Ser Leu Ser Cys Arg Lys Glu Leu Ser Asn Leu
1               5                   10                  15

Thr Glu Glu Glu Gly Gly Glu Gly Gly Val Ile Ile Thr Gln Phe Ile
            20                  25                  30

Ala Ile Ile Val Ile Thr Ile Phe Val Cys Leu Gly Asn Leu Val Ile
        35                  40                  45

Val Val Thr Leu Tyr Lys Lys Ser Tyr Leu Leu Thr Leu Ser Asn Lys
    50                  55                  60

Phe Val Phe Ser Leu Thr Leu Ser Asn Phe Leu Leu Ser Val Leu Val
65                  70                  75                  80

Leu Pro Phe Val Val Thr Ser Ser Ile Arg Arg Glu Trp Ile Phe Gly
                85                  90                  95

Val Val Trp Cys Asn Phe Ser Ala Leu Leu Tyr Leu Leu Ile Ser Ser
            100                 105                 110

Ala Ser Met Leu Thr Leu Gly Val Ile Ala Ile Ala Arg Tyr Tyr Ala
        115                 120                 125

Val Leu Tyr Pro Met Val Tyr Pro Met Lys Ile Thr Gly Asn Arg Ala
    130                 135                 140

Val Met Ala Leu Val Tyr Ile Trp Leu His Ser Leu Ile Gly Cys Leu
145                 150                 155                 160

Pro Pro Leu Phe Gly Trp Ser Ser Val Glu Phe Asp Glu Phe Lys Trp
                165                 170                 175

Met Cys Val Ala Ala Trp His Arg Glu Pro Gly Tyr Thr Ala Phe Trp
            180                 185                 190

Gln Ile Trp Cys Ala Leu Phe Pro Phe Leu Val Met Leu Val Cys Tyr
        195                 200                 205

Gly Phe Ile Phe Arg Val Ala Arg Val Lys Ala Arg Lys Val His Cys
    210                 215                 220

Gly Thr Val Val Ile Val Glu Glu Asp Ala Gln Arg Thr Gly Arg Lys
225                 230                 235                 240

Asn Ser Ser Thr Ser Thr Ser Ser Ser Gly Ser Arg Arg Asn Ala Phe
                245                 250                 255

Gln Gly Val Val Tyr Ser Ala Asn Gln Cys Lys Ala Leu Ile Thr Ile
            260                 265                 270

Leu Val Val Leu Gly Ala Phe Met Val Thr Trp Gly Pro Tyr Met Val
        275                 280                 285

Val Ile Ala Ser Glu Ala Leu Trp Gly Lys Ser Ser Val Ser Pro Ser
    290                 295                 300

Leu Glu Thr Trp Ala Thr Trp Leu Ser Phe Ala Ser Ala Val Cys His
305                 310                 315                 320

Pro Leu Ile Tyr Gly Leu Trp Asn Lys Thr Val Arg Lys Glu Leu Leu
                325                 330                 335

Gly Met Cys Phe Gly Asp Arg Tyr Tyr Arg Glu Pro Phe Val Gln Arg
            340                 345                 350

Gln Arg Thr Ser Arg Leu Phe Ser Ile Ser Asn Arg Ile Thr Asp Leu
        355                 360                 365

Gly Leu Ser Pro His Leu Thr Ala Leu Met Ala Gly Gly Gln Pro Leu
    370                 375                 380

Gly His Ser Ser Ser Thr Gly Asp Thr Gly Phe Ser Cys Ser Gln Asp
385                 390                 395                 400

Ser Gly Asn Leu Arg Ala Leu
                405

<210>  191
<211>  309
<212>  PRT
<213>  Artificial

<220>
<223>  GPR35D113A

<400> 191

Met Asn Gly Thr Tyr Asn Thr Cys Gly Ser Ser Asp Leu Thr Trp Pro
1               5                   10                  15

Pro Ala Ile Lys Leu Gly Phe Tyr Ala Tyr Leu Gly Val Leu Leu Val
            20                  25                  30

Leu Gly Leu Leu Leu Asn Ser Leu Ala Leu Trp Val Phe Cys Cys Arg
        35                  40                  45

Met Gln Gln Trp Thr Glu Thr Arg Ile Tyr Met Thr Asn Leu Ala Val
    50                  55                  60

Ala Asp Leu Cys Leu Leu Cys Thr Leu Pro Phe Val Leu His Ser Leu
65                  70                  75                  80

Arg Asp Thr Ser Asp Thr Pro Leu Cys Gln Leu Ser Gln Gly Ile Tyr
                85                  90                  95

Leu Thr Asn Arg Tyr Met Ser Ile Ser Leu Val Thr Ala Ile Ala Val
            100                 105                 110

Ala Arg Tyr Val Ala Val Arg His Pro Leu Arg Ala Arg Gly Leu Arg
        115                 120                 125

Ser Pro Arg Gln Ala Ala Ala Val Cys Ala Val Leu Trp Val Leu Val
    130                 135                 140

Ile Gly Ser Leu Val Ala Arg Trp Leu Leu Gly Ile Gln Glu Gly Gly
145                 150                 155                 160

Phe Cys Phe Arg Ser Thr Arg His Asn Phe Asn Ser Met Arg Phe Pro
                165                 170                 175

Leu Leu Gly Phe Tyr Leu Pro Leu Ala Val Val Val Phe Cys Ser Leu
            180                 185                 190

Lys Val Val Thr Ala Leu Ala Gln Arg Pro Pro Thr Asp Val Gly Gln
        195                 200                 205

Ala Glu Ala Thr Arg Lys Ala Ala Arg Met Val Trp Ala Asn Leu Leu
    210                 215                 220

Val Phe Val Val Cys Phe Leu Pro Leu His Val Gly Leu Thr Val Arg
225                 230                 235                 240

Leu Ala Val Gly Trp Asn Ala Cys Ala Leu Leu Glu Thr Ile Arg Arg
                245                 250                 255

Ala Leu Tyr Ile Thr Ser Lys Leu Ser Asp Ala Asn Cys Cys Leu Asp
            260                 265                 270

Ala Ile Cys Tyr Tyr Tyr Met Ala Lys Glu Phe Gln Glu Ala Ser Ala
        275                 280                 285

Leu Ala Val Ala Pro Arg Ala Lys Ala His Lys Ser Gln Asp Ser Leu
    290                 295                 300

Cys Val Thr Leu Ala
305

<210> 192
<211> 337
<212> PRT
<213> Artificial

<220>
<223> GPCR25D111A

<400> 192

Met Asn Ser Thr Cys Ile Glu Glu Gln His Asp Leu Asp His Tyr Leu
1               5                   10                  15

Phe Pro Ile Val Tyr Ile Phe Val Ile Ile Val Ser Ile Pro Ala Asn
            20                  25                  30

Ile Gly Ser Leu Cys Val Ser Phe Leu Gln Pro Lys Lys Glu Ser Glu
        35                  40                  45

Leu Gly Ile Tyr Leu Phe Ser Leu Ser Leu Ser Asp Leu Leu Tyr Ala
    50                  55                  60

Leu Thr Leu Pro Leu Trp Ile Asp Tyr Thr Trp Asn Lys Asp Asn Trp
65                  70                  75                  80

Thr Phe Ser Pro Ala Leu Cys Lys Gly Ser Ala Phe Leu Met Tyr Met
                85                  90                  95

Lys Phe Tyr Ser Ser Thr Ala Phe Leu Thr Cys Ile Ala Val Ala Arg
            100                 105                 110

Tyr Leu Ala Val Val Tyr Pro Leu Lys Phe Phe Phe Leu Arg Thr Arg
        115                 120                 125

Arg Ile Ala Leu Met Val Ser Leu Ser Ile Trp Ile Leu Glu Thr Ile
    130                 135                 140

Phe Asn Ala Val Met Leu Trp Glu Asp Glu Thr Val Val Glu Tyr Cys
145                 150                 155                 160

Asp Ala Glu Lys Ser Asn Phe Thr Leu Cys Tyr Asp Lys Tyr Pro Leu
                165                 170                 175

Glu Lys Trp Gln Ile Asn Leu Asn Leu Phe Arg Thr Cys Thr Gly Tyr
            180                 185                 190

Ala Ile Pro Leu Val Thr Ile Leu Ile Cys Asn Arg Lys Val Tyr Gln
        195                 200                 205

Ala Val Arg His Asn Lys Ala Thr Glu Asn Lys Glu Lys Lys Arg Ile
    210                 215                 220

Ile Lys Leu Leu Val Ser Ile Thr Val Thr Phe Val Leu Cys Phe Thr
225                 230                 235                 240

Pro Phe His Val Met Leu Leu Ile Arg Cys Ile Leu Glu His Ala Val
                245                 250                 255

Asn Phe Glu Asp His Ser Asn Ser Gly Lys Arg Thr Tyr Thr Met Tyr
            260                 265                 270

Arg Ile Thr Val Ala Leu Thr Ser Leu Asn Cys Val Ala Asp Pro Ile
        275                 280                 285

Leu Tyr Cys Phe Val Thr Glu Thr Gly Arg Tyr Asp Met Trp Asn Ile
    290                 295                 300

Leu Lys Phe Cys Thr Gly Arg Cys Asn Thr Ser Gln Arg Gln Arg Lys
305                 310                 315                 320

Arg Ile Leu Ser Val Ser Thr Lys Asp Thr Met Glu Leu Glu Val Leu
                325                 330                 335

Glu

<210>  193
<211>  361
<212>  PRT
<213>  Artificial

<220>
<223>  PGMO334E135F

<400>  193

Met Ser Pro Glu Cys Ala Arg Ala Ala Gly Asp Ala Pro Leu Arg Ser
1               5                   10                  15

Leu Glu Gln Ala Asn Arg Thr Arg Phe Pro Phe Phe Ser Asp Val Lys
            20                  25                  30

Gly Asp His Arg Leu Val Leu Ala Ala Val Glu Thr Thr Val Leu Val
        35                  40                  45

Leu Ile Phe Ala Val Ser Leu Leu Gly Asn Val Cys Ala Leu Val Leu
    50                  55                  60

Val Ala Arg Arg Arg Arg Arg Gly Ala Thr Ala Cys Leu Val Leu Asn
65                  70                  75                  80

Leu Phe Cys Ala Asp Leu Leu Phe Ile Ser Ala Ile Pro Leu Val Leu
                85                  90                  95

Ala Val Arg Trp Thr Glu Ala Trp Leu Leu Gly Pro Val Ala Cys His
            100                 105                 110

Leu Leu Phe Tyr Val Met Thr Leu Ser Gly Ser Val Thr Ile Leu Thr
        115                 120                 125

Leu Ala Ala Val Ser Leu Phe Arg Met Val Cys Ile Val His Leu Gln
    130                 135                 140

Arg Gly Val Arg Gly Pro Gly Arg Arg Ala Arg Ala Val Leu Leu Ala
145                 150                 155                 160

Leu Ile Trp Gly Tyr Ser Ala Val Ala Ala Leu Pro Leu Cys Val Phe
                165                 170                 175

Phe Arg Val Val Pro Gln Arg Leu Pro Gly Ala Asp Gln Glu Ile Ser
            180                 185                 190

Ile Cys Thr Leu Ile Trp Pro Thr Ile Pro Gly Glu Ile Ser Trp Asp
        195                 200                 205

Val Ser Phe Val Thr Leu Asn Phe Leu Val Pro Gly Leu Val Ile Val
    210                 215                 220

Ile Ser Tyr Ser Lys Ile Leu Gln Ile Thr Lys Ala Ser Arg Lys Arg
225                 230                 235                 240

Leu Thr Val Ser Leu Ala Tyr Ser Glu Ser His Gln Ile Arg Val Ser
                245                 250                 255

Gln Gln Asp Phe Arg Leu Phe Arg Thr Leu Phe Leu Leu Met Val Ser
            260                 265                 270

Phe Phe Ile Met Trp Ser Pro Ile Ile Ile Thr Ile Leu Leu Ile Leu
        275                 280                 285

Ile Gln Asn Phe Lys Gln Asp Leu Val Ile Trp Pro Ser Leu Phe Phe
    290                 295                 300

Trp Val Val Ala Phe Thr Phe Ala Asn Ser Ala Leu Asn Pro Ile Leu
305                 310                 315                 320

Tyr Asn Met Thr Leu Cys Arg Asn Glu Trp Lys Lys Ile Phe Cys Cys
                325                 330                 335

Phe Trp Phe Pro Glu Lys Gly Ala Ile Leu Thr Asp Thr Ser Val Lys
            340                 345                 350

Arg Asn Asp Leu Ser Ile Ile Ser Gly
        355                 360

<210> 194
<211> 361
<212> PRT
<213> Artificial

<220>
<223> PGM0334E135Q

<400> 194

Met Ser Pro Glu Cys Ala Arg Ala Ala Gly Asp Ala Pro Leu Arg Ser
1               5                   10                  15

Leu Glu Gln Ala Asn Arg Thr Arg Phe Pro Phe Phe Ser Asp Val Lys
            20                  25                  30

Gly Asp His Arg Leu Val Leu Ala Ala Val Glu Thr Thr Val Leu Val
        35                  40                  45

Leu Ile Phe Ala Val Ser Leu Leu Gly Asn Val Cys Ala Leu Val Leu
    50                  55                  60

Val Ala Arg Arg Arg Arg Arg Gly Ala Thr Ala Cys Leu Val Leu Asn
65                  70                  75                  80

Leu Phe Cys Ala Asp Leu Leu Phe Ile Ser Ala Ile Pro Leu Val Leu
                85                  90                  95

Ala Val Arg Trp Thr Glu Ala Trp Leu Leu Gly Pro Val Ala Cys His
            100                 105                 110

Leu Leu Phe Tyr Val Met Thr Leu Ser Gly Ser Val Thr Ile Leu Thr
        115                 120                 125

Leu Ala Ala Val Ser Leu Gln Arg Met Val Cys Ile Val His Leu Gln
    130                 135                 140

Arg Gly Val Arg Gly Pro Gly Arg Arg Ala Arg Ala Val Leu Leu Ala
145                 150                 155                 160

Leu Ile Trp Gly Tyr Ser Ala Val Ala Ala Leu Pro Leu Cys Val Phe
                165                 170                 175

Phe Arg Val Val Pro Gln Arg Leu Pro Gly Ala Asp Gln Glu Ile Ser
            180                 185                 190

Ile Cys Thr Leu Ile Trp Pro Thr Ile Pro Gly Glu Ile Ser Trp Asp
        195                 200                 205

Val Ser Phe Val Thr Leu Asn Phe Leu Val Pro Gly Leu Val Ile Val
    210                 215                 220

Ile Ser Tyr Ser Lys Ile Leu Gln Ile Thr Lys Ala Ser Arg Lys Arg
225                 230                 235                 240

Leu Thr Val Ser Leu Ala Tyr Ser Glu Ser His Gln Ile Arg Val Ser
                245                 250                 255

Gln Gln Asp Phe Arg Leu Phe Arg Thr Leu Phe Leu Leu Met Val Ser
            260                 265                 270

Phe Phe Ile Met Trp Ser Pro Ile Ile Ile Thr Ile Leu Leu Ile Leu
        275                 280                 285

Ile Gln Asn Phe Lys Gln Asp Leu Val Ile Trp Pro Ser Leu Phe Phe
    290                 295                 300

Trp Val Val Ala Phe Thr Phe Ala Asn Ser Ala Leu Asn Pro Ile Leu
305                 310                 315                 320

Tyr Asn Met Thr Leu Cys Arg Asn Glu Trp Lys Lys Ile Phe Cys Cys
                325                 330                 335

Phe Trp Phe Pro Glu Lys Gly Ala Ile Leu Thr Asp Thr Ser Val Lys
            340                 345                 350

Arg Asn Asp Leu Ser Ile Ile Ser Gly
        355                 360

<210>  195
<211>  361
<212>  PRT
<213>  Artificial

<220>
<223>  PGMO334E135A

<400>  195

Met Ser Pro Glu Cys Ala Arg Ala Ala Gly Asp Ala Pro Leu Arg Ser
1               5                   10                  15

Leu Glu Gln Ala Asn Arg Thr Arg Phe Pro Phe Phe Ser Asp Val Lys
            20                  25                  30

Gly Asp His Arg Leu Val Leu Ala Ala Val Glu Thr Thr Val Leu Val
        35                  40                  45

Leu Ile Phe Ala Val Ser Leu Leu Gly Asn Val Cys Ala Leu Val Leu
    50                  55                  60

Val Ala Arg Arg Arg Arg Arg Gly Ala Thr Ala Cys Leu Val Leu Asn
65                  70                  75                  80

Leu Phe Cys Ala Asp Leu Leu Phe Ile Ser Ala Ile Pro Leu Val Leu
                85                  90                  95

Ala Val Arg Trp Thr Glu Ala Trp Leu Leu Gly Pro Val Ala Cys His
            100                 105                 110

Leu Leu Phe Tyr Val Met Thr Leu Ser Gly Ser Val Thr Ile Leu Thr
        115                 120                 125

Leu Ala Ala Val Ser Leu Ala Arg Met Val Cys Ile Val His Leu Gln
    130                 135                 140

Arg Gly Val Arg Gly Pro Gly Arg Arg Ala Arg Ala Val Leu Leu Ala
145                 150                 155                 160

Leu Ile Trp Gly Tyr Ser Ala Val Ala Ala Leu Pro Leu Cys Val Phe
                165                 170                 175

Phe Arg Val Val Pro Gln Arg Leu Pro Gly Ala Asp Gln Glu Ile Ser
            180                 185                 190

Ile Cys Thr Leu Ile Trp Pro Thr Ile Pro Gly Glu Ile Ser Trp Asp
        195                 200                 205

Val Ser Phe Val Thr Leu Asn Phe Leu Val Pro Gly Leu Val Ile Val
    210                 215                 220

Ile Ser Tyr Ser Lys Ile Leu Gln Ile Thr Lys Ala Ser Arg Lys Arg
225                 230                 235                 240

Leu Thr Val Ser Leu Ala Tyr Ser Glu Ser His Gln Ile Arg Val Ser
                245                 250                 255

Gln Gln Asp Phe Arg Leu Phe Arg Thr Leu Phe Leu Leu Met Val Ser
            260                 265                 270

Phe Phe Ile Met Trp Ser Pro Ile Ile Ile Thr Ile Leu Leu Ile Leu
        275                 280                 285

Ile Gln Asn Phe Lys Gln Asp Leu Val Ile Trp Pro Ser Leu Phe Phe
    290                 295                 300

Trp Val Val Ala Phe Thr Phe Ala Asn Ser Ala Leu Asn Pro Ile Leu
305                 310                 315                 320

Tyr Asn Met Thr Leu Cys Arg Asn Glu Trp Lys Lys Ile Phe Cys Cys
                325                 330                 335

Phe Trp Phe Pro Glu Lys Gly Ala Ile Leu Thr Asp Thr Ser Val Lys
            340                 345                 350

Arg Asn Asp Leu Ser Ile Ile Ser Gly
        355                 360

<210>  196
<211>  361
<212>  PRT
<213>  Artificial

<220>
<223>  PGM0334D259S

<400>  196

Met Ser Pro Glu Cys Ala Arg Ala Ala Gly Asp Ala Pro Leu Arg Ser
1               5                   10                  15

Leu Glu Gln Ala Asn Arg Thr Arg Phe Pro Phe Phe Ser Asp Val Lys
            20                  25                  30

Gly Asp His Arg Leu Val Leu Ala Ala Val Glu Thr Thr Val Leu Val
        35                  40                  45

Leu Ile Phe Ala Val Ser Leu Leu Gly Asn Val Cys Ala Leu Val Leu
    50                  55                  60

Val Ala Arg Arg Arg Arg Arg Gly Ala Thr Ala Cys Leu Val Leu Asn
65                  70                  75                  80

Leu Phe Cys Ala Asp Leu Leu Phe Ile Ser Ala Ile Pro Leu Val Leu
                85                  90                  95

Ala Val Arg Trp Thr Glu Ala Trp Leu Leu Gly Pro Val Ala Cys His
            100                 105                 110

Leu Leu Phe Tyr Val Met Thr Leu Ser Gly Ser Val Thr Ile Leu Thr
        115                 120                 125

Leu Ala Ala Val Ser Leu Glu Arg Met Val Cys Ile Val His Leu Gln
    130                 135                 140

Arg Gly Val Arg Gly Pro Gly Arg Arg Ala Arg Ala Val Leu Leu Ala
145                 150                 155                 160

Leu Ile Trp Gly Tyr Ser Ala Val Ala Ala Leu Pro Leu Cys Val Phe
                165                 170                 175

Phe Arg Val Val Pro Gln Arg Leu Pro Gly Ala Asp Gln Glu Ile Ser
            180                 185                 190

Ile Cys Thr Leu Ile Trp Pro Thr Ile Pro Gly Glu Ile Ser Trp Asp
        195                 200                 205

Val Ser Phe Val Thr Leu Asn Phe Leu Val Pro Gly Leu Val Ile Val
    210                 215                 220

Ile Ser Tyr Ser Lys Ile Leu Gln Ile Thr Lys Ala Ser Arg Lys Arg
225                 230                 235                 240

Leu Thr Val Ser Leu Ala Tyr Ser Glu Ser His Gln Ile Arg Val Ser
                245                 250                 255

Gln Gln Ser Phe Arg Leu Phe Arg Thr Leu Phe Leu Leu Met Val Ser
            260                 265                 270

Phe Phe Ile Met Trp Ser Pro Ile Ile Ile Thr Ile Leu Leu Ile Leu
        275                 280                 285

Ile Gln Asn Phe Lys Gln Asp Leu Val Ile Trp Pro Ser Leu Phe Phe
    290                 295                 300

Trp Val Val Ala Phe Thr Phe Ala Asn Ser Ala Leu Asn Pro Ile Leu
305                 310                 315                 320

Tyr Asn Met Thr Leu Cys Arg Asn Glu Trp Lys Lys Ile Phe Cys Cys
                325                 330                 335

Phe Trp Phe Pro Glu Lys Gly Ala Ile Leu Thr Asp Thr Ser Val Lys
            340                 345                 350

Arg Asn Asp Leu Ser Ile Ile Ser Gly
        355                 360

<210>  197
<211>  330
<212>  PRT
<213>  Artificial

<220>
<223>  GPR43R217P

<400>  197

Met Leu Pro Asp Trp Lys Ser Ser Leu Ile Leu Met Ala Tyr Ile Ile
1               5                   10                  15

Ile Phe Leu Thr Gly Leu Pro Ala Asn Leu Leu Ala Leu Arg Ala Phe
            20                  25                  30

Val Gly Arg Ile Arg Gln Pro Gln Pro Ala Pro Val His Ile Leu Leu
        35                  40                  45

Leu Ser Leu Thr Leu Ala Asp Leu Leu Leu Leu Leu Leu Leu Pro Phe
    50                  55                  60

Lys Ile Ile Glu Ala Ala Ser Asn Phe Arg Trp Tyr Leu Pro Lys Val
65                  70                  75                  80

Val Cys Ala Leu Thr Ser Phe Gly Phe Tyr Ser Ser Ile Tyr Cys Ser
                85                  90                  95

Thr Trp Leu Leu Ala Gly Ile Ser Ile Glu Arg Tyr Leu Gly Val Ala
            100                 105                 110

Phe Pro Val Gln Tyr Lys Leu Ser Arg Arg Pro Leu Tyr Gly Val Ile
        115                 120                 125

Ala Ala Leu Val Ala Trp Val Met Ser Phe Gly His Cys Thr Ile Val
    130                 135                 140

Ile Ile Val Gln Tyr Leu Asn Thr Thr Glu Gln Val Arg Ser Gly Asn
145                 150                 155                 160

Glu Ile Thr Cys Tyr Glu Asn Phe Thr Asp Asn Gln Leu Asp Val Val
                165                 170                 175

Leu Pro Val Arg Leu Glu Leu Cys Leu Val Leu Phe Phe Ile Pro Met
            180                 185                 190

Ala Val Thr Ile Phe Cys Tyr Trp Arg Phe Val Trp Ile Met Leu Ser
        195                 200                 205

Gln Pro Leu Val Gly Ala Gln Arg Pro Arg Arg Ala Val Gly Leu Ala
    210                 215                 220

Val Val Thr Leu Leu Asn Phe Leu Val Cys Phe Gly Pro Tyr Asn Val
225                 230                 235                 240

Ser His Leu Val Gly Tyr His Gln Arg Lys Ser Pro Trp Trp Arg Ser
                245                 250                 255

Ile Ala Val Val Phe Ser Ser Leu Asn Ala Ser Leu Asp Pro Leu Leu
            260                 265                 270

Phe Tyr Phe Ser Ser Ser Val Val Arg Arg Ala Phe Gly Arg Gly Leu
        275                 280                 285

Gln Val Leu Arg Asn Gln Gly Ser Ser Leu Leu Gly Arg Arg Gly Lys
    290                 295                 300

Asp Thr Ala Glu Gly Thr Asn Glu Asp Arg Gly Val Gly Gln Gly Glu
305                 310                 315                 320

Gly Met Pro Ser Ser Asp Phe Thr Thr Glu
                325                 330

<210> 198
<211> 330
<212> PRT
<213> Artificial

<220>
<223> GPR43R217P/E106D

<400> 198

Met Leu Pro Asp Trp Lys Ser Ser Leu Ile Leu Met Ala Tyr Ile Ile
1               5                   10                  15

Ile Phe Leu Thr Gly Leu Pro Ala Asn Leu Leu Ala Leu Arg Ala Phe
            20                  25                  30

Val Gly Arg Ile Arg Gln Pro Gln Pro Ala Pro Val His Ile Leu Leu
        35                  40                  45

Leu Ser Leu Thr Leu Ala Asp Leu Leu Leu Leu Leu Leu Leu Pro Phe
    50                  55                  60

Lys Ile Ile Glu Ala Ala Ser Asn Phe Arg Trp Tyr Leu Pro Lys Val
65                  70                  75                  80

Val Cys Ala Leu Thr Ser Phe Gly Phe Tyr Ser Ser Ile Tyr Cys Ser
                85                  90                  95

Thr Trp Leu Leu Ala Gly Ile Ser Ile Asp Arg Tyr Leu Gly Val Ala
            100                 105                 110

Phe Pro Val Gln Tyr Lys Leu Ser Arg Arg Pro Leu Tyr Gly Val Ile
        115                 120                 125

Ala Ala Leu Val Ala Trp Val Met Ser Phe Gly His Cys Thr Ile Val
    130                 135                 140

Ile Ile Val Gln Tyr Leu Asn Thr Thr Glu Gln Val Arg Ser Gly Asn
145                 150                 155                 160

Glu Ile Thr Cys Tyr Glu Asn Phe Thr Asp Asn Gln Leu Asp Val Val
                165                 170                 175

Leu Pro Val Arg Leu Glu Leu Cys Leu Val Leu Phe Phe Ile Pro Met
            180                 185                 190

Ala Val Thr Ile Phe Cys Tyr Trp Arg Phe Val Trp Ile Met Leu Ser
        195                 200                 205

Gln Pro Leu Val Gly Ala Gln Arg Pro Arg Arg Ala Val Gly Leu Ala
    210                 215                 220

Val Val Thr Leu Leu Asn Phe Leu Val Cys Phe Gly Pro Tyr Asn Val
225                 230                 235                 240

Ser His Leu Val Gly Tyr His Gln Arg Lys Ser Pro Trp Trp Arg Ser
                245                 250                 255

Ile Ala Val Val Phe Ser Ser Leu Asn Ala Ser Leu Asp Pro Leu Leu
            260                 265                 270

Phe Tyr Phe Ser Ser Ser Val Val Arg Arg Ala Phe Gly Arg Gly Leu
        275                 280                 285

Gln Val Leu Arg Asn Gln Gly Ser Ser Leu Leu Gly Arg Arg Gly Lys
    290                 295                 300

Asp Thr Ala Glu Gly Thr Asn Glu Asp Arg Gly Val Gly Gln Gly Glu
305                 310                 315                 320

Gly Met Pro Ser Ser Asp Phe Thr Thr Glu
                325                 330

## Description

### Technical Field

The present invention relates to a method of isolating a DNA encoding a peptide capable of acting as an agonist, an antagonist or an inverse agonist for a receptor; to a method of detecting or isolating the peptide; a cell line derived from an endocrine cell used for these methods; to a process for producing a peptide using the cell line derived from the endocrine cell; to a B-cell line in which a receptor is expressed and which is used for the above methods; a method of isolating a DNA encoding a peptide which is capable of reacting with a substance to be tested using the B-cell line; a method of isolating a DNA encoding a peptide using the B-cell line; to a constitutively activated mutant G-protein coupled receptor obtained by the method of isolating a DNA; to a method of isolating an agonist, an antagonist or an inverse agonist for a G-protein coupled receptor using the B-cell line; to a method of isolating an activator or an inhibitor for a peptide using the B-cell line; and to a host-vector system using the B-cell line as a host.

### Background Art

It has been known that, in general, peptide hormones and neurotransmitters are produced as precursors (preproforms) and finally become active peptides as a result of action of plural processing enzymes or modifying enzymes [*Curr*. *Opin*. *Chem*. *Biol*., 2, 31 (1998)].

Specific processing enzymes and modifying enzymes are expressed only in specific endocrine cells and, therefore, active peptides are not usually produced even when a precursor gene of an active peptide (such as vasopressin and glucagon) produced in nerve cells and endocrine cells of hypothalamus, pituitary gland or Langerhans islets of pancreas, for example, is expressed in non-endocrine cells such as COS cells and CHO cells which are frequently used in gene expression.

Cells having an inherent property of producing specific active peptides, such as endocrine cells, express processing enzymes or modifying enzymes for the active peptide and, therefore, it is likely that, when a precursor gene of the peptide is expressed using the cells as a host, the peptide is able to be expressed in an active form. With regard to other active peptide which is able to be subjected to normal processing or modification by processing enzymes or modifying enzymes being expressed in the cells, it is also likely that the active peptide is able to be expressed by means of expression of a precursor gene using the cells as a host.

With regard to a method for producing an active peptide where a known active peptide precursor gene is transfected into a cell line which is established from endocrine cells (hereinafter, referred to as endocrine cell line) and expressed, there have been known a method where the preproinsulin gene or the preproamyrin gene is expressed by transfecting into RIN cell established from cancer cells of Langerhans islets of rat induced by irradiation of radioactive ray whereupon insulin or amyrin is produced (US 6194176 and US 6110707), a method where the preproinsulin gene is expressed by transfecting into MIN6 cell line established from tumor β cell produced in transgenic mouse whereupon C peptide is produced [*Nat. Cell Biol.,* 2, 805 (2000)], etc.

As mentioned above, there have been known several methods for producing known active peptides using endocrine cell lines but there has been no literature mentioning or suggesting that an endocrine cell line is useful for screening novel active peptides.

In addition, in order to efficiently find a peptide having new activity and a gene encoding the peptide, there has been demanded further improvement concerning endocrine cell lines using as hosts for gene expression, endocrine cell lines using as sources for genes and assay system for detecting activity of peptides.

With regard to endocrine cell lines, there have been known the following (1) cell lines derived from hypothalamus and (2) those derived from Langerhans islets.

### (1) Cell lines derived from hypothalamus

With regard to the cell lines derived from hypothalamus, those of the following (i) to (iv) have been known.
(i) Immortalized cells obtained by infection of primary culture cells derived from hypothalamus of mouse embryo (14-day-old) with SV40.
   As to the above immortalized cells, there have been known HT9, single clone derived from the HT9 cell (HT9-C7), etc. HT9-C7 expresses neurophysin and vasopressin [*Proc.Natl.Acad. Sci. USA*, 71, 3575 (1974)].
(ii) Immortalized cell lines obtained by infection of primary culture cells derived from hypothalamus of embryo of rat (16-day-old) with SV40.
   As to the above immortalized cells, there have been known RCF-8, RCA-6, RCF-27, RCD-15, D12, etc. Those cell lines express estrogen receptor [*Endocrinology*, 126, 235 (1990); *Endocrinology*, 140, 23928 (1999)].
(iii) Immortalized cell lines derived from two kinds of suprachiasmatic nuclei obtained by infection of primary culture cells derived from suprachiasmatic nuclei of rat embryos (15-day- and 16-day-old) with retrovirus which is able to express adenovirus 2 and hybrid E1A 12S of adenovirus 5
   With regard to the above immortalized cell lines, there have been known SCN1.4, SCN2.2, etc. [*J*. *Neurobiol*., 39, 1 (1999)]. Most of cell lines of SCN1.4 and SCN2.2 show a morphology like glia cells but expression of glia cell-specific antigen is limited. A part of the cells show a property characteristic to nerve cells, express nerve cell-specific antigens and neuropeptides expressed in nerve cells of suprachiasmatic nucleus , which include somatostatin, vasoactive intestinal polypeptide (VIP), gastrin-releasing peptide (GRP) or arginine vasopressin (AVP), and do not express oxytocin and corticotropin-releasing hormone (CRH) which are not expressed in nerve cells of suprachiasmatic nucleus. When cultivation is carried out under the condition of promoting the differentiation of cells, the number of cells expressing the neuropeptide which is noted to be expressed hereinabove increases.
(iv) Cell lines established from tumors generated in a hindbrain of a transgenic mouse where SV40 large T antigen is expressed using a promoter of the gonadotropin-releasing hormone (GnRH) gene expressed in hypothalamus
   With regard to the above cell lines, there have been known GT1, GT1-1, GT1-3, GT1-7, etc. [Neuron, 5, 1 (1990)]. GT1-1 and GT1-7 cells retain a GnRH-producing ability and are used for analysis of regulation mechanism of expression of GNRH [*Proc. Natl. Acad. Sci. USA*, 89, 1852 (1992); *Endocrinology*, 140, 1423 (1999)].
(v) Cell lines established from tumors generated in olfactory bulb of transgenic mouse in which SV40 large T antigen is expressed using a promoter of the GnRH gene expressed in hypothalamus

With regard to the above cell lines, there have been known GN, etc. GN cell expresses GnRH [*Proc. Natl. Acad. Sci. USA,* 88, 3402 (1991)].

When cell lines derived from hypothalamus expressing an endogenous leptin receptor are obtained, it is possible to analyze a biological function of leptin to each nerve cell but any of the above-mentioned cell lines derived from hypothalamus does not express a leptin receptor and, up to now, no cell line derived from hypothalamus expressing an endogenous leptin receptor has been known [*Diabetes*, 49, 1443 (2000); *Neuron*, 5, 1 (1990)].

Development of leptin has progressed as an anti-obesity agent or antidiabetic agent but, as a result of resistance to leptin, drugs in place of leptin have been demanded. It is believed that clarification of action mechanism of leptin results in finding of novel physiologically active substances and identification of targets for the development of new drugs. When cell lines derived from hypothalamus expressing an endogenous leptin receptor are obtained, it is possible to screen and evaluate the above-mentioned drugs using the cell lines.

There are not known hypothalamus derived cell lines that express preproneuromedin U gene, RF amide-related peptide (RFRP) preproprotein gene, preproorexin gene, preproopiomelanocortin gene, preproneuropeptide Y gene, preproneuropeptide FF gene, preprocorticotropin-releasing hormone gene, preprothyrotropin-releasing hormone gene, preproghrelin gene, prepromelanin concentration hormone gene, cocaine- and amphetamine-regulated transcript (CART) gene, type 2 neuromedin U receptor (NMU2R), RFRP receptor gene, type 4 melanocortin receptor (MC4R) gene, type 1 neuropeptide Y receptor (NPY1R) gene, type 5 neuropeptide Y receptor (NPY5R) gene, type 2 neuropeptide FF receptor (NPFF2) gene, type 1 corticotropin-releasing hormone receptor (CRHR-1) gene, type 2 corticotropin-releasing hormone receptor (CRHR-2) gene, ghrelin receptor gene, type 1 melanin concentration hormone receptor (MCHR1) gene, sulfonylurea receptor gene or preproagouti-related peptide gene, in addition to leptin receptor gene, or polypeptides encoded by those genes.

Hypothalamus has the most important controlling function for maintaining the life and has been well noted as a target site for drugs. Therefore, if hypothalamus derived cell lines maintaining the function are able to be obtained, function of each hypothalamus nerve cell is able to be studied in a cellular level and in a molecular level. That is also very useful for the development of drugs where the hypothalamus nerve cell is a target. It has been known that endocrine cells existing in hypothalamus produce various active peptides and, therefore, it is believed that various cell lines derived from hypothalamus are very useful sources for screening novel active peptides.

### (2) Cell lines derived from Langerhans islets

Until now, cell lines of the following (i) to (vii) have been known as cell lines derived from Langerhans islets.
(i) Cell lines established from cancer derived from Langerhans islets induced by radioactive ray
   With regard to the above-mentioned established cell lines, there have been known RIN-m cells (rat cells), INS-1 cells (rat cells), single clone derived from RIN-m cells (RIN-5F, RIN-14B, RIN-m5F), etc. [*Proc*. *Natl*. *Proc*. *Soc. Exp*. *Biol*. *Med*., 175, 35 (1984); *Endocrinology*, 130, 167 (1992)]. Since frequency of cancer induction by radioactive ray is low, it is believed that only specific cells (β cells, etc.) existing in Langerhans islets become cancerous. Accordingly, the above-mentioned established cell lines are believed to be derived from malignant transformants of specific cells.
(ii) Cell lines established from insulinoma of hamster
   With regard to the above-mentioned established cell lines, there have been known In-R1-G1, In-R1-G3, In-R1-G7, In-R1-G9, In-R1-G10, In-R1-G11, etc. [*In Vitro Cell. Dev. Biol.,* 22, 120 (1986)]. Those established cell lines are also believed to be cancerous cell lines of specific cells existing in Langerhans islets.
(iii) Cell lines established by transformation of primary culture cells of Langerhans islets by SV40
   With regard to the above-mentioned established cell lines, there have been known HIT cells derived fromhamster, etc. [*Proc. Natl. Acad. Sci. USA,* 78, 4339 (1981); *Biochem. J.,* 219, 547 (1984)]. Frequency of transformation by SV40 is low and, in addition, the frequency varies depending upon the type of the cell. Therefore, those established cell lines are alsobelieved to be cancerous cell lines of specific cells (such as β cells) existing in Langerhans islets.
(iv) Cell lines established from tumors believed to be derived from β cells generated in a transgenic mouse expressing SV40 large T antigen under the control of insulin promoter
   With regard to the above-mentioned established cell lines, there have been known βTC cells, NIT-1 cells, MIN6 cells, etc. [*Proc*. *Natl*. *Acad*. *Sci*. *USA*, 85, 9037 (1988); *Diabetes*, 40 , 842 (1991); *Endocrinology*, 127, 126 (1990)]. Here, SV40 large T antigen is expressed only in specific cells (such as β cells) existing in Langerhans islets and, therefore, only specific cells are believed to be immortalized. Accordingly, those established cell lines are also believed to be cancerous cell lines of specific cells existing in Langerhans islets.
(v) Cell lines established from tumors believed to be derived from β cells generated in mouse born by crossing of a transgenic mouse expressing a transcription factor under control of insulin promoter with a transgenic mouse expressing SV40 large T antigen under control of a promoter activated by the above transcription factor
   With regard to the above-mentioned established cell lines, there have been known βTC-tet cells, etc. [*Proc. Natl. Acad. Sci*. *USA,* 92, 3576 (1995)]. As same as in the above (iv), those established cell lines are also believed to be cancerous cell lines of specific cells existing in Langerhans islets.
(vi) Cell lines established from adenoma generated in a transgenic mouse expressing SV40 large T antigen under control of a preglucagon promoter
   With regard to the above established cell lines, there have been known αTC1 cells [*Diabetes,* 39, 406 (1990) ; *Diabetes,* 39, 415 (1990)], αTC1 clone 9 which is a single clone derived from αTC1 cells, etc. Here, SV40 large T antigen is expressed only in specific cells existing in Langerhans islets (such as α cells) and, therefore, only specific cells are believed to be immortalized. Accordingly, those established cell lines are also believed to be cancerous cell lines of specific cells existing in Langerhans islets.
(vii) Cell lines established from pancreatic cancer generated in a transgenic mouse expressing SV40 large T antigen under control of an elastase-1 promoter
   With regard to the above cell lines, there is TGP52 [*Carcinogenesis,* 15, 61 (1994)]. Here, SV40 large T antigen is expressedonly in specific cells existing in Langerhans islets and, therefore, only specific cells are believed to be immortalized. Accordingly, this established cell line is also believed to be a cancerous cell line of specific cells existing in Langerhans islets.

All of the above-mentioned cell lines established from cells of Langerhans islets are cancer cells or cells derived from cancer and, therefore, it is possible that they have different property from that of normal cells.

In screening novel physiologically active substances, receptors, ligands or active peptides, it is desired to obtain further various cell lines maintaining the property of normal cells. Since various cells of Langerhans islets are believed to control each other, it is possible to analyze the role of each cell when various cell lines are obtained and are subjected to co-incubation.

In addition, when various cell lines derived from Langerhans islets having the same genetic background are able to be obtained, that is useful for a comparative analysis of differentiation stage and function of each cell line. Until now, there has been no various immortalized cells derived from Langerhans islets having the same genetic background.

It has been known that endocrine cells existing in Langerhans islets produces various active peptides such as glucagon, insulin, somatostatin and pancreatic polypeptides and, therefore, the endocrine cells are believed to be very useful sources for the screening new active peptides as well.

In order to efficiently check the activity of a peptide which is produced from endocrine cell lines or endocrine cells into which any gene is transfected, a simple and highly-sensitive assay system by which activity of the peptide is able to be detected is necessary. In order to efficiently find a peptide having a novel activity or a gene encoding the peptide, various assay systems which are highly sensitive and simple arenecessary. It is also believed that, when various endocrine cell lines are used as peptide sources or gene sources, a peptide having a new activity or a gene encoding the peptide is able to be efficiently found.

Many assay systems have been constructed already. Examples of the assay systems having a good sensitivity include a bioassay system, etc.

In the case of abioassay system, the detection sensitivity, signal to noise ratio, multiplicity of use and simplicity depend greatly on which cell is used. When a transformant is used as the above cell, efficiency of the assay depends greatly on whether the efficiency of gene transfection is good, whether the expression of gene is high, whether induced expression of gene is possible, whether induction rate of the induced expression is high, etc. When animal cells are used, the assay efficiency depend greatly on whether cultivation is possible in a serum-free medium, whether they are non-adherent cells (floating cells) having a good growth, etc.

As an example of assay systems which are able to be used for screening active peptides (ligands), a bioassay system where G-protein coupled receptor (hereinafter, abbreviated as GPCR) is utilized has been known. By the GPCR bioassay system, there have been isolated novel ligands, agonists or antagonists. GPCR is coupled to a heterotrimeric G protein constituted from three kinds of G proteins (guanine nucleotide-binding proteins), i.e. Gα, Gβ and Gγ and, via activation of G protein, signal is transduced into cells. Since GPCR has seven transmembrane regions, it is also called a seven transmembrane receptor.

With regard to the GPCR bioassay system, there have been known many methods such as a method where an increase in the amount of intracellular Ca²⁺ is detected and a method where a reporter system is used, etc.

GPCRs transduce different signals depending upon the difference in subtypes of the coupled G protein. In the case of a method using a reporter system, there are differences in detectable signal, sensitivity, ratio of signal to noise ratio, etc. depending upon a factor that what promoter is used for the expression of the reporter gene.

With regard to a promoters, there have been utilized a promoter having TPA-responding elements (TRE), an NFAT (nuclear factor of activated T cells)-responding promoter, a promoter having cAMP-responding elements (CRE), a promoter having serum-responding elements (SRE), etc.

Steric structure of Gα coupled to GPCR has been clarified already and it has been known that, when amino acids of C terminal of Gα protein which are important for interaction with GPCR are substituted with the corresponding amino acids of other Gα protein, it is now able to be coupled to GPCR which is not primarily coupled [*Mol. Pharmacol.,* 57, 13 (2000)].

For example, a chimeric Gα protein (Gα_{q-i}) where 5 amino acids at C terminal of Gα_{q} are substituted with those of Gαᵢ is able to be coupled to GPCRs which transduce signals by coupling to Gαᵢ, a chimeric Gα protein (Gα_{s-q}) where 5 amino acids at C terminal of Gαₛ are substituted with those of Gα_{q} is able to coupled to GPCRs which transduce signals by coupling to Gα_{q} and a chimeric Gα protein (Gαₛ₋ᵢ) where 5 amino acids at C terminal of Gαₛ are substituted with those of Gαᵢ is able to be coupled to GPCRs which transduce signals by coupling to Gαᵢ.

With regard to GPCRs, there has been known a GPCR called a constitutively activated GPCR which transduces signals even when no ligand is present provided that it is excessively expressed in cells. A signal which is transduced in the absence of a ligand called a constitutive activity. In the constitutively activated GPCRs, there are that which is present in nature and a mutated GPCR which is constructed by introduction of modification such as substitution, deletion, etc. of amino acid(s) [*Mol. Pharmacol.,* 57, 890 (2000); WO 98/46995]. In some cases, the mutated GPCR increases the affinity with an agonist and, therefore, it is useful for screening ligands [*J*. *Biol. Chem.,* 272, 1822 (1997)].

In a GPCR bioassay system, there have been utilized animal cells such as CHO cells, COS-7 cells, 293 cells and HEK293 EBNA cells, etc., melanophores of frog [*Mol. Pharmacol.,* 57, 125 (2000)] or yeast [*Trends Biotechnol.,* 15, 487 (1997) ; G Protein Receptors, CRC Press, 49-68 (2000)], etc. as cells for the assay.

Although the above-mentioned GPCR bioassay systems are good assay systems, there have been demanded further improvements in detection sensitivity, signal to noise ratio, multiplicity of use, simplicity/convenience, host-vector system used, etc.

When an expression cloning is carried out using the above-mentioned assay cells as a host, it is possible to efficiently obtain a useful gene and, up to now, there have been obtained genes of β chain of interferon γ receptor and PACAP (pituitary adenylate cyclase-activating polypeptide) receptor using expression of reporter gene as an index [*Nature,* 365, 170 (1993); *Cell,* 76, 803 (1994)]. However, with regard to the expression cloning method, the same improvement as in GPCR bioassay system has been demanded as well. Incidentally, there has been neither description nor suggestion that constitutively activated GPCR gene is able to be isolated by an expression cloning method.

### Disclosure of the Invention

Objects of the present invention are to provide the following (1) to (5).
(1) Various cell lines derived from mammalian endocrine cells.
(2) Expression cloning systems of an active peptide precursor gene and a process for producing an active peptide using the cell lines as a host.
(3) A method for screening and evaluating a substance capable of acting on the cell lines using the cells.
(4) A method for screening and isolating a useful gene and a useful peptide using the cell line.
(5) A high sensitive and simple assay system for GPCR ligands used for the above-mentioned expression cloning systems.

The present invention relates to the following (1) to (106).
(1) A method of isolating a DNA encoding a peptide capable of acting as an agonist, antagonist or inverse agonist for an aimed receptor, which comprises the following steps [1] to [5] :
   [1] transfecting a cDNA or a DNA derived from a chromosome into a cell line derived from an endocrine cell to obtain a transformant;
   [2] culturing the transformant of the above [1] to express the transfected DNA and contacting a culture supernatant, a cell extract or a membrane fraction of the transformant or the transformant *per se* with a cell where the aimed receptor is expressed;
   [3] detecting a response reaction of the cell on the basis of the receptor;
   [4] selecting a transformant where the culture supernatant, the cell extract or the membrane fraction of the transformant or the transformant *per se* shows the aimed activity; and
   [5] identifying the DNA transfected in the transformant of the above [4] as a DNA for giving the aimed activity to the transformant.
(2) A method of isolating a DNA encoding a peptide acting capable of as an agonist, antagonist or inverse agonist for an aimed receptor, which comprises the following steps [1] to [7]:
   [1] dividing a cDNA library prepared using an expression vector into pools each having 1 to 10,000 clone(s);
   [2] transfecting a mixture of cDNA clones derived from each pool into a cell line derived from an endocrine cell to obtain a transformant;
   [3] culturing the transformant of the above [2] for each pool to express the transfected cDNA and then contacting a culture supernatant, a cell extract or a membrane fraction of the transformant or the transformant *per se* for each pool with a cell where the aimed receptor is expressed;
   [4] detecting a response reaction of the cell on the basis of the receptor for each pool;
   [5] selecting a pool where the culture supernatant, cell extract or membrane fraction of the transformant or the transformant *per se* shows the aimed activity and dividing the selected pool into smaller pools than those in [1];
   [6] repeating the operations of [2] to [5] until each pool consists of 1 clone; and
   [7] identifying the cDNA which gives the aimed activity to the transformant.
(3) The method of isolating a DNA according to (1) or (2), wherein a cDNA or the gene derived from a chromosome is a gene encoding an active peptide precursor.
(4) The method of isolating a DNA according to (1) or (2), wherein the receptor is a G-protein coupled receptor.
(5) The method of isolating a DNA according to (4), wherein the G-protein coupled receptor is an orphan G-protein coupled receptor.
(6) The method of isolating a DNA according to (1) or (2), wherein the cell line derived from an endocrine cell is a cell line derived from an endocrine cell where the large T antigen gene of SV40 is expressed.
(7) The method of isolating a DNA according to (1) or (2), wherein the cell line derived from an endocrine cell is a cell line derived from an endocrine cell where the large T antigen gene of temperature-sensitive mutant strain of SV40 is expressed.
(8) The method of isolating a DNA according to (1), (2), (6) or (7), wherein the cell line derived from an endocrine cell is a cell line derived from a cell of a non-human transgenic animal.
(9) The method of isolating a DNA according to (8), wherein the non-human transgenic animal is a transgenic rat.
(10) Themethodof isolating a DNA according to (7), wherein the temperature-sensitive mutant strain of SV40 is SV40tsA58.
(11) The method of isolating a DNA according to (1), (2), (6), (7) or (8), wherein the cell line derived from an endocrine cell is a cell line derived from hypothalamus or Langerhans islets.
(12) The method of isolating a DNA according to (1), (2), (6), (7) or (8), wherein the cell line derived from an endocrine cell is a cell derived from hypothalamus where at least one gene selected from the group consisting of leptin receptor (Ob-Rb) gene, preproneuromedin U gene, RFamide-related peptide (RFRP) preproprotein gene, preproorexin gene, preproopiomelanocortin gene, preproneuropeptide Y gene, preproneuropeptide FF gene, preprocorticotropin-releasing hormone gene, preprothyrotropin-releasing hormone gene, preproghrelin gene, prepromelanin concentration hormone gene, cocaine- and amphetamine-regulated transcript (CART) gene, type 2 neuromedin U receptor (NMU2R) gene, RFRP receptor gene, type 4 melanocortin receptor (MC4R) gene, type 1 neuropeptide Y receptor (NPY1R) gene, type 5 neuropeptide Y receptor (NPY5R) gene, type 2 neuropeptide FF receptor (NPFF2) gene, type 1 corticotropin-releasing hormone receptor (CRHR-1) gene, type 2 corticotropin-releasing hormone receptor (CRHR-2) gene, ghrelin receptor gene, type 1 melanin concentration hormone receptor (MCHR1) gene, preproagouti-related peptide gene, sulfonylurea receptor gene, ciliary neurotrophic factor (CNTF) receptor gene, type 1 neuromedin U receptor (NMU1R) gene, type 1 orexin receptor (OX1R) gene, type 2 orexin receptor (OX2R) gene, type 1 angiotensin II receptor gene, galanin receptor, glucagon-like peptide-1 (GLP-1) receptor gene and glucagon-like peptide-2 (GLP-2) receptor gene is endogenously expressed.
(13) The method of isolating a DNA according to (1), (2), (6), (7) or (8), wherein the cell line derived from an endocrine cell is a cell derived from hypothalamus where at least one peptide selected from the group consisting of leptin receptor (Ob-Rb), neuromedin U, RFamide-related peptide (RFRP) protein, orexin, opiomelanocortin, neuropeptide Y, neuropeptide FF, corticotropin-releasing hormone, thyrotropin-releasing hormone, ghrelin, melanin concentration hormone, cocaine- and amphetamine-regulated transcript (CART), type 2 neuromedin U receptor (NMU2R), RFRP receptor, type 4 melanocortin receptor (MC4R), type 1 neuropeptide Y receptor (NPY1R), type 5 neuropeptide Y receptor (NPY5R), type 2 neuropeptide FF receptor (NPFF2), type 1 corticotropin-releasing hormone receptor (CRHR-1), type 2 corticotropin-releasing hormone receptor (CRHR-2), ghrelin receptor, type 1 melanin concentration hormone receptor (MCHR1), agouti-related peptide, sulfonylurea receptor, ciliary neurotrophic factor (CNTF) receptor, type 1 neuromedin U receptor (NMU1R), type 1 orexin receptor (OX1R), type 2 orexin receptor (OX2R), type 1 angiotensin II receptor, galanin receptor, glucagon-like peptide-1 (GLP-1) receptor, glucagon-like peptide-2 (GLP-2) receptor and endorphin is endogenously expressed.
(14) The method of isolating a DNA according to (1), (2), (6), (7) or (8), wherein the cell of the cell line derived from an endocrine cell is a cell derived from Langerhans islets where at least one gene selected from the group consisting of preproinsulin gene, prepro-glucagon gene, preprosomatostatin gene, prepropancreatic polypeptide gene, prohormone convertase 1 (PC1) gene, prohormone convertase 2 (PC2) gene, glucagon-like peptide-1 (GLP-1) receptor gene, PDX1 (pancreatic-duodenal homeobox 1) gene, Pax 4 gene, Pax 6 gene, neurogenin 3 gene, neuro D gene, Nkx 2.2 gene, Nkx 6.1 gene, glucokinase gene, type 2 glucose transporter gene, beta-cellulin gene, sulfonylurea gene, P2Y₁ receptor gene, glucagon-like peptide-1 (GLP-1) receptor gene, type 1 somatostatin receptor gene, type 2 somatostatin receptor gene, type 3 somatostatin receptor gene, type 4 somatostatin receptor gene, type 5 somatostatin receptor gene, insulin receptor gene, glucose transporter gene and nestin gene is endogenously expressed.
(15) The method of isolating a DNA according to (1) or (2), wherein the cell in which the receptor is expressed is a B-cell line which is adapted for serum-free culture and in which the EBNA-1 gene of Epstein-Barr virus is expressed, where at least one of the following [1] to [3] is integrated in chromosomal DNA:
   [1] DNA construct for expression of a transcription factor necessary for construction of an inducible expression system;
   [2] DNA construct where a reporter gene is ligated at the downstream area of promoter having a responsive element of a transcription factor; and
   [3] DNA construct for expression of Gα protein or chimeric Gα protein.
(16) The method of isolating a DNA according to (15), wherein the B-cell line is a Namalwa cell adapted for serum-free culture.
(17) The method of isolating a DNA according to (16), wherein the Namalwa cell adapted for serum-free culture is Namalwa KJM-1 cell.
(18) The method of isolating a DNA according to (15), wherein the transcription factor necessary for construction of the inducible expression system is a chimeric protein of a ligand binding domain of estrogen receptor and yeast Gal4p.
(19) The method of isolating a DNA according to (15), wherein the responsive element of the transcription factor is cAMP responsive element (CRE), TPA responsive element (TRE), NFAT (nuclear factor of activated T cells) responsive element or serum responsive element (SRE).
(20) The method of isolating a DNA according to (15), wherein the reporter gene is firefly luciferase gene, *Renilla reniformis* luciferase gene, chloramphenicol acetyltransferase gene, β-galactosidase gene, β-lactamase gene or green fluorescent protein gene.
(21) The method of isolating a DNA according to (15), wherein the Gα protein is at least one Gα protein selected from the group consisting of Gα₁₆, Gα₁₅, Gα_{q}, Gα₁₁, Gαₛ, Gαᵢ, Gαₒ, Gα_{z}, Gα₁₂, Gα₁₃, Gα_{gust}, Gαₜ and Gα₁₄.
(22) The method of isolating a DNA according to (15), wherein the chimeric Gα protein is at least one chimeric Gα protein selected from the group consisting of the following [1] to [20]:
   [1] chimeric Gα protein where C-terminal 5 amino acids of Gαₛ are substituted with C-terminal 5 amino acids of Gα_{q};
   [2] chimeric Gα protein where C-terminal 5 amino acids of Gαₛ are substituted with C-terminal 5 amino acids of Gαᵢ;
   [3] chimeric Gα protein where C-terminal 5 amino acids of Gαₛ are substituted with C-terminal 5 amino acids of Gαₒ;
   [4] chimeric Gα protein where C-terminal 5 amino acids of Gαₛ are substituted with C-terminal 5 amino acids of Gα_{z};
   [5] chimeric Gα protein where C-terminal 5 amino acids of Gαₛ are substituted with C-terminal 5 amino acids of Gα₁₂;
   [6] chimeric Gα protein where C-terminal 5 amino acids of Gαₛ are substituted with C-terminal 5 amino acids of Gα₁₃;
   [7] chimeric Gα protein where C-terminal 5 amino acids of Gαₛ are substituted with C-terminal 5 amino acids of Gα_{Gust};
   [8] chimeric Gα protein where C-terminal 5 amino acids of Gαₛ are substituted with C-terminal 5 amino acids of Gαₜ;
   [9] chimeric Gα protein where C-terminal 5 amino acids of Gαₛ are substituted with C-terminal 5 amino acids of Gα₁₄;
   [10] chimeric Gα protein where C-terminal 5 amino acids of Gαₛ are substituted with C-terminal 5 amino acids of Gα₁₆;
   [11] chimeric Gα protein where C-terminal 5 amino acids of Gα_{q} are substituted with C-terminal 5 amino acids of Gαₛ ;
   [12] chimeric Gα protein where C-terminal 5 amino acids of Gα_{q} are substituted with C-terminal 5 amino acids of Gαᵢ;
   [13] chimeric Gα protein where C-terminal 5 amino acids of Gα_{q} are substituted with C-terminal 5 amino acids of Gαₒ;
   [14] chimeric Gα protein where C-terminal 5 amino acids of Gα_{q} are substituted with C-terminal 5 amino acids of Gα_{z};
   [15] chimeric Gα protein where C-terminal 5 amino acids of Gα_{q} are substituted with C-terminal 5 amino acids of Gα₁₂;
   [16] chimeric Gα protein where C-terminal 5 amino acids of Gα_{q} are substituted with C-terminal 5 amino acids of Gα₁₃;
   [17] chimeric Gα protein where C-terminal 5 amino acids of Gα_{q} are substituted with C-terminal 5 amino acids of Gα_{Gust};
   [18] chimeric Gα protein where C-terminal 5 amino acids of Gα_{q} are substituted with C-terminal 5 amino acids of Gαₜ;
   [19] chimeric Gα protein where C-terminal 5 amino acids of Gα_{q} are substituted with C-terminal 5 amino acids of Gα₁₄; and
   [20] chimeric Gα protein where C-terminal 5 amino acids of Gα_{q} are substituted with C-terminal 5 amino acids of Gα₁₆.
(23) The method of isolating a DNA according to (15), wherein the transcription factor necessary for construction of the inducible expression system is a chimeric protein of a ligand binding domain of estrogen receptor and yeast Gal4p, the promoter having a responsive element of the transcription factor is a promoter having a cAMP responsive element (CRE) and the reporter gene is firefly luciferase gene or *Renilla reniformis* luciferase gene.
(24) The method of isolating a DNA according to (15), wherein the transcription factor necessary for construction of the inducible expression system is the chimeric protein of the ligand binding domain of estrogen receptor and yeast Gal4p, the promoter having a responsive element of the transcription factor is a promoter having a cAMP responsive element (CRE), the reporter gene is firefly luciferase gene or *Renilla reniformis* luciferase gene and the chimeric Gα protein is a chimeric Gα protein where C-terminal 5 amino acids of Gαₛ are substituted with C-terminal 5 amino acids of Gα_{q} or a chimeric Gα protein where C-terminal 5 amino acids of Gαₛ are substituted with C-terminal 5 amino acids of Gαᵢ.
(25) The method of isolating a DNA according to (1) or (2), wherein the response reaction of the cell is at least one response reaction selected from the group consisting of release of arachidonic acid, release of acetylcholine, increase of intracellular Ca²⁺, production of intracellular cAMP, decrease of intracellular cAMP, production of intracellular cGMP, production of inositol phosphate, change in cell membrane potential, phosphorylation of intracellular protein, activation of c-fos, change in intracellular pH, cell growth, expression level of reporter gene and expression level of marker gene.
(26) The method of isolating a DNA according to (1) or (2), wherein the contact to the cell expressing the aimed receptor is a contact by layering of the cell onto a transformant.
(27) A cell line derived from hypothalamus where at least one gene selected from the group consisting of leptin receptor (ob-Rb) gene, preproneuromedin U gene, RFamide-related peptide (RFRP) preproprotein gene, preproorexin gene, preproopiomelanocortin gene, preproneuropeptide Y gene, preproneuropeptide FF gene, preprocorticotropin-releasing hormone gene, preprothyrotropin-releasing hormone gene, preproghrelin gene, prepromelanin concentration hormone gene, cocaine- and amphetamine-regulated transcript (CART) gene, type 2 neuromedin U receptor (NMU2R) gene, RFRP receptor gene, type 4 melanocortin receptor (MC4R) gene, type 1 neuropeptide Y receptor (NPY1R) gene, type 5 neuropeptide Y receptor (NPY5R) gene, type 2 neuropeptide FF receptor (NPFF2) gene, type 1 corticotropin-releasing hormone receptor (CRHR-1) gene, type 2 corticotropin-releasing hormone receptor (CRHR-2) gene, ghrelin receptor gene, type 1 melanin concentration hormone receptor (MCHR1) gene, preproagouti-related peptide gene, sulfonylurea receptor gene, ciliary neurotrophic factor (CNTF) receptor gene, type 1 neuromedin U receptor (NMU1R) gene, type 1 orexin receptor (OX1R) gene, type 2 orexin receptor (OX2R) gene, type 1 angiotensin II receptor gene, galanin receptor gene, glucagon-like peptide-1 (GLP-1) receptor gene and glucagon-like peptide-2 (GLP-2) receptor gene is endogenously expressed.
(28) A cell line derived from hypothalamus where at least one peptide selected from the group consistingof leptin receptor (Ob-Rb), neuromedin U, RFamide-related peptide (RFRP) protein, orexin, opiomelanocortin, neuropeptide Y, neuropeptide FF, corticotropin-releasing hormone, thyrotropin-releasing hormone, ghrelin, melanin concentration hormone, cocaine- and amphetamine-regulated transcript (CART), type 2 neuromedin U receptor (NMU2R), RFRP receptor, type 4 melanocortin receptor (MC4R), type 1 neuropeptide Y receptor (NPY1R), type 5 neuropeptide Y receptor (NPY5R), type 2 neuropeptide FF receptor (NPFF2), type 1 corticotropin-releasing hormone receptor (CRHR-1), type 2 corticotropin-releasing hormone receptor (CRHR-2), ghrelin receptor, type 1 melanin concentration hormone receptor (MCHR1), agouti-related peptide, sulfonylurea receptor, ciliary neurotrophic factor (CNTF) receptor, type 1 neuromedin U receptor (NMU1R), type 1 orexin receptor (OX1R), type 2 orexin receptor (OX2R), type 1 angiotensin II receptor, galanin receptor, glucagon-like peptide-1 (GLP-1) receptor, glucagon-like peptide-2 (GLP-2) receptor and endorphin is endogenously expressed.
(29) The cell line according to (27) or (28), wherein the cell line is a cell line in which the large T antigen gene of SV40 is expressed.
(30) The cell line according to (27) or (28), wherein the cell line is a cell line in which the large T antigen gene of temperature-sensitive mutant strain of SV40 is expressed.
(31) The cell line according to any of (27) to (30), wherein the cell line is a cell line derived from a non-human transgenic animal cell.
(32) An immortalized cell line obtained from Langerhans islets or hypothalamus of a non-human transgenic animal transfected with the large T antigen gene of temperature-sensitive mutant strain of SV40.
(33) The cell line according to (32), wherein the immortalized cell line obtained from Langerhans islets is a cell line where at least one gene of the genes selected from the group consisting of preproinsulin gene, prepro-glucagon gene, preprosomatostatin gene, prepropancreatic polypeptide gene, prohormone convertase 1 (PC1) gene, prohormone convertase 2 (PC2) gene, glucagon-like peptide-1 (GLP-1) receptor gene, PDX1 (pancreatic-duodenal homeobox 1) gene, Pax 4 gene, Pax 6 gene, neurogenin 3 gene, neuro D gene, Nkx 2.2 gene, Nkx 6.1 gene, glucokinase gene, type 2 glucose transporter gene, beta-cellulin gene, sulfonylurea gene, P2Y₁ receptor gene, glucagon-like peptide-1 (GLP-1) receptor gene, type 1 somatostatin receptor gene, type 2 somatostatin receptor gene, type 3 somatostatin receptor gene, type 4 somatostatin receptor gene, type 5 somatostatin receptor gene, insulin receptor gene, glucose transporter gene and nestin gene is endogenously expressed.
(34) The cell line according to (30) or (32), wherein the temperature-sensitive mutant strain of SV40 is SV40tsA58.
(35) The cell line according to (31) or (32), wherein the non-human transgenic animal is a transgenic rat.
(36) A process for producing a peptide, which comprises the following steps [1[and [2]:
   [1] culturing the cell line mentioned in any of (27) to (35) to produce and accumulate a peptide which is expressed by the cell endogenously, in the culture; and
   [2] recovering the peptide from the culture obtained in the above [1].
(37) A process for producing a peptide, which comprises the following steps [1] to [3]:
   [1] transfecting a DNA encoding an aimed peptide into a host cell to obtain a transformant wherein the cell line mentioned in any of (27) to (35) is used as the host cell;
   [2] culturing the transformant to produce and accumulate a peptide in the culture; and
   [3] recovering the peptide from the culture obtained in the above [2].
(38) The process for producing a peptide according to (36) or (37), wherein culturing is carried out at the temperature where activity of the large T antigen of the temperature-sensitive mutant strain of SV40 is not suppressed.
(39) The process for producing a peptide according to any of (36) to (38), which comprises a step for culturing in a serum-free medium, a medium containing not more than 2% of serum or a serum-free medium to which an N-supplement is added.
(40) The process for producing a peptide according to any of (36) to (39), which comprises a step for culturing in a medium containing 5 to 30 mmol/L of glucose.
(41) The process for producing a peptide according to any of (36) to (40), which comprises a step for culturing in a medium to which an agonist or antagonist for a receptor, a transporter or a channel expressed in the host cell is added.
(42) The process for producing a peptide according to (41), wherein the receptor is G-protein coupled receptor, nuclear receptor, growth factor receptor, sulfonylurea receptor, ciliary neurotrophic factor receptor, leptin receptor, cytokine receptor or sulfonylurea receptor.
(43) The process for producing a peptide according to (41), wherein the transporter is a glucose transporter.
(44) The process for producing a peptide according to (41), wherein the channel is Ca channel, K channel, Cl channel or Na channel.
(45) The process for producing a peptide according to any of (36) to (44), which comprises a step for culturing in a medium to which a substance capable of substituting a signal of a receptor expressed in a host cell is added.
(46) The process for producing a peptide according to (45), wherein the substance capable of substituting the signal of the receptor is at least one substance selected from the group consisting of adenylate cyclase, protein kinase, phosphodiesterase, low-molecular G protein, substance capable of changing intracellular cAMP or intracellular Ca²⁺ content, forskolin, 8-bromo-cyclic AMP (8-Br-cAMP), phorbol 12-myristate 13-acetate (PMA), ionomycin and 3-isobutyl-1-methylxanthine.
(47) The process for producing a peptide according to any of (36) to (46), which comprises a step for culturing on a dish coated with laminin or gelatin.
(48) The process for producing a peptide according to any of (36) to (46), wherein culturing is carried out in a medium to which succinylated concanavalin A is added.
(49) The process for producing a peptide according to any of (36) to (48), wherein culturing is carried out in a medium to which at least one substance selected from the group consisting of activin, glucagon-like peptide-1 (GLP-1), follistatin, glucose, hepatocyte growth factor, epidermal growth factor, nicotinamide, beta-cellulin, parathyroid hormone-related peptide, thyrotropin-releasing hormone, vascular endothelial growth factor, islet neogenesis-associated protein, platelet-derived growth factor, insulin-like growth factor I, fibroblast growth factor, nerve growth factor and Reg protein is added.
(50) The process for producing a peptide according to any of (36) to (49), wherein a secretagogue is added after an active peptide is produced and accumulated in the culture.
(51) The process for producing a peptide according to (50), wherein the secretagogue is potassium, glucose, tolbutamide or ATP.
(52) The process for producing a peptide according to any of (37) to (51), wherein DNA encoding one or more peptide (s) is introduced into a host cell to produce plural peptides.
(53) A method of detecting or isolating a peptide capable of acting as an agonist, an antagonist or an inverse agonist for an aimed receptor, which comprises the following steps [1] to [4]:
   [1] isolating a DNA encoding a peptide capable of acting as an agonist, an antagonist or an inverse agonist for an aimed receptor by the method mentioned in any of (1) to (26);
   [2] contacting the peptide encoded by the DNA or a partial peptide thereof with a cell where the aimed receptor is expressed;
   [3] detecting a response reaction of the cell on the basis of the receptor; and
   [4] identifying the peptide or the partial peptide which gives a response reaction in the above [3].
(54) A method of detecting or isolating a peptide capable of acting as an agonist, an antagonist or an inverse agonist for an aimed receptor, which comprises the following steps [1] to [4]:
   [1] preparing a peptide by the method of producing a peptide mentioned in any of (36) to (52);
   [2] contacting the peptide with a cell where an aimed receptor is expressed;
   [3] detecting a response reaction of the cell on the basis of the receptor; and
   [4] identifying the peptide which gives a response reaction in the above [3].
(55) The method of detecting or isolating a peptide according to (53) or (54), wherein the receptor is a G-protein coupled receptor.
(56) The method of detecting or isolating a peptide according to (55), wherein the G-protein coupled receptor is an orphan G-protein coupled receptor.
(57) The method of detecting or isolating a peptide according to (53) or (54), wherein the cell where the receptor is expressed is a B-cell line which is adapted for serum-free culture and in which the EBNA-1 gene of Epstein-Barr virus is expressed, where at least one of the following [1] to [3] is integrated into a chromosomal DNA:
   [1] DNA construct for expression of transcription factor necessary for construction of an inducible expression system;
   [2] DNA construct where a reporter gene is ligated at the downstream area of promoter having a responsive element of a transcription factor; and
   [3] DNA construct for expres sion of Gα protein or a chimeric Gα protein.
(58) The method of detecting or isolating a peptide according to (57), wherein the B-cell line is a Namalwa cell adapted for serum-free culture.
(59) The method of detecting or isolating a peptide according to (58), wherein a Namalwa cell adapted for serum-free culture is Namalwa KJM-1 cell.
(60) The method of detecting or isolating a peptide according to (57), wherein the transcription factor necessary for construction of the inducible expression system is a chimeric protein of a ligand binding domain of estrogen receptor and yeast Gal4p.
(61) The method of detecting or isolating a peptide according to (57), wherein the responsive element of the transcription factor is cAMP responsive element (CRE), TPA responsive element (TRE), NFAT (nuclear factor of activated T cells) responsive element or serum responsive element (SRE).
(62) The method of detecting or isolating a peptide according to (57), wherein the reporter gene is firefly luciferase gene, *Renilla reniformis* luciferase gene, chloramphenicol acetyltransferase gene, β-galactosidase gene, β-lactamase gene or green fluorescent protein gene.
(63) The method of detecting or isolating a peptide according to (57), wherein the Gα protein is at least one Gα protein selected from the group consisting of Gα₁₆, Gα₁₅, Gα_{q}, Gα₁₁, Gαₛ, Gαᵢ, Gαₒ, Gα_{z}, Gα₁₂, Gα₁₃, Gα_{gust}, Gαₜ and Gα₁₄.
(64) The method of detecting or isolating a peptide according to (57), wherein the chimeric Gα protein is at least one chimeric Gα protein selected from the group consisting of the following [1] to [20]:
   [1] chimeric Gα protein where C-terminal 5 amino acids of Gαₛ are substituted with C-terminal 5 amino acids of Gα_{q};
   [2] chimeric Gα protein where C-terminal 5 amino acids of Gαₛ are substituted with C-terminal 5 amino acids of Gαᵢ;
   [3] chimeric Gα protein where C-terminal 5 amino acids of Gαₛ are substituted with C-terminal 5 amino acids of Gαₒ;
   [4] chimeric Gα protein where C-terminal 5 amino acids of Gαₛ are substituted with C-terminal 5 amino acids of Gα_{z};
   [5] chimeric Gα protein where C-terminal 5 amino acids of Gαₛ are substituted with C-terminal 5 amino acids of Gα₁₂;
   [6] chimeric Gα protein where C-terminal 5 amino acids of Gαₛ are substituted with C-terminal 5 amino acids of Gα₁₃;
   [7] chimeric Gα protein where C-terminal 5 amino acids of Gαₛ are substituted with C-terminal 5 amino acids of Gα_{gust};
   [8] chimeric Gα protein where C-terminal 5 amino acids of Gαₛ are substituted with C-terminal 5 amino acids of Gαₜ;
   [9] chimeric Gα protein where C-terminal 5 amino acids of Gαₛ are substituted with C-terminal 5 amino acids of Gα₁₄;
   [10] chimeric Gα protein where C-terminal 5 amino acids of Gαₛ are substituted with C-terminal 5 amino acids of Gα₁₆;
   [11] chimeric Gα protein where C-terminal 5 amino acids of Gα_{q} are substituted with C-terminal 5 amino acids of Gαₛ ;
   [12] chimeric Gα protein where C-terminal 5 amino acids of Gα_{q} are substituted with C-terminal 5 amino acids of Gαᵢ;
   [13] chimeric Gα protein where C-terminal 5 amino acids of Gα_{q} are substituted with C-terminal 5 amino acids of Gαₒ;
   [14] chimeric Gα protein where C-terminal 5 amino acids of Gα_{q} are substituted with C-terminal 5 amino acids of Gα_{z};
   [15] chimeric Gα protein where C-terminal 5 amino acids of Gα_{q} are substituted with C-terminal 5 amino acids of Gα₁₂;
   [16] chimeric Gα protein where C-terminal 5 amino acids of Gα_{q} are substituted with C-terminal 5 amino acids of Gα₁₃;
   [17] chimeric Gα protein where C-terminal 5 amino acids of Gα_{q} are substituted with C-terminal 5 amino acids of Gα_{gust};
   [18] chimeric Gα protein where C-terminal 5 amino acids of Gα_{q} are substituted with C-terminal 5 amino acids of Gαₜ;
   [19] chimeric Gα protein where C-terminal 5 amino acids of Gα_{q} are substituted with C-terminal 5 amino acids of Gα₁₄; and
   [20] chimeric Gα protein where C-terminal 5 amino acids of Gα_{q} are substituted with C-terminal 5 amino acids of Gα₁₆·
(65) The method of detecting or isolating a peptide according to (57), wherein the transcription factor necessary forconstructionof the inducible expression systemis a chimeric protein of a ligand binding domain of estrogen receptor and yeast Gal4p, the promoter having a responsive element of the transcription factor is a promoter having a cAMP responsive element (CRE) and the reporter gene is firefly luciferase gene or *Renilla reniformis* luciferase gene.
(66) The method of detecting or isolating a peptide according to (57), wherein the transcription factor necessary for construction of the inducible expression systemis a chimeric protein of a ligand binding domain of estrogen receptor and yeast Gal4p, the promoter having a responsive element of the transcription factor is a promoter having a cAMP responsive element (CRE), the reporter gene is firefly luciferase gene or *Renilla reniformis* luciferase gene and the chimeric Gα protein is a chimeric Gα protein where C-terminal 5 amino acids of Gαₛ are substituted with C-terminal 5 amino acids of Gα_{q} or a chimeric Gα protein where C-terminal 5 amino acids of Gαₛ are substituted with C-terminal 5 amino acids of Gαᵢ.
(67) The method of detecting or isolating a peptide according to (53) or (54), wherein the response reaction of the cell is at least one response reaction selected from the group consisting of release of arachidonic acid, release of acetylcholine, increase of intracellular Ca²⁺, production of intracellular cAMP, decrease of intracellular cAMP, production of intracellular cGMP, production of inositol phosphate, change in cell membrane potential, phosphorylation of intracellular protein, activation of c-fos, change in intracellular pH, cell growth, expression level of reporter gene and expression level of marker gene.
(68) A cell line derived from a B-cell line which is adapted for serum-free culture and in which the EBNA-1 gene of Epstein-Barr virus is expressed, where at least one of the following [1] to [3] is integrated into a chromosomal DNA:
   [1] DNA construct for expression of a transcription factor necessary for construction of an inducible expression system;
   [2] DNA construct where a reporter gene is ligated at the downstream area of a promoter having a responsive element of a transcription factor; and
   [3] DNAconstruct for expression of Gα protein or a chimeric Gα protein.
(69) The cell line according to (68), wherein the cell line is a Namalwa cell adapted for serum-free culture.
(70) The cell line according to (69), wherein the Namalwa cell adapted for serum-free culture is Namalwa KJM-1 cell.
(71) The cell line according to (68), wherein the transcription factor necessary for construction of the inducible expression system is a chimeric protein of a ligand binding domain of estrogen receptor and yeast Gal4p.
(72) The cell line according to (68), wherein the responsive element of the transcription factor is cAMP responsive element (CRE), TPA responsive element (TRE), NFAT (nuclear factor of activated T cells) responsive element or serum responsive element (SRE).
(73) The cell line according to (68), wherein the reporter gene is firefly luciferase gene, *Renilla reniformis* luciferase gene, chloramphenicol acetyltransf erase gene, β-galactosidase gene, β-lactamase gene or green fluorescent protein gene.
(74) The cell line according to (68), wherein the Gα protein is at least one Gα protein selected from the group consisting of Gα₁₆, Gα₁₅, Gα_{q}, Gα₁₁, Gαₛ, Gαᵢ, Gαₒ, Gα_{z}, Gα₁₂, Gα₁₃, Gα_{gust}, Gαₜ and Gα₁₄.
(75) The cell line according to (68), wherein the chimeric Gα protein is at least one chimeric Gα protein selected from the group consisting of the following [1] to [20]:
   [1] chimeric Gα protein where C-terminal 5 amino acids of Gαₛ are substituted with C-terminal 5 amino acids of Gα_{q};
   [2] chimeric Gα protein where C-terminal 5 amino acids of Gαₛ are substituted with C-terminal 5 amino acids of Gαᵢ;
   [3] chimeric Gα protein where C-terminal 5 amino acids of Gαₛ are substituted with C-terminal 5 amino acids of Gαₒ;
   [4] chimeric Gα protein where C-terminal 5 amino acids of Gαₛ are substituted with C-terminal 5 amino acids of Gα_{z};
   [5] chimeric Gα protein where C-terminal 5 amino acids of Gαₛ are substituted with C-terminal 5 amino acids of Gα₁₂;
   [6] chimeric Gα protein where C-terminal 5 amino acids of Gαₛ are substituted with C-terminal 5 amino acids of Gα₁₃;
   [7] chimeric Gα protein where C-terminal 5 amino acids of Gαₛ are substituted with C-terminal 5 amino acids of Gα_{gust};
   [8] chimeric Gα protein where C-terminal 5 amino acids of Gαₛ are substituted with C-terminal 5 amino acids of Gαₜ;
   [9] chimeric Gα protein where C-terminal 5 amino acids of Gαₛ are substituted with C-terminal 5 amino acids of Gα₁₄;
   [10] chimeric Gα protein where C-terminal 5 amino acids of Gαₛ are substituted with C-terminal 5 amino acids of Gα₁₆;
   [11] chimeric Gα protein where C-terminal 5 amino acids of Gα_{q} are substituted with C-terminal 5 amino acids of Gαₛ;
   [12] chimeric Gα protein where C-terminal 5 amino acids of Gα_{q} are substituted with C-terminal 5 amino acids of Gαᵢ;
   [13] chimeric Gα protein where C-terminal 5 amino acids of Gα_{q} are substituted with C-terminal 5 amino acids of Gαₒ;
   [14] chimeric Gα protein where C-terminal 5 amino acids of Gα_{q} are substituted with C-terminal 5 amino acids of Gα_{z};
   [15] chimeric Gα protein where C-terminal 5 amino acids of Gα_{q} are substituted with C-terminal 5 amino acids of Gα₁₂:
   [16] chimeric Gα protein where C-terminal 5 amino acids of Gα_{q} are substituted with C-terminal 5 amino acids of Gα₁₃;
   [17] chimeric Gα protein where C-terminal 5 amino acids of Gα_{q} are substituted with C-terminal 5 amino acids of Gα_{gust};
   [18] chimeric Gα protein where C-terminal 5 amino acids of Gα_{q} are substituted with C-terminal 5 amino acids of Gαₜ;
   [19] chimeric Gα protein where C-terminal 5 amino acids of Gα_{q} are substituted with C-terminal 5 amino acids of Gα₁₄; and
   [20] chimeric Gα protein where C-terminal 5 amino acids of Gα_{q} are substituted with C-terminal 5 amino acids of Gα₁₆.
(76) The cell line according to (68), wherein the transcription factor necessary for construction of the inducible expression system is a chimeric protein of a ligand binding domain of estrogen receptor and yeast Gal4p, the promoter having a responsive element of the transcription factor is a promoter having a cAMP responsive element (CRE) and the reporter gene is firefly luciferase gene or *Renilla reniformis* luciferase gene.
(77) The cell line according to (68), wherein the transcription factor necessary for construction of the inducible expression system is a chimeric protein of a ligand bindingdomainof estrogen receptor and yeast Gal4p, thepromoter having a responsive element of the transcription factor is a promoter having a CAMP responsive element (CRE), the reporter gene is firefly luciferase gene or *Renilla reniformis* luciferase gene and the chimeric Gα protein is a chimeric Gα protein where C-terminal 5 amino acids of Gαₛ are substituted with C-terminal 5 amino acids of Gα_{q} or a chimeric Gα protein where C-terminal 5 amino acids of Gαₛ are substituted with C-terminal 5 amino acids of Gαᵢ.
(78) A method of isolating a DNA encoding a peptide capable of reacting with a substance to be tested, which comprises the following steps [1] to [4]:
   [1] transfecting a cDNA or a DNA derived from a chromosome into the cell line mentioned in any of (68) to (77) to obtain a transformant;
   [2] measuring a response reaction of the transformant in the presence of the substance to be tested using the transformant in which the transfected cDNA or chromosomal DNA is expressed;
   [3] measuring a response reaction of the transformant in the absence of the substance to be tested using the transformant in which the transfected cDNA or chromosomal DNA is expressed; and
   [4] comparing the above response reactions [2] and [3], selecting the transformant showing different response reaction and identifying the DNA transfected in the transformant.
(79) A method of isolating a DNA encoding a peptide capable of reacting with a substance to be tested, which comprises the following steps [1]) to [7]:
   [1] dividing a cDNA library prepared using expression vector into pools each having 1 to 10,000 clone(s);
   [2] transfecting a mixture of cDNA clones derived from each pool into a cell line mentioned in any of (68) to (77) to obtain a transformant;
   [3] measuring a response reaction of the transformant in the presence of the substance to be tested for each pool using the transformant in which the transfected cDNA is expressed;
   [4] measuring a response reaction of the transformant in the absence of the substance to be tested for each pool using the transformant in which the transfected cDNA is expressed;
   [5] comparing the above response reactions of [3] and (4), selecting the pool showing different response reaction and dividing the selected pool into smaller pools than those in [1];
   [6] repeating the operations of (2) to (5) until each pool consists of one clone; and
   [7] identifying the transformant showing difference response reaction in the presence and the absence of the substance to be tested and identifying the DNA which is transfected in the transformant.
(80) The method of isolating a DNA according to (78) or (79), wherein the response reaction of the cell is at least one response reaction selected from the group consisting of release of arachidonic acid, release of acetylcholine, increase of intracellular Ca²⁺, production of intracellular cAMP, decrease of intracellular cAMP, production of intracellular cGMP, production of inositol phosphate, change in cell membrane potential, phosphorylation of intracellular protein, activation of c-fos, change in intracellular pH, cell growth, expression level of a reporter gene and expression level of a marker gene.
(81) The method of isolating a DNA according to (78) or (79), wherein the substance to be tested is a substance which is prepared by culturing the cell line mentioned in any of (27) to (35).
(82) The method of isolating a DNA according to any of (78) to (81), wherein culturing is carried out at the temperature where activity of large T antigen of temperature-sensitive mutant of SV40 is suppressed or disappeared.
(83) A method of isolating a DNA encoding a peptide, which comprises the following steps [1] to [5]:
   [1] selecting a cell line where a DNA construct comprising a reporter gene ligated to downstream area of promoter having a response element of transcription factor is integrated in chromosomal DNA, which is selected from the cell lines mentioned in any of (68) to (77), as a host cell;
   [2] transfecting a cDNA or a DNA derived from a chromosome into the host cell to obtain a transformant;
   [3] measuring the expression level of the reporter gene in the transformant obtained in the above [2] when the transfected cDNA or DNA is expressed;
   [4] measuring the expression level of the reporter gene in the host cell or in the transformant obtained in the above (2) when the transfected cDNA or DNA is not expressed; and
   [5] comparing the expression levels of the reporter gene in the above [3] and [4], selecting the transformant showing different expression level of the reporter gene, and identifying a DNA transfected in the transformant.
(84) A method of isolating a DNA encoding a peptide, which comprises the following steps [1] to [8]:
   [1] dividing a cDNA library prepared using the inducible expression vector into pools each having 1 to 10,000 clone(s);
   [2] selecting a cell line where DNA construct comprising a reporter gene ligated to downstream area of promoter having a response element of transcription factor is integrated in chromosomal DNA, which is selected from the cell lines mentioned in any of (68) to (77), as a host cell;
   [3]transfecting a mixture of cDNA clones derived from each pool divided in the above [1] into the host cell obtained in the above [2] to obtain a transformant for each pool;
   [4] measuring the expression level of reporter gene in the transformant for each pool of the above [3] when the transfected cDNA is expressed;
   [5] measuring the expression level of reporter gene in the transformant for each pool of the above [3] when the transfected cDNA is not expressed;
   [6] comparing the expression levels of reporter genes in the above [4] and [5] for each pool, selecting the transformant showing higher expression level of reporter gene in [4] and dividing the selected pool into smaller pools than those in [1];
   [7] repeating the operations of the above [2] to [6] until each pool consists of one clone; and
   [8] identifying a transformant showing higher expression level of reporter gene in case the transfected cDNA is expressed than in case the transfected cDNA is not expressed and identifying the DNA transfected in the transformant.
(85) The method of isolating a DNA according to (78), (79), (83) or (84), wherein the peptide is a receptor, a transcription factor, a signal transduction molecule or an enzyme.
(86) The method of isolating a DNA according to (85), wherein the receptor is a G-protein coupled receptor.
(87) The method of isolating a DNA according to (85), wherein the receptor is a constitutively activated G-protein coupled receptor.
(88) The method of isolating a DNA according to (86) or (87), wherein the G-protein coupled receptor is an orphan G-protein coupled receptor.
(89) The method of isolating a DNA according to (78), (79), (83) or (84), wherein the peptide is a transcription factor, a signal transduction molecule or an enzyme, each of which has an activity to increase the activity of promoter having a responsive element of a transcription factor.
(90) The method of isolating a DNA according to (78), (79), (83) or (84), wherein the cDNA is a cDNA with a random mutation, encoding a mutant G-protein coupled receptor, and the peptide is a constitutively activated G-protein coupled receptor.
(91) The method of isolating a DNA according to (90), wherein the site into which the random mutation is introduced is from the second-half part of the third transmembrane region to the first-half part of the second intracellular region, or from the second-half part of the third intracellular region to the first-half part of the sixth transmembrane region in the G-protein coupled receptor.
(92) The method of isolating a DNA according to (90) or (91), wherein the site into which v random mutation is introduced is an amino acid which is the 20th residue or the 22nd residue directed to the N-terminal side from a proline residue existing in the sixth transmembrane region, in which the proline residue is one of conserved amino acid residues in a G-protein coupled receptor, or an amino acid residue corresponding to the proline residue.
(93) A constitutively activated mutant G-protein coupled receptor which is isolated by the method mentioned in any of (90) to (92).
(94) A constitutively activated mutant G-protein coupled receptor having a mutation at an amino acid which is the 20th residue or the 22nd residue directed to the N-terminal side froma proline residue existing in the sixth transmembrane region, in which the proline residue is one of conserved amino acid residues in a G-protein coupled receptor, or an amino acid residue corresponding to the proline residue.
(95) A constitutively activated mutant G-protein coupled receptor selected from the group consisting of a constitutively activated mutant G-protein coupled receptor where the 221st serine from N-terminal of OGR1 is substituted with asparagine; a constitutively activated mutant G-protein coupled receptor where the 118th aspartic acid from N-terminal of OGR1 is substituted with alanine; a constitutively activated mutant G-protein coupled receptor where the 118th aspartic acid from N-terminal of OGR1 is substituted with alanine and serine which is the 221st one is substituted with asparagine; a constitutively activated mutant G-protein coupled receptor where the 124th aspartic acid fromN-terminal of RE2 is substitutedwith alanine; a constitutively activated mutant G-protein coupled receptor where the 113th aspartic acid from N-terminal of GPR35 is substituted with alanine; a constitutively activated mutant G-protein coupled receptor where the 111th aspartic acid from N-terminal of GPCR25 is substituted with alanine; a constitutively activated mutant G-protein coupled receptor where the 135th glutamic acid from N-terminal of PGMO334 is substituted with phenylalanine, glutamine or alanine; a constitutively activated mutant G-protein coupled receptor where the 259th aspartic acid from N-terminal of PGMO334 is substituted with serine; a constitutively activated mutant G-protein coupled receptor where the 217th arginine from N-terminal of GPR43 is substituted with proline; and a constitutively activated mutant G-protein coupled receptor where the 217th arginine and the 106th glutamic acid from N-terminal of GPR43 is substituted with proline and aspartic acid, respectively.
(96) A method of screening or isolating an antagonist for MC1R using a type 1 melanocortin receptor (MC1R) and proadrenomedullin N-20 terminal peptide (PAMP) or a peptide consisting of the 9th to 20th amino acid residues from N terminal of PAMP.
(97) A method of screening or isolating an antagonist for GPR43 using an orphan G-protein coupled receptor GPR43 and acetic acid, propionic acid, acetate or propionate.
(98) A method of screening or isolating an antagonist for GPR41 using an orphan G-protein coupled receptor GPR41 and cyclopropanecarboxylic acid, propionic acid, cyclopropanecarboxylate or propionate.
(99) A method of screening or isolating an antagonist for G10d using an orphan G-protein coupled receptor G10d and an α-melanocyte stimulating hormone or adrenocorticotropic hormone.
(100) A method of isolating an agonist for a G-protein coupled receptor, which comprises the following steps [1] to [4]:
   [1] transfecting a DNA encoding any G-protein coupled receptor into the cell line mentioned in any of (68) to (77) to obtain a transformant;
   [2] measuring a response reaction of the transformant in the presence of a substance to be testedusing the transformant in which the transfected DNA is expressed;
   [3] measuring a response reaction of the transformant in the absence of a substance to be tested using the transformant in which the transfected DNA is expressed; and
   [4] comparing the response reactions of the above [2] and [3] and isolating the substance to be tested which induces the changes in the response reaction as an agonist.
(101) A method of isolating an antagonist for a G-protein coupled receptor, which comprises the following steps [1] to [4]:
   [1] transfecting a DNA encoding a G-protein coupled receptor into the cell line mentioned in any of (68) to (77) to obtain a transformant;
   [2] measuring a response reaction of the transformant in the presence of an agonist for the G-protein coupled receptor usingthetransformant in which the transfectedDNAis expressed;
   [3] measuring a response reaction of the transformant in the presence of both an agonist for the G-protein coupled receptor and a substance to be tested using the transformant in which the transfected DNA is expressed; and
   [4] isolating the substance to be tested which disappears the response reaction on the basis of the agonist of the transformant as an antagonist.
(102) The method of isolating an agonist or an antagonist for a G-protein coupled receptor according to (100) or (101), wherein the response reaction of the cell is at least one response reaction selected from the group consisting of release of arachidonic acid, release of acetylcholine, increase of intracellular Ca²⁺, production of intracellular cAMP, decrease of intracellular cAMP, production of intracellular cGMP, production of inositol phosphate, change in cell membrane potential, phosphorylation of intracellular protein, activation of c-fos, change in intracellular pH, cell growth, expression level of reporter gene and expression level of marker gene.
(103) A method of isolating an antagonist or inverse agonist for a G-protein coupled receptor, which comprises the following steps [1] to [5] :
   [1] selecting a cell line where a DNA construct comprising a reporter gene ligated to downstream area of promoter having a response element of transcription factor is integrated in chromosomal DNA, which is selected from the cell lines mentioned in any of (68) to (77), as a host cell;
   [2] transfecting a DNA encoding a constitutively activated G-protein coupled receptor into the host cell to obtain a transformant;
   [3] measuring the expression level of a reporter gene in the transformant in which the transfected DNA is expressed in the absence of a substance to be tested;
   [4] measuring the expression level of a reporter gene in the transformant in which the transfected DNA is expressed in the presence of a substance to be tested; and
   [5] comparing the expression levels of the reporter gene of the above [3] and [4] and isolating a substance to be tested which decrease the expression level of the reporter gene as an antagonist or an inverse agonist for the G-protein coupled receptor of [2].
(104) A method of isolating an activator or inhibitor for a peptide selected from the group consisting of transcription factors, signal transduction molecules and enzymes, which comprises the following steps [1] to [4]:
   [1] transfecting a DNA encoding a peptide selected from the group consisting of transcription factors, signal transduction molecules and enzymes into the cell line mentioned in any of (68) to (77) to obtain a transformant;
   [2] measuring a response reaction of the transformant in the absence of a substance to be tested using the transformant in which the transfected DNA is expressed;
   [3] measuring a response reaction of the transformant in the presence of a substance to be tested using the transformant in which the transfected DNA is expressed; and
   [4] comparing the response reactions of the above [2] and [3] and isolating the substance to be tested which changes the response reaction as an activator or an inhibitor for the peptide.
(105) The method of isolating an activator or inhibitor according to (104), wherein the response reaction of the cell is at least one response reaction selected from the group consisting of release of arachidonic acid, release of acetylcholine, increase of intracellular Ca²⁺, production of intracellular cAMP, decrease of intracellular cAMP, production of intracellular cGMP, production of inositol phosphate, change in cell membrane potential, phosphorylation of intracellular protein, activation of c-fos, change in intracellular pH, cell growth, expression level of reporter gene and expression level of marker gene.
(106) A host-vector systemwhich is characterized in that a cell line mentioned in any of (68) to (77) is used as a host cell and an expression vector having a promoter and oriP of Epstein-Barr virus is used as a vector.
(107) The host-vector system according to (106), wherein the any promoter is a Gal4p-responsible inducible expression promoter.
(108) The host-vector system according to (106) or (107), wherein the expression vector is pAMo, pAMo-nd, pAMo-d, pAGal9-nd or pAGal9-d.

In the present specification, a peptide means a substance where two or more amino acids are linked by peptide bond and includes polypeptide and oligopeptide.

### [I] Construction of an expression cloning system of an active peptide precursor gene and an efficient production system of an active peptide

An expression cloning system of an active peptide precursor gene and an efficient production system of an active peptide are able to be constructed as follows.

### 1. Preparing cell lines derived from endocrine cells

Processing from a precursor of an active peptide and modif ication after the processing are different for each peptide and are diverse. Known endocrine cell lines such as MIN6 are able to be utilized as a host but, for such a purpose of transfecting a precursor gene of an active peptide into a host cell to produce the active peptide efficiently or of cloning a precursor gene using activity of the expressed peptide as an index, it is preferred that many kinds of endocrine cell lines are obtained and a cell expressing a processing enzyme or modifying enzyme suitable for the active peptide is selected among them and is utilized as a host. Endocrine cell line is able to be established by the following methods.

### (1) Establishment of cell lines derived from endocrine cells

Cell lines derived from endocrine cells of the present invention is able to be established by isolating cells from tissues containing endocrine cells derived from a non-human transgenic animal transfected with an oncogene, followed by culturing the cells.

Examples of the non-human transgenic animal include a transgenic mouse, a transgenic rat, a transgenic pig and a transgenic rabbit, etc.

With regard to an oncogene, an oncogene derived from a virus such as SV40 and adenovirus can be exemplified and examples include the large T antigen gene of SV40 and E1A and E1B of adenovirus, etc. To be more specific, the large T antigen gene derived from SV40tsA58 which is a temperature-sensitive mutant and E1A 12S which is a hybrid of adenovirus 2 and adenovirus 5 , etc. can be exemplified. In order to establish a good cell line, it is preferred to use the large Tantigen gene of SV40tsA58. In order to express the oncogene in endocrine cells, it is necessary that an appropriate promoter capable of working in endocrine cells is present upstream of the coding region of the oncogene product. With regard to a promoter, an endogenous promoter of the oncogene *per se* derived from a virus, a promoter of an animal gene expressed in endocrine cells, etc. can be used. For example, the endogenous promoter of the large T antigen gene of SV40tsA58, H-2K^{b} class I prompter, etc. can be used.

A transgenic mouse transfected with the large T antigen gene of SV40tsA58 can be prepared by the methods mentioned in Japanese Published Unexamined Patent Application No. 292,958/1993, *Jpn*. *J*. *Cancer Res*., 82, 1344 (1991) and *Proc*. *Natl*. *Acad*. *Sci*. *USA*, 88, 5096 (1991). A transgenic rat transfected with the large T antigen gene of SV40tsA58 can be prepared by a method mentioned in Japanese Published Unexamined Patent Application 228,930/2000 or *Exp. Anim.,* 48, 255 (1999). To be more specific, a plasmid SV tsA 58 ori(-)-2 [*Cytotechnology,* 7, 165 (1991)], constructed by inserting the total genomic DNA of SV40tsA58 cleaved to open the circle with a restriction enzyme BamHI into aplasmidpBR322 , is cleavedwith a restriction enzyme BamHI to remove the vector site whereupon a DNA (5,240 bp) containing the large T antigen gene of SV40tsA58 is prepared. The endogenous promoter of the large T antigen gene of SV40tsA58 is present in the DNA and, therefore, the large T antigen gene of SV40tsA58 is expressed in somatic cell transfected with the DNA. The above DNA containing the large T antigen gene of SV40tsA58 is microinjected into a male pronucleus of a rat fertilized egg in the pronuclear stage which is a totipotent cell. The resulting egg is transplanted into an oviduct of a foster rat to give offsprings, then offsprings having the injected gene are selected and the individual rats into which the gene is integrated in a stable manner are obtained whereupon it is possible to efficiently produce a transgenic rat where the large T antigen gene of SV40tsA58 is integrated into the chromosome of cells of each tissue during the ontogenesis.

Known methods can be used as to a method for isolating cells from the tissues containing endocrine cells derived from a non-human transgenic animals transfected with an oncogene.

For example, cells derived from hypothalamus can be isolated by an enzymatic method mentioned in *J. Pharmacol. Toxicol. Method,* 36, 45-52 (1996), etc. and cells of Langerhans islets can be isolated by a method mentioned in *Diabetes,* 46, 1755 (1997), etc.

Culturing the isolated cells can be carried out according to conventional methods for culturing animal cells.

With regard to a medium for culturing the cells, a medium used generally, such as RPMI 1640 medium [*J*. *Am*. *Med*. *Assoc*., 199, 519 (1967)], Eagle's MEM [*Science*, 122, 501 (1952)], Dulbecco's modified Eagle medium (DMEM) [*Virology*, 8, 396 (1959)], 199 medium *[Proc*. *Soc*. *Exp*. *Biol*. *Med*., 73, 1 (1950)], a medium where fetal bovine serum or the like is added to those media, etc can be used.

Cultivation is usually carried out for 1 to 7 day(s) under the conditions of pH 6 to 8 at 30 to 40°C in the presence of 5% CO₂, etc. In order to grow the cells derived from a transgenic animal transfected with the large T antigen gene of temperature-sensitive mutant SV40tsA58, it is preferred to culture at the temperature by which activity of the temperature-sensitive large T antigen mutant is not inhibited (30 to 33°C or, preferably, 33°C).

During the cultivation, an antibiotic such as kanamycin or penicillin can be added to the medium if necessary.

When the cells are subcultured for 2 to 3 months, immortalized cells are able to be obtained.

According to the above-mentioned method, a large number of cell lines derived from endocrine cells such as cell lines derived fromhypothalamus and cell lines derived fromLangerhans islets are able to be prepared.

Many different kinds of cells are present in endocrine tissues such as hypothalamus and Langerhans islets etc., and it is possible to obtain many kinds of immortalized cell lines by the above method.

In the present invention, it has been found for the first time that many kinds of immortalized cell lines are able to be obtained abundantly in very efficient manner with good reproducibility particularly from endocrine cells of transgenic animals transfected with the large T antigen gene of temperature-sensitive mutant SV40tsA58 [*Exp*. *Anim*., 48, 255 (1999)].

A mixture of the obtained cell lines is able to be frozen and preserved according to a general cryopreservation method for animal cells and, upon necessity, the cells are able to be thawed to use for various analyses and experiments.

A mixture of the above-obtained cell lines is separated into single clones according to a general method such as a colony formation method [*Endocrinology,* 136, 4084 (1995)], and the resulting clones can be isolated as each cell line.

Examples of the cell line which is obtained by the above method include those obtained in the following Examples such as 798 single clones (including the clones as shown in Tables 2 to Table 5-2) derived from hypothalamus and 261 single clones (R-1 to R-45, M-1 to M-63, F-1 to F-9, D-1 to D-72 and D2-1 to D2-72) derived from Langerhans islets. Cell lines having the identical property as the above cell lines are able to be obtained by the above-mentioned method of the present invention with good reproducibility.

### (2) Selection of endocrine cell lines

With regard to many kinds of the immortalized cell lines obtained in the above (1), it is possible to select endocrine cells suitable for the expression of an aimed active peptide by checking the expression of the genes or gene products of various processing enzymes and modifying enzymes which have been known to be expressed in endocrine cells.

With regard to cell lines derived from hypothalamus or cell lines derived from Langerhans islets, for example, it is possible to select endocrine cell lines suitable for the object by checking the expression of the gene or the gene product of prohormone convertase (P1/P3, PC2, furin, PC4, PACE4, PC5/PC6, PC7/SPC7/LPC/PC8), carboxypeptidase E (CPE) or peptidylglycine α-amidating monooxigenase (PAM), etc..

It is also possible to select endocrine cells suitable for the expression of an aimed active peptide by checking the expression of active peptides expressed in endocrine cells (such as insulin, glucagon, somatostatin and pancreatic polypeptide, etc.) from the above-mentioned cell lines.

It is further possible to select endocrine cell line suitable for the expression of an active peptide by transfecting an active peptide precursor gene to the above-mentioned cell lines according to the method mentioned in the following 3. and checking whether the active peptide is produced.

Examples of amethod for checking the expression of various genes include RT-PCR and Northern blotting. Examples of a method for checking the expression of various gene products or the production of active peptides include a cell staining using various antibodies and Western blotting.

The endocrine cell lines prepared as above are also very useful as sources for peptides and for cDNAs for screening of various active peptides.

### (3) Characterization of immortalized cell lines

Characterization of many kinds of immortalized cell lines prepared in the above (1) and endocrine cell lines selected in the above (2) is able to be carried out by checking the expression of gene which has been known to be expressed in cell of the tissue wherefrom the cell line is derived and the gene product.

For example, in cell lines derived from hypothalamus, it is possible to conduct the characterization by checking the expression of leptin receptor gene, preproopiomelanocortin gene, CART gene, preproagouti-related peptide gene, preproneuromedin U gene, RFRP preproprotein gene, preprocorticotropin-releasing hormone gene, prepromelanin concentration hormone gene, preproorexin gene, preproghrelin gene, preprothyrotropin-releasing hormone gene, preproneuropeptide FF gene, MC4R gene, NPY1R gene, NPY5R gene, NMU2R gene, RFRP receptor gene, CRHR-1 gene, CRHR-2 gene, MCHR1 gene, ghrelin receptor gene, NPFF2 gene, etc.

In the cell lines derived from Langerhans islets, it is possible to conduct the characterization by checking the expression of preproinsulin gene, preproglucagon gene, preprosomatostatin gene, prepro pancreatic polypeptide gene, prohormone convertase 1 (PC1) gene, prohormone convertase 2 (PC2) gene, glucagon-likepeptide-1 (GLP-1) receptor gene, PDX1 (pancreatic-duodenal homeobox 1) gene, Pax4 gene, Pax6 gene, neurogenin 3 gene, neuro D gene, Nkx 2.2 gene, Nkx 6.1 gene, glucokinase gene, type 2 glucose transporter gene, etc. [*Adv. Pharmacol.,* 47, 255 (2000)].

Expression of various kinds of gene and expression of various kinds of gene products are able to be carried out according to the method mentioned in the following 2.

It is also possible to characterize the above-mentioned cell lines by checking the reactivity of a ligand, an agonist or an antagonist for a receptor which has been known to be expressed in cell of the tissue wherefrom the cell line is derived. Specific examples are as follows.

### [Leptin receptor]

It is possible to check whether a functional leptin receptor (Ob-Rb) is expressed in a cell by stimulating the cell with leptin and then subjecting the cell to cell staining using anti-STAT antibody (manufactured by NEB) or anti-phosphorylated STAT antibody or to Western blotting or by examining the change of the expression level of c-fos gene or SOCS-3 gene. In addition, it is able to confirm whether the leptin receptor is functional by checking the change of the expression level of the reporter gene using STAT reporter.

### [Ciliary neurotrophic factor (CNTF) receptor]

It is possible to check whether a functional CNTF receptor is expressed in a cell by stimulating the cell with CNTF and then subjecting the cell to a cell staining using anti-STAT antibody or anti-phosphorylated STAT antibody or to Western blotting.

### [GPCR coupled to Gα_{q} protein, Gα₁₁ protein, Gα₁₅ protein or Gα₁₆ protein]

When cell is stimulated by a ligand or an agonist and then checked whether the intracellular Ca²⁺ level is increased, it is possible to check whether the functional receptor is expressed in the cell.

When a reporter gene whose expression increases by an increase of Ca²⁺ level or by activation of protein kinase C is previously transfected into the cell, it is possible to check whether the functional receptor is expressed in the cell by checking the expression of the reporter gene.

Examples of such a receptor in the case of cell lines derived from hypothalamus include type 1 NMU receptor (NMU1R), type 2 NMU receptor (NMU2R), ghrelin receptor, type 1 orexin receptor (OX1R), type 2 orexin receptor (OX2R) and type 1 angiotensin II receptor, etc. and those in the case of cell lines derived from Langerhans islets include P2Y₁ receptor, etc.

### [GPCR coupled to Gαₛ protein]

When a cell is stimulated by a ligand or an agonist and then checked whether intracellular cAMP level is increased, it is possible to check whether the functional receptor is expressed in the cell.

When a reporter gene whose expression increases as a result of increase of the cAMP level is previously transfected into the cell, it is also possible to check the increase of the intracellular cAMP level by expression of the reporter gene.

Examples of such a receptor in the case of cell lines derived from hypothalamus include type 4 melanocortin receptor (MC4R), type 1 CRH receptor (CRHR-1), type 2 CRH receptor (CRHR-2), galanin receptor, GLP-1 receptor and GLP-2 receptor , etc. and those in the case of cell lines derived from Langerhans islets include GLP-1 receptor, etc.

### [GPCR coupled to Gαᵢ protein]

When cAMP levels in the cell stimulated by forskolin solely and in the cell stimulated forskolin and a ligand (or an agonist) are compared and a reduction in an increase of cAMP level in the cell in the latter case is checked, it is possible to check whether the functional receptor is expressed in the cell.

When a reporter gene whose expression increases as a result of the increase in cAMP level is previously transfected to the cell, it is also possible to check the increase of the intracellular cAMP level by expression of the reporter gene.

Examples of such a receptor in the case of cell lines derived from hypothalamus include type 1 NPY receptor (NPY 1R), type 5 NPY receptor (NPY 5R), RFRP receptor and type 2 NPFF receptor (NPFF 2R), etc. and those in the case of cell lines derived from Langerhans islets include five types of somatostatin receptors (type 1 to type 5), etc.

### [Sulfonylurea receptor]

When cell is stimulated by sulfonylurea and then it is checked whether the Ca²⁺ level in the cell increases, it is possible to check whether the functional receptor is expressed in the cell.

When a reporter gene whose expression increases as a result of an increase in Ca²⁺ level is previously transfected to the cell, it is also possible to check the increase of the intracellular Ca²⁺ level by expression of the reporter gene.

### [Insulin receptor]

When cell is stimulated by insulin and then phosphorylation of insulin receptor or phosphorylation of insulin receptor substrate 1 (IRS-1) or IRS-2 is checked, it is possible to check whether the functional insulin receptor is expressed in the cell.

### [Glucose transporter]

When cell is stimulated by glucose at a high concentration (15 to 30 mmol/L) and then it is checkedwhether Ca²⁺ concentration in the cell increases, it is possible to check whether the functional glucose transporter is expressed in the cell. It is also possible to check that by measuring the membrane potential of the cell.

In Langerhans islets, there is a cell which is activated (for example, Ca²⁺ concentration in the cell increases) by the reaction with glucose at a high concentration (15 to 30 mmol/L) like in the case of β-cell whereupon synthesis and secretion of insulin are promoted. Accordingly, with regard to the cell lines derived from Langerhans islets, it is also possible to characterize each cell line by checking the reactivity with glucose.

It is further possible to conduct the characterization by checking the expression of glucagon which is able to be utilized as a marker for α cell, insulin which is able to be utilized as a marker for β cell, somatostatin which is able to be utilized as a marker for δ cell, pancreatic polypeptide which is able to be utilized as a marker for γ cell and nestin which is able to be utilized as a marker for multipotential stem cell [*Diabetes,* 50, 521 (2001)].

### (4) Control of differentiation characteristics of immortalized cell line

When the immortalized cell line prepared in the above 1.(1) is cultured under different conditions, it is possible to control the differentiation of the cell line. It is also possible that, with regard to the cell line where differentiation characteristics is changed, to select an endocrine cell line or to characterize immortalized cell line using the method mentioned in the above (2) or (3).

With regard to a method for culturing to control the differentiation characteristics, the culturing methods mentioned in the following (a) to (h) and those where such methods are combined can be exemplified.
(a) Cultivation is carried out under such a condition that expression of cancer gene used for preparing the above immortalized cell line and activity of the oncogene product are not inhibited.
   The immortalized cell line where temperature-sensitive mutant of SV40 large T antigen is expressed is cultured at the temperature where activity of the mutant is not inhibited (30 to 33°C or, preferably, 33°C) to control the differentiation characteristics and to grow the cell line.
   When the differentiation is to be induced, the cell line can be cultured under the condition where expression of the oncogene and activity of the oncogene product are inhibited. The immortalized cell line in which the temperature-sensitive mutant of SV40 large T antigen is expressed can be cultured at 37 to 39°C or, preferably, at 37°C.
(b) Cultivation is carried out in a serum-free medium, a medium containing not more than 2% of serum or a serum-free medium to which low concentration (1%) of N-2 supplement (manufactured by Gibco) is added to .
   In the activity measuring method mentioned in the following 6., there are some cases where reporter activity of the background increases if serum is present and, therefore, an culturing method using a serum-free or low-serum medium as such is apreferredculturingmethodwhenmeasurementof activity of active peptide is necessary.
(c) Cultivation is carried out by addition of a ligand, an agonist or an antagonist for a receptor, a transporter or a channel which are expressed in the above immortalized cell line or addition of a substrate of the transporter or the channel.
   Examples of the receptor in the case of host cell line derived fromhypothalamus include GPCR (such as GLP-1 receptor), nuclear receptor, growth factor receptor or leptin receptor and those in the case of cell line derived from Langerhans islets are GPCR (such as GLP-1 receptor), nuclear receptor, growth factor receptor (such as insulin receptor), cytokine receptor (such as activin A receptor) and sulfonylurea receptor, etc.
   An example of the transporter includes a glucose transporter.
   Examples of the channel include Ca channel, K channel, Cl channel and Na channel, etc.
   When Cultivation is carried out by addition of 5 to 30 mmol/L of glucose for example, it is possible to control the differentiation characteristics of cell and the expression of gene during the cultivation.
(d) Cultivation is carried out by addition of a substance which is able to substitute for a signal of a receptor expressed in the above immortalized cell line
   Examples of the substance which is able to substitute for a signal of the receptor include protein kinase such as protein kinase A, protein kinase B, protein kinase C and MAP kinase, etc., a substance which activates or suppresses a low-molecular G protein, forskolin which is able to substitute for signal of the G-protein coupled receptor, 8-bromo-cyclic AMP (8-Br-cAMP), 3-isobutyl-1-methylxanthine, phorbol 12-myristate 13-acetate (PMA) and ionomycin, etc.
(e) Cultivation is carried out by a culturing method to control differentiation characteristics of nerve cells and glia cells. Examples of such a culturing method include a method to culture on a dish coated with laminin, and a method to culture by adding succinylated concanavalin A [*J*. *Neurobiol*., 39, 1 (1999)], etc. and the method is suitable for culturing a cell line derived from hypothalamus.
(f) Cultivation is carried out by a culturing method to control differentiation characteristics of Langerhans islets cells.
   Examples of such a culturing method include a method to culture on a dish coated with gelatin [*Diabetes*, 48, 1402 (1999)] and a method to culture by adding activin, GLP-1, follistatin, glucose, hepatocyte growth factor, epidermal growth factor, nicotinamide, beta-cellulin, parathyroid hormone-related protein, thyrotropin-releasing hormone, vascular endothelial growth factor, islet neogenesis-associated protein, platelet-derived growth factor, insulin-like growth factor I, fibroblast growth factor, nerve growth factor or Reg protein [*Saishin Igaku,* 54, 2522 (1999); *Nat. Med.,* 6, 278 (2000) : *Eur. J. Biochem.,* 267, 971 (2000); *J. Biol. Chem.,* 274, 6360 (1999); *Int. J. Mol. Med.,* 3, 247 (1999)], etc. and the method is suitable for culturing the cell line derived from Langerhans islets.
(g) Co-culturing is carried out with cells in which a ligand or an antagonist for the receptor expressed in the above immortalized cell line is expressed.
(h) Cultivation is carried out wherein a gene of a transcription factor or a signal transduction molecule participating in intracellular signal transduction of the receptor expressed in the above immortalized cell line or a gene of an important transcription factor for differentiation and function expression of the immortalized cell line is transfected to be expressed.
   Examples of the transcription factor gene in the case of a cell line derived from hypothalamus cell include Gsh-1 gene, and those in the case of cell line derived from Langerhans islets include PDX1 gene, Pax4 gene, Pax6 gene, neurogenin 3 gene, neuro D gene, Nkx 2.2 gene, Nkx 6.1 gene, Is1-1 gene and upstream stimulatory factor gene, etc. [*Adv*. *Pharmacol*., 47, 255 (2000); *Biochem*. *J*., 341, 315 (1999)]. It is also possible that a gene of a dominant negative mutant or a constitutively activated mutant of the above-mentioned receptor, signal transduction molecule or transcription factor is transfected and expressed. the gene can be transfected according to the method 3. which will be mentioned later.

### 2. Isolation of an active peptide precursor gene

With regard to the active peptide precursor gene, any gene can be used so far as it is a DNA encoding the active peptide precursor. The DNA encoding the active peptide precursor can be isolated as follows. On the basis of the nucleotide sequence of cDNA of the active peptide precursor, a region of the cDNA including the active peptide precursor coding region is appropriately selected. A DNA comprising the sequence of 20 to 40 bases of the 5'-terminal nucleotide sequence of the selected region at the 3'-terminal and a DNA comprising the sequence complementary to 20 to 40 bases of the 3'-terminal nucleotide sequence of the selected region at the 3' -terminal are synthesized by a DNA synthesizer, respectively. cDNA is prepared from tissues and cells in which the active peptide is expressed. By way of PCR using the prepared cDNA as a template and the two kinds of synthetic DNAs as primers, it is possible to amplify and isolate the DNA encoding the active peptide precursor. Preparation of the cDNA from the tissues and the cells and PCR can be carried out by a method mentioned in "Molecular Cloning: A Laboratory Manual" 3rd ed., Cold Spring Harbor Laboratory Press (2001) (hereinafter, referred to as "Molecular Cloning; 3rd ed.").

It is also possible to express and produce any peptide by the method mentioned in the following 3. and 5. where the active peptide coding region of the active peptide precursor gene is substituted with a DNA encoding a peptide. It is further possible to prepare a random peptide library using a mixture of plural DNAs having random sequences as the DNA encoding a peptide in the above method.

### 3. construction of an expression vector for an active peptide precursor gene and transfection of the vector into a host

A recombinant vector where an active peptide precursor gene is inserted downstream of a promoter of an appropriate expression vector is constructed and the resulting vector is transfected into a host cell using the endocrine cell line selected in the above 1. as the host cell for gene expression whereupon an expression system of the active peptide precursor gene can be constructed.

### (1) Transfection of a gene using a plasmid vector

Examples of a component necessary for a plasmid vector for the expression of the active peptide precursor gene include a plasmid having an expression unit (a structure wherein a restriction enzyme site into which a DNA encoding the aimed active peptide precursor can be inserted and a polyadenylation signal are linked to downstream of a promoter in this order) for a foreign gene to be expressed.

With regard to the promoter, any promoter can be used so far as it functions in animal cells and examples of the promoter include a promoter of IE (immediate early) gene of human cytomegalovirus (CMV), an early promoter of SV40, long terminal repeat (LTR) promoter of Moloney murine leukemia virus, LTR promoter of Rous sarcoma virus, a promoter of herpes simplex virus (HSV) thymine kinase (TK) gene, retrovirus promoter, heat shock promoter, SRα promoter and metallothionein promoter, etc. It is also possible to use a promoter having a responsive element for the specific transcription factor which will be mentioned in 6. (2) later. It is further possible to use an enhancer of IE gene of human CMV, etc. together with the above promoter. With regard to promoter, it is also possible to use a promoter which reacts with a specific transcription factor in animal cells and functions.

In that case, it is necessary to utilize animal cells which are ale to express the transcription factor or animal cells into which expression plasmid of the transcription factor is transfected so that the transcription factor is able to be expressed.

With regard to such a promoter, an example includes a promoter which responds the transcription factor Gal4p derived from a yeast (*Saccharomyces cerevisae*), etc.

The promoter responding to Gal4p reacts not only with Gal4p but also with chimeric protein of a DNA-binding domain of Gal4p and a transcription activation domain of any transcription factor.

Examples of the transcription activation domain of the transcription factor include a transactivation domain of VP16 of herpes simplex virus [*Nature*, 335, 563 (1988)] and a ligand binding domain of estrogen receptor [*Cell.,* 54, 199 (1988) ; *Proc. Natl. Acad. Sci. USA,* 90, 1657 (1993)]. When the ligand binding domain of estrogen receptor is utilized, transcription is able to be started by addition of estrogen to the cell.

With regard to a polyadenylation signal, any polyadenylation signal can be utilized and, for example, a polyadenylation signal of SV40 early gene, a polyadenylation signal of rabbit β-globulin gene, a polyadenylation signal of bovine growth hormone, etc. can be used.

In order to increase the copy numbers of expression plasmid transfected to a host or in order to maintain the expression plasmid transfected to a host in a state of a plasmid, a plasmid vector having a replication origin necessary for replication in animal cells is used. With regard to the replication origin, the replication origin of SV40 and oriP, the replication origin of Epstein-Barr virus, can be used. A plasmid vector having the replication origin of SV40 increases its copy numbers in a host in which SV40 large T antigen is expressed and, therefore, it is advantageous for high-level expression of the target gene. With regard to the immortalized cell line prepared in the above 1., in which the temperature-sensitive SV40 large T antigen is expressed, the aimed gene is able to be highly expressed using a plasmid vector having SV40 replication origin in the cell line cultured under the condition where the large T antigen functions. A vector comprising oriP is not integrated into a chromosome of a host in which EBNA-1 (Epstein-Barr virus nuclear antigen-1) of Epstein-Barr virus is expressed, but is maintained outside the chromosome.

Whether the vector comprising oriP is stably maintained outside the chromosome is dependent on the host. When B-cell line in which EBNA-1 is expressed is used as a host, the vector is usually stably maintained outside the chromosome. It has been known that, even EBNA-1 is not expressed inherently in a cell, there may be a case that the vector comprising oriP is maintained in a state of a plasmid when foreign EBNA-1 gene is transfected into the cell and expressed.

Further, in the plasmid vector, a drug-resistant gene containing an endogenous promoter for *Escherichia coli* and a replication origin necessary for replication in *E. coli* are necessary for the preparation of the vector using *E*. *coli*. Examples of a drug-resistant gene for *E*. *coli* include ampicillin-resistant gene (β-lactamase gene), tetracycline-resistant gene and kanamycin-resistant gene, etc. derived from *E*. *coli*. Examples of a replication origin necessary for replication in *E*. *coli* include the replication origin of pBR322 and the replication origin of co1E1, etc.

In order to obtain a stable transformant, not for transient expression, a plasmid having an expression unit of a drug-resistant gene for animal cells (a structure wherein a drug-resistant gene and a polyadenylation signal are linked to downstream of a promoter in this order) is further preferred. Examples of the drug-resistant gene for animal cells include neomycin-resistant gene, hygromycin-resistant gene, blasticidin-resistant gene, puromycin-resistant gene and zeocin-resistant gene. With regard to the promoter and the polyadenylation signal, those which are the same as those used for the expression unit into which active peptide precursor gene is inserted can be used.

Examples of the plasmid vector for expression include pcDNAI/Amp, pcDNA3.1(+), pcDNA3.1(-), pcDNA3.1(+)Hygro, pcDNA3.1 (-) Hygro (all of those are manufactured by Invitrogen) , pCDM8,pREP4, pAGE107, pAGE103, pAMo [*J.Biol. Chem.,* 268, 22782 (1993); another name: pAMoPRC3Sc (Japanese Published Unexamined Patent Application No. 336,963/1993)], pAMoA, pAS3-3, pAMoh (Example 19), pAGal9-nd (refer to Example 18 mentioned later) and pAGal9-d (refer to Example 18 mentioned later).

A DNA encoding the active peptide precursor is inserted downstream of the promoter of the expression unit for a foreign gene to construct a recombinant vector.

With regard to a method for transfecting the recombinant vector, any method can be used so far as it is a method for transfecting a DNA into animal cells and examples of the method include electroporation method [*Cytotechnology,* 3, 133 (1990)], calcium phosphate method (Japanese Published Unexamined Patent Application No. 227,075/1990), a lipofection method [*Proc. Natl*. *Acad*. *Sci*. *USA*, 84, 7413 (1987)] and a method mentioned in *Virology,* 52, 456 (1973), etc.

It is also possible to use a commercially available reagent for transfection of a DNA. Examples of the reagent for transfection of a DNA include Lipofectamine plus (manufactured by Gibco BRL), LipofectAMINE 2000 (manufactured by Gibco BRL), LipofectAMINE (manufactured by Gibco BRL), Lipofectin (manufactured by Gibco BRL), DMRIE-C (manufactured by Gibco BRL), Superfect (manufactured by Qiagen), Effectene (manufactured by Qiagen),TransFast (manufactured by Promega), GeneJammer (manufactured by Strantagene), FuGENE (manufactured by Roche), DuoFect(manufactured by Q-biogene) and Transfectram (manufactured by BioSEPRA), etc.

In the gene transfection according to the above method, it is preferred to use an expression vector having expression level as high as possible for each endocrine cell line used as a host. Therefore, it is preferred to attempt the optimization of the expression vector, the promoter and the method for gene transfection for each host used.

The optimum condition for gene transfection can be determined by detecting the activity of the produced peptide in a high sensitivity utilizing an assay method using a reporter systemwhichwill be mentioned later in 6. The optimum condition for gene transfection can be determined very efficiently by utilizing a 96-well plate in the assay system and setting a condition for gene transfection for each well.

### (2) Gene transfection using a virus vector

With regard to a virus vector for the expression of the aimed active peptide precursor gene, it is possible to use a vector which is able to produce a recombinant virus in a packaging cell and comprises a promoter suitable for the expression of the aimed gene in a host cell line and examples of the vector include MFG [*Proc*. *Natl. Acad*. *Sci*. *USA*, 92, 6733-6737 (1995)], pBabePuro [*Nucleic Acids Research*, 18, 3587-3596 (1990)], LL-CG, CL-CG, CS-CG, CLG [*Journal of Virology*, 72 , 8150-8157 (1998)] and pAdex1 [*Nucleic Acids Res*., 23, 3816-3821 (1995)], etc. In those virus vectors, at least one gene encoding a protein necessary for packaging of the virus is deficient.

Examples of the protein necessary for the packaging in the case of retrovirus vector include gag, pol and env derived from mouse retrovirus, etc.; those in the case of lentivirus vector include gag, pol, env, vpr, vpu, vif, tat, rev and nef derived from HIV virus; those in the case of adenovirus vector include E1A and E1B derived from adenovirus, etc.; and those in the case of adeno-associated virus include proteins such as Rep(p5, p19, p40) and Vp(Cap).

With regard to the promoter, the promoter mentioned in the above (1) can be used.

The active peptide precursor gene is inserted downstream of the promoter in the virus vector to construct a recombinant virus vector.

The constructed recombinant virus vector plasmid is transfected into a packaging cell suitable for the virus vector plasmid.

With regard to the packaging cell, any packaging cell can be used so far as it is a cell which is able to supplement the protein necessary for packaging and encoding the above gene deficient in the virus vector and, for example, HEK 293 cell derived from human kidney, mouse fibroblast NIH 3T3, etc. in which the gene is expressed can be used.

The above-mentioned recombinant virus vector is transfected into the above-mentioned packaging cell to produce a recombinant virus.

Examples of the method for the transfection of the above virus vector into the above packaging cell include calcium phosphate method (Japanese Published Unexamined Patent Application No. 227,075/1990) and lipofection method [*Proc. Natl. Acad. Sci. USA,* 84, 7413 (1987)].

The virus vector is able to be transfected into a host by infecting a host cell with the produced recombinant virus.

It is preferred to attempt the optimization of the expression vector, the promoter, and the method for gene transfection, for each host used. The optimum condition for gene transfection is determined by the same method as in the above (1).

### 4. Expression cloning system of an active peptide precursor gene

An expression cloning system of an active peptide precursor gene is able to be constructed as follows.

### (1) Preparation of a cDNA library

In an expression cloning system of an active peptide precursor gene, a cDNA library is prepared by inserting cDNAs isolated fromcells and tissues into an expression vector instead of a specific active peptide precursor gene.

With regard to a source for a cDNA, any tissue and cell of human being or animals can be used but, in order to screen active peptides, it is preferred to use a cell having an ability of expression of the active peptide or to use a tissue containing the cell.

For example, total brain, various sites of brain (hypothalamus, thalamus, pituitary gland, cerebellum, hippocampus, striate body, substantia nigra, caudate nucleus, amygdaloid body and callosum), adrenal gland, kidney, small intestine, colon, heart, lymph node, spinal cord, trachea, pancreas, bone marrow, liver, mammary gland, uterus, lung, placenta, stomach, thyroid gland, skeletal muscle, pancreatic Langerhans islets, α cell, β cell, δ cell, γ cell, etc. can be used. The immortalized cell line prepared in the above 1. can be used as well.

mRNA is isolated from the above tissues or cells, double-stranded cDNA is synthesized from the mRNA and the cDNA is inserted downstream of the promoter of the expression vector mentioned in 3.(1) to construct a recombinant vector. The recombinant vector is transfected into *E*. *coli* whereupon a cDNA library is able to be prepared. An example of the cDNA library prepared by such a method includes a cDNA library derived from human hypothalamus prepared using the inducible expression vector pAGal9-nd (refer to Example 23), etc. The cDNA library is diluted to an appropriate concentration and cultured on an agar medium whereupon each of the resulting colonies is able to be isolated as clone (*E*. *coli*). In addition, when a plasmid (a recombinant vector) is isolated from each clone (*E*. *coli*), a cDNA clone (a plasmid) is able to be isolated. The plasmid is able to be isolated by a general method mentioned in Molecular Cloning, 3rd ed., etc. or using a kit such as QIAprep 96 Turbo Miniprep Kit (manufactured by Qiagen), etc.

### (2) Screening a cDNA encoding an active peptide precursor

The clones (*E*. *coli*) isolated from the cDNA library are divided into pools each having 1 to 10,000 clone(s) or, preferably, 10 to 100 clones and *E*. *coli* is cultured for each pool to isolate plasmids (a mixture of cDNA clones). plasmids (recombinant vectors) derived from each pool are transfected into host cells by a method mentioned in 3. where the cells prepared in 1. is used as host cells. The resulting transformants are cultured by the method mentioned in 5. (1) later and a protein encoded by each cDNA clone is expressed. The transformant or the culture supernatant is used as a sample, its activity is measured by the method mentioned in 6. later and the pool where activity is detected is selected. The selected pool is divided into smaller pools again and the same operation is carried out. The step is repeated and, finally, the above operation is carried out for each 1 clone whereupon a cDNA encoding the aimed active peptide precursor is able to be identified.

When a nucleotide sequence of a cDNA contained in each cDNA clone is apparent, cDNAs having a high possibility of encoding an active peptide precursor are selected and the above operation is carried out whereupon a DNA having the aimed activity is able to be screened efficiently. An example of the cDNA having a high possibility of encoding an active peptide precursor is a DNA encoding a peptide characterized in that it has a signal sequence, that it consists of 80 to 250 amino acids, that there is a sequence where two continuous basic amino acids in a region other than a signal sequence and that it has no membrane-binding domain. A cDNA having a high possibility of encoding an active peptide precursor can be selected by analyzing the above-mentioned characteristics for an amino acid sequence which is able to be encoded by each cDNA. A signal sequence and a transmembrane region are able to be predicted from the amino acid sequence using a program such as PSORT [*Trends Biochem. Sciences,* 24 , 34 (1999)] and SignalP [Signal P web site (http://www.cbs.dtu.dk/services/SignalP/)], etc. or using analytical software MacMolly 3.5 (Aroka), analytical software SOSUI system ver 1.0/10 (Mitsui Joho Kaihatsu), etc.

### 5. Production of an active peptide

### (1) Production of an active peptide using a transformant

An aimed active peptide is able to be produced from culture medium or cell obtained by culturing the transformant obtained in the above 3. or an extract from the cell.

Cultivation is able to be carried out according to the culturing method of the above-mentioned 1. (1) or the culturing method to control the differentiation characteristics of the immortalized cell line of the above 1.(4).

### (2) Production of an active peptide using an endocrine cell line

Many kinds of endocrine cell lines established by the method mentioned in the above 1. are cultured according to the methods of above 1.(1) or 1.(4) whereupon an active peptide specific for each cell line is able to be produced.

Examples of the known active peptide produced by a cell line derived from hypothalamus include NPY, α-MSH, NPFF, CRH and TRH, etc. and examples of the known active peptide produced by a cell line derived from Langerhans islets include insulin, glucagon, somatostatin, pancreatic polypeptide and ghrelin, etc. It is considered that a novel active peptide is also produced from many kinds of endocrine cell lines established by the method mentioned in the above 1. and such a cell line is very useful for screening a novel active peptide as well.

### (3) Release of an active peptide by secretory stimulation

It has been known that, in endocrine cells, an active peptide is accumulated in secretory granules and, when an appropriate secretory stimulation is applied, it is at once released outside the cells. Therefore, even in the production of an active peptide using the cell line derived from an endocrine cell according to the present invention, there are some cases where it is preferred to apply an appropriate secretory stimulation to the cell.

Examples of the secretory stimulant include high-potassium (such as 50 to 100 mmol/L), high-glucose (such as 25 mmol/L), tolbutamide (such as 100 µmol/L) and ATP (such as 1 µmol/L to 10 mmol/L), etc.

Substances which are able to be used as secretory stimulants can also be selected as follows.

A substance to be tested is added to the cell line derived from an endocrine cell and a substance to be tested having an activity of increasing the Ca²⁺ concentration in the cells is selected. Measurement of Ca²⁺ concentration can be carried out according to known methods using an instrument such as CAF-110 (manufactured by Nippon Bunko), FLIPR (manufactured by Molecular Devices) and FDSS 6000 system (manufactured by Hamamatsu Photonics), etc.

An active peptide precursor gene (such as vasopressin precursor gene or CRH precursor gene) is transfected into an endocrine cell line, cultivation is carried out for 2 to 4 days, a substance to be tested is added thereto and the amount of the active peptide (such as vasopressin or CRH) in the medium is measured. The amount of the active peptide is compared with that in the case where a substance to be tested is not added, and a substance which increases the amount of the active peptide by adding is selected as a secretory stimulant.

With regard to a secretory stimulant which is used conclusively, it is important to select the one which does not affect the detection of the activity. In the case of an assay cell using a reporter system of the following 6., it is necessary to select a stimulant which does not increase the reporter activity by acting on the assay cell.

It is possible to prepare a peptide solution of a high concentration by using as little medium as possible, adding an appropriate secretory stimulant and releasing a peptide at once. Such a peptide solution of a high concentration is preferred for the above-mentioned assay system because the activity is detected efficiently.

When an assay is carried out by layering the assay cells of the following 6., concentration of peptide near the assay cell is able to be enhanced by addition of an appropriate secretory stimulant even if a medium in a small amount is not used and, therefore, activity is able to be detected more efficiently as comparedwith the case when no secretory stimulant is used.

In some cases, accumulation of the produced active peptide to secretory granules is able to be controlled depending upon the culturing condition.

For example, when cultivation is carried out using a medium containing high amount of glucose (25 mmol/L glucose) in the production of insulin by MIN6 which is a β cell line derived from a mouse, insulin is produced in a high amount but is secreted into the medium constantly while, when cultivation is carried out using a medium containing low amount of glucose (5.5 mmol/L glucose), the produced amount is low but insulin is accumulated in secretory granules.

Accordingly, a lot of active peptide is able to be released at once when MIN6 in which active peptide precursor gene is expressed is cultured in a high-glucose-containing medium [such as DMEM (HG): DMEM containing 25 mmol/L glucose, 15% of fetal bovine serum, 25 U/ml of penicillin and 25 µg/ml of streptomycin (manufactured by Nissui Seiyaku)], then it is cultured in a low-glucose-containing medium [such as DMEM (LG): DMEM containing 5.5 mmol/L of glucose, 15% of fetal bovine serum, 25 U/ml of penicillin and 25 µg/ml of streptomycin (manufactured byNissui Seiyaku)], the resulting active peptide is accumulated in secretory granules and, after that, an appropriate secretory stimulation is carried out.

### (4) Modification of active peptide

An appropriate modification necessary for activity of peptide is able to be applied when the endocrine cell line prepared in 1. or, particularly, the endocrine cell line which expresses gene of various kinds of processing enzymes and repair enzymes mentioned in 1.(2) is used to produce a peptide. Examples of modification of peptide as such are amidation of amino acid at C terminal, addition of pyroglutamic acid to N-terminal, addition of fatty acid and addition of sugar chain, etc.

### 6. Measurement of activity of peptide

After an active peptide precursor gene is transfected into and expressed in endocrine cell, activity of the peptide is able to be measured using culture supernatant of the cell, cell extract of the cell, membrane fraction of the cell or the cell *per se.* With regard to a method for the measurement of activity, any method which is able to measure the activity can be used and it is preferred to use a method having a sensitivity of as high as possible. Examples of themethod include a sandwich ELISA using an antibody which binds to the active peptide and a bioassay specific to the active peptide.

When an active peptide is a ligand for a receptor such as GPCR, the active peptide is able to be detected simply and conveniently with a high sensitivity and a high signal/noise ratio by an assay system using a receptor.

Hereinafter, specific examples using a reporter gene in an assay system utilizing a receptor will be shown. A cell used for the assay (hereinafter, sometimes referred to as an assay cell) has a reporter gene ligated downstream of a promoter responding to the signal from the receptor such as GPCR and is a cell having an expression unit of the receptor gene of an active peptide. When the active peptide is bound to a receptor on the assay cell, signal transduces from the receptor and expression of the reporter gene is induced. Accordingly, when a sample is contacted with the assay cell and the expression level of the reporter gene is measured, it is possible to detect the active peptide in the sample. The receptor for the active peptide is GPCR which is a type of inducing an increase in Ca²⁺ in the cell, it is also possible to detect the active peptide in the sample when the sample is contacted to the assay cell and an increase in Ca²⁺ in the cell is measured. An assay cell is able to be constructed by the following methods.

### (1) Construction of a host-vector system for expression of a DNA encoding the receptor of the active peptide

A host-vector system for expression of a DNA encoding the receptor of the active peptide is able to be constructed by a general methodmentioned, for example, in Molecular Cloning, 3rd ed. In a system where a B-cell line which is adapted for serum-free culture and expresses EBNA-1 gene of Epstein-Barr virus constructed in the present invention is used as a host and a plasmid having a replication origin oriP of Epstein-Barr virus is a vector, vector is stably present in a state of plasmid outside the chromosome of host cell and has the following properties (i) to (iii) which is preferred in view of construction of assay cell.

(i) Efficiency of transfection of a vector into host cell is high. (ii) Since host cell and the resulting assay cell are non-adherent cells (suspended cells), manipulation of culture is simple and easy and simplicity and multiplicity of use of the assay are high. When a cell where the expression of the peptide is to be checked is an adherent cell, it is easy to assay by layering assay cells on the cells and, therefore, detecting sensitivity of the assay system is able to be increased in some assay methods. (iii) a host cell and the resulting assay cell are able to be cultured in a serum-free medium. Since various components are contained, there is no need of using serum which can increase the background of the assay system in an assay system and, therefore, detection sensitivity of assay system, signal/noise ratio, multiplicity of use and simplicity/convenience are high. The system constructed in the present invention will be shown.

### (a) Preparing a B-cell line which is adapted for serum-free culture and in which EBNA-1 gene of Epstein-Barr virus is expressed

With regard to B-cell line for expressing EBNA-1 gene of Epstein-Barr virus, any B-cell line can be used so far as it is a B-cell line in which EBNA-1 gene of Epstein-Barr virus is expressed but, in view of safety, it is preferred to use a cell line which does not produce Epstein-Barr virus. With regard to such a cell, Namalwa cell (ATCC No: CRL-1432), Raji cell (ATCC No: CCL-86) and Daudi cell (ATCC No: CCL-213) can be used for example. More advantageously, Namalwa cell can be used. When the above-mentioned cell line is adapted for serum-free culture according to a known method [*Cytotechnology*, 1, 151 (1988); *Dev*. *Biol*. *Stand*., 99, 153 (1999); *Pharmacol*. *Ther*., 53, 355 (1992); *Cytotechnology*, 5, 3 (1991); *Adv*. *Biochem*. *Eng*. *Biotechnol*., 34, 95 (1987); Japanese Patent No. 1,653,986], it is possible to prepare a B-cell line which is adapted for serum-free culture and in which EBNA-1 gene of Epstein-Barr virus is expressed. With regard to B-cell line in which EBNA-1 gene of Epstein-Barr virus is expressed and which is adapted for serum-free culture, Namalwa cell adapted for serum-free culture is used preferably and, more preferably, Namalwa KJM-1 cell [*Cytotechnology*, 1, 151 (1988)] prepared by adapting Namalwa cell for serum-free culture.

With regard to a medium for culturing the B-cell line which is adapted for serum-free culture and in which EBNA-1 gene of Epstein-Barr virus is expressed, amediumusedgenerally, such as RPMI 1640 medium [*J. Am. Med. Assoc.*, 199, 519 (1967)], Eagle's MEM [*Science*, 122, 501 (1952)], DMEM [*Virology,* 8, 396 (1959)], 199 medium [*Proc. Soc. Exp. Biol*. *Med.*, 73, 1 (1950)] or media in which additives for serum-free culture are added to those media [*Cytotechnology*, 1, 151 (1988); *Dev. Biol. Stand*., 99, 153 (1999); *Pharmcol. Threr*., 53, 355 (1992); *Cytotechnology,* 5, 3 (1991); *Adv. Biochem. Eng. Biotechnol.,* 34, 95 (1987)], etc. can be used. Also, a commercially available medium for serum-free culture can be used.

Cultivation is usually carried out for 1 to 7 day(s) under the condition of pH 6 to 8, at 30 to 40°C, in the presence of 5% CO₂, etc. During the cultivation, an antibiotic substance such as kanamycin and penicillin can be added to the medium according to need. In addition, when plasmid containing drug-resistant gene is introduced into the cell as will be mentioned later, cultivation can be carried out by addition of the corresponding drug to the medium.

### (b) Construction of a vector

An example of the expression vector into which a DNA encoding a receptor of an active peptide is inserted includes a vector which is able to express the DNA in a host animal cell and has oriP of Epstein-Barr virus as a replication origin necessary for replication in the animal cell. Examples of such an expression vector include pAMo, pAMoA, pAMoh, etc. or that where the oriP is introduced to the expression vector of the above 3.(1), etc.

DNA encoding a receptor of active peptide is inserted downstream of a promoter of expression unit for foreign gene in an expression vector to construct a plasmid where the receptor is expressed. DNA encoding the receptor is able to be isolated as follows. On the basis of a nucleotide sequence of cDNA encoding the receptor, a region of the cDNA including the receptor coding region is appropriately selected. A DNA comprising a sequence of 20 to 40 bases of the 5'-terminal nucleotide sequence of the selected region at 3'-terminal and a DNA comprising a sequence complementary to 20 to 40 bases of the 3' -terminal nucleotide sequence of the selected region at 3'-terminal are synthesized by a DNA synthesizer, respectively. A cDNA is prepared from tissues and cells in which the receptor is expressed. It is possible to amplify and isolate the DNA encoding the receptor by means of a PCR using the two kinds of the synthetic DNAs as primers and using the prepared cDNA as a template. Preparation of cDNA from the tissues or the cells and PCR are able to be carried out by a method mentioned in Molecular Cloning, 3rd ed.

### (c) Transfecting the expression plasmid into a host and preparing a transformant

The expression plasmid is transfected into the host cell prepared in 6.(1)(a) by the method mentioned in the above 3.(1). A transformant can be prepared and cultured according to a known method mentioned in Japanese Published Unexamined Patent Application No. 227,075/1990 or Japanese Published Unexamined Patent Application No. 257,891/1990.

### (2) Construction of a host-vector system for inducible expression of a DNA encoding a receptor for an active peptide

An inducible expression systemof a DNA encoding a receptor for an active peptide is incorporated in a host-vector system where a B-cell line which is adapted for serum-free culture and in which EBNA-1 gene of Epstein-Barr virus is expressed and which is prepared in the above 6. (1) is a host and plasmid having oriP of Epstein-Barr virus is a vector whereupon a host-vector system capable of an efficient inducible expression of the DNA can be constructed. Although the cell, in which a DNA encoding a receptor for foreign active peptide is expressed, often shows poor growth, it is possible that the assay cell is able to be well grown when expression of the DNA is suppressed until being used for the assay. In case a receptor is GPCR, when GPCR is transiently highly expressed using an inducible expression system as shown in the following [II](2), it is now possible to detect a constitutive activity of GPCR.

Inducible expression system is such a system that an inducible expression vector having a structure of being ligated a DNA downstream of a promoter responding to activated transcription factor is transfected to a host where a transcription factor which is activated by addition of specific drug, etc. is expressed and the DNA is induced and expressed by addition of drug, etc. With regard to the inducible expression system, for example, a system where estrogen is used [*Cell*, 54, 199 (1988): *Proc*. *Natl*. *Acad*. *Sci*. *USA*, 90, 1657 (1993)], a system where tetracycline-resistant operon is used [*Proc*. *Natl*. *Acad*. *Sci*. *USA*, 89, 5547 (1992); *Methods Enzymol*., 283, 159 (1997)], a system where insect hormone ecdysone is used [*Proc*. *Natl*. *Acad*. *Sci*. *USA*, 93, 3346 (1996)], a system where lactose operon is used [*Cell*, 48, 555 (1987)], etc. can be used.

In a system where estrogen is used [*Cell*, 54, 199 (1988): *Proc*. *Natl*. *Acad*. *Sci. USA*, 90, 1657 (1993)], a chimeric protein (Gal4-ER) of a ligand binding domain of estrogen receptor and a DNA binding domain of a transcript factor Gal4p derived from yeast (*Saccharomyces cerevisiae*) is used as a transcription factor to be expressed in the host while, as a promoter for expression vector, Gal4p-responding promoter is used. In this system, as a result of addition of estrogen, expression of any gene under the promoter is able to be induced. Moreover, when concentration of estrogen used is changed, expression level of gene is able to be controlled.

In a system where tetracycline-resistant operon is used [*Proc*. *Natl. Acad. Sci. USA*, 89, 5547 (1992); *Science Methods* *Enzymol*., 283, 159 (1997)], tetracycline-controlled transactivator (a chimeric protein of tetracycline repressor of E. coli and a transcription activation region of VP16 of HSV) or reverse tetracycline-controlled transactivactor (a chimeric protein of a mutated tetracycline repressor which activates the transcription upon binding to tetracycline and a transcription activation region of VP16 of HSV) is used as a transcription factor to be expressed in a host while, as a promoter for expression vector, promoter having a tetracycline-responding element is used. In this system, it is possible to induce the expression of any DNA under the above promoter by removal of tetracycline or doxycycline in case the tetracycline-controlled transactivator is used while, in case the reverse tetracycline-controlled transactivator is used, it is possible to do that by addition of the drug. When concentration of the existing drug is changed, expression level of gene can be controlled.

In a system where lactose operon of *E*. *coli* is used, a lactose repressor of *E*. *coli* is used as a transcription factor to be expressed in a host while, as a promoter for vector, a promoter having a lactose operator is used. In this system, expression of any DNA under the promoter can be induced by addition of isopropyl-β-D-thiogalactoside.

In a system using insect hormone, a ecdysone receptor derived from vinegar fly and a heterodimer of retinoic acid receptor RXR are used as transcription factors to be expressed in a host while, as a promoter for vector, a promoter having an ecdysone-responding element is used. In this system, expression of any DNA under the promoter can be induced by addition muristerone A or ponasterone A.

Inducible expression vector can be constructed, for example, by the following method.

The above promoter having a responsive element corresponding to a transcription factor to be expressed in a host is introduced into a plasmid vector for expression mentioned in 6.(1)(b).

The promoter can also be constructed by insertion of DNA containing one or more responsive element (s) into promoter for expression of exogenous gene mentioned, for example, in 3. (1). Its examples include Gal4p-responsive promoter having five Gal4p responsive elements (corresponding to transcription factor Gal4-ER) (pAGalSd1 of Example 18, etc.), promoter having a tetracycline-responsive element ligated to CMV promoter under downstream side of tetracycline-responsive element (pTRE2 of Clontech, etc.), promoter having a lactose operator ligated to lactose operator at downstream side of LTR promoter of RSV (pORSVI/MCS of Stratagene, etc.) and promoter having ecdysone-responsive element ligated to heat shock promoter at downstream side of five ecdysone-responsive elements (pEGSH of Stratagene, pIND of Invitrogen, etc.).

With regard to responsive element and TATA element in the promoter, it is possible to optimize sensitivity and signal/noise ratio by investigating each element, number and position. As will be mentioned later in 6.(3), a plasmid where reporter is inducibly expressed where reporter gene is inserted in downstream s ide of a promoter to be investigated, each plasmid where reporter is inducibly expressed is transfected to a host cell to obtain a transformant, a reporter is subjected to an inducible expression by the transformant and a promoter where expression level of reporter upon non-induction is low and ratio of the expression level of reporter upon induction to that upon non-induction is high is selected whereupon optimization of promoter is able to be carried out.

Examples of the vector for inducible expression subjected to optimization of promoter as such include pAGal9-d and pAGal9-nd (both in Example 18), etc.

When DNA encoding a receptor of active peptide is inserted at downstream side of promoter for inducible expression mentioned above, it is possible to construct a plasmid which is able to inducibly express the DNA encoding a receptor of active peptide.

Host which expresses transcription factor for conducting the inducible expression of the DNA is able to be constructed as follows.

With regard to a vector for construction of expression plasmid, any vector can be used so far as it is a vector which is able to express the transcription factor in animal cells and example of the vector includes the vector mentioned in 3. (1). Expression plasmid of transcription factor constructed as such is constructed according to a common method such as that in Molecular Cloning, 3rd ed. Examples of the expression plasmid of transcription factor as such include expression plasmid pGERbsrR2 of Gal4-ER (Example 18), expression plasmid pTet-Off of tetracycline-controlled transactivator (manufactured by Clontech), expression plasmid pTet-On (manufactured by Clontech) of reverse tetracycline-controlled transactivactor, expression plasmid pCMVLacI (manufactured by Stratagene) of lactose repressor, expression plasmid pERV3 (manufactured by Stratagene) of retinoic acid receptor RXR and ecdysone receptor and pVgRXR (manufactured by Invitrogen), etc.

With regard to a drug-resistant gene for animal cells used in the above vector for expression of transcription factor, it is preferred to use other drug-resistant gene than the drug-resistant gene for animal cells used for the above vector for inducible expression.

Expression plasmid of transcription factor is transfected by the method mentioned in 6.(1) into a B-cell line which is adapted for serum-free culture and in which EBNA-1 gene of Epstein-Barr virus is expressed and which is prepared in 6. (1) to obtain many transformants. Reporter inducible expression plasmid where the reporter gene which will be mentioned later in 6.(3) instead of DNA encoding a receptor of active peptide is inserted into the above-mentioned vector for inducible expression is prepared and induced into those transformants. For each of the transformants, expression of reporter gene is inducedbyadditionofdrug, etc. forinductionoftranscription, expression levels of reporter gene upon induction and upon non-induction are measured and the ratio of inducible expression ([expression amount of reporter upon induction]/[expression amount of reporter upon non-induction]) is calculated. A transformant having excellent property in the construction of assay cell (transformant where expression level upon non-induction is low and the ratio of inducible expression is high) is selected as a host cell.

When no host cell having excellent property is obtained even when many transformants are subjected to selection, strength of promoter for the expression of transcription factor, promoter sequence in the inducible expression plasmid, etc. are checked and selection of a host cell can be carried out according to the above-mentioned method once again.

Examples of a host cell having excellent property obtained by the above-mentioned method include a cell line KJMGER8 (Example 18) where Gal4-ER expression plasmid pGERbsrR2 is transfected into Namalwa KJM-1 cell and integrated into chromosomal DNA, etc.

A host-vector systemusingKJMGER8 as a host and pAGal9-nd or pAGal9-d as a vector for inducible expression is a very good inducible expression system where there is no omission of gene expression upon non-induction and, in addition, induction ratio of gene expression is high.

### (3) Construction of host into which reporter system is integrated

### (a) Construction of a reporter plasmid

Vector for expression into which reporter gene is inserted is able to be constructed by the induction of promoter having a responsive element corresponding to transcription factor into plasmid vector used for the expression of active peptide precursor gene mentioned in 3.(1). The promoter can also be constructed, for example, by insertion of DNA containing one or more responsive element (s) into promoter for expression of exogenous gene mentioned in 3.(1).

With regard to promoter for expression of reporter gene in the case of a reporter system corresponding to signal of receptor, that having a responsive element corresponding to signal of receptor is used. When a receptor is GPCR for example, a responsive element corresponding to the kind of G protein where GPCR bound to the active peptide to be measured is coupled is used. To be more specific, in the case of GPCR coupled to Gas, intracellular cAMP increases by binding to a ligand whereby a promoter having cAMP-responsive element (CRE) can be used; in the case of GPCR coupled to Gαᵢ, intracellular cAMP decreases or MAP kinase cascade is activated by binding to a ligand whereby a promoter having CRE or serum responsive element (SRE) can be used; and, in the case of GPCR coupled to Gα_{q}, Gα₁₁, Gα₁₅ or Gα₁₆, protein kinase C is activated or intracellular Ca²⁺ is increased by binding to a ligand whereby a promoter having TPA responsive element (TRE) or NFAT (nuclearfactorof activated T cells) responsive element can be used.

With regard to responsive element and TATA element in the promoter, it is possible to optimize sensitivity and signal/noise ratio by investigating each element, number and position. To be more specific, each reporter plasmid having promoter to be investigated is constructed, each reporter plasmid is induced into a host cell to obtain a transformant as will be mentioned in (b) later, the transformant is stimulated to express a reporter and a promoter in which the expression level of reporter upon non-stimulation is low and the ratio of expression level of reporter upon stimulation to that upon non-stimulation is high is selected.

With regard to a reporter gene, any reporter gene can be utilized and examples of the reporter gene include chloramphenicol acetyltransf erase gene, β-galactosidase gene, β-lactamase gene, firefly luciferase gene, *Renilla reniformis* luciferase gene and green fluorescent protein (GFP) gene [*Mol. Biotechnol.,* 13, 29 (1999); *Anal. Chem.,* 70, 579A (1998); *J*. *Recept*. *Signal*. *Transduct*. *Res*., 19, 395 (1999); *J*.*Recept*.*Signal Transduct*.*Res*., 20, 189 (2000); *Methods Mol. Biol*., 130, 165 (2000)].

With regard to drug-resistant gene for animal cells, it is preferred to use a drug-resistant gene for animal cells which is different from the drug-resistant gene for animal cells [the drug-resistant gene for animal cells used for expression plasmid of DNA encoding a receptor of active peptide mentioned in 6. (1) or (2) or that used for expression plasmid for transcription factor mentioned in 6. (2)] being already transfected to a host cell into which the present reporter plasmid is transfected. In conducting an optimization of promoter of reporter gene expression unit, it is preferred to use oriP of Epstein-Barr virus where plasmid stably exists outside the chromosome of a host cell as a replication origin necessary for replication in animal cells.

Examples of the reporter plasmid constructed as such include pACREpluc which expresses firefly luciferase under the control of promoter having 16 CREs which is a responsive element corresponding to signal of GPCR and pACRERluc which expresses *Renilla reniformis* luciferase under the control of promoter having 16 CREs.

### (b) Incorporation of reporter system into host cell and selection of excellent transformant

The reporter plasmid constructed by the method mentioned in the above (a) is transfected to a host cell constructed by the method mentioned in the above (1) or (2) to obtain many stable transformants. A transformant having excellent property in view of construction of assay cell (having good response to stimulation and having low background) is selected from those as follows and used as a host cell for the construction of assay cell. With regard to a method for transfecting a reporter plasmid, the method mentioned in the above 3. (1) can be used. When the reporter plasmid contains oriP of Epstein-Barr virus, the host cell is transfected after disruption of the function of oriP or after removal of oriP by cleaving with restriction enzyme whereby the plasmid is able to be integrated into a chromosome of the host cell.

With regard to the transformant, expression level of reporter gene upon induction by a drug, etc. for induction of the transcription and that upon non-induction are compared and a cell line having a good response to stimulation is selected. For example, when a promoter having CRE in a host cell where GPCR is expressed is used, the cell is stimulated by forskolin whereby reporter gene is able to be expressed. When TRE is used in the same host cell, a reporter gene is able to be expressed by PMA (phorbol 12-myristate 13-acetate). In that case, a cell line where expression level (background) of reporter gene without stimulation by a substance promoting the transcription from the responsive element is as low as possible is selected. It is also possible that expression level of reporter gene upon stimulation of the cell with an agonist or a ligand for GPCR and that upon non-stimulation are compared and a cell line having a good response to stimulation is selected. In that case, a cell line where the expression level (background) of reporter gene upon non-stimulation is as low as possible is selected. For example, Namalwa KJM-1 and KJMGER8 endogenously express adenosine receptor of a 2a type (A2a) coupled to Gαₛ and, therefore, in the case of a transformant where a reporter plasmid containing CRE as responsive element is introduced into such a cell, it is possible to use 5' -N-ethylcarboxamide adenosine (NECA) which is an agonist for A2a as a stimulant.

Expression level of reporter gene is able to be measured using activity and amount of reporter polypeptide as an index according to known methods [*Mol. Biotechnol*., 13, 29 (1999); *Anal*. *Chemi*., 70 , 579A (1998); *J. Recept*. *Signal Transduct*. *Res*., 19, 395 (1999); *J*. *Recept*. *Signal Transduct*. *Res.,* 20, 189 (2000); *Methods Mol*. *Biol*., 130, 165 (2000)].

Examples of the transformant transfected with a reporter system and having good response to stimulation and lowbackground prepared as such include GBC7 which is KJMGER8 transfected with pACREpluc which is a reporter plasmid for expression of a firefly luciferase under the control of CRE and GBCR2 which is KJMGER8 transfected with pACRERluc which is a reporter plasmid for expression of *Renilla reniformis* luciferase under the control of CRE, etc.

### (4) Construction of host into which Gα protein or a chimeric Gα protein is incorporated

Since signal is different depending upon G protein coupled to GPCR bound to an active peptide, it is usually necessary to change an assay method when measurement of activity of various peptides is carried out. Thus, for each coupling G protein, it is necessary to construct suitable a reporter plasmid and a host cell into which the reporter plasmid is introduced. In that case, expression level of each Gα protein is different depending upon the host cell used and, therefore, there are some cases where sensitivity and signal/noise ratio of the reporter system increase when Gα protein is expressed together. Examples of Gα protein to be expressed include Gαₛ , Gα_{q}, Gα₁, Gα₁₁, Gα₁₂, Gα₁₃, Gα₁₄, Gα₁₅, Gα₁₆, Gαₒ, Gα_{z}, Gαₜ or Gα_{gust}. Incidentally it has been known that Gα₁₅ or Gα₁₆ flow a signal which is same as that for Gαₒ coupled to many GPCRs. Therefore, when Gα₁₅ or Gα₁₆ is expressed in a host cell, it is now possible to detect many GPCR signals in a reporter system using TRE and NFAT responsive element. In the case of GPCR coupled to Gα_{q}, Gα₁₁, Gα₁₅ or Gα₁₆, although it is possible to detect the signal from GPCR using an increase in an intracellular Ca²⁺ as an index, there are some cases where an increase in an intracellular Ca²⁺ by stimulation with a ligand easily in the case of a host cell where Gα_{q}, Gα₁₁, Gα₁₅ or Gα₁₆ is expressed.

On the other hand, when chimeric Gαₛ where five amino acids at C terminal of Gαₛ are substituted with the corresponding amino acids of other Gα (Gα_{q}, Gαᵢ, Gα₁₁, Gα₁₂, Gα₁₃, Gα₁₄, Gα₁₅, Gα₁₆, Gαₒ, Gα_{z}, Gαₜ or Gα_{gust}) is expressed in a host cell, GPCR inherently coupled to Gα other than Gαₛ couples to the chimeric Gas and, like the signal mediated by Gαs, it is possible to detect the signal from the GPCR by a reporter system using CRE.

Further, when chimeric Gα_{q} where five amino acids at C terminal of Gα_{q} are substituted with the corresponding amino acids of other Gα (Gαₛ, Gαᵢ, Gα₁₁, Gα₁₂, Gα₁₄, Gα₁₅, Gα₁₆, Gαₒ, Gα_{z}, Gαₜ or Gα_{gust}) is expressed in a host cell, GPCR inherently coupled to Gα other than Gα_{q} couples to the chimeric Gα_{q} and, like the signal mediated by Gα_{q}, it is possible to detect the signal from the GPCR by a reporter system using NFAT responsive element or TRE. In that case, it is also possible to detect the signal from GPCR using an increase in an intracellular Ca²⁺ as an index.

Accordingly, for example, when both a chimeric Gα protein where five amino acids at C terminal of Gαₛ are substituted with the corresponding amino acids of Gα_{q} and a chimeric Gα protein where five amino acids at C terminal of Gαₛ are substituted with the corresponding amino acids of Gαᵢ are expressed in a host cell which expresses reporter gene under the control of CRE prepared in 6. (3), it is possible to prepare a multipurpose host cell useful for construction of assay cell where signal of not only GPCR coupled to Gαₛ but also GPCR coupled to Gαᵢ and GPCR coupled to Gα_{q} are detectable.

Expression unit of a chimeric Gα protein gene is able to be incorporated, by the methods mentioned in the following (a) and (b), into a transformant (such as GBC7 or GBCR2) which is to be a host for the expression of GPCR constructed by the above-mentioned methods 6.(1) to (3).

### (a) Construction of expression plasmid of a chimeric Gα protein

DNA encoding a chimeric Gα protein is able to be constructed according to a general method [*Science*, 249, 662 (1990); *Nature*, 363, 274 (1993); *Mol*. *Pharmacol*., 50, 885 (1996); *FEBS lett.,* 406, 165 (1997); *Mol*. *Pharmacol*., 57, 13 (2000)].

When the DNA encoding the chimeric Gα protein is inserted into the downstream area of promoter of the expression vector mentioned in the above 3. (1), expression plasmid of the chimeric Gα protein can be constructed. It is preferred that the drug-resistant gene for animal cells used for the chimeric Gα protein expression plasmid is a drug-resistant gene for animal cells which is different from the drug-resistant gene for animal cells used for expression vector mentioned in 6. (1), inducible expression vector mentioned in 6.(2), expression plasmid of transcription factor mentioned in 6.(2) and reporter plasmid mentioned in 6.(3).

For example, when a DNA encoding a chimeric Gα protein (Gα_{s-q}) where five amino acids at C terminal of Gαₛ₄ which is a subtype of Gαₛ are substituted with five amino acids at C terminal of Gα_{q} is incorporated in expression plasmid pAMoh, it is possible to construct a Gα_{s-q} expression plasmid pAMoh-Gs-q. Further, when a DNA encoding a chimeric Gα protein (Gαₛ₋ᵢ) where five amino acids at C terminal of Gαₛ₄ are substituted with five amino acids at C terminal of Gαᵢ is incorporated in expression plasmid pAMoh, it is possible to construct a Gαₛ₋ᵢ expression plasmid pAMoh-Gs-i. Furthermore, it is possible to construct a plasmid pAMopGs-qMoGs-i for co-expression of Gα_{s-q} and Gαₛ₋ᵢ using pAMoh-Gs-q and pAMoh-Gs-i.

It is also possible to use virus which expresses the chimeric Gα protein instead of plasmid.

### (b) Incorporation of chimeric Gα protein gene expression unit into host cell

It is possible to obtain a stable transformant when any chimeric Gα protein expression plasmid constructed by the method mentioned in the above (a) is transfected according to the method mentioned in 3.(1) into a host cell constructed by the method mentioned in the above 6.(1) to (3). When oriP of Epstein-Barr virus is contained in the chimeric Gα protein expression plasmid, it is possible to incorporate the chimeric Gα protein expression plasmid into chromosome of a host cell when oriP is removed or function of oriP is destructed by means of cleavage with a restriction enzyme followed by introducing into a host cell.

In the case of a stable transformant obtained by introduction of chimeric Gα protein expression plasmid into a host cell into which the reporter system constructed by the method mentioned in 6.(3) is incorporated, it is possible to select a trans formant having good property (having good response to stimulation and having little background) using expression level of reporter gene which is and is not stimulated with an agonist or a ligand for the GPCR as an index by the same manner as in the method mentioned in the above 6.(3)(b).

In the case of a stable transformant obtained by transfecting a chimeric Gα protein expression plasmid into a host cell into which reporter plasmid constructed by the method mentioned in 6.(1) or (2) is not , it is possible to select a transformant having good property (having good response to stimulation and having little background) by the same manner as above by means of co-transfecting a reporter plasmid having a responsive element corresponding to chimeric Gα protein and a GPCR expression plasmid into each transformant.

Examples of the host cell constructed as above include GBCC13 selected from the transformants where pAMopGs-qMoGs-i is introduced into GBC7 and GBCRC6 selected from the transformants where pAMopGs-qMoGs-i is transfected to GBCR2, etc. When GBCC13 is used, it is now possible to detect the signal from GPCR coupled to Gαₛ , Gα_{q} and Gαᵢ using activity of firefly luciferase as an index. When GBCRC6 is used, it is now possible to detect the signal from GPCR coupled to Gαₛ , Gα_{q} and Gαᵢ using activity of *Renilla reniformis* luciferase as an index.

### (5) Construction of assay system

### (a) Assay system using reporter

Expression plasmid of receptor of active peptide is prepared by the method mentioned in 6.(1)(b) or (2)(a), the plasmid is introduced into a host cell constructed in 6.(3) or (4) and the resulting stable transformant is able to be used as an assay cell.

It is also possible that a stable transformant obtained by co-transfecting an expression plasmid of the receptor for the above active peptide and the reporter plasmid which is able to be constructed by the method mentioned in the above 3(a) into the host cell constructed in 6.(1) or (2) is used as an assay cell.

It is furtherpossible that a stable transformant obtained by a co-transfecting an expression plasmid of receptor of the above active peptide, the above reporter plasmid and the expression plasmid of a chimeric Gα protein which is able to be constructed by the method mentioned in the above 6.(4) (a) into the host cell constructed in 6.(1) or (2) is used as an assaycell. It is furthermore possible to use a reporter plasmid into which expression unit of a chimeric Gα protein is incorporated instead of expression plasmid of the above chimeric Gα protein and the above reporter plasmid.

It is still further possible that a stable transformant obtained by co-transfecting an expression plasmid of receptor for the above active peptide and an expression plasmid of the above chimeric Gα protein into the host cell constructed in 6.(3) is used as an assay cell.

It is preferred that the host cell constructed in 6. (2) to (4) is cultured in a medium to which an appropriate amount of drug for the selection corresponding to the drug-resistant gene owned by the vector which is incorporated into each cell is added. Cultivation can be carried out using a method mentioned in 6. (1) (a). For example, it is preferred to culture in a medium to which blasticidin S (2.0 µg/ml) is added in the case of KJMGER8, a medium to which blasticidin S (2.0 µg/ml) and hygromycin B (300 µg/ml) are added in the case of GBC7 and GBCR2 and a medium to which blasticidin S (2.0 µg/ml), hygromycin B (300 µg/ml) and puromycin (2.0 µg/ml) are added in the case of GBCC13 and GBCRC6.

A stable transformant is able to be prepared by culturing in a medium to which an appropriate amount of drug for selection corresponding to the drug-resistant gene contained in the introduced plasmid is added. For example, when expression vector using pAGal9-nd having G418-resistant gene is transfected into a host cell GBCRC6, a medium containing 0.5 mg/ml of geneticin is used. It is preferred that a drug which is preferably added to the medium for culturing the above-mentioned host is added to the medium at the same time.

When inducible expression of receptor of active peptide is carried out, a host cell expressing a transcription factor necessary for the inducible expression is used as mentioned in (2) and a vector for inducible expression having a responsive element corresponding to the transcription factor is used. For example, when a host cell where Gal4-ER is expressed for the inducible expression is used as in the case of GBCC13 or GBCRC6, inducible expression vector containing Gal4p-responding element such as pAGal9-nd or pAGal9-d is used for the construction of an expression plasmid of a receptor for an active peptide.

In the case of expression by constitutive promoter as in the case of expression plasmid mentioned in 6. (1) (b), it is possible to express the receptor of active peptide on the cell surface of the assay cell merely by culturing assay cell. In the case of inducible expression, a drug which is necessary for the inducible expression mentioned in 6.(2) is added and the assay cell which is constructed above is cultured whereby the receptor of active peptide is expressed on the cell surface of the assay cell. For example, in the case of an assay cell obtained by transfecting an inducible expression plasmid of a receptor of an active peptide containing Gal4p responsive element into a host cell wherein Gal4-ER is expressed, 0.1 to 1000 nmol/L or, preferably, 0.1 to 10 nnmol/L of 17-β estradiol is added and cultivation is carried out for not shorter than 3 hours or, preferably, 6 to 48 hours whereupon receptor of the active peptide is able to be expressed on the cell surface of the assay cell.

After a sample where the activity is to be measured is added to the assay cell in which receptor of active peptide is expressed on the cell surface and cultivation is carried out for 2 to 24 hours or, preferably 6 hours, expression level of reporter gene, expression level of reporter polypeptide or activity of reporter polypeptide is measured according to known methods [*Mol. Biotechnol.,* 13, 29 (1999); *Anal. Chemi.,* 70, 579A (1998); *J.Recept.Signal Transduct.Res.,* 19, 395 (1999) ; *J. Recept. Signal Transduct.Res.,* 20, 189 (2000); *Methods Mol. Biol.,* 130 , 165 (2000)]. As a control, the same assay is carried out using active peptide samples of various concentrations, a sample containing no such an active peptide, or a supernatant liquid of cultured solution of cell line into which no active peptide precursor gene is introduced, etc. When comparison is conducted with the results of the controls, it is possible to detect and measure the active peptide in the sample.

When an assay cell is constructed using a GPCR where it is not apparent whether a peptide is a ligand or an orphan GPCR instead of the known GPCR, a ligand peptide for the GPCR is able to be identified. Further, when such an assay cell is used and activity is measured in the case of expression cloning of the active peptide precursor gene mentioned in the above 4., it is possible to screen a novel active peptide precursor gene.

### (b) Assay system using no reporter

An expression plasmid for receptor of active peptide is prepared by the method mentioned in 6. (1) (b) or (2)(a), the plasmid is transfected to the host cell constructed in 6. (1) to (4) and the resulting stable transformant is able to be used as an assay cell. The assay cell can be prepared and cultured and a peptide receptor can be expressed on the cell surface by the same manner as in the above (a). It is also possible that the immortalized cell prepared in the above 1. is used as an assay cell.

When signal in the assay cell obtained by binding of active peptide to the receptor is measured, the peptide in the sample is able to be detected. Signal in the cell is dependent on the receptor and examples of the signal include release of arachidonic acid, release of acetylcholine, increase in intracellular Ca²⁺, production of intracellular cAMP, decrease in intracellular cAMP, production of intracellular cGMP, production of inositol phosphate, change in the cell membrane potential, phosphorylation of intracellular protein (such as CREB, STAT1, STAT2, STAT3, STAT4, STAT5a, STAT5b, STAT6, MAP kinase, ATF-2, c-Jun, c-fos, Iκ-Bα, Iκ-Bβ, Smad1, Smad2, Smad3, Smad5 and Smad8), activation of c-fos, change of intracellular pH and cell growth which can be measured according to the known methods mentioned in literatures [*J*. *Biol. Chem*., 271, 1857 (1996); *Science*, 268, 98 (1995); *J*. *Pharmacol*. *Exp*. *Ther*., 275, 1274 (1995); *J*. *Biol*. *Chem*., 272, 1822 (1997); *J. Recept*. *Signal Transduct*. *Res*., 17, 57 (1997); *Endocrinology*, 138, 1400 (1997); *Endocrinology*, 138, 1471 (1997); *Nat*. *Biotechnol*., 16, 1334 (1998); *Biochem*. *Biophys. Res. Commun*., 251, 471 (1998); *Brit*. *J*. *Pharmacol*., 125, 1387 (1998); *Trends Biotechnol*., 15, 487 (1997); *Anal*. *Biochem*., 252, 115 (1997); *Nature*, 358, 325 (1992); *Nature*, 393, 272 (1998); *Cell*, 92, 573 (1998); *J. Biol*. *Chem*., 272, 27497 (1997); *Trends Pharmacol*. *Sci*., 18, 430 (1997); *Trends Pharmacol*. *Sci*., 20, 370 (1999); WO 98/46995]. Further, when gene where expression level changes or protein where expression level or state changes upon binding of active peptide to receptor is measured, it is possible to detect an active peptide binding to the receptor.

When an assay cell is constructed using a GPCR where it is not apparent whether a peptide is a ligand or an orphan GPCR instead of the known GPCR, a ligand peptide for the GPCR is able to be identified. Further, when such an assay cell is used and activity is measured in the case of expression cloning of the active peptide precursor gene mentioned in the above 4., it is possible to screen a novel active peptide precursor gene.

### [II] Utilization of host-vector system

The host-vector system constructed in the above [I] 6. is able to be utilized not only for the construction of assay system of receptor of active peptide but also for the followings.

### 1. Construction of an assay system for a GPCR and screening of a ligand, an agonist or an antagonist for a GPCR using the assay system

### (a) An assay system using a reporter

An expression plasmid of any GPCR is constructed in the same manner as the method mentioned in [I] 6.(1)(b) or [I] 6.(2)(a), an assay cell is prepared in the same manner as in [I] 6.(5)(a) and the GPCR is expressed on a cell surface. The cell is incubated after addition of any substance such as peptide or compound as a substance to be tested, expression level of reporter gene is measured, comparison is conducted with the control (in case where incubation is conducted only by addition of a solvent used for dissolving the substance) and a substance where expression level of reporter gene increases is selected whereupon a ligand or an agonist for the GPCR is able to be isolated.

With regard to the substance to be tested, an experiment of addition of the same substance to be tested is conducted using an assay cell to which control vector into which no GPCR gene is inserted is transfected and it is able to be confirmed that, in case expression level of reporter gene is not increased, the substance does not act on signal transduction of the GPCR or on the transcription of the reporter gene but is a ligand or an agonist capable of acting on GPCR. When the same experiment is carried out using an assay cell in which other GPCR is expressed concerning the ligand or agonist, a GPCR specificity of the ligand or the agonist is able to be evaluated.

After incubation is carried out by addition of a ligand or an agonist of a GPCR and any substance as a substance to be tested to an assay cell inwhich a GPCR is expressed, expression level of the reporter gene is measured and a substance where expression level of the reporter gene decreases as compared with the control (the case where only a ligand or an agonist is added) is selected whereupon it is possible to isolate an antagonist for the GPCR.

With regard to the substance, the same experiment is carried out using an assay cell where other GPCR is expressed and an agonist to the GPCR and it is able to be confirmed that, in case the expression level of reporter gene is not decreased, the substance does not act on the signal transduction and on the transcription of reporter gene but is an antagonist merely acting on a specific GPCR.

### (b) An assay system using no reporter

It is possible to screen a ligand, an agonist or an antagonist for a GPCR by measuring a signal using a method for the measurement of the signal in the assay cell mentioned in [I] 6. (5) (b) instead of measuring the expression of the reporter gene in the above (a),.

### 2. Evaluation of constitutive activity of a GPCR

When a GPCR gene is highly expressed transiently in an assay system of GPCR using an inducible expression system, constitutive activity of the GPCR is able to be measured.

A stable transformant which is obtained by transfecting the inducible expression plasmid of a GPCR prepared according to the method mentioned in [I] 6.(2)(a) to a host cell which expresses a transcription factor necessary for inducible expression of GPCR and constructed by the method mentioned in [1] 6.(3) or [I] 6.(4) wherein reporter system is incorporated is used as a cell for the evaluation.

The GPCR of the transformant is subjected to an inducible expression by the same manner as in [I] 6. (5) and expression level of reporter gene is compared with the case where no GPCR is subjected to an inducible expression. When expression level of reporter gene increases in case GPCR is subjected to an inducible expression, it can be judged that the GPCR has a constitutive activity. As such, it is now possible to select the GPCR showing a constitutive activity (a constitutively activated GPCR).

### 3. Screening of inverse agonist or agonist using a constitutively activated GPCR

By the same manner as in 2., an assay cell which is able to inducibly express any constitutively activated GPCR is constructed. After any substance such as peptide or compound is added as a substance to be tested to the cell, the GPCR is subjected to an inducible expression, expression level of reporter gene is measured and a substance where expression level of reporter gene decreases as compared with the control (where only a solvent in which the substance is dissolved added) is selected whereupon it is possible to isolate an inverse agonist of the constitutively activated GPCR.

When a substance where expression level of reporter gene increases as compared with the control is selected, an agonist of the constitutively activated GPCR is able to be isolated.

With regard to the substance, when an experiment of addition of the same substance to be tested is carried out using an assay cell to which a control vector is transfected where no GPCR gene is inserted thereinto and when the expression level of reporter gene does not change, it is able to be confirmed that the substance to be tested does not act on the signal transduction of GPCR and on the transcription of reporter gene but is inverse agonist or agonist acting on GPCR. When the same experiment is carried out using an assay cell in which another constitutively activated GPCR is expressed concerning the inverse agonist or the agonist, it is nowpossible to evaluate the specificity of the inverse agonist or the agonist to GPCR.

In addition, when incubation is carried out by addition of an agonist for the constitutively activated GPCR and any substance in the same manner as in [II] 1. , expression level of reporter gene is measured and a substance where expression level of reporter gene decreases as compared with the control (in case only agonist is added) is selected and further when only the substance is added without addition of the agonist, it is now possible that a substance where expression level of reporter gene does not decrease as compared with the case where neither agonist nor the substance is added is selected as antagonist (neutral antagonist) for the GPCR. When the same experiment is carried out using an assay cell to which another constitutively activated GPCR is transfected concerning the antagonist, it is possible to evaluate the specificity of the antagonist to GPCR.

### 4. Expression cloning

cDNA library is prepared by insertion of cDNA isolated from cells or tissues into an expression vector mentioned in [I] 6.(1)(b) or [I] 6.(2)(a).

With regard to a source for cDNA, any tissue or cell of human being or animals can be used. For example, various kinds of tissues, cells or cell lines derived from human being and animals can be used.

mRNA is isolated from those cells, double-stranded cDNA is synthesized from the mRNA, the cDNA is inserted into a downstream side of promoter of vector for the expression mentioned in [I] 6.(1)(b) or [I] 6.(2)(a) to prepare a recombinant vector and the resulting recombinant vector is transfected to *E*. *coli* to prepare a cDNA library. Preparation of the cDNA library as mentioned above can be carried out according to publicly known methods such as those mentioned in Molecular Cloning, 3rd ed. and Japanese Published Unexamined Patent Application No. 336,963/1993. The resulting cDNA library (*E*. *coli* to which the recombinant vector is transfected) is diluted to an appropriate concentration and cultured on an agar medium and each of the resulting colonies is able to be isolated as clone. It is also possible that plasmid (recombinant vector) is isolated as each cDNA clone from each clone (*E*. *coli*). The plasmid is able to be isolated by a common method mentioned, for example, in Molecular Cloning, 3rd ed. or using a kit such as QIAprep 96 Turbo Miniprep Kit (manufactured by Qiagen).

The cDNA library is divided into pools each comprising 1 to 10,000 clone(s) (*E*. *coli*) or, preferably, 10 to 100 clones and *E*. *coli* is cultured for each pool to isolate a plasmid (cDNA clone mixture). The resulting recombinant vector derived from each pool in transfected to a host cell into which the reporter system prepared in [I] 6. (3) (b) or [I] 6. (4) (b) is incorporated by the method mentioned in [I] 3. (1) to give a stable transformant by the method mentioned in [I] 6.(5).

The resulting stable transformant is cultured by the method mentioned in [I] 6.(5) to express each cDNA clone. Expression level of the reporter gene of the transformant is measured and, at the same time, expression level of the reporter gene of the case where no cDNA is expressed (in the case of an inducible expression, the casewhere expression is not induced while, in the case of constitutive expression, a host cell into which empty vector is transfected) is also measured. Both are compared and a pool where expression level of reporter gene when cDNA is expressed is selected. The selected pool is divided into smaller pools again and the same operation is carried out. Such a step is repeated and, finally, the above operation is carried out for each 1 clone whereupon it is possible to isolate cDNA encoding a molecule (such as a constitutively activated GPCR, a transcription factor, a signal transduction molecule or an enzyme, etc.) having an activity of increasing the transcription from responsive element controlling the expression of reporter gene in the host cell.

Further, after cDNA for each pool is expressed in the transformant by the same manner as above, any substance is added to the transformant followed by culturing the transformants for 2 to 12 hours (preferably, for 6 hours), then expression level of reporter gene is measured and, at the same time, expression level of reporter gene is measured in case the transformant is cultured without addition any substance as well. Both are compared and a pool where expression level of reporter gene when any substance is added is selected. The selected pool is divided again into smaller pools and the same operation is carried out. The step is repeated and, finally, the above operation is carried out for each 1 clone whereupon it is possible to isolate a cDNA encoding a peptide (such as GPCR where any substance is ligand or agonist, transcription factor which activates or inactivates any substancewhen it is present, signal transduction molecule which activates or inactivates any substance when it is present or enzyme which activates or inactivates any substance when it is present) where transcription from responsive element controlling the expression of reporter gene is increased by reacting with any substance.

It is also possible to conduct an expression cloning without dividing into pools as follows.

From the total cDNA library prepared by the above method, plasmid is prepared by a common method or using a kit such as Plasmid Maxi Kit (manufactured by Qiagen). The plasmid is introduced into a host cell which is able to express cell surface molecule or a molecule detectable in a state of living cell such as GFP as a reporter whereupon a stable transformant is prepared. After cDNA is expressed by the same manner as above, cell wherein expression of the detectable molecule as such is high is isolated. Alternatively, after any substance is added to the cell where cDNA is expressed followed by incubating, cell where expression of the molecule is high is isolated. When the molecule is a cell surfacemolecule, the cell is stained with an antibody which recognizes the molecule and a cell where a binding ability to antibody is high is collected using a fluorescence activated cell sorter (hereinafter, abbreviated as FACS). When the molecule is GFP, a cell where expression level of GFP is high is collected using an FACS. After the collected cells are incubated, the same operation is carried out whereupon it is possible to concentrate the cells in which expression of the molecule increases upon expression of cDNA or the cells in which expression of the molecule increases upon addition of any substance. From the cells, introduced plasmid is able to be recovered by a known method such as a Hart method [*Mol*. *Cell*. *Biol*., 8, 2837 (1988)]. The plasmid is introduced into a host cell again and the same operation is carried out whereupon it is able to be confirmed that whether expression of the molecule increases when cDNA in the plasmid is expressed or whether expression of the molecule increases when any substance is added to the cell in which the cDNA is expressed.

With regard to a nucleotide sequence of the cDNA isolated by the above method, the DNA fragment *per se* or that after being cleaved by an appropriate restriction enzyme or the like is incorporated into vector by a common method mentioned, for example, in Molecular Cloning, 3rd ed. whereupon the nucleotide sequence is able to be decided by a commonly used nucleotide sequence analyzing method such as a dideoxy method by Sanger, et al. [*Proc. Natl. Acad. Sci. USA*, 74, 5463 (1977)] or using a commercially available kit (Dye Terminator Cycle Sequencing FS Ready Reaction Kit, dRhodamine Terminator Cycle Sequencing FS Ready Reaction Kit or BigDye Terminator Cycle Sequencing FS Ready Kit; manufactured by Applied Biosystems) and a DNA sequencer such as ABI PRISM 377 (manufactured by Applied Biosystems). From the nucleotide sequence, it is possible to decide an amino acid sequence of peptide encoded by the resulting cDNA.

Expression plasmid of peptide prepared as such is prepared according to a method mentioned in [II] 1. or [II] 3. to construct an assay cell. When the peptide has an action of activating the transcription either constitutively or by being activated, any substance is added followed by incubating and expression level of reporter gene is measured whereupon it is possible to select and isolate an inhibitor or an activator for the peptide.

### 5. Construction of a constitutively activated mutant GPCR

Into a DNA encoding GPCR which is not a constitutively activated type is introduced a site-specific mutation or a random mutation according to a method mentioned in literatures [Molecular Cloning, ed. 3; Current Protocols in Molecular Biology, John Wiley & Sons, (1987-2001) (hereinafter, referred to as Current Protocols in Molecular Biology); *Nucleic Acids Res*., 10, 6487 (1982); *Proc*. *Natl*. *Acad*. *Sci*. *USA*, 79, 6409 (1982); *Gene*, 34, 315 (1985); *Nucleic Acids Res*., 13, 4431 (1985); *Proc*. *Natl*. *Acad*. *Sci. USA*, 82, 48 (1985); *Methods Mol. Biol*., 57, (1996); *BioTechniques,* 25, 958 (1998); BioTechniques, 25, 958 (1998); BioTechniques, 24, 428 (1998); *Proc. Natl. Acad. Sci. USA,* 93, 9670 (1996)].

DNA encoding a mutated GPCR is inserted into a downstream side of promoter of vector for inducible expression mentioned in [I] 6. (2) (a) to prepare a library of many mutated DNAs. The mutated DNA library is divided into pools by the same method as mentioned in [II] 4. and DNA in each pool is introduced into a host cell into which expression unit of transcription factor necessary for inducible expression and reporter system mentioned in [I] 6.(3) or [I] 6.(4) and a stable transformant is prepared. Expressions of reporter gene when expression of DNA is and is not induced are compared in accordance with the method mentioned in [II]2 and a pool where expression level of reporter gene increases when the expression is induced is selected. The selected pool is more finely divided into pools and the same operation is carried out. The step is repeated and, finally, the above operation is carried out for each 1 clone whereupon it is possible to isolate a DNA encoding a constitutively activated mutant GPCR. It is also possible to conduct a selection using an FACS by the method mentioned in [II]4.

Depending upon the site of mutation and upon the type of amino acid to be mutated, strength of constitutive activity of GPCR encoded by the mutated DNA and reactivity to a ligand, an agonist or an antagonist change. To screen an inverse agonist and agonist, it is preferred that specificity to a ligand, an agonist and an antagonist is not different from that of a wild type GPCR and a mutant GPCR as such is able to be isolated by a randommutagenesis into a site where there is a high possibility of not changing the specificity to the ligand, agonist and antagonist as compared with the wild type GPCR [for example, from the second half of the third transmembrane region to the first half of the second transmembrane region, each of amino acids corresponding to (Asp or Glu)-Arg Tyr sequence (hereinafter, referred to as D/ERY motive) existing in the above region and preserved by many GPCRs and from the second half of the third intracellular domain to the first half of the sixth transmembrane region] on the basis of the findings up to now [*J*. *Biol*. *Chem*., 271, 1857 (1996); *Science*, 268, 98 (1995); *J*. *Pharmacol*. *Exp*. *Ther*., 275, 1274 (1995); *J*. *Biol*. *Chem*., 272, 1822 (1997); *J. Recept*. *Signal Transduct*. *Res*., 17, 57 (1997); *Mol*. *Neurobiol*., 17, 109 (1998); *J*. *Biol*. *Chem*., 274, 18574 (1999); *Proc*. *Natl*. *Acad*. *Sci*. *USA*, 97, 7615 (2000); *Mol*. *Pharmacol*., 57, 890 (2000); WO 98/46995]. It is also possible to isolate by a random mutagenesis into the 20th or the 22nd amino acid residue in N-terminal counting (proline residue as the 0th one) from proline residue preserved by many G-protein coupled receptor existing in the sixth transmembrane region (in the case of G-protein coupled receptor where no proline residue is preserved, from an amino acid residue corresponding to the position of the proline residue). It is also possible that mutation is randomly introduced throughout the whole regions of a DNA encoding GPCR, then a constitutively activated GPCR is selected therefrom and evaluation is conducted whether the selected one is an aimed mutant whereupon it is possible to select a mutant having strong constitutive activity being suitable for exploring an inverse agonist in high sensitivity and in high signal/noise ratio.

When site and type (substitution, deletion or addition of amino acid(s)) of mutation introduced in a constitutively activated mutant GPCR are determined, it is possible to obtain information concerning the correlation between structure and activity of a GPCR. A constitutively activated mutant GPCR is often a cause for diseases and, therefore, it is possible to check the relation to the disease on the basis of the information.

With regard to molecule (such as transcription factor, signal transduction molecule or enzyme) having an activity of increasing the transcription from the used transcription factor-responsive element (such as CRE and NKκ responsive element) either directly or indirectly, it is also possible to isolate a constitutively activated mutant by introducing random mutations by the same manner as above.

It is further possible to isolate a dominant negative mutant and a mutant which no longer shows a constitutive activity by introducing random mutations to the above molecule.

### [III] Utilization of immortalized cell line derived from hypothalamus and pancreatic Langerhans islets

### 1. Method for screening or evaluation of drug, physiologically active substance and receptor using the cell line of the present invention

In accordance with the method of the present invention, it is now possible to obtain many kinds of cell lines keeping the character of endocrine cells such as hypothalamic cells and Langerhans islets cells, etc. and, therefore, when such cell lines are used, it is now for the first time possible to efficiently screen or evaluate the substances (peptide, compound, drug, etc.) acting on various endocrine cells such as hypothalamic cells and Langerhans islets cells, etc.

In addition, when the cell line of the present invention is used, specific endocrine cells can be increased in large quantities and, therefore, it is now possible to isolate physiologically active substances and receptors expressing in specific endocrine cells.

Hereinafter, specific examples using cell lines derived from hypothalamus or Langerhans islets will be given in (a) and (b).

(a) When any substance (peptide, compound, drug, etc.) is contacted with a mixture of many kinds of cell lines derived from hypothalamus or each cell line derived from hypothalamus or a mixture of many kinds of cell lines derived from Langerhans islets or each cell line derived from Langerhans islets prepared in the above [I] 1. (1) and reactivity of the cell line(s) to any substance is checked, it is now possible to screen or evaluate the substance acting on the cell line(s).

With regard to a method for culturing the cell, a method mentioned in the above [I] 1.(1) or [I] 1.(4) can be used.

Measurement of reactivity of the cell with any substance is able to be carried out according to a method, for example, where reaction of assay cell is measured mentioned in the above [I] 6.(5)(b).

With regard to a drug to be contacted with the cell, any drug can be used and examples of the drug include a drug showing a pharmaceutical effect by acting on hypothalamus (such as anti-obesity drug, remedy for cachexia, anti-allergic drug, anti-immune drug, anti-inflammatory drug, remedy for tumor, remedy for virus and psychoneurotic drug), drug showing pharmaceutical effect acting on Langerhans islets (such as antidiabetic drug, remedy for cachexia, anti-immune drug, anti-inflammatory drug, remedy for tumor, remedy for virus and anti-obesity drug), drug where adverse action in hypothalamus or Langerhans islets is a problem and drug where molecule (such as various receptor) expressed in hypothalamic cell or Langerhans islets cell is a target.

With regard to a peptide to be contacted to cell, any peptide can be used and, for example, peptide where receptor thereof is unknown and secretory protein, membrane protein and peptide where function thereof is unknown can be used. Instead of such a peptide, it is also possible to use a cell which expresses the peptide.

With regard to a compound to be contacted to the cell, any compound can be used and, for example, substances which have been known to be present in organism of animals can be used.

When a substance acting on the cell line is found, it is possible to isolate a molecule (receptor, enzyme, transcription factor, etc.) interacting on the substance from the cell line reacting with the substance. With regard to a method for isolating the molecule interacting on the substance, for example, a purifying method where affinity to the substance is utilized, or an expression cloning method can be used.

(b) It is possible to screen physiologically active substances using culture medium or cell after culturing the cell line of the present invention under various conditions or extract of the cell. With regard to a method for culturing, the method mentioned in the above [I] 1.(1) or [I] 1.(4) can be used.

For example, each of the above-mentioned culture solution or cell of the above cell line and extract of the cell is contacted to cell in which any protein (such as receptor, enzyme, transcription factor, etc.) is expressed and cell in which such a protein is not expressed and it is checked whether cell in which any protein (receptor, enzyme, transcription factor, etc.) is expressed reacts strongly whereupon it is now possible to screen a physiologically active substance acting on any protein.

Measurement of reactivity of the cell can, for example, be carried out according to the method for measuring the reaction of assay cell mentioned in the above [I] 6.(5)(b).

When activity is detected in culture solution, cell or cell extract, a physiologically active substance is isolated by a common method [Spectrometric Identification of Organic Compounds, 6th Edition, John Wiley & Sons (1997)] and its structure (total or partial structure) can be determined. When a physiologically active substance is a peptide, the corresponding gene can be isolated on the basis of the structure (total or partial structure) determined using a common method [Structure Analysis of Protein for Gene Cloning, Tokyo Kagaku Dojin, (1993)]. When a physiologically active substance is a peptide, a gene encoding the active substance is isolated by means of expression cloning using a gene library or a cDNA library prepared using the cell showing the activity as a source whereby structure of the active substance can be clarified.

### 2. Isolation of useful gene and useful peptide expressed in hypothalamic cell or Langerhans islets cell

Many kinds of cells are present in hypothalamus and Langerhans islets and each cell is believed to play a specific function important for the maintenance of homeostasis of living body. Such a function is achieved by way of action of various receptors and various physiologically active substances expressed by hypothalamic cells or Langerhans islets cells. It is believed that gene and peptide specifically expressed in specific hypothalamic cells or specific Langerhans islets cells participate in the cell-specific function. Particularly, secretory peptide specifically expressed in specific hypothalamic cells or specific Langerhans islets cells has a high possibility of functioning as a physiologically active substance. It is also believed that gene where expression varies upon stimulation of hypothalamic cells or Langerhans islets cells has a high possibility of encoding receptor and physiologically active substance which are important for controlling the function of hypothalamic cells or Langerhans islets cells.

Therefore, when gene or peptide specifically expressed in specific hypothalamic cells or specific Langerhans islets cells is screened, it is believed that peptide (various kinds of receptors, various kinds of physiologically active substance, various kinds of transcription factors, etc.) important for expression of function of the cells or gene coding therefor is able to be isolated. When a cell line keeping the character of hypothalamic cells or Langerhans islets cells obtained by the present invention is used, it is possible to increase one kind of hypothalamic cell or Langerhans islets cells in large quantities and, therefore, it is now possible to isolate a gene and a peptide which are specifically expressed in specific hypothalamic cells or specific Langerhans islets cells. In the present invention, it has been succeeded in preparing many kinds of cell lines keeping the character of hypothalamic cells or Langerhans islets cells and, therefore, it is now possible to screen and isolate a gene and a peptide specifically expressed in various hypothalamic cells or Langerhans islets cells, a gene and a peptide where expression changes upon stimulation of specific hypothalamic cells or specific Langerhans islets cells or secretory protein, etc. expressed in specific hypothalamic cells or Langerhans islets cells. Hereinafter, specific examples will be shown in (a), (b), (c) and (d). It is also possible to isolate the corresponding gene of human being and other mammals such as mouse utilizing the homeostasis of the rat hypothalamic cells or rat Langerhans islets cells by the methods of (a) to (d).

### (a) Isolation of a gene encoding a peptide having a secretory signal sequence using a signal sequence trap method

A peptide ligand and a receptor existing on cell surface usually have a signal sequence and, therefore, when a signal sequence trap method [*Science*, 261, 600 (1993); *Nat*. *Biotechnol*., 17, 487 (1999)] is carried out using a cell line of the present invention as a source, it is possible to efficiently isolate a ligand and a receptor expressed in the cell line.

It has been known that, in neuropeptide and hormone or receptors thereof, expression level of gene thereof changes by various stimulations. Therefore, when a signal sequence trap method is carried out using the cell line of the present invention being subjected to various stimulations as a source, it is possible to more efficiently isolate a ligand and a receptor expressed in the cell line.

With regard to a method for culturing or stimulating the cell line, the methods mentioned in the above [I]1.(1) and [I] 1.(4) can be used.

cDNA used in a signal sequence trap method is a partial fragment and, therefore, a full-length cDNA is isolated by a hybridization using a probe specific to the sequence of the cDNA. When a nucleotide sequence of the full-length cDNA is determined, a peptide encoded by the cDNA can be identified.

When the amino acid sequence of the peptide is subjected to homology search, motive search or hydrophobicity search, it is possible to estimate the function of the peptide. For example, as a result of the above analysis, it can be estimated whether the peptide functions as a receptor, ligand, transporter, enzyme, etc. When it is checked whether it has a secretory signal sequence or transmembrane domain, it is also possible to confirm whether the peptide is a secretory protein or a membrane protein.

When DNA encoding the peptide is expressed in an appropriate cell, it is possible to produce the peptide and to experimentally check the activity of the peptide. With regard to the method for the expression, a method similar to that mentioned in the following 3. can be exemplified.

### (b) Isolation of a gene specifically expressed in specific cell line

With regard to a method for the isolation, a subtraction method [*Proc. Natl. Acad. Sci. USA,* 85, 5738 (1988)] where a gene in which expression is different in two different samples is isolated and a method by means of a representational difference analysis [*Nucleic Acids Res.,* 22, 5640 (1994)] can be exemplified. It is also possible that, using a DNA chip method [*Tampakushitsu Kakusan Koso,* 45, 1841 (2000); *Nippon Rinsho,* 57, 465 (1999)], etc., gene expressed in each cell line is analyzed and compared with gene expressed in other cell lines whereby gene which is specifically expressed in each cell line is identified.

Hereinafter, an example using a subtraction method will be given.

A cDNA library prepared from any cell line of the present invention is subjected to subtraction using mRNA isolated from a cell other than the cell line (for example, a cell line derived from the same tissue (hypothalamus or Langerhans islets) but showing different character or cell or cell line derived from other tissues). After a differential cDNA library where the cell line-specific gene is concentrated is prepared, a nucleotide sequence of inserted cDNA in the differential cDNA library is randomly decided from 5' side. Primer which is specific to the decided sequence is prepared and a PCR is carried out using the primer whereupon it is possible to check the expression level of gene having the sequence in two kinds of cells used for the differentiation. As a result, gene which is specifically expressed in the cell line is able to be identified.

When the whole nucleotide sequence of cDNA corresponding to the specifically expressed gene is determined, a peptide encoded by the cDNA is able to be identified. When cDNA in the differential library is a partial fragment, a full-length cDNA is able to be isolated by a hybridization using a probe which is specific to the sequence of the cDNA. When a nucleotide sequence of the full-length cDNA is decided, a peptide encoded by the cDNA is able to be identified.

When the amino acid sequence of the peptide is subjected to homology search, motive search or hydrophobicity search, function of the peptide can be estimated. For example, as a result of the above analysis, it can be estimated whether the peptide functions as a receptor, a ligand, a transcription factor, a transporter, an enzyme, etc. When it is checked whether it has a secretory signal sequence or transmembrane domain, it is also possible to determine whether the peptide is a secretory protein or a membrane protein.

When DNA encoding the peptide is expressed in an appropriate cell, it is possible to produce the peptide and to experimentally check the activity of the peptide.

When the above signal sequence trap method is carried out using the differentiated cDNA, a ligand and a receptor which are specifically expressed in a cell line of the present invention is able to be efficiently isolated. When a library for a signal sequence trap using a vector which is able to be subtracted is prepared, it is possible to efficiently carry out the subtraction and the signal sequence trap.

### (c) Efficient isolation of a gene encoding a ligand and a receptor

It has been known that, with regard to neuropeptide, hormone or receptor thereof, expression level of gene thereof changes by various kinds of stimulations. Therefore, with regard to any cell line of the present invention forwhich various kinds of stimulations are conducted, when genes where expression level changes in case various stimulations are and are not conducted are compared, it is now possible to more efficiently isolate a gene encoding a ligand and a receptor. As to a specific method therefor, a subtraction method mentioned in the above (b), a representational difference analysis method or a DNA chip method can be used. With regard to a method for culturing and that for stimulation, the methods mentioned in the above [I] 1.(1) and [I] 1.(4) can be used.

When the above-mentioned signal sequence trap method is carried out using a differentiated cDNA isolated by a subtraction method, it is possible to efficiently isolate a ligand and a receptor whose expression level changes when any cell line of the present invention is stimulated. When a library for a signal sequence trap is prepared using a vector where subtraction is possible, it is possible to efficiently conduct subtraction and signal sequence trap.

### (d) Identification and isolation of a peptide specifically expressed in a specific cell line or a peptide whose expression level changes upon stimulation of a specific cell line

When analysis is carried out using a commonly-known proteomics means [Proteome Research: New Frontiers in Functional Genomics, Springer (1997); Proteome and Protein Analysis, Springer (2000)], it is possible to identify a peptide which is specifically expressed in a specific cell line or a peptide whose expression level changes upon stimulation of a specific cell line. Commercially available instruments can be used as well.

For example, after any cell line of the present invention is cultured under various conditions, a peptide existing in culture medium or cell extract is analyzed. When the same analysis is carried out for a peptide existing in culture medium or extract of other cell line and results of both analyses are compared, apeptidewhich is specifically expressed in a specific cell line is able to be identified. A secretory peptide which is specifically expressed in a cell line derived from hypothalamus is expected to act as neuropeptide or hormone. Therefore, it is considered to be possible that, when a peptide specifically existing in a cultured mediumof a cell line derived from hypothalamus is identified, it is possible to isolate neuropeptide and hormone.

It has been also known that expression level and secreted amount vary in neuropeptide and hormone by various kinds of stimulations. Therefore, when any hypothalamic cell line is cultured under various conditions and a secretory peptide whose expression level changes depending upon the cultivation condition is identified, it is believed to be possible that neuropeptide and hormone can be efficiently isolated.

With regard to a method for culturing or for stimulation, the methods mentioned in the above [1] 1. (1) and [I] 1. (4) can be used.

A partial amino acid sequence of the peptide isolated above is able to be decided using a known method. It is also possible to isolate the corresponding gene on the basis of the amino acid sequence using a known method.

### 3. Method for the production of a peptide expressed in hypothalamic cells or Langerhans islets cells

When a gene encoding a peptide isolated by the method mentioned in [I] 4. or [III] 2. or gene corresponding to other mammals such as human being and mouse isolated by utilizing a homology to the gene is expressed in an appropriate host cell, a peptide encoded by the gene is able to be manufactured. With regard to a method therefor, the method mentioned, for example, in Molecular Cloning, 3rd ed., Current Protocols in Molecular Biology can be used.

Thus, when a recombinant vector where gene encoding a peptide expressed by the cell line of the present invention is inserted into a downstream of a promoter of an appropriate expression vector is constructed and then the vector is transfected to a host cell, it is possible that a transformant expressing the peptide is obtained and that the transformant is cultured to produce the peptide.

With regard to a host cell, any cell can be used so far as it is able to express the aimed gene such as prokaryotic cell, yeast, animal cell, insect cell and animal cell. It is also possible to use individual animal and individual plant.

With regard to an expression vector, that where autonomous replication is possible in the host cell or incorporation into chromosome is possible and that which contains promoter at the position suitable for the transcription of gene to be expressed (preliminarily called as gene A) can be used.

When prokaryote such as bacterium is used as a host cell, it is preferred that the expression vector of gene A is able to be autonomously replicable in prokaryote and also that it is constituted from promoter, ribosome-binding sequence, gene A and transcription termination sequence. Gene which controls a promoter can be also contained therein.

Examples of the expression vector include pLEX (manufactured by Invitrogen), pRSET (manufactured by Invitrogen), pGEMEX-1 (manufactured by Promega), pQE-30 (manufactured by Qiagen), pKYP 10 (Japanese Published Unexamined Patent Application No. 110,600/1983), pKYP 200 [*Agric.Biol.Chem.,* 48, 669 (1984)], pLSA1 [*Agric.Biol. Chem.,* 53, 277 (1989)], pGEL1 [*Proc. Natl. Acad. Sci., USA*, 82 , 4306 (1985)], pCAL-n (manufactured by Stratagene), pTrs30 (FERM BP-5407), pTrs32 (FERM BP-5408), pGHA2 (FERM BP-400), pGKA2 (FERM B-6798), pTerm2 (Japanese Published Unexamined Patent Application No. 22979/1991, US 5,342,775), pKK233-2 (manufactured by Amersham Biosciences), pGEX (manufactured by Amersham Biosciences), pET3-a (manufactured by Novagene), pSupex, pUB110, pTP5, pC194, pMAL-c2X (manufactured by New England Biolabs), etc.

With regard to a promoter, any promoter can be used so far as it is able to initiate a transcription in a host cell such as *Escherichia coli.* examples of the promoter include a promoter derived from *E. coli*, phage, etc. such as trp promoter (Ptrp), lac promoter (Plac), P_{L} promoter and P_{R} promoter, SP01 promoter, SP02 promoter and penP promoter, etc. It is also possible to use an artificially designed and modified promoter, etc. such as (Ptrp × 2) which is a promoter where two Ptrp are connected in series, tac promoter, lacT7 promoter and let I promoter.

With regard to a ribosome-binding sequence, it is preferred to use a plasmid where a distance between Shine-Dalgarno sequence and initiation codon is appropriately adjusted (such as 6 to 18 bases).

Although a transcription termination sequence is not always necessary for the expression of gene A, it is preferred to place a transcription termination sequence immediately beneath a structural gene.

Examples of a host cell include microorganisms belonging to genus *Escherichia,* genus *Serratia,* genus *Bacillus,* genus *Brevibacterium,* genus *Corynebacterium,* genus *Microbacterium,* genus *Pseudomonas,* etc. such as *Escherichia coli* XL1-Blue, *Escherichia coli* XL2-Blue *Escherichia coli* DH1, *Escherichia coli* MC1000, *Escherichia coli* KY3276, *Escherichia coli* W1485, *Escherichia coli* JM109, *Escherichia coli* HB101, *Escherichia coli* No. 49, *Escherichia coli* W3110, *Escherichia coli* NY49, *Escherichia coli* BL21 (DE3), *Escherichia coli* BL21 (DE3) pLysS, *Escherichia coli* HMS174 (DE3), *Escherichia coli* HMS 174 (DE3) pLysS, *Serratia ficaria*, *Serratia fonticola, Serratia liquefaciens, Serratiamarcescens* , *Bacillus subtilis, Bacillus amyloliquefaciens*, *Brevibacterium ammoniagenes*, *Brevibacterium immariophilum* ATCC 14068, *Brevibacterium saccharolyticum* ATCC 14066, *Corynebacterium glutamicum* ATCC 13032, *Corynebacterium glutamicum* ATCC 14067, *Corynebacterium glutamicum* ATCC 13869, *Corynebacterium acetoacidophilum* ATCC 13870, *Microbacterium ammoniaphilum* ATCC 15354, *Pseudomonas* sp. D-0110, etc.

With regard to a method for transfecting a recombinant vector, any method can be used so far as it is a method for transfecting a DNA into the above host cell and examples of the method include an electroporation method [*Nucleic Acids Res*., 16, 6127 (1988)], a method using calcium ion [*Proc*. *Natl*. *Acad*. *Sci*. *USA*, 69, 2110 (1972)] a protoplast method (Japanese Published Unexamined Patent Application No. 248,394/1988) and a method mentioned in *Gene*, 17, 107 (1982) and *Mol*. *Gen*. *Genet*., 168, 111 (1979).

When a yeast strain is used as a host cell, examples of the expression vector include YEp13 (ATCC 37115), YEp24 (ATCC 37051), YCp50 (ATCC 37419), pHS19, pHS15, etc. With regard to a promoter, any promoter can be used so far as it is able to initiate a transcription in a yeast strain and examples of the promoter include a promoter such as PH05 promoter, PGK promoter, GAP promoter, ADH promoter, gal 1 promoter, gal 10 promoter, heat shock protein promoter, MF α1 promoter, CUP 1 promoter, etc.

With regard to a host cell, yeast strain belonging to genus *Saccharomyces,* genus *Schizosacchromyces,* genus *Kluyveromyces,* genus *Trichosporon,* genus *Schwanniomyces* are exemplified and, to be more specific, *Saccharomyces cerevisiae, Schizosaccharomyces pombe, Kluyveromyces lactis, Trichosporon pullulans, Schwanniomyces alluvis,* etc. can be exemplified.

With regard to a method for tansfecting a recombinant vector, any method can be used so far as it is a method for tansfecting a DNA into yeast and examples of the method include an electroporationmethod [*Methods*. *Enzymol*., 194, 182 (1990)], a spheroplast method [*Proc*. *Natl*. *Acad*. *Sci*. *USA*, 84, 1929 (1978)], a lithium acetate method [*J*. *Bacteriol*., 153, 163 (1983) and a method mentioned in *Proc*. *Natl*. *Acad*. *Sci*. *USA*, 75, 1929 (1978).

A transformant wherein an animal cell is used as a host cell is able to be obtained by the method mentioned in [I] 3.

Examples of a host cell include a mouse myeloma cell, a rat myeloma cell, a mouse hybridoma, CHO cell which is a Chinese hamster cell, BHK cell, a renal cell of African green monkey, Namalwa cell or Namalwa KJM-1 cell which is a human cell, a human fetal renal cell, ahuman leukemia cell, HBT 5637 (Japanese Published Unexamined Patent Application No. 000,299/1988) and a human colorectal cancer cell line, etc.

Examples of a mouse myeloma include SP2/0, NS0, etc.; examples of a rat myeloma cell include YB2/0, etc.; examples of a human fetal renal cell include HEK293, 293, etc.; examples of a human leukemia cell include BALL-1, etc.; examples of a renal cell of African green monkey include COS-1, COS-7, etc.; and examples of a human colorectal cancer cell line include HCT-15, etc.

As mentioned in the above [I] 3., the immortalized cell line of the present invention can be used as well.

When insect cell is used as a host cell, it is able to express a peptide by a method mentioned, for example, in Baculovirus Expression Vectors: A Laboratory Manual, W. H. Freeman and Company, New York (1992), Molecular Biology, 3rd ed., Current Protocols in Molecular Biology, etc.

Thus, arecombinant gene transfervector andabaculovirus are co-transfected into an insect cell to obtain a recombinant virus on culture supernatant of the insect cell and insect cells are further infected with the resulting recombinant virus whereupon a peptide is able to be expressed.

Examples of the gene transfer vector used in the above method include pVL1392 (manufactured by Pharmingen), pVL1393 (manufactured by Pharmingen) and pBlueBac4. 5 (manufactured by Invitrogen), etc.

With regard to the baculovirus, *Autographa californica* nuclear polyhedrosis virus which is a virus infecting an insect belonging to Noctuidae family, etc. can be used for example.

With regard to an insect cell, an ovarian cell of *Spodoptera frugiperda*, an ovarian cell of *Trichoplusiani,* a cultured cell derived from ovary of silkworm, etc. Examples of the ovarian cell of *Spodoptera frugiperda* include Sf9 and sf21 [Baculovirus Expression Vectors: A Laboratory Manual, W. H. Freeman and Company, New York (1992)], etc.; examples of the ovarian cell of *Trichoplusia ni* include High Five (manufactured by Invitrogen), etc.; and examples of the cultured cell derived from ovary of silkworm include *Bambyx mari* N4, etc.

Examples of a method for the co-transfection of the above-mentioned recombinant gene transfer vector and the above-mentioned baculovirus into insect cell to obtain a recombinant virus include a calcium phosphate method (Japanese Published Unexamined Patent Application No. 227,025/1990), a lipofectionmethod [*Proc*. *Natl*. *Acad*. *Sci*. *USA*, 84, 7413 (1987)], etc.

It is also possible to transfect a DNA to an insect cell using the same method as a method for transfecting a DNA into animal cell and examples of the method include an electroporation method [*Cytotechnology*, 3, 133 (1990)], a calcium phosphate method (Japanese Published Unexamined Patent Application No. 227,075/1990), a lipofection method [*Proc*. *Natl*. *Acad*. *Sci*. *USA*, 84, 7413 (1987)], etc.

When plant cell or plant individual is used as a host, it is possible to produce a peptide expressed by the cell line of the present invention according to a known method [*Soshiki Baiyo*, 20 (1994); *Soshiki Baiyo,* 21 (1995); *Trends in Biotechnology,* 15, 45 (1997)].

With regard to a promoter used for gene expression, any promoter can be used so far as it is able to initiate a transcription in plant cell and examples of the promoter include 35S promoter of cauliflower mosaic virus, rice actin 1 promoter, etc. It is also possible to improve the efficiency of gene expression by insertion of intron 1 of corn alcohol dehydrogenase gene, etc. between a promoter and a gene to be expressed.

Examples of a host cell include plant cells or the like, such as cells of potato, tobacco, corn, rice, rape, soybean, tomato, wheat, barley, rye, alfalfa, flax, etc. With regard to a method for transfecting a recombinant vector, any method can be used so far as it is a method for transfecting a DNA into a plant cell and examples of the method include *Agrobacterium* (Japanese Published Unexamined Patent Application No. 140,885/1984, No. 070,080/1985; WO 94/00977), an electroporation method [*Cytotechnology*, 3, 133 (1990); Japanese Published Unexamined Patent Application No. 251,887/1985], a method using a particle gun (Japanese Patent No. 2,606,856, No. 2,517,813), etc.

Cell and organ of plant to which gene is transfected can be cultured in large quantities using a jar fermenter. It is also possible to prepare a plant individual (transgenic plant) transfected with a gene by means of re-differentiation of plant cell transfected with the gene.

It is possible to produce a peptide where the cell lines of thepresent invention is expressed using an animal individual. For example, in accordance with a known method [*Am*. *J*. *Clin*. *Nutr*., 63, 639S (1996); *Am*. *J*. *Clin*. *Nutr*., 63, 627S (1996); *Bio*/*Technology*, 9, 830 (1991)], the peptide is able to be produced in an animal to which a gene encoding the peptide is transfected.

With regard to a promoter, any promoter can be used so far as it is able to initiate a transcription in animals and, for example, a promoter which is specific to mammary gland cell such as α-casein promoter, β-casein promoter, β-lactoglobulin promoter and whey acidic protein promoter can be advantageously used.

When a transformant derived from a microorganism, an animal cell or a plant cell having recombinant vector where a gene encoding the peptide expressed by the cell line of the present invention is inserted is cultured according to the conventional culturing method, the peptide is produced and accumulated and the peptide is then recovered from the culture to produce the peptide.

When a transformant is an animal individual or a plant individual, it is bred or cultivated according to the conventional method to produce and accumulate the peptide and the peptide is then recovered from the animal individual or the plant individual to produce the peptide.

In the case of animal individual, for example, a non-human transgenic animal comprising a gene encoding the peptide is bred to produce and accumulate the peptide in the animal and the peptide is then recovered from the animal whereupon the peptide is able to be produced. Examples of the place for production and accumulation in the animal include milk, egg, etc. of the animal.

In the case of plant individual, for example, a transgenic plant comprising a gene encoding the peptide is cultivated to produce and accumulate the peptide in the plant and the peptide is then recovered from the plant whereupon the peptide is able to be produced.

When the transformant for producing the peptide is prokaryote such as *E. coli* or eukaryotic microbe such as yeast, a medium in which such a living organism is cultured can be either natural or synthetic medium so far as it is a medium containing carbon source, nitrogen source, inorganic salt, etc. which are able to be assimilated by the living organism and are able to efficiently conduct cultivation of the transformant.

With regard to a carbon source, any carbon source can be used so far as it is able to be assimilated by the transformant and there can be used carbohydrate such as glucose, fructose, sucrose, molasses containing them, starch or hydrolyze starch; organic acid such as acetic acid and propionic acid; and alcohol such as ethanol and propanol.

With regard to a nitrogen source, there can be used ammonia, ammonium salt of various inorganic and organic acids such as ammonium chloride, ammonium sulfate, ammonium acetate and ammonium phosphate, other nitrogen-containing compounds, peptone, meat extract, yeast extract, corn steep liquor, hydrolyzed casein, soybean cake, hydrolyzed soybean cake, various fermented microbe cells and digested product thereof, etc.

With regard to an inorganic salt, there can be used potassium primary phosphate, potassium secondary phosphate, magnesium phosphate, magnesium sulfate, sodium chloride, ferrous sulfate, manganese sulfate, copper sulfate, calcium carbonate, etc.

Cultivation is carried out under an aerobic condition such as shake culture or deep aeration stirring culture. With regard to cultivation temperature, 15 to 40°C is preferred and cultivation time is usually 16 to 96 hours. During cultivation, pH is kept at 3.0 to 9.0. Adjustment of pH is carried out using inorganic or organic acid, alkaline solution, urea, calcium carbonate, ammonia, etc.

During the cultivation, an antibiotic substance such as ampicillin or tetracycline can be added to a medium if necessary.

When microorganisms transfectedwith an expression vector using inducible promoter as a promoter are cultured, an inducer can be added to a medium if necessary. For example, when microorganisms transfected with an expression vector using lac promoter is cultured and when microorganisms transfected with an expression vector using trp promoter is cultured, isopropyl-β-D-thiogalactoside or the like and indoleacrylic acid or the like can be added to the medium respectively.

When a transformant for the manufacture of the peptide is animal cell, a commonly-used RPMI 1640 medium [*J*. *Am*. *Med*. *Assocn*., 199, 519 (1967)], Eagle's MEM medium [*Science*, 122, 501 (1952)], DMEM medium [*Virology*, 8, 396 (1959)], 199 medium [*Proc*. *Soc*. *Exp*. *Biol*. *Med*., 73, 1 (1950)] or the above-mentioned medium to which FCS or the like is added can be used as a medium for culturing the cell.

Culturing is carried out usually for 1 to 7 day(s) under the condition of pH 6 to 8, at 30 to 40°C, in the presence of 5% CO₂, etc.

During the cultivation, an antibiotic substance such as kanamycin and penicillin can be added to the medium if necessary.

With regard to a medium for culturing a transformant prepared using insect cell as a host cell, commonly-used TNM-FH medium (manufactured by Pharmingen), Sf-900 II SFM medium (manufactured by Gibco), ExCell 400 and ExCell 405 (both manufactured by JRH Biosciences), Grace' s insect medium [*Nature*, 195, 788 (1962)], etc. can be used.

It is preferred that pH is 6 to 7 and cultivation temperature is 25 to 30°C and, usually, cultivation time is 1 to 5 day(s).

During the cultivation, an antibiotic substance such as gentamycin can be added to a medium if necessary.

With regard to a method for the expression of gene, it is also possible to express a partial peptide in addition to express a full-length peptide.

The above-mentioned full-length peptide or partial peptide is also able to be expressed as a secretory protein or a fused protein according to a method mentioned, for example, in Molecular Cloning, 3rd ed. , in addition to be solely expressed in a cell. Examples of the protein to be fused include β-galactosidase, protein A, IgG-binding domain of protein A, chloramphenicol acetyltransferase, polyarginine, polyglutamic acid, protein G, maltose binding protein, glutathione S-transferase, polyhistidine chain (His-tag), S peptide, DNA-binding protein domain, Tac antigen, thioredoxin, green fluorescent protein, FLAG peptide and epitope of any antibody [*Jikken Igaku*, 13, 469 (1995)].

With regard to a method for the production of peptide (including partial peptide) expressed by the cell line of the present invention, there is a method to produce in a host cell, a method to secrete outside the host cell or a method to produce on an outer membrane of host cell and, by changing the host cell used or the structure of the peptide to be produced, the method therefor is able to be selected.

When the peptide (including partial peptide) is produced in a host cell or on an outer membrane of a host cell, it is possible to positively secrete the peptide outside the host cell by a method by Paulson, et al. [*J*. *Biol*. *Chem*., 264, 17619 (1989)], a method by Row, et al. [*Proc*. *Natl*. *Acad*. *Sci*., *USA*, 86, 8227 (1989); *Genes Develop*., 4, 1288 (1990)] or a method in accordance with that mentioned in Japanese Published Unexamined Patent Application No. 336,963/1993, WO 94/23021, etc.

Thus, when expression is carried out in such a form that signal peptide is added before the peptide (including partial peptide) by means of genetic recombination, an aimed peptide is able to be positively secreted outside the host cell. It is also possible that tag for purification and detection is added between the signal peptide and the peptide or at the C terminal of the peptide.

Examples of the tag for purification and detection include β-galactosidase, protein A, IgG-binding domain of protein A, chloramphenicol acetyltransferase, polyarginine, polyglutamic acid, protein G, maltose binding protein, glutathione S-transferase, polyhistidine chain (His-tag), S peptide, DNA-binding protein domain, Tac antigen, thioredoxin, green fluorescent protein, FLAG peptide and epitope of any antibody [*Jikken Igaku,* 13, 469 (1995)], etc.

It is also possible that production amount is increased utilizing a gene amplification system using a dihydrofolate reductase gene, etc. according to a method mentioned in Japanese Published Unexamined Patent Application No. 227,075/1990.

In order to isolate and purify the peptide from the culture of transformant for producing the peptide (including partial peptide), a common isolation/purification method for enzymes can be used. For example, When the peptide is accumulated in a cell of the transformant in a dissolved state, the culture is centrifuged to recover the cells in the culture and the cells are washed and then crushed by sonicator, French press, Manton-Gaulin homogenizer, Dynomill, etc. to give a cell-free extract.

The cell-free extract is centrifuged and the resulting supernatant liquid is subjected to a means such as extraction with solvent, removal of salt by salting-out using ammonium sulfate or the like, precipitating method using an organic solvent, anion-exchange chromatography using resin such as diethylaminoethyl(DEAE)-Sepharose or Diaion HPA-75 (manufactured by Mitsubishi Chemical), cation-exchange chromatography using resin such as S-Sepharose FF (manufactured by Pharmacia), hydrophobic chromatography using resin such as butyl Sepharose and phenyl Sepharose, gel filtration using a molecular sieve, affinity chromatography method, chromatofocusing method, electrophoretic method such as isoelectric electrophoresis, etc. to give a purified specimen.

When the peptide (including partial peptide) is expressed by formation of insoluble matter in cells, the cells are recovered in the same manner, crushed and centrifuged, the peptide is recovered from the resulting precipitated fraction by a common method and the insoluble matter of the peptide is made soluble by a peptide denaturing agent. The solubilized liquid is diluted or dialyzed to such an extent that no peptide denaturing agent is contained therein or that the concentration of the peptide denaturing agent no longer denatures the peptide and the peptide is reconstructed to a normal steric structure and then subjected to the same isolating/purifying method as above to give a purified specimen.

When the peptide is secreted outside the cells, the culture is treated by means of centrifugal separation or the like to give a soluble fraction. It is possible to prepare a purified specimen of the peptide from the soluble fraction by the same means as in the isolating/purifying method from the above supernatant liquid of the cell-free extract.

It is also possible that the peptide (including partial peptide) is produced as a fused protein with other protein and is purified utilizing affinity chromatography using a substance having affinity to the fused protein [*Jikken Igaku,* 13, 469 (1995)]. For example, the peptide is produced as a fused protein with protein A according to a method of Row, et al. [*Proc. Natl*. *Acad*. *Sci*., *USA*, 86 , 8227 (1989); *Genes Develop.,* 4, 1288 (1990)] or a method in accordance with that mentioned in Japanese Published Unexamined Patent Application No. 336,963/1993, WO 94/23021 and is able to be purified by affinity chromatography using immunoglobulin G. It is also possible that the peptide is produced as a fused protein with FLAG peptide and is purified by affinity chromatography using anti-FLAG antibody [*Proc*. *Natl*. *Acad. Sci*., *USA*, 86, 8227 (1989)]; *Genes Develop*., 4, 1288 (1990)].

It is further possible to purify by means of affinity chromatography using an antibody to the peptide *per se*.

It is furthermore possible to produce the peptide using an *in vitro* transcription/translation system in accordance with a known method [*J*. *Biomol*. *NMR*, 6, 129 (1995); *Science*, 242, 1162 (1988); *J. Biochem*., 110, 166 (1991)].

The peptide is also able to be manufactured by a chemical synthetic method such as a Fmoc method (fluorenylmethyoxycarbonyl method) and a tBoc method (tert-butyloxycarbonyl method). It is further possible to chemically synthesize it utilizing a peptide synthesizer such as that manufactured by Advanced ChemTech, Applied Biosystems, Amersham Biosciences, Shimadzu, etc.

Structure analysis of the purified peptide is able to be carried out by a method which is commonly used in protein chemistry such as a method mentioned in "Protein Structure Analysis for Gene Cloning" (by Hisashi Hirano, published by Tokyo Kagaku Dojin, 1993).

### 4. Method for preparing antibody recognizing a peptide expressed in hypothalamic cells or Langerhans islets cells

It is possible to prepare an antibody recognizing a peptide expressed in hypothalamic cells or Langerhans islets cells using a peptide prepared by a method mentioned in [I] 5. or [III] 3. as an antigen. It is also possible to use the synthetic peptide as an antigen. It is further possible that a cell line of the present invention or a cell membrane fraction prepared from the cell line is used as an antigen to prepare an antibody which specifically recognizes a surface antigen of the cell line.

Method for the production of polyclonal antibody and monoclonal antibody will be shown in the following (i) and (ii).

### (i) Production of polyclonal antibody

Purified specimen of a full-length or a partial fragment of the peptide prepared by the above mentioned method 3. or a peptide (partial peptide) having a partial amino acid sequence of the above peptide is used as an antigen and administered to animal whereupon a polyclonal antibody is able to be produced. It is also possible to use the cell line of the present invention or a cell membrane fraction prepared form the cell line as an antigen.

With regard to animals for the administration, rabbit, goat, rat of 3 to 20 weeks age, mouse, hamster, etc. can be used. Dose of the antigen is preferred to be 50 to 100 µg per animal. When a partial peptide is used, it is preferred that a product where the partial peptide is subjected to a covalent bond to a carrier protein such as keyhole limpet hemocyanin or bovine thyroglobulin is used as an antigen. Apartial peptide used as an antigen is able to be synthesized by a peptide synthesizer. When the cell line of the present invention is used as an antigen, 3 to 5 × 10⁵ cells are administered to one animal. When a cell membrane fraction prepared from the cell line is used as an antigen, 1 to 10 mg is administered to one animal.

Administration of the antigen is carried out for 3 to 10 times every one to two week (s) after the first administration. After each administration, blood is collected from venous plexus of fundus on the 3rd to the 7th day and it is confirmed that the serum reacts with the antigen used for immunization by an enzyme-linked immunosorbent assay (ELISA) ["Enzyme Immunoassay Method" published by Igaku Shoin, 1976; Antibodies: ALaboratory Manual, Cold Spring Harbor Laboratory (1988)], etc.

Serum is prepared from non-human mammal where a serum shows a sufficient antibody value to the antigen used for immunization and the serum is isolated and purified whereupon a polyclonal antibody is able to be prepared.

Examples of a method for isolation and purification include treatments by means of centrifugal separation, salting-out using 40 to 50% saturated ammonium sulfate, precipitation with caprylic acid [Antibodies: A Laboratory Manual, Cold Spring Harbor Laboratory (1988)] or a method where chromatography using DEAE-Sepharose column, anion-exchange column, protein A or G-column or gel filtration column, etc. either solely or jointly.

### (ii) Preparation of monoclonal antibody

### (a) Preparation of antibody-producing cells

Mouse or rat where a serum thereof shows a sufficient antibody value to a peptide used in (i) for immunization where the cell line of the present invention is expressed or a partial fragment or a partial peptide of the peptide is used as a source for supplying antibody-producing cells.

An antigen substance is subjected to a final administration to mouse or rat showing the antibody value and, on the 3rd to 7th day thereafter, spleen is excised. The spleen is finely cut in an MEM medium (manufactured by Nissui Seiyaku), unwound using a forceps and centrifuged at 1,200 rpm for 5 minutes and the supernatant liquid is discarded. Spleen cells of the resulting precipitation fraction are treated with a Tris-ammonium chloride buffer (pH 7.65) for 1 to 2 minute(s) to remove red blood corpuscles and, after that, washed with an MEM medium for three times and the resulting spleen cells are used as antibody-producing cells.

### (b) Preparation of myeloma cells

With regard to myeloma cells, established cells prepared from mouse or rat are used.

For example, 8-azaguanine-resistant mouse (derived from BALB/b) myeloma cell line P3-X6 3Ag8-U1 [*Curr. Top. Microbiol. Immunol*., 81, 1 (1978); *Eur*. *J*. *Immunol.,* 6, 511 (1976)], SP2/0-Ag14 [*Nature*, 276, 269 (1978)], P3-X63-Ag8653 [*J*. *Immunol*., 123, 1548 (1979)], P3-X63-Ag8 [*Nature*, 256, 495 (1975)], etc. can be used.

Those cell lines are subcultured using a 8-azaguanine medium [to a medium (hereinafter, referred to a normal medium) where 1.5 mmol/L glutamine, 50 µmol/L 2-mercaptoethanol, 10 µg/mL gentamycin and 10% fetal bovine serum (FCS) are added are added to an RPMI 1640 medium is further added 15 µg/mL 8-azaguanine] although, before 3 to 4 days of cell fusion, they are cultured in a normal medium and, in fusion, not less than 2 × 10⁷ cells are used.

### (c) Production of hybridoma

The antibody-producing cells prepared in (a) and the myeloma cells prepared in (b) are well washed with an MEM medium or a PBS (1.83 g of disodium phosphate, 0.21 g of monosodium phosphate, 7.65 g of salt and 1 liter of distilled water; pH 7.2), mixed so as to make cell numbers of (antibody-producing cells): (myeloma cell) = 5 to 10:1 and centrifuged at 1,200 rpm for 5 minutes and a supernatant liquid is discarded therefrom.

Cell group of the resulting precipitated fraction is well unwound, then 0.2 to 1 mL of a solution prepared by mixing 2 g of polyethylene glycol 1000 (PEG-1000), 2 ml of MEM and 0.7 ml of dimethyl sulfoxide (DMSO) is added to the cell group (per 10⁸ antibody-producing cells) with stirring at 37°C and 1 to 2 mL of an MEM is further added thereto for several times every 1 to 2 minute(s). After the addition, an MEM is added so as to make the total volume 50 mL.

The solution prepared as such is centrifuged at 900 rpm for 5 minutes and the supernatant liquid is discarded. Cells in the resulting precipitated fraction are gently unwound and gently suspended in 100 mL of HAT medium [a medium where hypoxanthine (0.1 mmol/L), thymidine (15 µmol/L) and aminopterin (0.4 µmol/L) are added to a normal medium] by means of sucking and spouting using a measuring pipette.

The suspension is placed on a 96-well culture plate in an amount of 100 µL/well and cultured in a 5% CO₂ incubator at 37°C for 7 to 14 days. After culturing, a part of the culture supernatant is subjected to an ELISA mentioned, for example, in Antibodies: A Laboratory Manual, Cold Spring Harbor Laboratory, Chapter 14 (1988) to select a hybridoma which specifically reacts with the peptide expressed by the cell line of the present invention.

With regard to specific examples of ELISA, the following methods can be exemplified. Peptide where the cell line of the present invention is expressed in antigen upon immunization, a partial fragment of the peptide or partial peptide is coated on an appropriate plate, made to react with culture supernatant of cultured hybridoma or purified antibodywhich will be prepared later in (d) as a primary antibody, then made to react with anti-rat or anti-mouse immunoglobulin antibody labeled with enzyme such as peroxidase as a secondary antibody and subjected to a coloration reaction corresponding to a labeled enzyme and aproductwhichspecificallyreactswiththepeptide, the partial fragment of the peptide or the partial peptide coated on the plate is selected as a hybridoma which produces a monoclonal antibody specifically recognizing the peptide expressed by the cell line of the present invention.

Cloning is repeated twice by a limiting dilution method using the hybridoma [the first cloning uses an HT medium (an HT medium wherefrom aminopterin is removed) and the second one uses a normal medium] and that where a stable and strong antibody value is noted is selected as a hybridoma strain which produces an antibody specifically recognizing the peptide expressed by the cell line of the present invention.

### (d) Preparation of monoclonal antibody

Mouse or nude mouse of 8 to 10 weeks age treated with pristane [0.5 ml of 2,6,10,14-tetramethylpentadecane (pristane) is intraperitoneally administered and raised for two weeks] is intraperitoneally injected with 5 to 20 × 10⁶ monoclonal antibody-producing hybridoma cells (per mouse) prepared in (c) which specifically recognizes the peptide expressed by the cell line of the present invention. Within 10 to 21 days, the hybridoma becomes an ascites tumor. Ascites is collected from the mouse having the ascites tumor and centrifuged at 3,000 rpm for 5 minutes to remove a solid. From the resulting supernatant liquid, a monoclonal antibody is able to be purified and prepared by the same method as that used for polyclonal one.

Decision of subclass of the antibody is carried out using a mouse monoclonal antibody typing kit or a rat monoclonal antibody typing kit. Protein amount is calculated by a Lawry method or from absorbance at 280 nm.

### 5. Method for the utilization of antibody recognizing a peptide expressed in hypothalamic cells or Langerhans islets cells

When cells or tissues are stained using the antibody prepared in the above 4., it is possible to identify the cell which expresses a peptide expressed in hypothalamic cells or Langerhans islets cells. In addition, after the cell is stained using the antibody prepared in the above 4., an FACS is used whereupon it is possible to isolate the cell which expresses a peptide expressed in hypothalamic cells or Langerhans islets cells.

Thus, when the antibody prepared in the above [III] 4. is used, it is now possible to check the distribution of specific hypothalamic cells or specific Langerhans islets cells or to isolate specific hypothalamic cells or specific Langerhans islets cells. It is also possible that, after nerve stem cells, somatic stem cells or ES cells are cultured under various culture conditions, cells which are differentiated in specific hypothalamic cells or specific Langerhans islets cells are detected and isolated using the antibody.

With regard to a method for immunological detection and quantification of antigen using antibody, there are a fluorescent antibody method, an enzyme immunoassay (EIA), a radio-labeled immunoassay (RIA), an immunohistochemical staining method, an immunocytochemical staining method, a western blotting method, a dot blotting method, an immunoprecipitation method, a sandwich ELISA [Manual for Experiments of Single Clone Antibody (Kondansha Scientific) (1987); Lectures on Chemical Experiments, No. 5, Second Series, Method for Studies of Immunobiochemistry (Tokyo Kagaku Dojin) (1986)], etc.

A fluorescent antibody method is a method where microbe, animal cell or insect cell or tissue in which the peptide expressed by the cell line of the present invention is expressed inside or outside of the cells is made to react with an antibody which specifically recognizes the peptide, then further made to react with an antibody which reacts with the above antibody recognized by a fluorescent substance such as fluoresceine isothiocyanate (FITC) (such as anti-mouse IgG antibody when the peptide or an antibody which specifically recognizes the peptide is a mouse IgG class) and then the fluorescent dye is measured by a flow cytometer.

An EIA is a method where, with a sample to be measured containing the peptide expressed by the cell line of the present invention, an antibody which specifically recognizes the peptide is made to react and then an antibody which reacts with the antibody labeled with enzyme such as peroxidase (such as anti-mouse IgG antibody when the peptide or an antibody specifically recognizes the peptide is a mouse IgG class) is made to further react therewith and a coloration reaction using enzyme is carried out whereupon the colored amount is measured by a spectrophotometer.

An RIA is a method where, with a sample to be measured containing the peptide expressed by the cell line of the present invention, an antibody which specifically recognizes the peptide is made to react and then an antibody which reacts with the antibody labeled with radioisotope (such as anti-mouse IgG antibody when an antibody specifically recognizes the peptide is a mouse IgG class) is made to further react therewith and the amount of radioactivity is measured by a scintillation counter or the like.

An immunohistochemical staining method and an immunocytochemical staining method are the methods where, with microbe, animal cells or insect cells or tissues where the peptide expressed by the cell line of the present invention is expressed inside or outside of the cells, an antibody which specifically recognizes the peptide is made to react, then an antibody which reacts with the antibody being labeled with a fluorescent substance such as FITC, an enzyme such as peroxidase, biotin, etc. (such as anti-mouse IgG antibody when the antibody which specifically recognizes the peptide is a mouse IgG class) or a fragment thereof is made to react therewith and an observation under a microscope is carried out.

A western blotting is a method where an extract of microbe, animal cells or insect cells or tissues in which the peptide expressed by the cell line of the present invention is expressed inside or outside of the cell is fractionated by an SDS-polyacrylamide gel electrophoresis [Antibodies: A Laboratory Manual, Cold Spring Harbor Laboratory (1988)], the gel is blotted on a polyvinylidene fluoride (PVDF) membrane or a nitrocellulose membrane, an antibody which specifically recognizes the peptide is made to react with the membrane, then an antibody which reacts with the antibody labeled with a fluorescent substance such as FITC, an enzyme such as peroxidase, biotin or the like (such as anti-mouse IgG antibody when an antibody which specifically recognizes the peptide is a mouse IgG class) or a fragment thereof is made to react therewith and a reaction corresponding to the labeled substance is carried out.

A dot blotting method is a method where an extract of microbe, animal cells or insect cells or tissues in which the peptide expressed by the cell line of the present invention is expressed inside or outside of the cells is blotted on a nitrocellulose membrane, an antibody which specifically recognizes the peptide is made to react with the membrane, then an antibody which reacts with the antibody labeled with a fluorescent substance such as FITC, an enzyme such as peroxidase, biotin or the like (such as anti-mouse IgG antibody when an antibody which specifically recognizes the peptide is a mouse IgG class) or a fragment thereof is made to react therewith and a reaction corresponding to the labeled substance is carried out.

An immunoprecipitation method is a methodwhere an extract of microbe, animal cells or insect cells or tissues in which the peptide expressed by the cell line of the present invention is expressed inside or outside of the cells is made to react with an antibody which specifically recognizes the peptide, then a carrier having a specific binding ability to immunoglobulin such as protein G Sepharose is added and an antigen-antibody complex is precipitated.

A sandwich ELISA is a method where, with a plate in which an antibody specifically recognizing the peptide expressed by the cell line of the present invention is adsorbed therewith, an extract of microbe, animal cells or insect cells or tissues expressing the peptide inside or outside of the cells is made to react and, after that, an antibody which specifically recognizes the peptide labeled with an enzyme such as peroxidase (where the site for recognizing the antigen is different from the above-mentioned antigen) is react therewith followed by subj ecting to a reaction corresponding to the labeled substance.

### Brief Description of Drawings

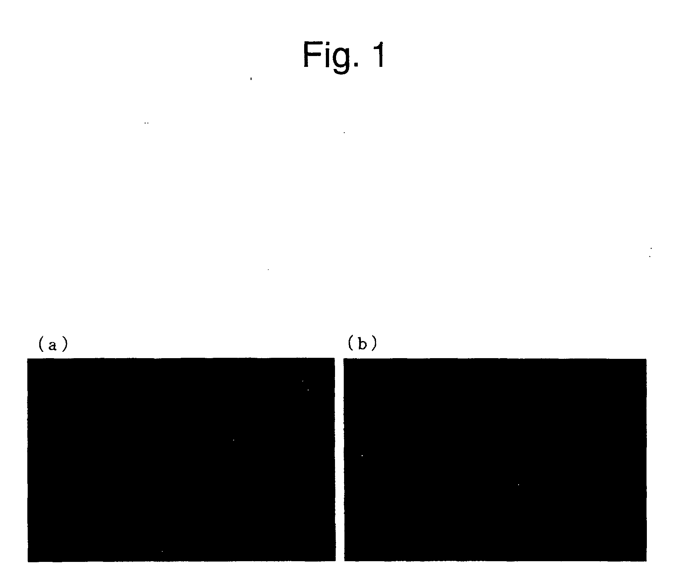

Fig. 1 shows an immunostaining of SV40 large T antigen of immortalized cells derived from hypothalamus. With regard to a mixture of immortalized hypothalamic cells derived from fetal transgenic rat cultured at 33°C, (a) shows the result of immunostaining of SV40 large T antigen and (b) shows the result of nucleus staining thereof.

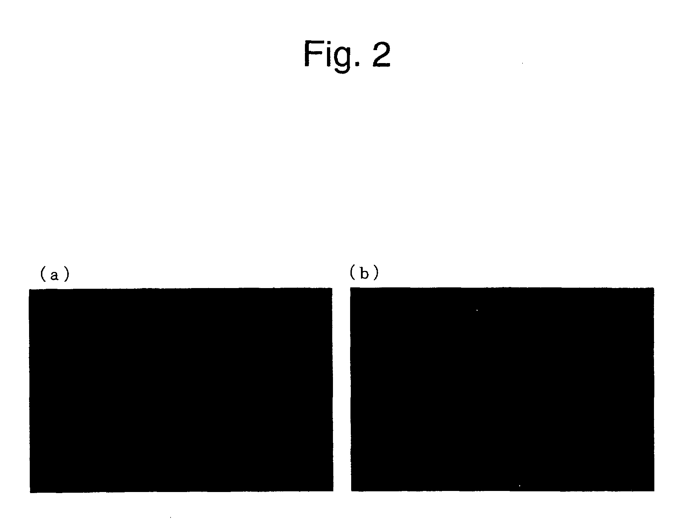

Fig. 2 shows the result of immunostaining of SV40 large Tantigen of a mixture of immortalized hypothalamic cells derived from fetal transgenic rat. (a) shows the case when cultured at 33°C and (b) shows the case when cultured at 39°C.

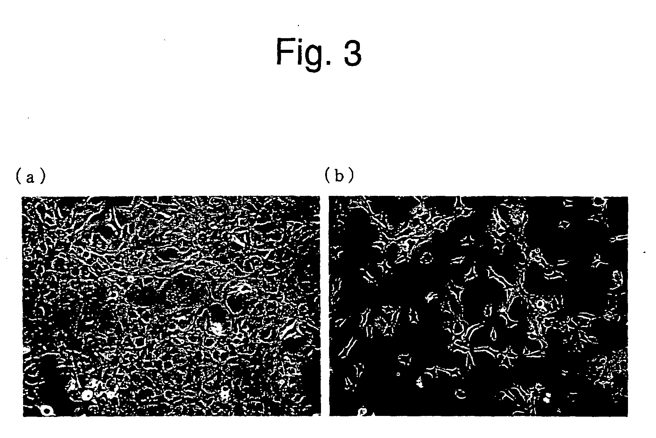

Fig. 3 shows the morphology of an immortalized hypothalamic cell line AMN2-100 when it was cultured in a medium containing 10% of serum at the temperature which was changed. (a) shows the case when cultured at 33°C and (b) shows the case when cultured at 39°C.

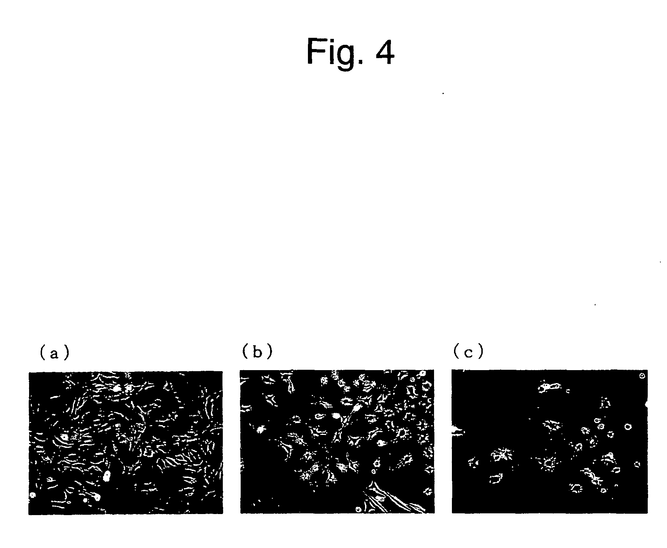

Fig. 4 shows the morphology of immortalized hypothalamic cell line AMF2-20 when it was cultured at 33°C in the medium which was changed. (a) shows the case when cultured in a medium containing 10% of serum, (b) shows the case when cultured in a DMEM/F-12 (N2) medium and (c) shows the case when cultured in a DMEM/F-12 (-FCS) medium.

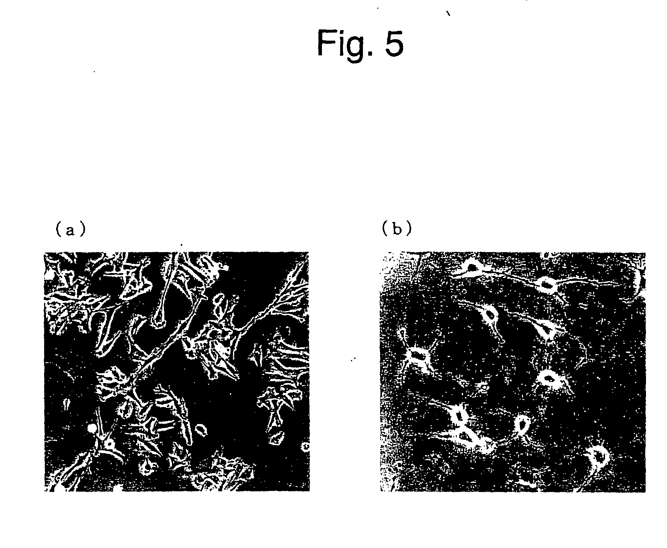

Fig. 5 shows the morphology of immortalized hypothalamic cell line AMF2-20 when it was cultured wherein the medium and the temperature were changed. (a) shows the case when cultured in a DMEM/F-12 (FCS) medium at 33°C for 8 days and (b) shows the case when cultured in a DMEM/F-12 (FCS) medium at 37°C for 5 days and, after that, the medium was changed to a DMEM/F-12 (-FCS) medium and further culturing at 37°C for 3 days.

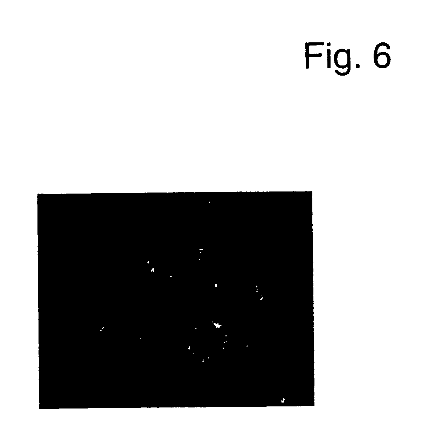

Fig. 6 shows the result when a mixture of immortalized hypothalamic cells derived from adult females of transgenic rat was subjected to an immunostaining of a leptin receptor.

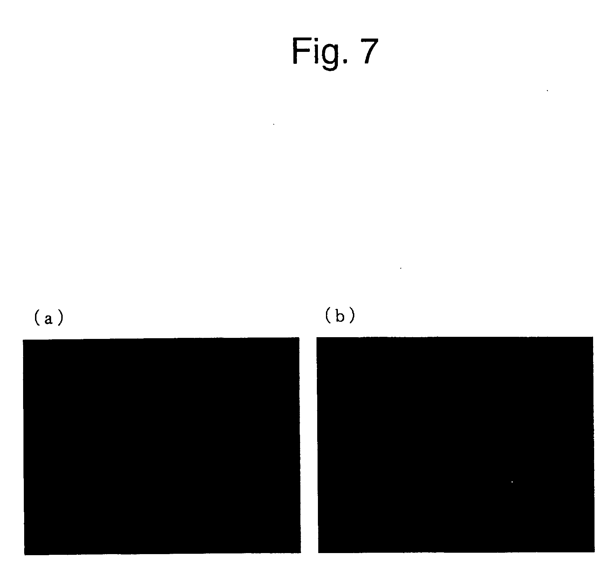

Fig. 7. (a) shows the result where immunostaining of NPY was carried out for hypothalamic cell line AMN2-25. (b) shows a negative control where no anti-NPY antibody was added.

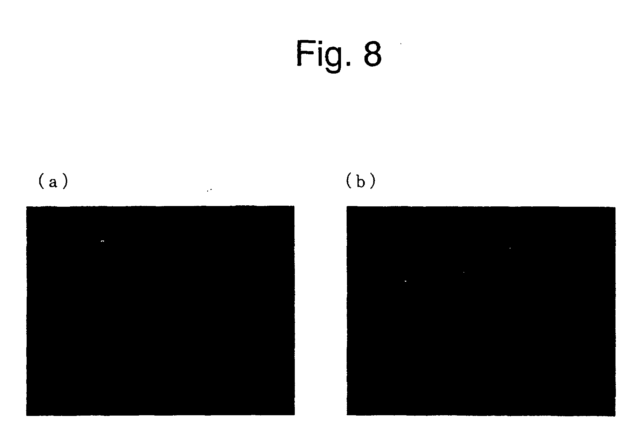

Fig. 8 shows the result of an immunostaining of STAT 3 for immortalized hypothalamic cell line NC-12. (a) shows the case where no stimulation with leptin was conducted and (b) shows the case where stimulation with leptin was conducted.

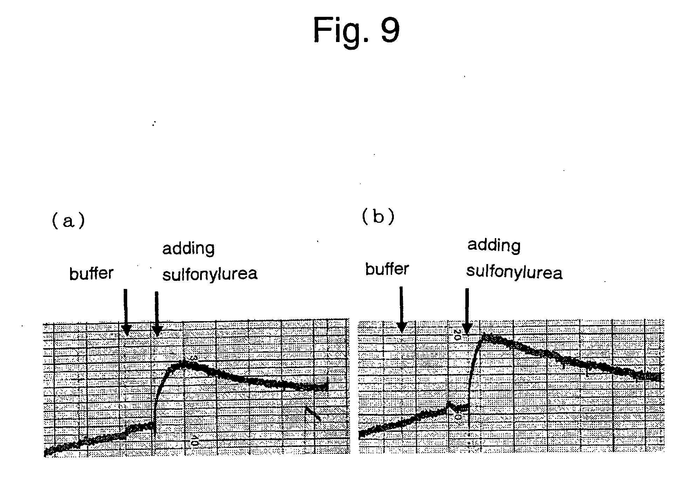

Fig. 9 shows the changes in intracellular calcium concentration by stimulation of cell line derived from immortalized hypothalamus with sulfonylurea stimulation. (a) shows the case of a mixture of immortalized cells derived from hypothalamus of female imago of a transgenic rat and (b) shows the case where an immortalized hypothalamic cell line AMN2-25 was used. Abscissa is time and, at the time shown by an arrow, HBSS buffer or sulfonylurea was added to the cells. Ordinate shows calcium concentration in terms of fluorescence intensity ratio in two excited wavelengths.

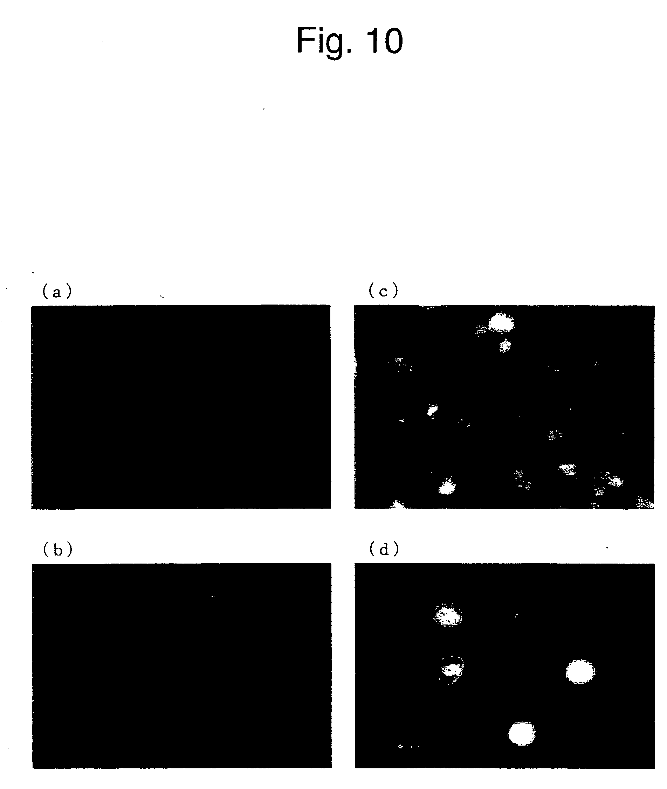

Fig. 10 shows the result where immortalized cell line F-8 derived from Langerhans islets was subjected to an immunostaining and a nuclear staining with PC2. (a) and (b) show immunostaining with PC2 while (c) and (d) show nuclear staining therewith. (a) and (c) shows the cases where cultivation was conducted in an RPMI 1640 (05918) medium while (b) and (d) show the cases where the cells cultured in an RPMI 1640 (05918) were cultured for two weeks after substituting the medium with a DMEM (D-5796) medium.

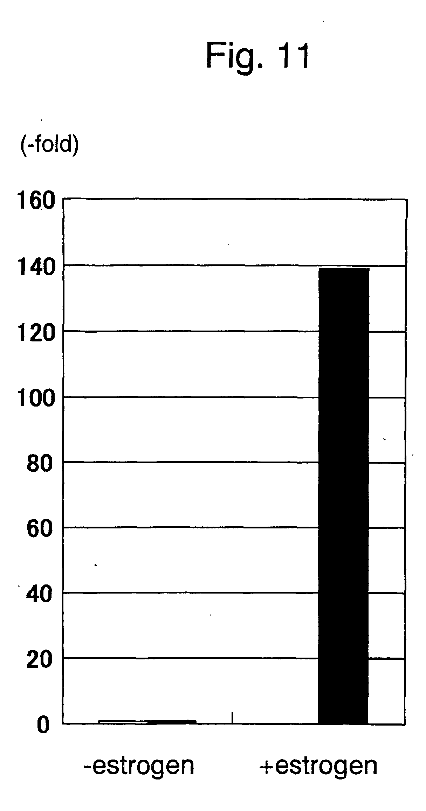

Fig. 11 shows *Renilla reniformis* luciferase activity upon stimulation with vasopressin of a stable transformant where pAGa 19-V2 was transfected to a host cell line GBCR 2. +Estrogen means the case when stimulation with 17β-estradiol was conducted whereupon inducible expression of V2 gene was carried out while - Estrogen means the case when no stimulation with 17β-estradiol was conducted. Each bar at the right side thereof shows activity when stimulation with vasopressin was conducted while each bar at the left side thereof shows activity when no stimulation with vasopressin was conducted. Ordinate means an activity ratio where the activity in case stimulation with vasopressin was not conducted was defined as 1.

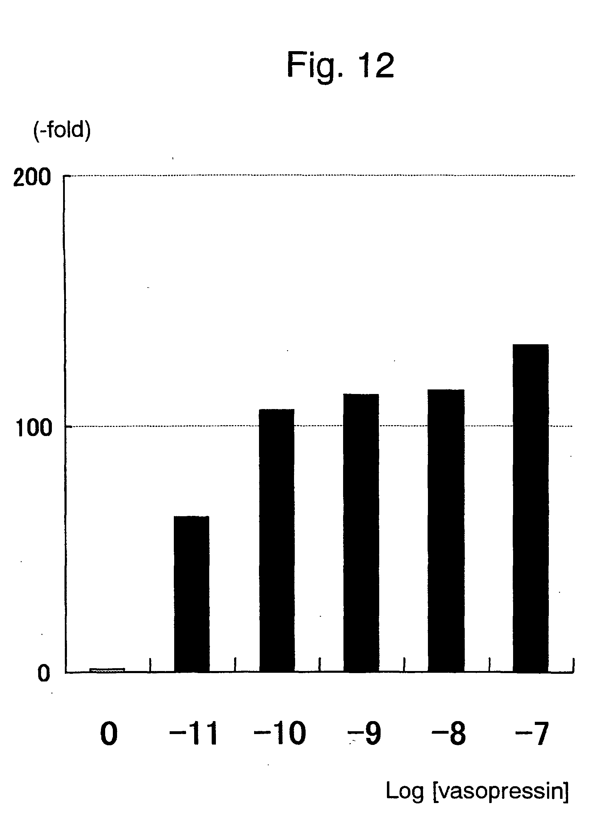

Fig. 12 shows *Renilla reniformis* luciferase activity when a stable transformant where pAGal9-V2 was transfected to a host cell line GBCRC 6 was subjected to an inducible expression of V2 gene expression with 17β-estradiol and then stimulation with various concentrations of vasopressin was conducted. Abscissa shows concentration of vasopressin [log(mol/L)] while ordinate shows activity ratio where the activity in case stimulation with vasopressin was not conducted was defined as 1.

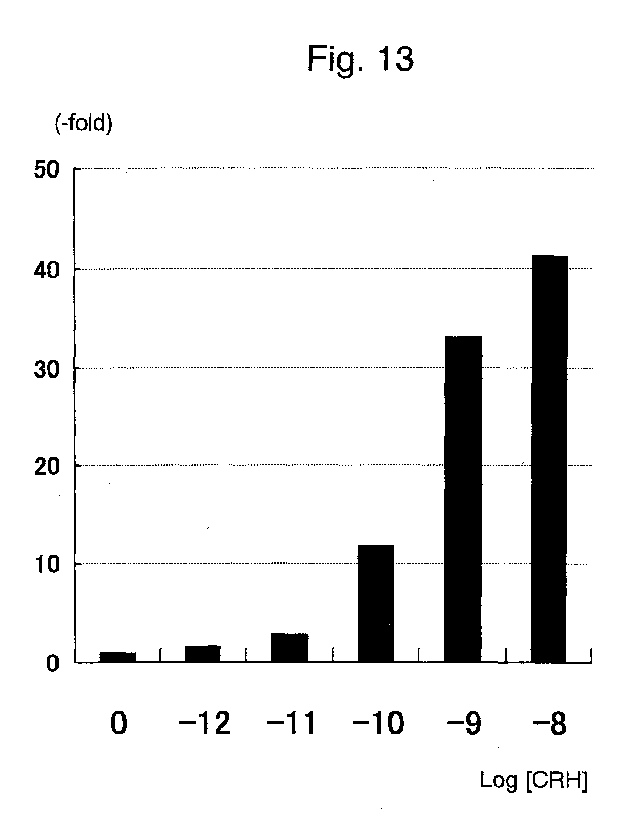

Fig. 13 shows *Renilla reniformis* luciferase activity when a stable transformant where pAGal9-CRHR1 was transfected to a host cell line GBCRC 6 was subjected to an inducible expression of CRHR1 gene expression with 17β-estradiol and then stimulation with various concentrations of corticotropin-releasing hormone was conducted. Abscissa shows concentration of corticotropin-releasing hormone [log(mol/L)] while ordinate shows activity ratio where the activity in case stimulation with corticotropin-releasing hormone was not conducted was defined as 1.

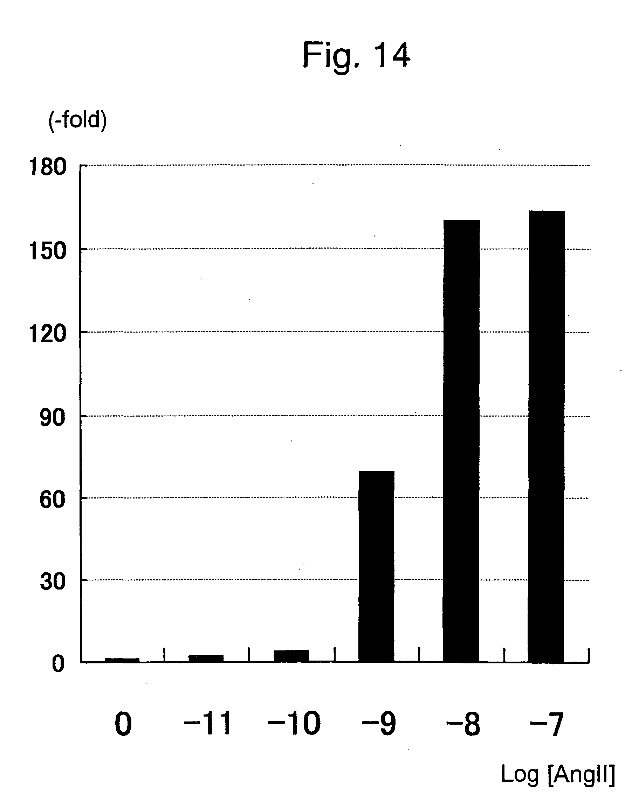

Fig. 14 shows *Renilla reniformis* luciferase activity when a stable transformant where pAGal9-AT1 was transfected to a host cell line GBCRC 6 was subjected to an inducible expression of AT1 gene expression with 17β-estradiol and then stimulation with various concentrations of angiotensin II was conducted. Abscissa shows concentration of angiotensin II [log(mol/L)] while ordinate shows activity ratio where the activity in case stimulation with angiotensin II was not conducted was defined as 1.

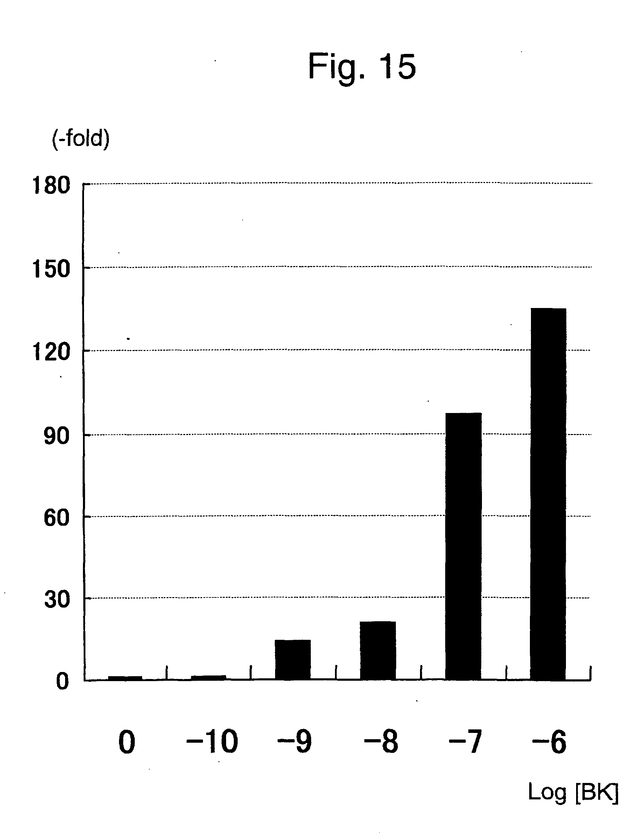

Fig. 15 shows *Renilla reniformis* luciferase activity when a stable transformant where pAGal9-B1 was transfected to a host cell line GBCRC 6 was subjected to an inducible expression of B1 gene expression with 17β-estradiol and then stimulation with various concentrations of Des-Arg9-bradykinin was conducted. Abscissa shows concentration of Des-Arg9-bradykinin [log(mol/L)] while ordinate shows activity ratio where the activity in case stimulation with Des-Arg9-bradykinin was not conducted was defined as 1.

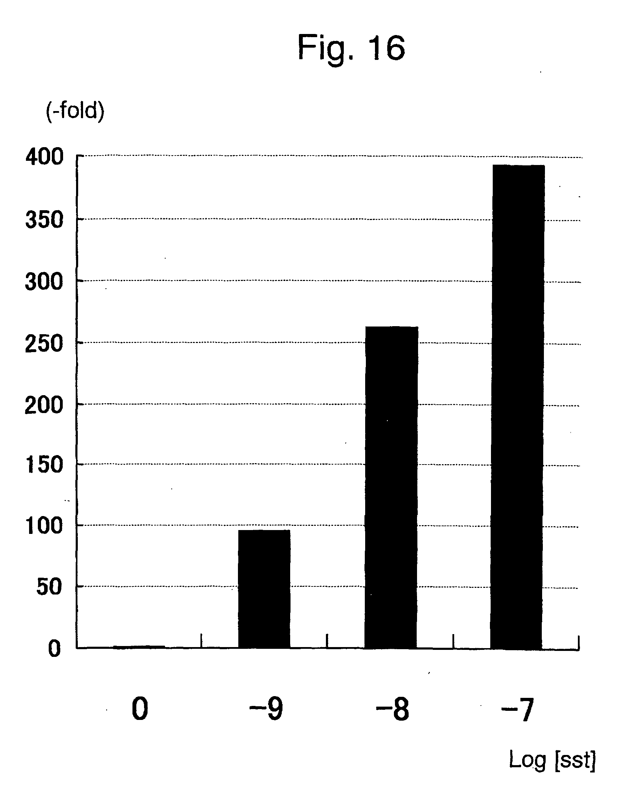

Fig. 16 shows *Renilla reniformis* luciferase activity when a stable transformant where pAGal9-sst5 was transfected to a host cell line GBCRC 6 was subjected to an inducible expression of sst5 gene expression with 17β-estradiol and then stimulation with various concentrations of somatostatin was conducted. Abscissa shows concentration of somatostatin [log(mol/L)] while ordinate shows activity ratio where the activity in case stimulation with somatostatin was not conducted was defined as 1.

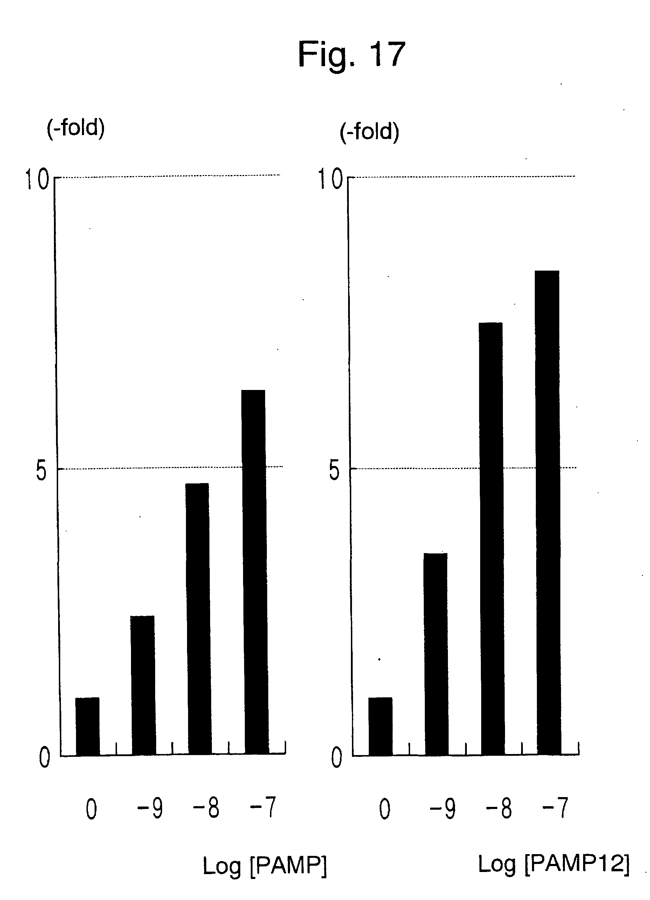

Fig. 17 shows *Renilla reniformis* luciferase activity when a stable transformant where pAGal9-MC1R was transfected to a host cell line GBCR 2 was subjected to an inducible expression of MC1R gene expression with 17β-estradiol and then stimulation with various concentrations of PAMP or PAMP-12 was conducted. A graph on the left side shows the case when stimulated with PAMP while a graphon the right side shows the case when stimulated with PAMP-12. Abscissa of the left graph shows concentration of PAMP [log(mol/L)] and that of the right graph shows concentration of PAMP-12 [log(mol/L)] while each ordinate shows activity ratio where the activity in case stimulation with PAMP or PAMP-12 was not conducted was defined as 1.

### Best Mode for Carrying Out the Invention

### [Example 1] Establishment of cell lines derived from hypothalamus of fetal rats

A domain including hypothalamus was excised from fetal transgenic rats (14-day-old, 17-day-old and 21-day-old fetal rats; 10 to 15 rats for each) transfectedwith the large T antigen gene of a temperature-sensitive mutant SV40tsA58, which was prepared by a method mentioned in *Exp*. *Anim*., 48, 255 (1999). Excision of the domain including hypothalamus was carried out according to a known method [*Endocrinology*, 137, 5651 (1996)]. The transgenic rats were purchased from YS New Technology Institute, Inc..

Separation and recovery of the cells derived from hypothalamus were carried out as follows using an enzymatic method. The excised hypothalamus was washed with Leibovitz' s L-15 medium (manufactured by Gibco), cut into small sizes using scissors and centrifuged at 800 rpm for 5 minutes. After the centrifugal separation, the supernatant was removed, 5 ml of 0.25% trypsin solution (manufactured by Gibco) and 50 µl of 10 µg/ml DNase I (manufactured by Boehringer Mannheim) were added and the mixture was subjected to an enzymatic treatment at 37°C for 10 minutes with stirring. After confirming the progress of the enzymatic treatment under an inverted microscope, 2 ml of fetal bovine serum cooled at 4°C were added and the mixture was well suspended. The suspension was centrifuged at 800 rpm for 5 minutes and the cells were recovered.

The cells were suspended in 10 ml of DMEM/F-12 (FCS) medium [a mixed medium comprising same amounts of DMEM and Ham' s F-12 medium containing 10% of fetal bovine serum, 50 U/ml of penicillin and 50 µg/ml of streptomycin (manufactured by Gibco)].

The cell suspension was plated on a 100-mm dish (manufactured by Becton-Dickinson) coated with poly-lysine and cultured at 33°C under the condition of 5% of carbon dioxide gas concentration and 100% humidity. When the cells grew to become confluent, the cells were sub-cultured to another 100-mm dish coated with poly-lysine so as to make the cell density to the cultured surface about 70%. The cultivation was continued for about three months to obtain a mixture of immortalized cell lines derived from hypothalamus. The resulting mixture of immortalized cell lines derived from hypothalamus was dispensed into 21 tubes at about 1 × 10⁶ cells for each and then cryopreserved by a known method.

A colony-forming method was used for single cloning of the cells. On a 100-mm dish coated with poly-lysine were plated 100 or 500 cells and cultured until colonies were formed. After the cultivation, each colony was peeled off by the top of a chip under microscopic observation and, at the same time, they were sucked into the chip to recover the cells. The recovered cells derived from each colony were plated on a 24-well plate coated with poly-lysine and cultured. When the cells grew to become confluent, they were cultured by a successive scaling-up to a 6-well plate and 100-mm dish (both being coated with poly-lysine). As such, four single-cloned cell lines were finally obtained. Each of the cell lines was named EA-1, EA-2, EA-4 and EA-8, respectively. EA-1 is a cell line derived from 14-day-old fetus, EA-2 and EA-4 are cell lines derived from 21-day-old fetuses and EA-8 is a cell line derived from 17-day-old fetus. Each of the resulting 4 cell lines was cryopreserved using a known method. A doubling time of the resulting cell lines was 36 to 72 hours. Even after one year or more from the establishment, no significant change was noted in the growing property of the cell lines.

### [Example 2] Establishment of cell lines derived from hypothalamus of newborn rats

Hypothalamus was excised by the same manner as in Example 1 from newborn transgenic rats (13 newborn ones on the 0th day after birth) transfected with the large T antigen gene of temperature-sensitive mutant Sv40tsA58 and the resulting cells were cultured for about 3 months to obtain a mixture of immortalized cell lines derived from hypothalamus. The resulting mixture of immortalized cell lines derived from hypothalamus was dispensed into 20 tubes at about 1 × 10⁶ cells for each and then cryopreserved by a known method.

According to the method mentioned in Example 1, 141 single-cloned cell lines were obtained from the above cryopreserved cell lines. The resulting 141 cell lines were also cryopreserved by the same manner as above. A doubling time for the resulting cell lines was 36 to 72 hours. Even after one year or more from the establishment, no significant change was noted in the growing property of the cell lines.

### [Example 3] Establishment of cell lines derived from hypothalamus of adult rats

Adult (eight-week-old after birth: one male and two females) transgenic rats transfected with the large T antigen gene of temperature-sensitive mutant SV40tsA58 were killed by cervical dislocation and, after that, brain was excised. The brain was divided into two equal parts along a midline and hypothalamus was excised with a careful attention so that no meninges was contained therein. Excision of the hypothalamus was carried out as follows.

After the rat was decapitated, skin and muscle were carefully removed from the head so that the skull was exposed. The skull was excised with scissors along the midline from the decapitated part and then excised with scissors in a right angle to the first cutting angle to remove the upper part of the skull whereupon the brain was exposed. The optic chiasma was removed from the base of the brain by excising with scissors carefully and the brain was taken out from the skull. The taken-out brain was placed in such a manner that the venter was upside and pituitary gland in the central area of the base of the brain was identified by referring to the drawings on page 52 of "Color Atlas - Guidebook for Anatomy of Rats" (published by Hirokawa Shoten). Using the pituitary gland as an index, hypothalamic domain was excised by referring to the drawings in "The Rat Brain in Stereotaxic Coordinate" Second Edition, Academic Press (1986).

By the same manner as in Example 1, the cells derived from the excised hypothalamus of male or female rats were separately cultured for about 3 months to obtain a mixture of immortalized hypothalamus-derived cell lines. Each of the resulting mixture of immortalized cell lines derived from hypothalamus of adult male or female rats was dispensed into 11 tubes at about 1 × 10⁶ cells for each and then cryopreserved by a known method.

From the mixture of immortalized cell lines derived from hypothalamus of the adult male were obtained 373 single-cloned cell lines according to the method mentioned in Example 1. Each of the resulting 373 cell lines was cryopreserved by a known method. A doubling time of the resulting cell lines was 36 to 72 hours. Even when after one year or more from the establishment, no significant change was noted in the growing property of the cell lines.

Similarly were prepared 280 single-cloned cell lines from a mixture of immortalized cell lines derived from hypothalamus of adult females. Each of the resulting 280 cell lines was cryopreserved by a known method. A doubling time of the resulting cell lines was 36 to 72 hours. Even when after one year or more from the establishment, no significant change was noted in the growing property of the cell lines.

### [Example 4] Analysis of expression of SV40 large T antigen in cell lines derived from hypothalamus

With regard to a mixture of immortalized cell lines derived from hypothalamus of fetuses, newborns, adult male and adult female of the rats obtained in Examples 1 to 3, expression of SV40 large T antigen was investigated at the protein level using an immunostaining method.

Each of the above-mentioned mixtures of immortalized cell lines was cultured according to the method mentioned in Example 1, the medium was removed, 4% paraformaldehyde solution was added and the reaction was carried out at 4°C for 10 minutes so that the cells were immobilized. The immobilized cells were treated with a 0.3% Triton X-100 solution, then antibody to SV40 large T antigen diluted to an extent of 10-fold (manufactured by Oncogene) was added thereto and a reaction was carried out at 37°C for 60 minutes. After the reaction, the cells were washed with PBS for one time, then a FITC-labeled anti-mouse IgG (manufactured by KPL) diluted to an extent of 40-fold was added and a reaction was carried out at room temperature for 60 minutes. After the reaction, the cells were washed with PBS for one time, 1 µg/ml of a Hoechst 33342 solution (manufactured by Calbiochem) was added thereto and a reaction was carried out at room temperature for 5 minutes to stain the nuclei.

The nucleus-stained cells were washed with PBS for one time, dried, mounted with a solution containing a discoloration inhibitor [PBS containing 90% of glycerol and 2.5% of 1,4-diazabicyclo-[2,2,2]-octane (DABCO); hereinafter, referred to as "a solution of discoloration inhibitor"] and observed under a fluorescence microscope. As shown in Fig. 1, a positive staining showing the expression of SV40 large T antigen was confirmed in most of the cells.

From the above-mentioned results, it is believed that the immortalized cell lines derived fromhypothalamus of fetuses, newborns, adult male and adult female of rats obtained in Examples 1 to 3 were immortalized due to expression of SV40 large T antigen.

Further, when staining was conducted similarly under the condition of culturing cells at 39°C, signals were decreased apparently as compared with the case of condition of culturing cells at 33°C. The result was shown in Fig. 2. From the result, it is believed that a temperature-sensitive SV40 large T antigen was expressed in the obtained group of cells.

### [Example 5] Study of method for culturing cell lines derived from hypothalamus

Immortalized cell lines derived from hypothalamus of fetuses, newborns, adult male and adult females of rats obtained in Examples 1 to 3 were used to study influence of culturing method on differentiation characteristics of cells using the morphology of the cells as an index.

### (1) Study of cultivation temperature

Each of the above-mentioned immortalized cell lines (single clones and a mixture) was cultured according to the method mentioned in Example 1. After the cultivation, about 2 × 10⁴ cells were plated on a 35-mm dish coated with poly-lysine and cultured at 33°C, 37°C or 39°C for 5 days and the morphology was observed under a microscope.

As a result, morphology of the cells changed when cultured at 37°C or 39°C whereupon cells showing the morphology characteristic to nerve cells, oligodendrocytes or astrocytes appeared. The result shows that, when cultivation was carried out at 37°C or 39°C, it is possible to differentiate the above-mentioned immortalized cell line derived from hypothalamus. Result of the experiment using immortalized cell line (AMN2-100) derived from hypothalamus of adult male obtained in Example 3 was shown in Fig. 3.

### (2) Study of a medium

Each of the above-mentioned immortalized cell lines (single clones andmixtures) was cultured according to the method mentioned in Example 1. After the cultivation, about 2 × 10⁴ cells were plated on a 35-mm dish coated with poly-lysine and cultured for 5 days in a DMEM/F-12 (FCS) medium, a DMEM/F-12 (N2) medium [a medium to which 1% of N-2 supplement (manufactured by Gibco) was added instead of 10% of fetal calf serum of the DMEM/F-12 (FCS) medium] or a DMEM/F-12 (FCS) medium to which no serum component was added [hereinafter, referred to as a DMEM/F-12 (-FCS) medium] and the morphology was observed under a microscope.

As a result, when the cells were cultured in a DMEM/F-12 (N2) medium or a DMEM/F-12 (-FCS) medium, morphology of the cell changed and cells showing the morphology characteristic to nerve cells, oligodendrocytes or astrocytes appeared. The result shows that, when cultivation was carried out in a medium containing no serum such as a DMEM/F-12 (N2) mediumor a DMEM/F-12 (-FCS), it is possible to differentiate the above-mentioned immortalized cell line derived from hypothalamus. Result of the experiment using immortalized cell line (AMF2-20) derived from hypothalamus of adult male obtained in Example 3 is shown in Fig. 4.

### (3) Study of cultivation temperature and medium

Each of the above-mentioned immortalized cell lines (single clones and mixtures) was cultured according to the method mentioned in Example 1. After the cultivation, about 2 × 10⁴ cells were plated on a 35-mm dish coated with poly-lysine and cultured under the following two conditions and the morphology was observed under a microscope. The condition 1 is a condition where the cells were cultured at 33°C for 8 days while the condition 2 is a condition where the cells were cultured at 37°C for 5 days, then the medium was exchanged to a DMEM/F-12 (-FCS) medium and cultivation was carried out for 3 days more.

As a result, under the condition 2, morphology of the cell changed and cells showing the morphology characteristic to nerve cells, oligodendrocytes or astrocytes appeared. The result shows that, when cultivation was carried out under the condition 2, it is possible to differentiate the above-mentioned immortalized cell line derived from hypothalamus.

### [Example 6] Analysis of expression of various genes in cell lines derived from hypothalamus

With regard to the cell lines obtained in Examples 1 to 3, expression profile of various genes expressed in hypothalamic cells as shown in Table 1 was analyzed according to the following method.

Four cell lines derived from fetal rats obtained in Example 1, 57 cell lines derived from newborn rats obtained in Example 2, 49 cell lines derived frommale adult rats obtained in Example 3 and 46 cell lines derived from female adult rats obtained in Example 3 were cultured according to the method mentioned in Example 1. After the cultivation, about 1 × 10⁵ cells were plated on a 100-mm dish coated with poly-lysine and cultured for 5 days by shifting the cultivation temperature from 33°C to 37°C.

After the cultivation, total RNA was prepared from each cell line using an RNeasy Mini Kit (manufactured by Qiagen). Five µg of the total RNA were used and a single-stranded cDNA was synthesized by Superscript First-Strand Synthesis System for RT-PCR (manufactured by Gibco). The single-stranded cDNA was diluted with water to an extent of 50-fold and used as a template for the PCR.

To the single-stranded cDNA (5 µl) were added a gene-specific primer (each 20 pmol) as shown in Table 1, 1.6 µl of 2.5 mmol/L dNTP mixed solution, 1 µl of DMSO, 0.1 µl of 5 units/µl Recombinant Ex Taq DNA Polymerase (manufactured by Takara Shuzo) and 2 µl of 10 × reaction buffer (manufactured by Takara Shuzo) and then sterilized water was added thereto to make the total volume 20 µl. The resulting solution was used and subj ected to a PCR under the following condition. After heating at 95°C for 5 minutes , a reaction in 25 to 35 cycles at 94°C for 1 minute, at 55, 60, 65 or 67°C (reaction temperature for each gene amplification is shown in Table 1) for 1 minute and at 72°C for 1 minute was conducted using a thermal cycler DNA Engine (manufactured by MJ Research).

**Table 1**

| No. | Gene | Primer Primer (SEQ ID NO) | Reaction Reaction Temp (°C) |
|---|---|---|---|
| 1 | Leptin receptor (Ob-Rb) | 1, 2 | 55 |
| 2 | Preproneuromedin U | 3, 4 | 65 |
| 3 | RFRP preproprotein | 5, 6 | 65 |
| 4 | Preproorexin | 7, 8 | 65 |
| 5 | Preproopiomelanocortin | 9, 10 | 65 |
| 6 | Preproneuropeptide Y | 11, 12 | 60 |
| 7 | Preproneuropeptide FF | 13, 14 | 65 |
| 8 | Preprocorticotropin-releasing hormone | 15, 16 | 65 |
| 9 | Preprothyrotropin-releasing hormone | 17, 18 | 65 |
| 10 | Preproghrelin | 19, 20 | 65 |
| 11 | Prepromelanin-concentrating hormone | 21, 22 | 60 |
| 12 | CART | 23, 24 | 60 |
| 13 | Type 2 neuromedin U receptor | 25, 26 | 65 |
| 14 | RFRP receptor | 27, 28 | 65 |
| 15 | Type 4 melanocortin receptor (MC4R) | 29, 30 | 60 |
| 16 | Type 1 neuropeptide Y receptor (NPY1R) | 31, 32 | 60 |
| 17 | Type 5 neuropeptide Y receptor (NYP5R) | 33, 34 | 60 |
| 18 | Type 2 neuropeptide FF receptor (NPFF2) | 35, 36 | 55 |
| 19 | Type 1 corticotropin-releasing hormone receptor (CRHR-1) | 37, 38 | 60 |
| 20 | Type 2 corticotropin-releasing hormone receptor (CRHR-2) | 39, 40 | 60 |
| 21 | Ghrelin receptor | 41, 42 | 67 |
| 22 | Type 1 melanin-concentrating hormone receptor (MCHR1) | 43, 44 | 65 |

After a part (8 µl) of the reaction solution was subjected to an agarose gel electrophoresis, the gel was stained with SYBR Green I nucleic acid stain (Molecular Probes). Pattern of amplified DNA fragment was analyzed by FluorImager SI (manufactured by Molecular Dynamics) to measure the amount of amplified DNA fragment.

Quantitative determination of transcription product of each gene was carried out by means of a semi-quantitative PCR according to a conventional method [PCR Protocols, Academic Press(1990)]. A transcript of rat glyceraldehydes 3-phosphate dehydrogenase (hereinafter, abbreviated as G3PDH) was quantified at the same time whereby it is confirmed that the amounts of mRNA among cells and conversion efficiency from mRNA to single-stranded cDNA by reverse transcriptase among the samples were not different significantly. Quantitative determination of the G3PDH transcript was carried out by a quantitative PCR according to a common method [*Proc*. *Natl*. *Acad*. *Sci*. *USA*, 87, 2725 (1990); *J*. *Biol*. *Chem*., 269, 14730 (1994); Japanese Published Unexamined Patent Application No. 181,759/1994].

Results of analysis are shown from Table 2 to Table 5-2. It was found that various cell lines where the expression patterns of genes of the above (1) to (22) were different were obtained.

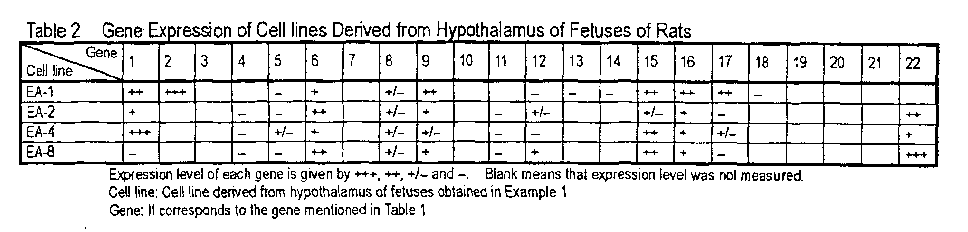

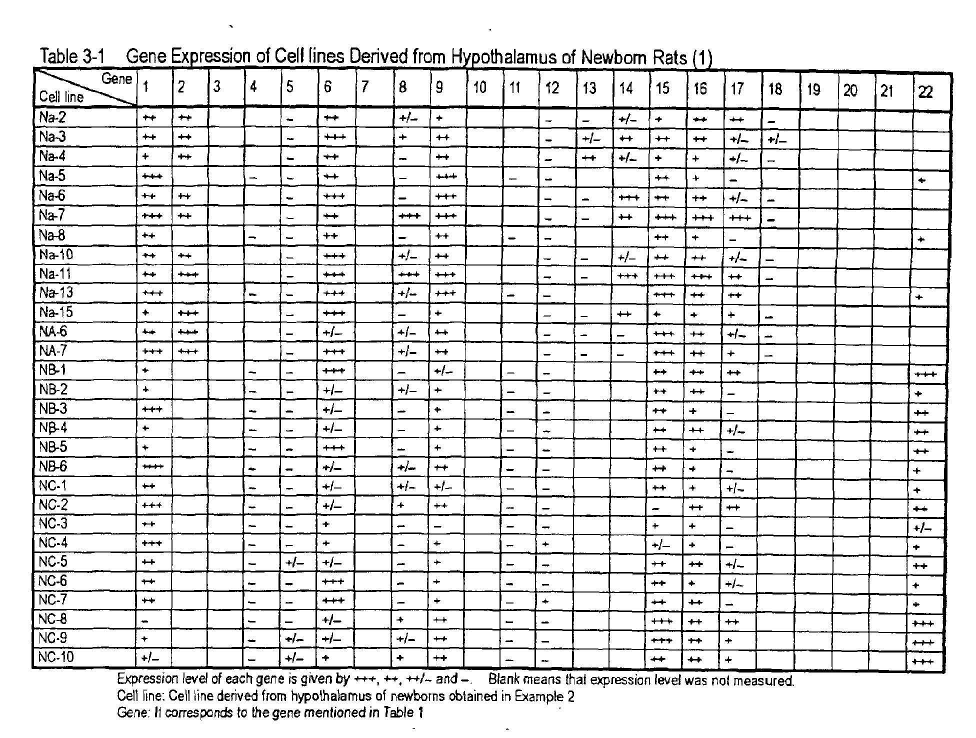

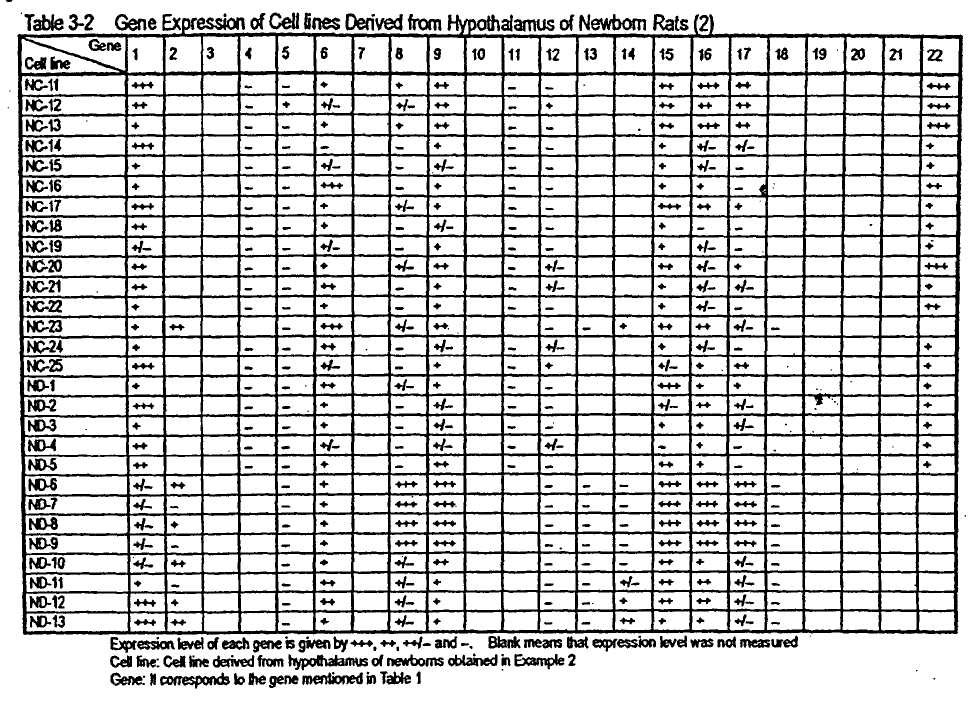

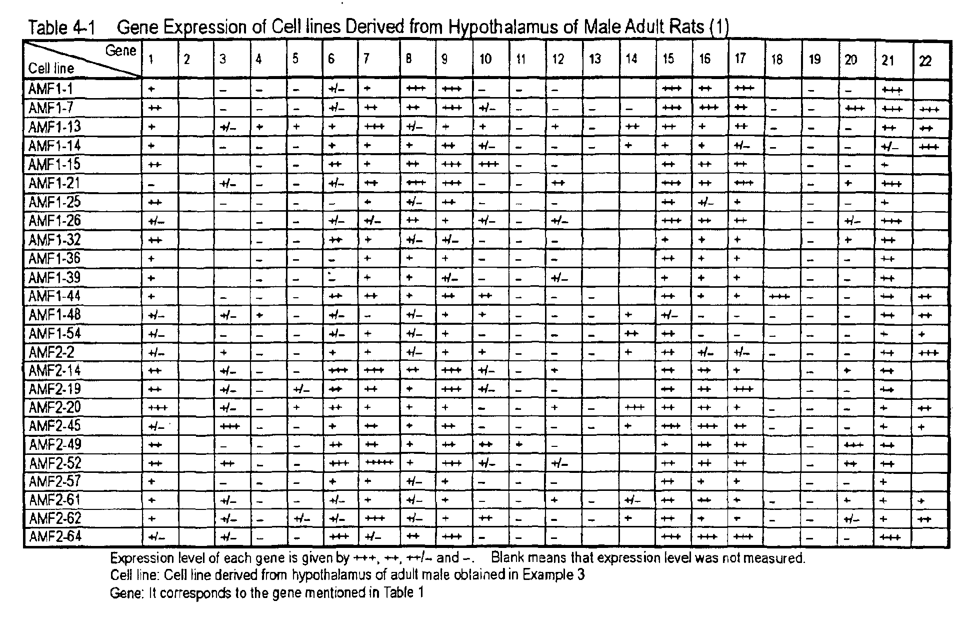

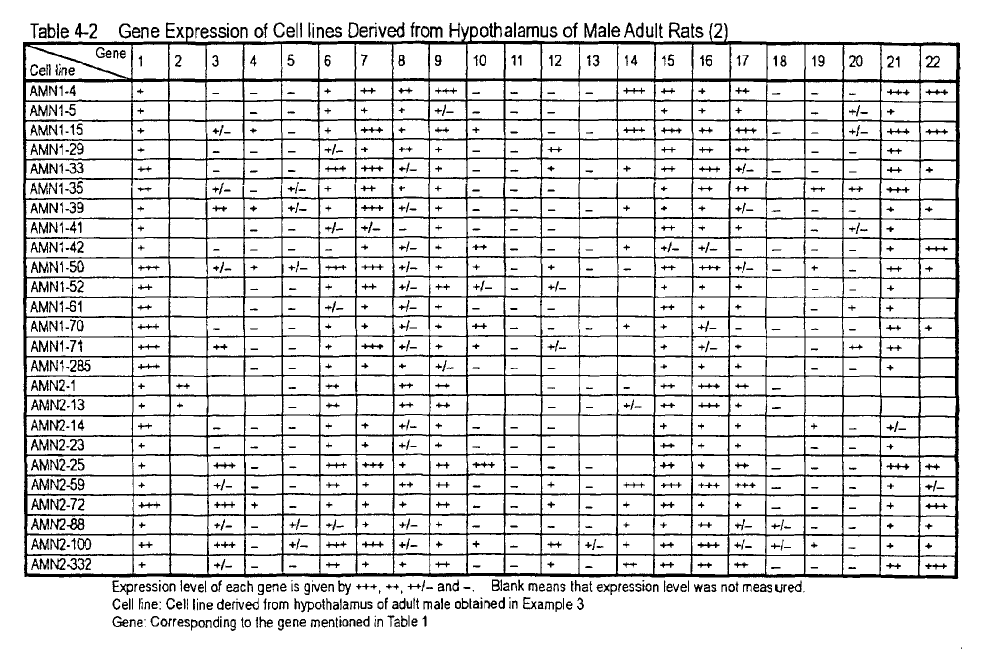

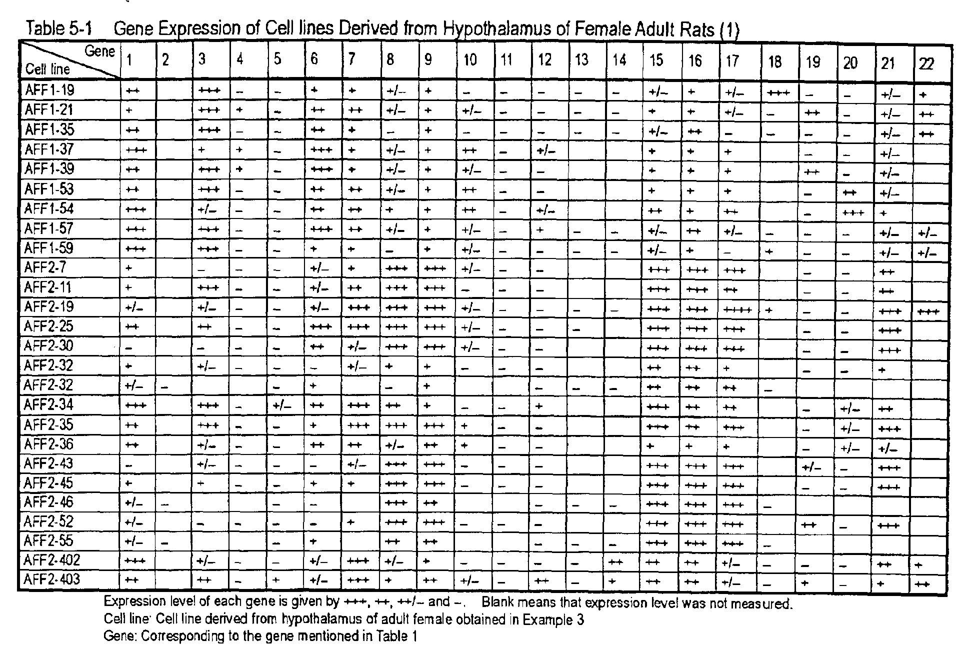

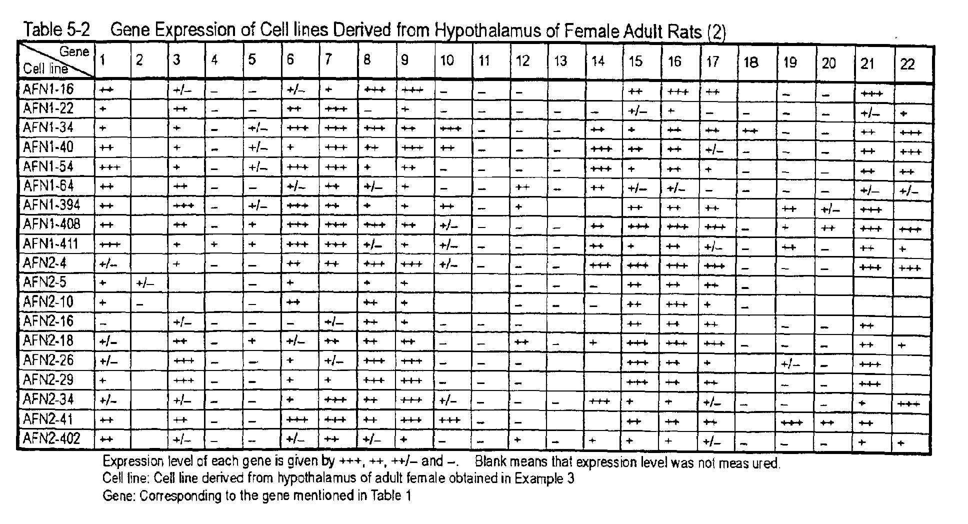

Leptin receptor (Ob-Rb) gene expressed in arcuate nucleus, paraventricular nucleus, lateral field, ventromedial nucleus and dorsomedial nucleus of hypothalamus was expressed in many cell lines. It has been believed that each of Ob-Rb-expressing cells existing in arcuate nucleus, paraventricular nucleus, lateral field, ventromedial nucleus and dorsomedial nucleus has different function and, since expression of the above mentioned genes of (1) to (22) differ depending upon cell lines, many different Ob-Rb gene-expressing cell lines were able to be obtained.

It is possible to presume what kind of cell the above-mentioned O6-Rb-expressing cell line is and from what nucleus it is derived using expression of gene which is characteristic to the nucleus as an index. With regard to cell line which does not express Ob-Rb, it is also possible to presume from what nucleus it is derived in a similar manner.

AMN2-25 expresses Ob-Rb gene, preproneuropeptide Y gene, preproghrelin gene, MC4R gene, NPY1R gene, NPY5R gene, ghrelin receptor gene, etc. and, therefore, that is presumed to be a NPY-producing cell derived from arcuate nucleus.

NC-12 expresses Ob-Rb gene, preproopiomelanocortin gene, CART gene, MC4R gene, NPY1R gene, NPYSR gene, etc. and, therefore, that is presumed to be an α-MSH-producing cell derived from arcuate nucleus.

AFN2-4, AFF2-19 and AFF2-25 abundantly express preprocorticotropin-releasing hormone gene and preprothyrotropin-releasing hormone gene and also express MC4R gene, NPY1R gene, NPY5R gene, etc. and, therefore, they are presumed to be cells derived from paraventricular nucleus. AFF2-25 expressed Ob-Rb gene too.

Cell lines such as AFF1-21, AFF1-37, AFF1-39, AFN1-411, AMF1-48 and AMN2-72 which express preproorexin gene, cell line (AMF2-14) which expresses melanin-aggregating hormone gene or cell lines (AFF1-19 and AMF1-44) which express NPFF2 gene are presumed to be cells derived from lateral field.

It is presumed that, in cell lines which express CRHR-1 gene or CRHR-2 gene, there are presumed to be a cell derived from ventromedial nucleus or dorsomedial nucleus.

### [Example 7] Analysis of expression of various gene products in cell line derived from hypothalamus

### (1) Analysis of expression of Ob-Rb

With regard to the immortalized cell line derived from hypothalamus wherein expression of Ob-Rb gene was confirmed in Example 6, expression of the gene product was analyzed by the following immunostaining method.

The immortalized cell line (mixture and single clones NC-6, NC-12 and AMN2-25) derived from hypothalamus wherein expression of Ob-Rb gene was confirmed in Example 6 was cultured according to the method mentioned in Example 1. After the cultivation, about 1 × 10³ cells were plated on 8 chambers coated with CELL-TAK (manufactured by Collaborative Biomedical Products), cultivation temperature was shifted from 33°C to 37°C and cultivation was carried out for 5 days.

After the cultivation, the medium was removed, 4% paraformaldehyde solution was added and the reaction was carried out at 4°C for 10 minutes to immobilize the cells. The immobilized cells were treated with a 0.3% Triton-X solution, an antibody against Ob-Rb (manufactured by Santa Cruz) diluted to an extent of 20-fold was added and the reaction was carried out at 37°C for 60 minutes. As a control, normal goat serum was used in place of the antibody against Ob-Rb. After the reaction, washing was conducted with PBS for one time, Alexa 488-labeled anti-goat IgG (manufactured by Molecular Probes) was added and the reaction was carried out at room temperature for 60 minutes. After the reaction, washing was conducted with PBS for one time, 1 µg/ml of Hoechst 33342 solution (manufactured by Calbiochem) was added and the reaction was carried out at room temperature for 5 minutes to stain the nuclei. After the staining, washing was conducted with PBS for one time, drying was done, mounting with a solution of a discoloration inhibitor was conducted and an observation under a f luorescence microscope was carried out.

As a result, a positive staining showing the expression of Ob-Rb was confirmed in any of mixture and single clones NC-6, NC-12 and AMN2-25. The result of immortalized cell line (mixture) derived from hypothalamus of adult female is shown in Fig. 5.

### (2) Analysis of expression of neuropeptide Y

Immortalized cell line (mixture or single clone) derived from hypothalamus where expression of preproneuropeptide Y (preproNPY) gene was confirmed in Example 6 was also analyzed in a similar manner using cyanine 3-labeled anti-goat IgG (manufactured by Jackson) diluted to an extent of 200-fold and an antibody against NPY (manufactured by Chemicon International) diluted to an extent of 200-fold in place of an antibody against Ob-Rb.

In the immortalized cell line derived from hypothalamus where expression of preproNPY gene was confirmed, positive staining showing the expression of NPY was confirmed. Result of immortalized cell line AMN2-25 derived from hypothalamus of adult male is shown in Fig. 6. It is able to be confirmed by the same immunostaining method that the gene where expression is confirmed in Example 6 is also expressed at the protein level.

### [Example 8] Investigation of leptin reactivity in hypothalamus-derived cell lines expressing leptin receptor

### (1) Analysis by immunostaining using an anti-STAT antibody

In a cell linewhere expression of a leptin receptor (Ob-Rb) protein is confirmed by the method mentioned in Example 7, it was analyzed by the following method whether a functional Ob-Rb is expressed. The following method is a method where the fact that a transcription factor STAT3 is phosphorylated upon stimulation by leptin and is transferred from cytoplasm to nucleus is utilized.

NC-6, NC-12 and AMN2-25 expressing leptin receptor (Ob-Rb) protein are cultured according to the method mentioned in Example 1. After the cultivation, about 1 × 10³ cells were plated on 8 chambers coated with CELL-TAK and cultured for 3 days where cultivation temperature was shifted from 33°C to 37°C. After the cultivation, the medium was removed and cultivation was carried out at 37°C for 17 hours after exchanging to a DMEM/F-12 (-FCS) medium. After the cultivation, exchange to a DMEM/F-12 (-FCS) medium was conducted. After 2 hours from the exchange of the medium, the cells were stimulated with 100 nmol/L of human leptin (manufactured by R&D Systems) at 37°C for 15 minutes.

After the stimulation, immunostaining was carried out using an anti-STAT antibody (manufactured by NEB) and a cyanine 3-labeled anti-rabbit IgG (secondary antibody) diluted to an extent of 200-fold. The immunostaining was carried out according to the manual of Phospho Plus Stat 3 (Tyr 705) Antibody Kit (NEB). Nuclear staining was also carried out by addition of a 1 µg/ml Hoechst 33342 solution (manufactured by Calbiochem) and by reacting at room temperature for 5 minutes. After the staining, drying was conducted, mounting with a discoloration inhibitor solution was done and an observation under a fluorescence microscope was carried out.

With regard to NC-6, NC-12 and AMN2-25 expressing Ob-Rb protein, cytoplasm was stained under the condition where there was no stimulation with leptin while, when stimulation with leptin was conducted, the area inside the nucleus was stained. Such a result show that NC-6, NC-12 and AMN2-25 express a functional OB-Rb. Result for the cell line NC-12 is shown in Fig. 7.

### (2) Analysis of change in expression of c-fos gene and SOCS-3 gene

Expression of c-fos gene and SOCS-3 gene, which has been known to be increased in hypothalamic cells by leptin stimulation, was analyzed by the following method utilizing a PCR whereby the leptin reactivity of the cell line, which was confirmed to be expressing a functional OB-Rb in the above (1), was confirmed from another view.

NC-6, NC-12 and AMN2-25 were cultured according to the method mentioned in Example 1. After the cultivation, about 2 × 10⁴ cells were plated on a 35-mm dish coated with poly-lysine and cultured out for 5 days by shifting the cultivation temperature from 33°C to 37°C. After the cultivation, the medium was removed and cultivation was carried out at 37°C for 17 hours by exchanging to a DMEM/F-12 (-FCS) medium containing no fetal calf serum. After the cultivation, exchange to a DMEM/F-12 (-FCS) medium was done.

After 2 hours from the exchange of the medium, 100 nmol/L of human leptin (manufactured by R&D Systems) was added. After incubating at 37°C for 0 minute, 30 minutes or 1 hour, total RNA was collected from each cell line using an RNeasy Mini Kit (manufactured by Qiagen). Single-stranded cDNA was synthesized from the total RNA according to the method mentioned in Example 6 and a PCR was carried out using the cDNA as a template. With regard to primers specific to c-fos gene, synthetic DNAs having the nucleotide sequences of SEQ ID NOS: 45 and 46 were used while, with regard to primers specific to SOCS-3 gene, synthetic DNAs having the nucleotide sequences of SEQ ID NOS: 47 and 48 were used. Expression of G3PDH was investigated as well.

Although there was no change in expression level of G3PDH gene in any of NC-6, NC-12 and AMN2-25, it was shown that the expression was increased by stimulation with leptin in the case of c-fos gene and SOCS-3 gene. Such a result also shows that NC-6, NC-12 and AMN2-25 express a functional OB-Rb. The result further shows that, by screening of genes which expression is changed by leptin stimulation of the cell line where a functional OB-Rb expression was confirmed by the above method, it is possible to screen leptin-response genes.

### [Example 9] Construction of peptide expression system using a cell line derived from hypothalamus as a host

### (1) Construction of an expression plasmid of preprovasopressin gene

Preprovasopressin gene was obtained by a PCR using a single-stranded cDNA prepared from mRNA (1 µg; manufactured by Clontech) derived from human thymus as a template and two kinds of synthetic DNAs having sequences mentioned in SEQ ID NO: 49 and SEQ ID NO: 50 as primers specific to preprovasopressin gene. To each of the above primers, HindIII site and NotI site are introduced respectively. After the above PCR-amplified fragment was cleaved by HindIII and NotI, a HindIII-NotI fragment wasobtained. After plasmid pCDM8 (manufactured by Invitrogen) was cleaved by HindIII and NotI, a HindIII-NotI fragment was obtained. pCDM8-VP was constructed by ligating the above-mentioned HindIII-NotI fragment derived from the above PCR amplification fragment and the HindIII-NotI fragment derived from pCDM8.

After pCDM8-VP was cleaved with HindIII and NotI, a HindIII-NotI fragment containing preprovasopressin gene was obtained. The HindIII-NotI fragment was inserted between HindIII-NotI of expression plasmid pcDNAI/Amp (manufactured by Invitrogen) whereupon pcDNAI/Amp-VP was constructed.

In addition, the HindIII-NotI fragment was inserted between HindIII-NotI of expression plasmid pcDNA3.1(+) (manufactured by Invitrogen) whereupon pcDNA3.1-VP was constructed.

### (2) Construction of an expression plasmid of preprocorticotropin-releasing hormone gene

Preprocorticotropin-releasing hormone gene was obtained by a PCR using a single-stranded cDNA prepared from mRNA (1 µg; manufactured by Clontech) derived from human hypothalamus as a template and two kinds of synthetic DNAs having sequences mentioned in SEQ ID NO: 51 and SEQ ID NO: 52 as primers specific to preprocorticotropin-releasing hormone gene. To each of the above primers, HindIII site and NotI site are introduced respectively. After the above PCR-amplified fragment was cleaved by HindIII and NotI, a HindIII-NotI fragmentwas obtained. After plasmid pCDM8 was cleaved by HindIII and NotI, a HindIII-NotI fragment was obtained. pCDM8-CRF was constructed by ligating the above-mentioned HindIII-NotI fragment derived from the above PCR amplification fragment and the HindIII-NotI fragment derived from pCDM8.

After pCDM8-CRF was cleaved with HindIII and NotI, a HindIII-NotI fragment comprising preprocorticotropin-releasing hormone gene was obtained. The HindIII-NotI fragment was inserted between HindIII-NotI of expression plasmid pcDNAI/Amp (manufactured by Invitrogen) whereupon pcDNAI/Amp-CRF was constructed.

In addition, the HindIII-NotI fragment was inserted between HindIII-NotI of expression plasmid pcDNA3.1(+) (manufactured by Invitrogen) whereupon pcDNA3.1-CRF was constructed.

### (3) Expression of a peptide using a single clone AFF2-34 as a host

The immortalized cell line (single clone AFF2-34) derived from hypothalamus of adult male rat obtained in Example 3 was cultured according to the method mentioned in Example 1. After the cultivation, about 1 × 10⁴ cells were plated on one well of a 96-well dish coated with poly-lysine and cultured at 33°C for one day.

After the cultivation, preprovasopressin expression plasmid (pCDM8-VP), preprocorticotropin-releasing hormone expression plasmid (pCDM8-CRF) or control plasmid (pCDM8) was transfected into AFF2-34 using Lipofectamine plus (manufactured by Gibco; comprising a Lipofectamine reagent and a plus reagent). The gene transfection was carried out according to a direction attached to the lipofactamine plus using 0.5 µg of plasmid, 0.5 µl of the Lipofectamine reagent and 1 µl of the plus reagent. AFF2-34 cell line transfected with the gene was cultured in 100 µl of a DMEM/F-12 (FCS) medium at 33°C for one day. After the cultivation, the medium was exchanged to 100 µl of a DMEM/F-12 (N2) medium and cultivation was further carried out at 33°C for 2 days.

After the cultivation, the culture supernatant was collected and a vasopressin activity and a corticotropin-releasing hormone activity in the supernatant were measured. The vasopressin activity was measured using assay cells constructed in (1) of the following Example 21. The corticotropin-releasing hormone activity was measured using assay cells constructed in (2) of Example 21.

In the culture supernatant derived from the cells into which preprovasopressin-expression plasmid was transfected, a vasopressin activity was detected while, in the culture supernatant derived from the cells to which preprocorticotropin-releasing hormone-expression plasmid was transfected, a corticotropin-releasing hormone activity was detected. In the culture supernatant derived from the cells to which control plasmidwas transfected, any of those activities was not detected.

From the above result, it has now been apparent that an active peptide is able to be produced when a precursor gene of active peptide is expressed using an immortalized cell line (single clone AFF2-34) derived from hypothalamus as a host.

### (4) Expression of a peptide using a single clone AFF2-404 as a host

The same experiment as in the above (3) was carried out using an immortalized cell line (single clone AFF2-404) derived from hypothalamus of male adult rat obtained in Example 3. With regard to the transfection of the gene to AFF2-404 however, that was carried out using 0.2 µg of plasmid and 0.5 µl of lipofectAMINE 2000 (manufactured by Gibco).

It has now been apparent that, even when AFF-2-404 is used as a host, an active peptide is able to be produced by expressing a precursor gene of active peptide as same as in the above.

### (5) Method for the selection of cell lines suitable for the production of active peptides

When immortalized cell lines (a mixture) derived from hypothalamus of male adult rat obtained in Example 3 were used and subjected to making into single clone again, 708 single clones were newly obtained. According to the method of the above (3), vasopressin activity of the cell line where preprovasopressin-expression plasmid (pcDNA3.1-VP) was transfected into single clone was measured.

Six kinds of single clones (AF2-C3, AF2-C11, AF2-E2, AF2-E11, AF2-F7 and AF2-G5) where activity of vasopressin was high were obtained as good cell lines.

### (6) Construction of an expression cloning systemof active peptide precursor genes

The single clones AF2-E2 and AF2-G5 obtained in the above (5) were cultured according to the method mentioned in Example 1. After the cultivation, about 2 × 10⁴ cells each were plated on each well of a 96-well dish coated with poly-lysine and cultured at 33°C for one day. After the cultivation, each of the plasmids of the following (i) to (iii) was transfected using a Lipofectamine plus (manufactured by Gibco). Transfection of the gene was carried out according to the direction attached to Lipofectamine plus using 0.2 µg of plasmid, 0.5 µl of a lipofectamine reagent and 1 µl of a plus reagent.
(i) Control plasmid [pcDNA3.1(+)]
(ii) Preprovasopressin expression plasmid (pcDNA3.1-VP)
(iii) Mixed plasmid where pcDNA3.1-VP is diluted with pcDNA3.1(+) to an extent of one-tenth

After the transfection, each cell was cultured in 100 µl of a DMEM/F-12 (FCS) medium at 33°C for one day. After the cultivation, the medium was exchanged to 100 µl of a DMEM/F-12 (N2) medium and cultivation was carried out at 33°C for 2 days more. Assay cells constructed in (1) of Example 21 were layered on the above-cultured cells and, after that, 1 mmol/L of ATP was added. After 6 hours from the addition, coelenterazine h (Molecular Probes) (final concentration: 250 nmol/L) was added and activity of a reporter (luciferase of *Renilla reniformis*) was measured using a VIM camera (Argus-50/2D luminometer/MP: manufactured by Hamamatsu Photonics).

When preprovasopressin expression plasmid pcDNA3.1-VP (not diluted) was transfected, activity was detected in each cell while, when only vector [pcDNA3.1(+)] was transfected, no activity was detected.

In each cell, activity was detected even when the expression plasmid was diluted to an extent of one-tenth using avector. Since activity is able to be detected even when diluted to an extent of one-tenth, it is now apparent that, when a suitable hypothalamic cell line is selected and used as a host, at least ten kinds of genes or libraries are transfected to a host cell at the same time whereupon the aimed activity is able to be measured.

In addition, when a gene encoding a random peptide is expressed using various cell lines suitable for the production of active peptides as hosts, various peptides are able to be efficiently produced. For example, when the region of the gene encoding an active peptide of any active peptide precursor gene is substituted with a gene encoding a random peptide, it is possible to produce a gene which expresses a random peptide.

### [Example 10] Identification of cell lines derived from hypothalamus reacting with sulfonylurea

### (1) Detection of cell lines reacting with sulfonylurea using immortalized cell lines (a mixture) derived from hypothalamus

The immortalized cell lines (a mixture) derived from hypothalamus of female adult rat obtained in Example 3 were cultured by the method mentioned in Example 1. After the cultivation, 5 µmol/L of Fura-2AM was added to the cells and cultivation was carried out at 37°C for 1 hour. The resulting cells were washed with an HBSS buffer (manufactured by Gibco) twice and the cells were floated. Sulfonylurea (Tolbutamide; manufactured by Alexis) was added to about 1 × 10⁶ cell to make its final concentration 0.5 mmol/L and a rise in Ca²⁺ concentration in the cells was checked. Measurement of the Ca²⁺ concentration in the cells was conducted using a CAF-110 instrument (manufactured by Nippon Bunko).

As a result, it was found that cell lines which reacted with sulfonylurea were present in the above-mentioned immortalized cell lines (a mixture). The result is shown in Fig. 8 (a).

### (2) Identification of a cell line reacting with sulfonylurea using an immortalized cell line (single clone) derived from hypothalamus

As same as in the above (1), reactivity of an immortalized cell line (single clone AMN2-25) derived from hypothalamus of male adult rat obtained in Example 3 with sulfonylurea was tested. As shown in Fig. 8 (b), it was found that AMN2-25 also reacted with sulfonylurea whereupon the Ca²⁺ concentration in the cell increased. The result shows that the single clone AMN2-25 expresses a sulfonylurea receptor.

It is possible by the above method to identify or select an immortalized cell line (single clone) derived from hypothalamus reacting with sulfonylurea. The above result also shows that a substance reacting with cell line derived from hypothalamus is able to be screened when the immortalized cell lines (a mixture or single clone) derived from hypothalamus of fetus, newborn, male adult or female adult of rat obtained in Examples 1 to 3 is contacted to any substances (such as proteins, peptides, compounds, drugs, cells, culture supernatant of cells or cell extracts) to search whether a cell line derived from hypothalamus reacting with such a substance is present.

### [Example 11] Establishment of cell lines derived from Langerhans islets of pancreas

Pancreas was excised according to a known method [Ryozaburo Takagi, Kazuyuki Hamaguchi and Junko Ono: "New Lectures on Biochemical Experiments", volume 18, Cell Culturing Technique, pages 154-159] from a transgenic rat (purchased from YS New Technology Institute, Inc.) transfected with the large T antigen gene of a temperature-sensitive mutant SV40tsA58, which was prepared by the method mentioned in Exp. Anim. 48, 255 (1999). Thus, a Hanks' solution containing 0.7 mg/ml of collagenase (manufactured by Wako Pure Chemical) was infused into pancreas of rat and the pancreas was excised.

The pancreas was placed in a 50-ml tube, shaken for 30 minutes in a thermostat bath of 37°C, 8 ml of a Hanks' solution [137 mmol/L of NaCl, 5.4 mmol/L of KCl, 0.3 mmol/L of Na₂HPO₄, 0.4 mmol/L of KH₂PO₄, 2.8 mmol/L of glucose, 0.8 mmol/L of MgSO₄, 1.3 mmol/L of CaCl₂, 10 mmol/L of HEPES/NaOH (pH 7.4) and 5 mmol/L of sodiumpyruvate] containing ice-cold 0.5% bovine serum albumin [manufactured by Sigma: hereinafter, abbreviated to as BSA] was added thereto and the mixture was vigorously shaken to loosen the pancreatic tissues.

The pancreatic tissues were centrifuged for 2 minutes at 1,200 rpm using a centrifugal separator (himacSCT-5BA) manufactured by Hitachi to prepare a precipitate. Ice-cold Hanks' solution (40 ml) was added to the precipitate to suspend and the suspension was centrifuged at 1,200 rpm for 2 minutes to prepare a precipitate. This operation was carried out until there was no turbidity in the supernatant after centrifugal separation.

A Hanks' solution (40 ml) was added to the resulting precipitate to suspend and the suspension was filtered using a tea strainer made of metal (manufactured by Tiger Crown). Ice-cold Hanks' solution (20 ml) was added to the resulting filtrate and the mixture was centrifuged at 1,200 rpm for 2 minutes to collect a precipitate. This operation was repeated for two times more.

To the resulting precipitate was added 10 ml of Histopaque 1077 (manufactured by Sigma) to suspend and each 5 ml thereof was placed in a 15-ml tube (Falcon). A Hanks' solution (5 ml) of room temperature was gently layered on each of the dispensed suspension using a Pasteur pipette and centrifuged for 15 minutes under the condition of room temperature and 2,300 rpm.

Langerhans islets gathering at the boundary was collected using a Pasteur pipette, 10 ml of a Hanks' solution containing 0.5% BSA was added and the mixture was centrifuged for 2 minutes at 1,200 rpm to collect a precipitate. A Hanks' solution (10 ml) containing 0.5% BSA was added to the precipitate and the mixture was centrifuged at 1,200 rpm for 2 minutes to collect a precipitate (Langerhans islets). The operation was carried out for one time more.

The resulting Langerhans islets was suspended in 10 ml of a Hanks' solution containing ice-cold 0.5% BSA and centrifuged at 800 rpm for 30 seconds to give a precipitate. The precipitate was suspended in 10 ml of a balanced salt solution not containing Ca²⁺ and Mg²⁺ [8 g/l of NaCl, 0.3 g/l of KCl, 0.05 g of NaH₂PO₄.H₂O, 0.025 g/l of KH₂PO₄, 1 g/l of NaHCO₃ and 2 g/l of glucose: hereinafter, abbreviated as a CMF solution] and the mixture was centrifuged at 800 rpm for 1 minute to give a precipitate.

To the precipitate was added 10 ml of a CMF solution containing 0.02% EDTA and, after the mixture was slowly stirred at room temperature for 5 minutes, it was centrifuged at 1,200 rpm for 1 minute to give a precipitate. The precipitate was suspended in 10 ml of a CMF solution and centrifuged at 1,200 rpm for 1 minute to give a precipitate. The precipitate was suspended in a CMF solution (10 ml) containing 3.3 mg/ml dispase (manufactured by Sanko Junyaku), slowly stirred at room temperature for 15 minutes and centrifuged at 1,200 rpm for 1 minute to give a precipitate (cells derived from Langerhans islets).

The resulting cells derived from Langerhans islets were washed with 10 ml of a CMF solution for three times, divided into two and cultured in a 100-mm dish (manufactured by Becton-Dickinson) coated with poly-lysine under the conditions of 33°C, 5% of carbon dioxide gas concentration and 100% of humidity using two kinds of media (each 10 ml) , i.e. (1) a DMEM medium to which 10% of fetal bovine serum, 50 U/ml of penicillin and 50 µg/ml of streptomycin were added (manufactured by Sigma; product No. D-5796) [hereinafter, referred to as DMEM (D-5796) medium; the medium contains 25 mmol/L of glucose] or (2) an RPMI1640 medium to which 10% of fetal bovine serum, 50 U/ml of penicillin and 50 µg/ml of streptomycin were added (manufactured by Nissui Seiyaku: product No. 05918) [hereinafter, referred to as RPMI1640 (05918) medium].

At the stage where the cells grew and saturated, they were subcultured in a 100-mm dish coated with poly-lysine using each medium so as to make the cell density to the culture surface about 70%. Cultivation was continued for about 3 months in each medium to give a mixture of immortalized cell lines derived from Langerhans islets. The resulting mixture of immortalized cell lines derived from Langerhans islets was dispensed into five tubes so that each tube contained about 5 × 10⁶ cells and then cryopreserved by a known method.

The cryopreserved cells were separated into single clones by the following colony formation method. Thus, 100 or 500 cells were plated on a 100-mm dish (manufactured by Becton-Dickinson) coated with poly-lysine and cultured using each medium until colonies were formed. After the cultivation, each colony was peeled off using the top of a chip under the microscopic observation, at the same time, it was sucked into a chip to recover the cells. The recovered cells derived from each colony were plated on a 24-well plate coated with poly-lysine and cultured in each medium. At the stage where the cells grew and saturated, they were successively scaled up to a 6-well plate and a 100-mm dish (both being coated with poly-lysine).

As a result of the above colony formation method, 117 and 144 cell lines made into single clones were obtained using an RPMI1640 (05918) medium and a DMEM (D-5796) medium, respectively. The cell lines obtained using the RPMI1640 (05918) medium were named R-1 to R-45, M-1 to M-63 and F-1 to F-9 while those obtained using the DMEM (D-5796) medium were named D-1 to D-72 and D2-1 to D2-72. Each of the resulting cell lines was cryopreserved by known methods. Doubling time of the resulting cell lines was 36 to 72 hours. Even after one year or more from the establishment, there was no significant change in the growing property of the cell lines.

### [Example 12] Analysis of expression of various genes in cell lines derived from pancreatic Langerhans islets

As for immortalized cell lines (a mixture or single clone) derived from Langerhans islets obtained in Example 11, expression of various kinds of genes expressed in Langerhans islets, which are shown in Table 6, was analyzed by the following method.

The cell lines obtained in Example 11 were cultured according to the method mentioned in Example 11. After the cultivation, about 1 × 10⁵ cells were plated on a 100-mm dish coated with poly-lysine and cultured for 5 days at the cultivation temperature of 33°C.

After the cultivation, total RNA was prepared from each cell line using an RNeasy Mini Kit (manufactured by Qiagen). The total RNA (5 µg) was used and single-stranded cDNA was synthesized using a Superscript First-Strand Synthesis System for RT-PCR (manufactured by Gibco). The single-stranded cDNA was diluted with water to an extent of 50-fold and used as a template for a PCR.

To the single-stranded cDNA (5 µl) were added the gene-specific primers shown in Table 6 (20 pmol each), 1.6 µl of 2.5 mmol/L dNTP mixed solution, 1 µl of DMSO, 0.1 µl of 5 units/µl of Recombinant Ex Taq DNA Polymerase (manufactured by Takara Shuzo) and 2 µl of 10 × reaction buffer (manufactured by Takara Shuzo) and then sterilized water was added thereto to make the total volume 20 µl. The solution prepared as such was used and subjected to a PCR under the following condition. Using a thermal cycler DNA Engine (manufactured by MJ Research), after a thermal treatment at 95°C for 5 minutes, a reaction comprising at 94°C for 1 minute, at 60, 65 or 67°C (reaction temperature for each gene amplification is shown in Table 6) for 1 minute and at 72°C for 1 minute was carried out for 25 to 35 cycles.

A part (8 µl) of the reaction solution was subjected to an agarose gel electrophoresis and the gel was stained with SYBR Green I nucleic acid stain (Molecular Probes). The pattern of the amplified DNA fragment was analyzed by a Fluor Image SI (manufactured by Molecular Dynamics) whereupon the amount of the amplified DNA fragment was measured.

Quantitative determination of the transcript of each gene was carried out by a semi-quantitative PCR method according to a common method [PCR Protocols, Academic Press (1990)]. The transcript of rat glyceraldehyde-3-phosphate dehydrogenase (hereinafter, abbreviated as G3PDH) was quantified at the same time whereupon it was confirmed that there was no significant difference in the mRNA level among the cells and also in conversion efficiency from mRNA to single-stranded cDNA by a reverse transcriptase among the samples. Quantitative determination of the transcript of G3PDH was carried out by a quantitative PCR method according to a common method [*Proc. Natl. Acad. Sci. USA,* 87, 2725 (1990) ; *J. Biol. Chem.,* 269, 14730 (1994); Japanese Published Unexamined Patent Application No. 181,759/1994].

**Table 6**

| No. | Gene | Primer (SEQ ID NO) | Reaction Temp (°C) |
|---|---|---|---|
| 1 | Preproinsulin | 53, 54 | 60 |
| 2 | Preproglucagon | 55, 56 | 60 |
| 3 | Preprosomatostatin | 57, 58 | 65 |
| 4 | Prepropancreatic polypeptide | 59, 60 | 60 |
| 5 | PC 1 | 61, 62 | 65 |
| 6 | PC 2 | 63, 64 | 65 |
| 7 | GLP-1 receptor (GLP-1R) | 65, 66 | 67 |
| 8 | PDX1 | 67, 68 | 65 |
| 9 | Neuro D | 75, 76 | 60 |
| 10 | Pax 4 | 69, 70 | 65 |
| 11 | Pax 6 | 71, 72 | 65 |
| 12 | Neurogenin 3 | 73, 74 | 65 |
| 13 | Nkx 2.2 | 77, 78 | 65 |
| 14 | Nkx 6.1 | 79, 80 | 65 |
| 15 | Glucokinase | 81, 82 | 65 |
| 16 | Type 2 glucose transporter | 83, 84 | 65 |
| 17 | beta-Cellulin | 85, 86 | 65 |

Among the analyzed results, expression levels of each gene for preproinsulin (referred to as Ins in the tables), preproglucagon (referred to as Glu in the tables), preprosomatostatin (referred to as SM in the tables), prepropancreatic polypeptide (referred to as PP in the tables) , PC1, PC2 , GLP-1 receptors (GLP-1R), PDX1 and neuro D in each of cell lines of D-1 to D-72, D2-1 to D2-72 and R-1 to R-45 were shown as +++, ++, +, +/- and - in Table 7-1 to Table 7-6. It was demonstrated that various cell lines with different expression pattern of the above (1) to (17) genes were obtained.

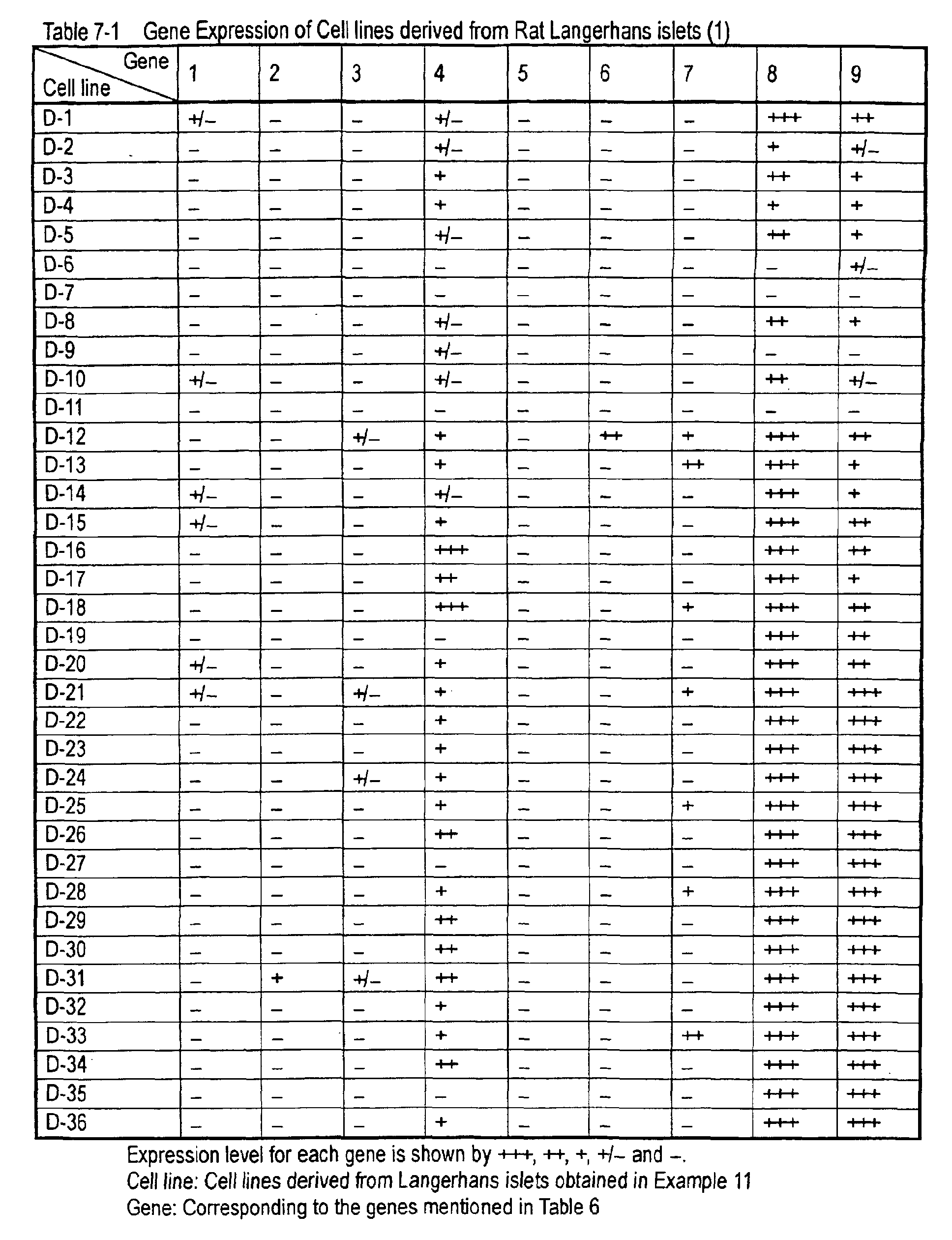

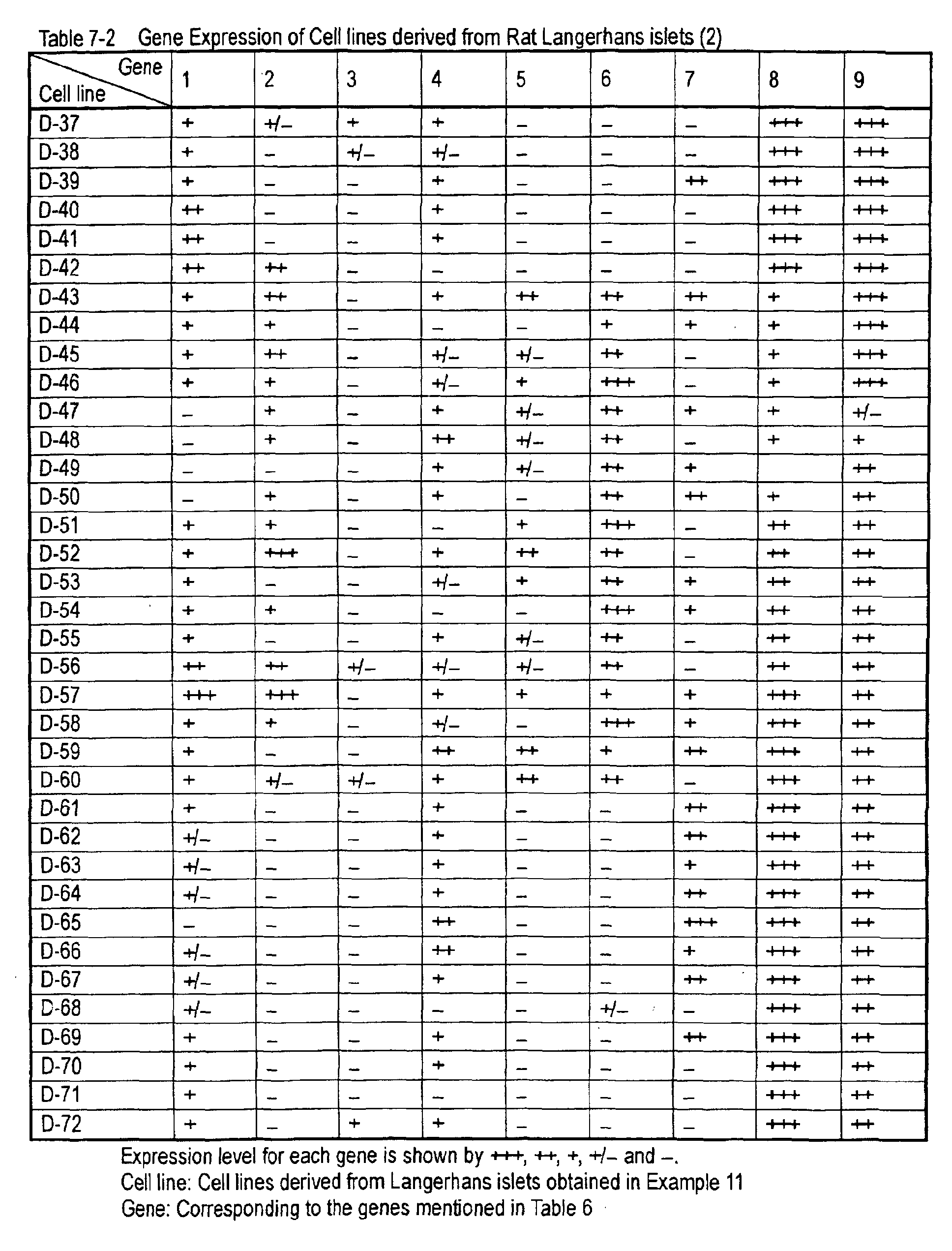

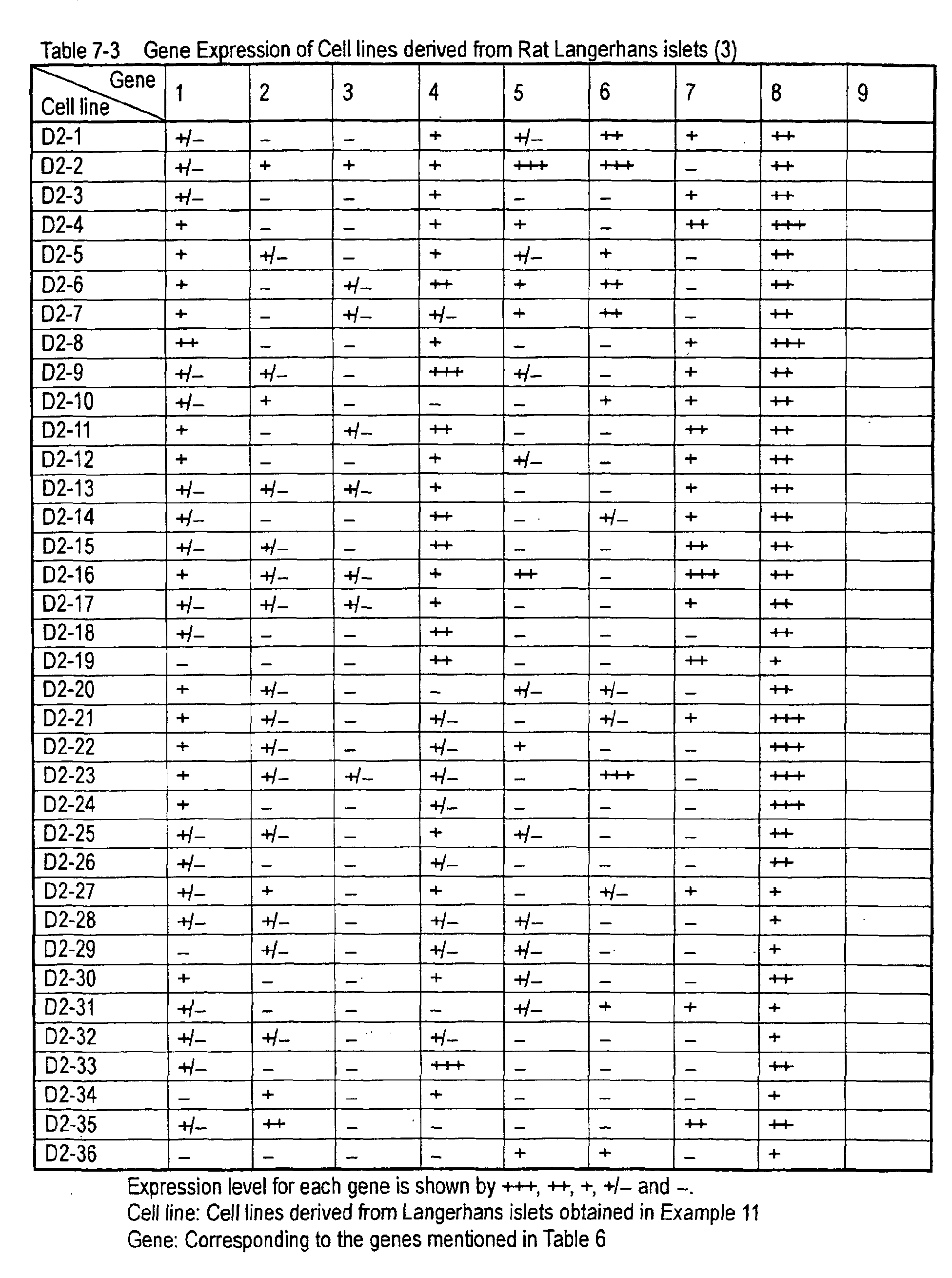

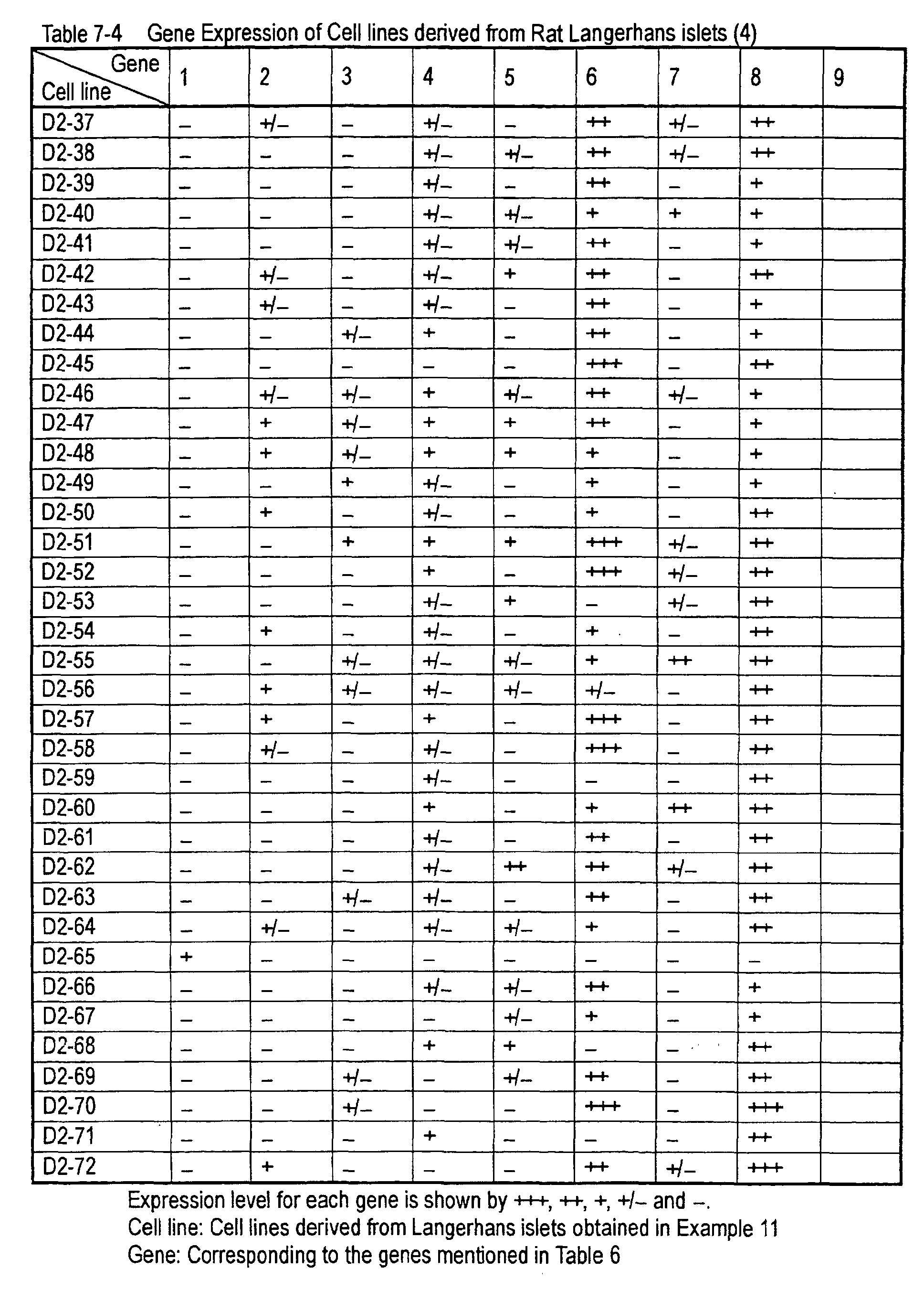

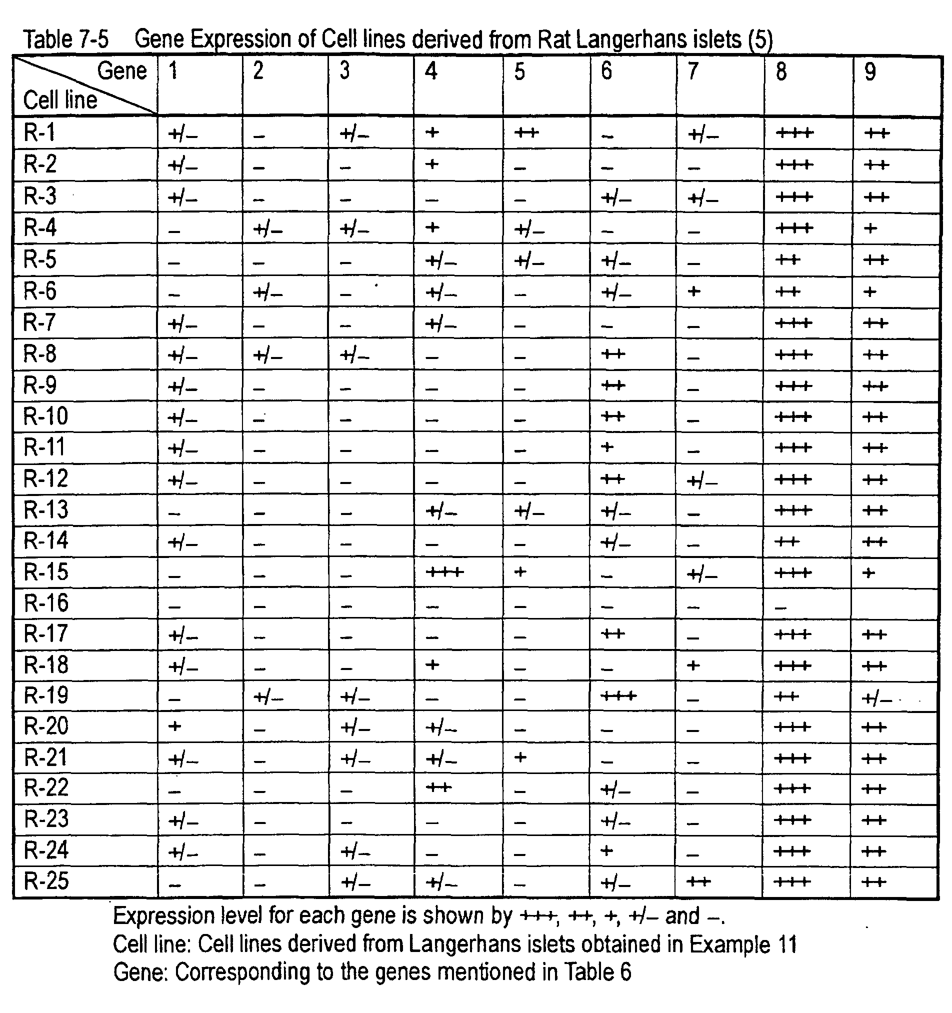

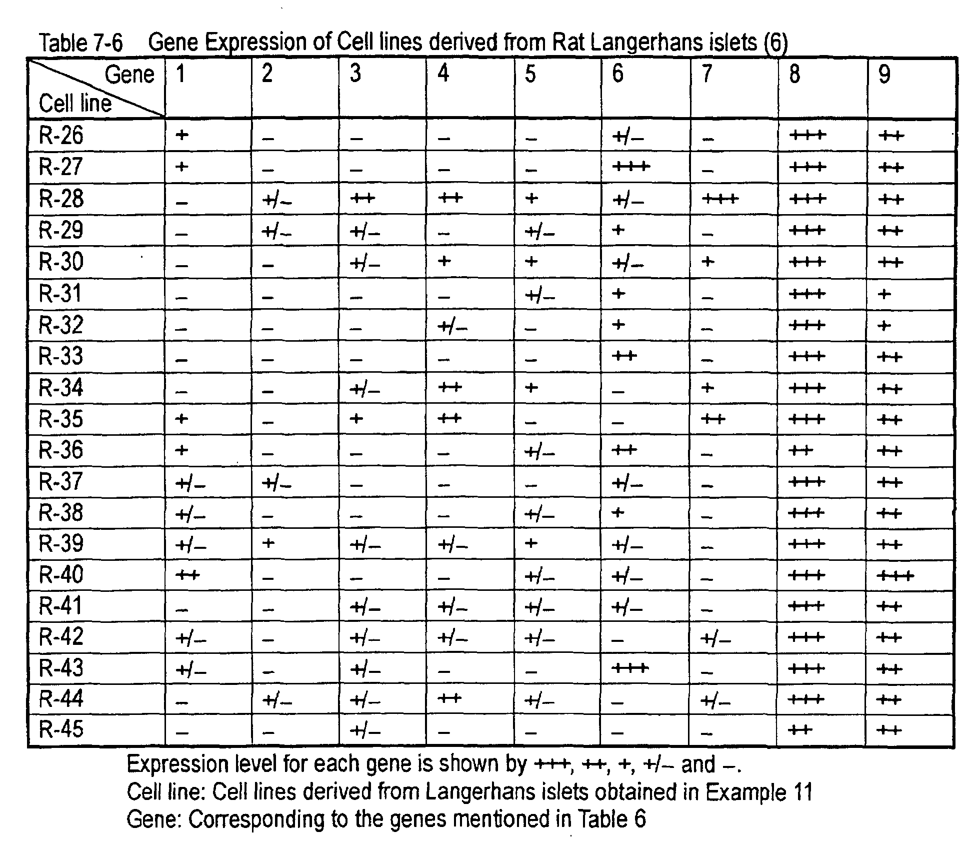

### [Example 13] Analysis of expression of various gene products in cell lines derived fromLangerhans islets of pancreas

### (1) Analysis of expression of insulin and preparing insulin-expressing cells

A mixture (about 1 × 10³ cells) of immortalized cell lines mentioned in Example 11 obtained using an RPMI 1640 (05918) medium was plated on an 8-chamber Lab-Tek chamber slide (manufactured by Nalge Nunc International) coated with CELL-TAK and cultured at 33°C for 2 days in an RPMI 1640 (05918) medium.

After the cultivation, the medium was removed, a 4% paraformaldehyde solution was added and reaction was carried out at 4°C for 10 minutes to immobilize the cells. After the immobilized cells were treated with a 0.3% Triton X-100 solution, PBS containing 1% of fetal bovine serum was added and the mixture was allowed to stand at roomtemperature for 30 minutes to conduct a blocking. After the blocking, an anti-insulin antibody (manufactured by Sigma) diluted to an extent of 1,000-fold was added and reaction was carried out at 37°C for 60 minutes. After the reaction, it was washed with PBS containing 1% of fetal bovine serum for three times, Alexa 488-labeled anti-goat IgG (manufactured by Molecular Probes) diluted to an extent of 200-fold was added and reaction was carried out at room temperature for 60 minutes. After the reaction, it was washed with PBS for two times, 1 µg/ml Hoechst 33342 solution (manufactured by Calbiochem) was added and reaction was carried out at room temperature for 5 minutes to stain the nuclei.

After staining, it was washed with PBS for two times, dried, mounted with a solution of a discoloration inhibitor and observed under a fluorescence microscope. As a result, it was found that cells expressing insulin were present.

Presence of insulin-expressing cells was also confirmed by the following method for each single clone of immortalized cell lines mentioned in Example 11 obtained using an RPMI 1640 (05918) medium.

Each single clone (about 1 × 10⁴ cells) mentioned in Example 11 obtained using an RPMI 1640 (05918) medium was plated on one well of a 96-well plate and cultured using an RPMI 1640 (05918) medium at 33°C for 2 days. After the cultivation, immunostaining using an anti-insulin antibody and staining of the nucleus using Hoechst 33342 were carried out by the same method as above. As a result, it was found that single clones M-8, M-9 and M-15 express insulin.

### (2) Analysis of expression of processing enzyme PC1 and preparing PC1-expressing cells

Each single clone (about 1 × 10⁴ cells) of the immortalized cell lines mentioned in Example 11 obtained using an RPMI 1640 (05918) medium was plated on a well of a 96-well plate and cultured at 33°C for 2 days using an RPMI 1640 (05918) medium.

After the cultivation, immunostaining using an anti-PC1 antibody and staining of nucleus usingHoechst 33342 were carried out according to the above-mentioned method (1). With regard to the antibody, an anti-PC1 antibody (manufactured by Chemicon International) diluted to an extent of 50-fold was used. As a result, it was found that single clones M-9 and M-33 express PC1.

### (3) Analysis of expression of processing enzyme PC2 and preparing PC2-expressing cells

Each single clone (about 1 × 10⁴ cells) of the immortalized cell lines mentioned in Example 11 obtained using an RPMI 1640 medium was plated on a well of a 96-well plate and cultured at 33°C for 2 days using an RPMI medium.

After the cultivation, immunostaining using an anti-PC1 antibody and staining of nucleus using Hoechst 33342 were carried out according to the above-mentioned method (1). With regard to the antibody, an anti-PC2 antibody (manufactured by Chemicon International) diluted to an extent of 50-fold was used. As a result, it was found that many cells such as single clones M-9, M-15, F-1 to F-9, etc. express PC2.

### [Example 14] Identification of cell line derived from Langerhans islets reacting with sulfonylurea

About 5 × 10⁶ cells of single clones (M-6, M-9, M-15, M-19, M-20, M-33, M-43, F-1 and F-9) of immortalized cell lines mentioned in Example 11 obtained using an RPMI 1640 (05918) medium were plated on a 60-mm dish and cultured at 33°C for 2 days using an RPMI 1640 (05918) medium.

After the cultivation, 5 µmol/L of Fura-2AM was added to those cells and cultivation was carried out at 37°C for 1 hour. After the cultivation, it was washed with an HBSS buffer (manufactured by Gibco) for two times and the cells were floated. Sulfonylurea (Tolbutamide, manufactured by Alexis) was added to about 1 × 10⁶ of the cells so as to make the final concentration 0.5 mmol/L whereupon an increase in Ca²⁺ concentration in the cells was checked. Measurement of the Ca²⁺ concentration in the cells was carried out using a CAF-100 instrument (manufactured by Nippon Bunko).

As a result, it was found that the Ca²⁺ concentration in the cells increased in response to sulfonylurea in the all single clones checked. The result shows that the all single clones checked express sulfonylurea receptor.

The above result also shows that substances reacting with a cell line derived fromLangerhans islets are able to be screened by contacting a immortalized cell line (a mixture or single clone) derived from rat Langerhans islets obtained in Example 11 with any substance (a protein, a peptide, a compound, an drug, a cell, a culture supernatant of cells, a cell extract or the like), and checking whether a cell line derived from Langerhans islets reacting with the substance is present.

### [Example 15] Investigation of culturing method for cell lines derived from pancreatic Langerhans islets

### (1) Investigation of medium

Single clones (18 clones) confirmed to express PC2 in (3) of Example 13 were cultured in an RPMI 1640 (05918) medium at 33°C and, after that, about 1 × 10⁴ cells were plated on a well of a 96-well plate and cultured at 33°C for 2 weeks in RPMI 1640 (05918) medium or DMEM (D-5796). After the cultivation, expression levels of PC1 and PC2 were investigated by the methods mentioned in (2) and (3) of Example 13.

As a result, it was found that, in many cell lines, growth became slow and expression of PC1 and PC2 increased when cultured in DMEM (D-5796). The result shows that, when the cells cultured in RPMI 1640 (05918) medium are cultured for 2 weeks by substituting with a DMEM (D-5796), differentiation characteristic of the above cell lines derived from Langerhans islets is able to be controlled. Fig. 9 shows the result obtained by the use of the cell line F-8.

### (2) Investigation of cultivation temperature

The same single clones as in the above (1) were cultured at 33°C in RPMI 1640 (05918) medium and about 1 × 10⁴ cells were plated on a well of a 96-well plate and cultured at 33°C or 37°C for 2 weeks in RPMI 1640 (05981) medium or DMEM (D-5796). After the cultivation, expression levels of PC1 and PC2 were investigated using the methods mentioned in (2) and (3) of Example 13.

As a result, it was clarified that, when a single clone F-1 was cultured at 37°C in DMEM (D-5796), expression of PC1 further increased as compared with the cultivation at 33°C in the same medium. The result shows that, when a cell cultured at 33°C is cultured at 37°C for 2 weeks, there may be the case where the differentiation character of the above cell line derived from Langerhans islets is able to be controlled.

### [Example 16] Construction of an active peptide-expressing system where a cell line derived from Langerhans islets is used as a host.

The immortalized cell lines derived from rat Langerhans obtained in Example 11 (single clones F-4, F-5 and F-8) were cultured at 33°C in RPMI 1640 (05918) medium and, after that, 2 to 5 × 10⁴ cells were plated on a well of a 96-well plate and cultured at 33°C for 1 day.

After the cultivation, preprovasopressin expression plasmid (pcDNA3.1-VP) prepared in (1) of Example 9, preprocorticotropin-releasing hormone expression plasmid (pcDNA3.1-CRF) prepared in (2) of Example 9 or control plasmid [pcDNA3.1(+)] was transfected into the cell using lipofectamine plus (manufactured by Gibco). The gene transfection was carried out using 0.2 µg of plasmid, 0.5 µl of the Lipofectamine reagent and 1 µl of the plus reagent according to the direction attached to the Lipofectamine plus.

The cell line into which the gene was transfected was cultured at 33°C for one day in 100 µl of RPMI 1640 (05918) medium. After the cultivation, the medium was exchanged to 100 µl of RPMI 1640 (05918) medium containing no serum and cultivation was carried out at 33°C for 2 days more. After the cultivation, the culture supernatant was collected and vasopressin activity and corticotropin-releasing hormone activity were measured in the culture supernatant. The vasopressin activity was measured using assay cells constructed in (1) of Example 21 which will be mentioned later. The corticotropin-releasing hormone activity was measured using assay cells constructed in (2) of Example 21.

In the culture supernatant derived from cells to which preprovasopressin expression plasmid was transfected, a vasopressin activity was detected while, in the culture supernatant derived from cells to which preprocorticotropin-releasing hormone expression plasmid was transfected, a corticotropin-releasing hormone activity was detected. In the culture supernatant derived from cells into which control plasmid was transfected, none of those activities was detected. In the above, the same result was obtained even when a DMEM (D-5796) medium was used in place of the RPMI 1640 (05918) medium. From the above results, it was found that, when a precursor gene of active peptide is expressed using immortalized cell lines (single clones F-4, F-5 and F-8) derived from Langerhans islets as hosts, active peptide is able to be produced.

### [Example 17] Construction of an expression cloning system of an active peptide using a cell line derived from pancreatic Langerhans islets as a host

### (1) Construction of an expression cloning systemof active peptide precursor genes

According to the method mentioned in Example 16, each of the following plasmids (i) to (iii) was transfected into an immortalized cell line (single clone F-8) derived from Langerhans islets.
(i) Control plasmid [pcDNA3.1(+)]
(ii) Mixed plasmids where preprocorticotropin-releasing hormone expression plasmid (pcDNA3.1-CRF) is diluted to an extent of 1/10 using pcDNA3.1(+)
(iii) Mixed plasmids where pcDNA3.1-CFR is diluted to an extent of 1/100 with pcDNA3.1(+)

After the transfection, each cell was cultured at 33°C for 2 days in 100 µl of DMEM (LG). After the cultivation, the medium was exchanged to 100 µl of DMEM (LG) containing no serum and cultivation was carried out at 33°C for 2 days more.

The assay cells constructed in (2) of Example 21 which will be mentioned later were layered on the resulting cells and then 25 mmol/L of glucose and 100 µmol/L of sulfonylurea (Tolbutamide) were added thereto. After 6 hours from the addition, coelenterazine h (Molecular Probes) (final concentration: 250 nmol/L) was added and activity of reporter (*Renilla reniformis* luciferase) was measured using a VIM camera (Argus-50/2D luminometer/MP; manufactured by Hamamatsu Photonics). The result was that, in all of the diluted samples, activity was detected while, when only expression vector [pcDNA3.1(+)] was transfected, no activity was detected.

Activity is able to be detected even when dilution is conducted to an extent of 1/10 and 1/100 and, therefore, it is now apparent that, when an appropriate cell line derived from pancreatic Langerhans islets is selected and used as a host, many (such as 10 to 100 cDNAs) were transfected to host cells at the same time and then culture supernatant of the cells, cell extract of the cells, membrane fraction of the cells or the cells *per se* is/are used, the aimed activity is able to be measured.

### (2) Expression cloning of active peptide precursor genes

A cDNA library (*Escherichia coli*) was constructed according to the method mentioned in Example 23 using an appropriate expression vector [pcDNA3.1(+) (manufactured by Invitrogen), pAGal9-nd (Example 18 which will be mentioned later) or pAGal9-d (Example 18 which will be mentioned later)].

The cDNA library was cultured after dividing into pools each comprising 1 to 100 clone(s) and then plasmids were recovered from each pool. The plasmids were transfected to an appropriate cell line derived from Langerhans islets using a 96-well plate for expression.

After 3 days, assay cells which were able to be prepared according to the method mentioned in Example 21 were layered and, after 6 hours, a reporter activity was measured. When pools each comprising 100 clones are used, it is possible to conduct a screening for 9,600 clones using one 96-well plate. When the pool where the activity is detected is divided into smaller pools and subjected to the same operation, it is finally possible to isolate an aimed active peptide precursor gene.

When a cDNA library which was constructed using pAGal9-nd or pAGal9-d as a vector is used, it is necessary to transfect a plasmid [such as PCR2.1/Gal4-VP16 (manufactured by Invitrogen), pAMo-Gal4VP16 (refer to the following) or pcDNA3-Gal4VP16 (refer to the following)] for the expression of a chimeric protein [*Nature*, 335, 563 (1988)] consisting of DNAbinding domain of Gal4p and transactivation domain of herpes simplex virus VP16 together with the cDNA library to the host cell at the same time.

Construction of pAMo-Gal4VP16 and pcDNA3-Gal4VP16 is mentioned as follows.

pMC1 [*J.Mol.Biol.,* 180, 1 (1984)] was cleaved with BstBI, subjected to a Klenow treatment and further cleaved with AccI to give a BstBI (blunt end) -AccI fragment comprising 3' -terminal region of the VP16 gene. pCMVGal4 [*EMBO J.,* 8, 2337 (1989)] was cleaved with HindIII and ClaI to give a HindIII-ClaI fragment comprising 5' -terminal sequence of the Gal4 gene. pAMo-nd was cleaved with NotI, subjected to a Klenow treatment and further cleaved with HindIII to give a NotI (blunt end)-HindIII fragment comprising the ampicillin-resistant gene. The BstBI (blunt end)-AccI fragment derived from pMC1, the HindIII-ClaI fragment derived frompCMVGal4 and the NotI (blunt end)-HindIII fragment derived from pAMo-nd were ligated to construct a Gal4-VP16 chimeric gene expression plasmid pAMo-Gal4VP16.

pAMo-Gal4VP16 was cleaved with HindI II and KpnI to give a HindIII-KpnI fragment containing the Gal4-VP16 chimeric gene. pcDNA3.1+ (manufactured by Invitrogen) was cleaved with HindIII and KpnI to give a HindIII-KpnI fragment comprising the ampicillin-resistant gene. HindIII-KpnI fragment derived from pAMo-Gal4VP16 and HindIII-KpnI fragment derived from pcDNA3.1+ were ligated to construct pcDNA3-Gal4VP16.

Genes encoding random peptides are expressed using various cell lines suitable for the production of active peptides as hosts whereupon many kinds of peptides are able to be produced efficiently. For example, a gene part encoding an active peptide of any active peptide precursor gene is substituted with a gene encoding a random peptide whereupon gene which expresses the random peptide is able to be prepared.

### [Example 18] Construction of a novel host-vector system (1)

### (1) Construction of Gal4-ER expression plasmid pGERbsrR2

pSV2bsr (manufactured by Kaken Seiyaku) was cleaved with PvuII and EcoRI and subjected to a Klenow treatment to prepare aPvuII (blunt end) -EcoRI (blunt end) fragment of 2.6 kb. ERαAF2 in pM containing the Gal4-ER chimeric gene [*Cell*, 54, 199 (1988); *Proc*. *Natl*. *Acad*. *Sci*., *USA*, 90, 1657 (1993)] (apportioned from Dr. Shigeaki Kato, University of Tokyo) was cleaved with AatII and NdeI and subjected to a Klenow treatment to prepare an AatII (blunt end)-NdeI (blunt end) fragment. The above mentioned PvuII (blunt end)-EcoRI (blunt end) fragment derived from pSV2bsr and the AatII (blunt end)-NdeI (blunt end) fragment derived from ERαAF2 in pM were ligated to construct a plasmid pGERbsrR2. pGERbsrR2 is able to express a chimeric protein (Gal4-ER) consisting of a DNA binding domain of transcription factor Gal4p derived from a yeast (*Saccharomyces cerevisiae*) and a ligand binding domain of estrogen receptor.

### (2) Construction of an inducible expression plasmid of firefly luciferase

pcDNA3 (Invitrogen) was cleaved with XhoI and subjected to a Klenow treatment to prepare a XhoI (blunt end) fragment. The fragment were ligated to construct pcDNA3 where cleaved sites by XhoI disappeared.

pcDNA3 where the cleaved site with XhoI disappeared was cleaved with KpnI and subjected to a Klenow treatment to prepare a KpnI (blunt end) fragment. The fragment were ligated to construct pcDNA3 where cleaved sites with XhoI and KpnI disappeared. The plasmid was cleaved with BglII and subjected to a Klenow treatment to a prepare BglII (blunt end) fragment. pAMoERC3Sc (Japanese Published Unexamined Patent Application No. 336,963/1993) was cleaved with XhoI and NsiI and subjected to a Klenow treatment to obtain a XhoI (blunt end)-NsiI (blunt end) fragment of 2.2 kb having an oriP sequence. The above-mentioned BglII (blunt end) fragment derived from pcDNA3 where XhoI-cleaved site and KpnI-cleaved site disappeared and the XhoI (blunt end)-NsiI (blunt end) fragment derived from pAMoERC3Sc were ligated to construct a plasmid pcDNA3-oriP.

pcDNA3-oriP was cleaved with XhoI and HindIII to obtain a XhoI-HindIII fragment. pSE0luc2 (WO 98/14474) was cleaved with XhoI and NcoI and subjected to a Klenow treatment to obtain a XhoI (blunt end)-NcoI (blunt end) fragment comprising the ampicillin-resistant gene. The fragments were ligated to construct a plasmid pASd1-luc1.

After pASd1-luc1 was cleaved with XhoI and HindIII, a XhoI-HindIII fragment of 0.11 kb were obtained. The above-mentioned XhoI-HindIII fragment derived from pcDNA3-oriP and the XhoI-HindIII fragment derived from pASd1-luc1 were ligated to construct a plasmid pcDNA3-oriP-Sd1.

pcDNA3-oriP-Sd1 was cleaved with XhoI and Asp 718 to obtain a XhoI-Asp718 fragment. Four kinds of DNAs having nucleotide sequences represented by SEQ ID NOS: 87, 88, 89 and 90,respectively were synthesized. When the synthetic DNAs were mixed and annealed, a double-stranded DNA having polyadenylation signal was constructed. Each of the synthetic DNAs was phosphorylated using T4 polynucleotide kinase, mixed and annealed to give a double-stranded DNA. When the double-stranded DNA was ligated to the XhoI-Asp718 fragment derived frompcDNA3-oriP-Sd1, a plasmid pcDNA3-oriP-Sd1-pA was constructed.

pcDNA3-oriP-Sdl-pA was cleaved with XhoI and subjected to a Klenow treatment to obtain a XhoI (blunt end) fragment. pFR-luc (manufactured by Stratagene) was cleaved with HindIII and BamHI and subjected to a Klenow treatment to obtain a HindIII (blunt end)-BamHI (blunt end) fragment of 0.14 kb. The above-mentioned XhoI (blunt end) fragment derived from pcDNA3-oriP-Sd1-pA and the HindIII-BamHI fragment derived from pFR-luc were ligated to obtain a plasmid pAGalSd1. The pAGalSd1 comprises a promoter having a sequence where Gal4p-responsive elements (UASG) are repeated for 5 times.

pAGalSd1 was cleaved with EcoRI and subjected to a Klenow treatment to obtain a EcoRI (blunt end) fragment. pSE0luc2 (WO 98/14474) was cleaved with HindIII and SacI and subjected to a Klenow treatment to prepare a HindIII (blunt end)-SacI (blunt end) fragment of 1. 7 kb comprising the firefly luciferase gene. The above-mentioned HindIII (blunt end)-SacI (blunt end) fragments derived from pSE01uc2 and the EcoRI (blunt end) derived from0 pAGalSd1 were ligated to construct a plasmid pAGalSd1-luc.

Among the two HindIII sites existing in pAGalSd1-luc, only a HindIII site far from the firefly luciferase gene was made disappeared by a Klenow treatment to construct pAGalSd4-luc.

pAGalSd4-luc was cleaved with Asp718 and subjected to a partial digestion with StuI to obtain a Asp718-StuI fragment of 9.5 kb derived from pAGalSd4-luc. The DNA fragments were subjected to a Klenow treatment and self-ligated to construct a plasmid pAGal9-luc.

### (3) Construction of inducible expression vectors pAGal9-d and pAGal9-nd

Expression plasmid pAGal9-luc havingoriP of Epstein-Barr viruswas cleaved with HindIII and SacI to prepare a HindIII-SacI fragment of 6.9 kb containing oriP. pAMo-d (Japanese Published Unexamined Patent Application No. 211,885/2001) was cleaved with HindIII and SacI to prepare a HindIII-SacI fragment comprising the tetracycline-resistant gene (Tc^{R}). The above-mentioned HindIII-SacI fragment derived from pAGal9-luc and the HindIII-SacI fragment derived from pAMo-d were ligated to construct a plasmid pAGal9-d where the firefly luciferase gene in pAGal9-luc was substituted with a stuffer sequence of pAMo-d.

pAGal9-luc was cleaved with HindIII and SacI to prepare a HindIII-SacI fragment of 6.9 kb. pAMo-nd (Japanese Published Unexamined Patent Application No. 211,885/2001) was cleaved with HindIII and SacI to prepare a HindIII-SacI fragment comprising the tetracycline-resistant gene. The above-mentioned HindIII-SacI fragment derived from pAGal9-luc and the HindIII-SacI fragment derived from pAMo-nd were ligated to construct a plasmid pAGal9-nd where the firefly luciferase gene in pAGal9-luc was substituted with a stuffer sequence of pAMo-nd.

### (4) Preparation of a cell line KJMGER8 where Gal4-ER expression plasmid pGERbsrR2 was integrated in chromosomal DNA of Namalwa KJM-1 cells

Gal4-ER chimeric transcription factor expression plasmid pGERbsrR2 was dissolved in a TE buffer [10 mmol/L Tris-HCl (pH 8.0) and 1 mmol/L of ethylenediamine tetraacetate] so as to make 1 µg/µL and, after that, the plasmid, 4 µg for 6 × 10⁶ cells, was transfected to Namalwa KJM-1 cells [*Cytotechnology,* 1, 151 (1988)] by an electroporation method [*Cytotechnology,* 3, 133 (1990)] to prepare transformed cells. Namalwa KJM-1 cell is a B-cell line adapted for serum-free culture, which expresses the EBNA-1 gene.

The transformant was suspended in 8ml of an RPMI 1640-ITPSG medium [a medium where a 1/40 amount of 7.5% NaHCO₃, 3% 200 mmol/L of L-glutamine solution (manufactured by Invitrogen), 0.5% penicillin-streptomycin solution (manufactured by Invitrogen comprising 5,000 units/ml of penicillin and 5,000 µg/ml of streptomycin), 10 mmol/L of N-2-hydroxyethylpiperazine-N'-2-hydroxypropane-3-sulfonic acid(HEPES), 3 µg/ml insulin, 5 µg/ml transferrin, 5 mmol/L sodiumpyruvate, 125 nmol/L sodium selenite and 1mg/ml galactose were added to an RPMI 1640 medium (manufactured by Nissui Seiyaku)] and cultured at 37°C in a CO₂ incubator for 24 hours.

After the cultivation, blasticidin S (KK-400: manufactured by Kaken Seiyaku) was added so as to make 2.0 µg/ml, dispensed in a 96-well plate (500 to 2,000 cells/well) and cultivation was carried out to obtain many stable transformants (single clones) where pGERbsrR2 was integrated in chromosomal DNA. Each transformant was subcultured in RPMI 1640-ITPSG medium containing 2.0 µg/ml of blasticidin S.

By the method as mentioned below, an excellent stable transformant KJMGER8 cell having high induction ratio and low background upon non-inducing stage was selected from the above-mentioned stable transformants.

An inducible expression plasmid pAGal5d1-luc of firefly luciferase was transfected to each transformant by an electroporation method and cultured for 2 days. After the cultivation, 17β-estradiol (E8875: manufactured by Sigma) (final concentration 10 nmol/L) was added and, after the cultivation for 24 hours more, the firefly luciferase activity was measured. For the measurement of the activity, a luminometer-LB 953 (manufactured by Berthold) was used, 100 µl of a buffer for dissolving the cells [1% Triton X-100, 100 mmol/L KH₂PO₄ (pH 7.8) and 1 mmol/L dithiothreitol] was automatically injected into the above culture solution, then 300 µl of a substrate solution [25 mmol/L glycylglycine (pH 7.8), 15 mmol/L MgSO₄, 5 mmol/L ATP and 0.33 mmol/L luciferin] was automatically injected and the amounts of emission of light during 10 seconds was measured for adopting as a luciferase activity. For comparison, luciferase activity under the condition where no 17β-estradiol was added was also measured.

Luciferase activity under the condition where 17β-estradiol was added and luciferase activity under the condition where no 17β-estradiol was added were compared, induction ratio for gene expression was calculated, and KJMGER8 cell was selected as a clone with high induction ratio and low luciferase activity in the condition without addition of 17β-estradiol.

### (5) Inducible expression of the firefly luciferase gene using KJMGER8 a host

Inducible expression plasmid pAGal9-luc of firefly luciferase or control plasmid (pAGal9-nd) was transfected to KJMGER8 using 4 µg to 1.6 × 10⁶ cells by an electroporation method to prepare transformed cells (KJMGER8/pAGal9-luc and KJMGER8/pAGal9-nd).

The transformed cells were suspended in 8 ml of RPMI 1640-ITPSG medium and cultured in a CO₂ incubator at 37°C for 24 hours. After the cultivation, blasticidin S (0.2 µg/ml) and genetic in (manufactured by Gibco) (0.5 mg/ml) were added and cultivation was carried out for 14 days more to obtain a stable transformant. The stable transformant was subcultured in RPMI 1640-ITPSG medium containing blasticidin S (0.2 µg/ml) and geneticin (manufactured by Gibco) (0.5 mg/ml).

17β-Estradiol (E8875: manufactured by Sigma) (final concentration 10 nmol/L) was added to the stable transformant and cultured for 24 hours and, after that, the luciferase activity was measured by the same manner as above. For comparison, the luciferase activity under the condition where no 17β-estradiol was added was also measured.

The luciferase activity under the condition where 17β-estradiol was added and the luciferase activity under the condition where no 17β-estradiol was addedwere compared whereby induction ratio for gene expression was calculated. As a result, induction ratio of gene expression in KJMGER8/pAGal9-luc was about 10,000-fold. Luciferase activity in KJMGER8/pAGal9-luc under the condition where no 17β-estradiol was added was very low (about 60 RLU/10 seconds). The result shows that, under the condition where no 17β-estradiol was added, the luciferase gene was rarely expressed.

As mentioned above, in a system where pAGal9-luc was used as an inducible expression plasmid and KJMGER8 as a host, luciferase activity at the non-induction state was very low and that at the induction state was very high. Structure of pAGal9-luc is such a structure that the firefly luciferase gene is inserted as a reporter gene for inducible expression in pAGal9-nd or pAGal9-d. Accordingly, a system where KJMGER8 is used as a host and pAGal9-nd or pAGal-d is used as an inducible expression vector has been found to be a very good inducible expression system where gene expression level in the non-induction state is very low and, in addition, induction ratio for gene expression is high.

### [Example 19] Development of a novel host-vector system (2)

### (1) Construction of a reporter plasmid pACREpluc where firefly luciferase is a reporter

pACREpluc which is a reporter plasmid capable of expressing a firefly luciferase gene under the control of cAMP-responding element (CRE) was constructed by the following method. pACREplus has oriP of Epstein Barr virus and the hygromycin-resistant gene.

pAMo [*J*. *Biol*. *Chem*., 268, 22782 (1993); another name: pAMoPRC3Sc (Japanese Published Unexamined Patent Application No. 336,963/1993)] was partially digested with ClaI to obtain a DNA fragment where one site was cleaved. The DNA fragment were partially digested by MluI to obtain a ClaI-MluI fragment of 9.5 kb. pAGE248 [*J*. *Biol*. *Chem*., 269, 14730 (1994)] was cleaved with ClaI and MluI to obtain a ClaI-MluI fragment of 1.5 kb comprising the hygromycin-resistant gene. The ClaI-MluI fragment derived from pAMo and the ClaI-MluI fragment derived from pAGE248 were ligated to construct a plasmid pAMoh.

pAMoh was cleaved with XhoI and HindIII to obtain a XhoI-HindIII fragment comprising the hygromycin-resistant gene. pAGal9-luc was cleaved with SalI and HindIII to obtain a SalI-HindIII fragment comprising oriP and Gal4UAS. The SalI- HindIII fragment derived from pAGal9-luc and the above-mentioned XhoI-HindIII fragment derived from pAMoh were ligated to construct a plasmid pAGal9h.

pBluescriptII KS+ (manufactured by Stratagene) was cleaved with SalI and XhoI and subjected to a dephosphorylation using phosphatase (Alkaline Phosphatase E. coli C75; manufactured by Takara Shuzo) to obtain a SalI-XhoI fragment comprising the ampicillin-resistant gene. As a result of annealing of synthetic oligonucleotides having nucleotide sequences of SEQ ID NOS: 91 and 92, respectively, a double-stranded DNA containing two CRE sequences was prepared. The double-stranded DNA was ligated to the above SalI-XhoI fragment derived from pBluescriptII KS+ to construct a plasmid pBS-CREI comprising two CRE sequences. The pBS-CREI is a plasmid where the double-stranded DNA is inserted in such a direction that cleaved site with SalI and cleaved site with XhoI are regenerated and has each one of the above cleaved sites.

pBS-CREI was cleaved with ScaI and XhoI to prepare a ScaI-XhoI fragment comprising ori of a phage f1. pBS-CREI was cleaved with ScaI and SalI to prepare a ScaI-SalI fragment comprising Co1E1 ori. The ScaI-XhoI fragment and the ScaI-SalI fragment derived from pBS-CREI were ligated to construct pBS-CREII comprising 4 CRE sequences.

pBS-CREII was cleaved with ScaI and XhoI to obtain a ScaI-XhoI fragment comprising ori of a phage f1. pBS-CREII was cleaved with ScaI and SalI to obtain a ScaI-SalI fragment comprising Co1E1 ori. The ScaI-XhoI fragment and the ScaI-SalI fragment derived from pBS-CREII were ligated to construct pBS-CREIV comprising 8 CRE sequences.

pBS-CREIV was cleaved with ScaI and XhoI to obtain a ScaI-XhoI fragment comprising ori of a phage f1. pBS-CREIV was cleaved with ScaI and SalI to obtain a ScaI-SalI fragment comprising ColEl ori. The ScaI-XhoI fragment and the ScaI-SalI fragment derived from pBS-CREIV were ligated to construct pBS-CREVIII comprising 16 CRE sequences.

pBS-CREVIII was cleaved with XhoI, subjected to a Klenow treatment, and further cleaved with HindIII to obtain a HindIII-XhoI (blunt end) fragment comprising 16 CREs. pAGalSd1 was cleaved with MluI and HindIII to obtain a MluI-HindIII fragment of 1.4 kb. pAGal19h was cleaved with XbaI, subjected to a Klenow treatment, and further cleaved with MluI to give a XbaI (blunt end)-MluI fragment. The HindIII-XhoI (blunt end) fragment derived from pBS-CREVIII, the MluI-HindIII fragment derived from pAGalSd1 and the XbaI (blunt end) -MluI fragment derived from pAGal19h were ligated to prepare a plasmid pACREh.

pAGal9-luc was cleaved with XhoI and NotI to obtain a XhoI-NotI fragment comprising the firefly luciferase gene. pACREh was cleaved with XhoI and NotI to obtain a XhoI-NotI fragment comprising CRE sequences. The XhoI-NotI fragment derived from pAGal9-luc and the XhoI-NotI fragment derived from pACREh were ligated to construct a plasmid pACREluc.

pACREluc was cleaved with HindIII, subjected to a Klenow treatment, and further cleaved with XhoI to obtain a HindIII (blunt end) -XhoI fragment comprising CRE and a HindIII (blunt end) -XhoI fragment comprising the firefly luciferase gene, respectively. The above-mentioned two HindIII (blunt end)-XhoI fragments derived from pACREluc were ligated to construct a plasmid pACRElucH in which HindIII site in upstream of CRE sequence in pACREluc disappeared.

pGL3-Enhancer vector (manufactured by Promega) was cleaved with HindIII and HpaI to obtain a HindIII-HpaI fragment comprising the luc+ gene (an improved firefly luciferase gene). pACRElucH was cleaved with NotI, subjected to a Klenow treatment, and further cleavedwith HindIII to obtain a HindIII-NotI (blunt end) fragment containing CRE. The HindIII-HpaI fragment derived from pGL3-Enhancer vector and the HindIII-NotI (blunt end) fragment derived from pACRElucH were ligated to construct a plasmid pACREpluc.

### (2) Preparing Namalwa KJM-1 cells into which a reporter plasmid pACREpluc was transfected

A reporter plasmid pACREpluc was dissolved in a TE buffer so as to make 1 µg/µl and the plasmid was transfected to Namalwa KJM-1 cells by an electroporation method [*Cytotechnology*, 3, 133 (1990)] using 4 µg to 6 × 10⁶ cells to give transformants.

The transformants were suspended in 8 ml of RPMI 1640-ITPSG medium and cultured in a CO₂ incubator at 37°C for 24 hours. After the cultivation, hygromycin B (300 µg/ml) was added and cultivation was carried out for 14 days more to obtain stable transformants. The transformants were subcultured in RPMI 1640-ITPSG medium containing hygromycin B (300 µg/ml).

In order to confirm whether gene expression via CRE took place, the transformants were stimulated under the following condition using 5'-N-ethylcarboxamide adenosine (NECA: manufactured by Sigma) which is an agonist of type 2a adenosine receptor (A2a), forskolin (manufactured by Sigma) which is an activator for adenylate cyclase or A23187 (manufactured by Research Biochemicals International) which is a calcium ionophore.

Thus, the above stable transformants were dispensed in an amount of 1 × 10⁵ cells per well of a 48-well plate, then NECA (final concentration 100 nmol/L), forskolin (final concentration 100 µmol/L) or A23187 (final concentration 10 µmol/L) was added and stimulation was conducted by incubating in a CO₂ incubator for 5 hours.

After stimulation, firefly luciferase activity was measured using the method mentioned in (4) of Example 18.

NECA and forskolin stimulation result in about 7-fold and about 10-fold increase of firefly luciferase activity, respectively. On the other hand, A23187 stimulation rarely changed firefly luciferase activity.

From the above result, it is apparent that, in Namalwa KJM-1 cells to which pACREpluc was transfected, the luciferase gene was expressed by stimulation which promotes the transcription from CRE.

Thus, when Namalwa KJM-1 cells to which pACREpluc was transfected are used, it is possible to screen a substance having activity for promoting the transcription from CRE.

When Namalwa KJM-1 cells to which pACREpluc was transfected were stimulated with NECA and firefly luciferase activity was measured after 6, 24, 48, 72 and 96 hours, the activity was highest after 6 hours and, after 24 hours, the activity was decreased to an extent of nearly one half. Accordingly, when the cells are stimulated by any substance, it is believed to be preferred to measure the activity after 6 hours from the stimulation.

### (3) Preparation of stable transformants in which a reporter plasmid pACREpluc was integrated into chromosomal DNA of KJMGER8

pACREpluc cleaved with HpaI, SpeI or BalI which is a restriction enzyme existing in one place in oriP of pACREpluc was dissolved in a TE buffer so as to make 1 µg/µl and, after that, it was transfected in an amount of 4 µg to 6 × 10⁶ cells to KJMGER8 constructed in Example 18 by an electroporation method [*Cytotechnology*, 3, 133 (1990)] to prepare transformants.

The transformants were suspended in 8 ml of RPMI 1640-ITPSG medium and cultured in a CO₂ incubator at 37°C for 24 hours. After the cultivation, blasticidin S (2.0 µg/ml) and hygromycin B (300 µg/ml) were added and cultivation was carried out for 7 days more. After the number of living cells were confirmed, they were diluted in RPMI 1640-ITPSG medium containing blasticidin S (2.0 µg/ml), hygromycin B (300 µg/ml) and 1 × 10⁵ cells/ml of KJMGER8 so as to make 150 cells/ml and cultured after dispensing in a 96-well plate (30 cells in average per well) and wells where one colony per well was actually formed were selected to obtain 420 stable transformants (single clones). Each transformant was subcultured using an RPMI 1640-ITPSG medium containing blasticidin S (2.0 µg/ml) and hygromycin B (300 µg/ml).

### (4) Selection of stable transformants (single clones) having good properties

Each of the clones prepared in the above (3) was plated on a 96-well plate (1 to 2 × 10⁴ cells/well), NECA (final concentration 100 nmol/L) was added thereto and the mixture was incubated in a CO₂ incubator for 6 hours. After the incubation, firefly luciferase activity in each well was measured using the method mentioned in (4) of Example 18. With regard to a device for the measurement, Micro Lumat LB96P (manufactured by Berthold) was used. Seventeen clones having high activity were selected.

The 17 clones were used, stimulation with NECA was applied by the same manner as in the above (2), comparison with the case where no stimulation with NECA was conducted and 8 clones where luciferase activity rose to an extent of 60-fold or more were selected. Such 8 clones were named GBC1 to GBC8 respectively.

To the 8 clones was transfected pAGal9-GPR12 [a plasmid for inducible expression of GPR12 which is a constitutively activated G-protein coupled receptor: refer to Example 22 which will be mentioned later] by the above electroporation method using 4 µg to 6 × 10⁶ cells to prepare transformants.

The transformants were suspended in 8 ml of RPMI 1640-ITPSG medium and cultured in a CO₂ incubator at 37°C for 24 hours. After the cultivation, blasticidin S (2.0 µg/ml), hygromycin B (300µg/ml) andgeneticin (500 µg/ml) were added andcultivation was carried out for 14 days more to obtain stable transformants. The transformants were subcultured in an RPMI 1640-ITPSG medium containing blasticidin S (2. 0 µg/ml), hygromycin B (300 µg/ml) and geneticin (500 µg/ml).

To each transformant was added 17β-estradiol (final concentration 10 nmol/L), incubation was carried out for 24 hours and luciferase activity was measured by the same method as in (4) of Example 18. For comparison, luciferase activity under the condition where no 17β-estradiol was added was also measured.

Three clones (GBC5, GBC6 and GBC7) where rising rate of luciferase activity upon addition of 17β-estradiol was high were selected. The rising rates in GBC5, GBC6 and GBC7 were 56-, 193- and 364-fold, respectively.

To the 3 clones was transfected pAGal9-V2 [a plasmid for inducible expression of type 2 vasopressin receptor (V2): refer to (1) of Example 21 which will be mentioned later] by the same method as above to prepare a stable transformant, then the transfected gene (V2 gene) was subjected to an inducible expression by means of stimulation with 17β-estradiol and luciferase activity upon vasopressin stimulation was checked. At the same time, activity where no stimulation with 17β-estradiol was conducted and activity where no stimulation with vasopressin was conducted were checked.

As a result thereof, GBC7 which reacted with vasopressin onlywhen stimulatedwith 17β-estradiol to give a high luciferase activity (599-fold) was selected as a good cell line.

### (5) Construction of a reporter plasmid pACRERluc where Renilla reniformis luciferase is a reporter

pRL-SV40 vector (manufactured by Promega) was cleaved with XbaI, subjected to a Klenow treatment, and further cleaved with HindIII to obtain a HindIII-XbaI (blunt end) fragment comprising the *Renilla reniformis* luciferase gene. pACRElucH constructed in the above (1) was cleaved with NotI, subjected to a Klenow treatment, and further cleaved with HindIII to obtain a HindIII-NotI (blunt end) fragment comprising CRE. The HindIII-XbaI (blunt end) fragment derived from pRL-SV40 vector and the HindIII-NotI (blunt end) fragment derived from pACRElucH were ligated to construct a plasmid pACRERluc.

### (6) Preparation of stable transformants where a reporter plasmid pACRERluc is integrated in chromosomal DNA of KJMGER8

pACRERluc was transfected into KJMGER8 according to the method mentioned in the above (3) to prepare 96 stable transformants (single clones). Each transformant was subcultured in an RPMI 1640-ITPSG medium containing blasticidin S (2.0 µg/ml) and hygromycin B (300 µg/ml).

### (7) Selection of stable transformants (single clones) having good properties

Clones having good properties were selected from the clones prepared in the above (6) according to the method mentioned in the above (4) except that activity of *Renilla reniformis* luciferase was measured instead of measurement of the activity of firefly luciferase.

Each clone obtained in the above (6) was plated on a 96-well plate (1 to 2 × 10⁴ cells/well), NECA (final concentration 100 nmol/L) was added thereto and incubation was carried out in a CO₂ incubator for 6 hours. After that, coelenterazine h (Molecular Probes) (final concentration 250 nmol/L) was added to measure the activity of *Renilla reniformis* luciferase. For the measurement of the activity, a Wallac 1420 ARVOsxMultilable Counter (manufactured by Wallac Berthold Japan) was used. Nine clones having high activity were selected. The 9 clones were named GBCR1 to GBCR9, respectively.

With regard to the 9 clones, stimulation with NECA was conducted and, as compared with the case no stimulation with NECA was done, 2 clones where the activity of *Renilla reniformis* luciferase increased to an extent of not less than 18-fold were selected. The 2 clones were named GBCR1 and GBCR2, respectively.

pAGal9-GPR12 was transfected to the 2 clones to prepare stable transformants. The transformants were stimulated for 24 hours with 17β-estradiol and, after that, the activity of *Renilla reniformis* luciferase was measured. For comparison, the activity of *Renilla reniformis* luciferase under the condition where no 17β-estradiol was added was also measured. One clone (GBCR2) where a rising rate of *Renilla reniformis* luciferase activity was high when 17β-estradiol was added was selected as a good clone. The rising rate was 364-fold.

pAGal9-V2 was transfected into GBCR2 to prepare a stable transformant. The transformant was stimulated with 17β-estradiol and, after the transfected V2 gene was subjected to inducible expression, it was stimulated with vasopressin and *Renilla reniformis* luciferase activity after the stimulation was measured. At the same time, activity where no stimulation with 17β-estradiol was conducted and activity where no stimulation with vasopressinwas conducted were checked. The result is shown in Fig. 10.

When GBCR2 was used as a host cell, it reacted with vasopressin only when stimulated with 17β-estradiol, showing high *Renilla reniformis* luciferase activity (139-fold) as compared with the case where GBCR1 was used, and it was found that GBCR2 is better in this system.

### [Example 20] Development of a novel host-vector system (3)

### (1) Construction of a Gαₛ₄ expression plasmid pAMoh-Gs4

A single-stranded cDNA was prepared from total RNA (5 µg) derived fromNamalwa KJM-1 according to the method mentioned in Example 6. To the above-mentioned single-stranded cDNA (10 µl) were added Gαₛ₄ gene-specific primers (each 20 pmol), 4 µl of 2.5 nmol/l dNTP mixed solution, 2.5 µl of DMSO, 0.25 µl of 5 units/µl Pyrobest DNA Polymerase (manufactured by Takara Shuzo) and 5 µl of 10 × reaction buffer (manufactured by Takara Shuzo) and then sterilized water was added thereto to make the total volume 50 µl. Synthetic DNAs having the sequences mentioned in SEQ ID NOS: 93 and 94 were used as Gαₛ₄ gene-specific primers. To those primers were introduced a HindIII site and a Asp718 site, respectively. Using a thermal cycler DNA Engine (manufactured by MJ Research), after the treatment at 95°C for 5 minutes, a PCR was carried out under the condition of 30 cycles of reactions each comprising 94°C for 30 seconds, 65°C for 1 minute and 72°C for 2 minutes.

DNA fragments amplified by a PCR were recovered by means of an agarose gel electrophoretic method. The amplified fragment was cleaved by HindIII and Asp718 to obtain a HindIII-Asp718 fragment. A plasmid pAMoh was cleaved with HindIII and Asp718, and a HindIII-Asp718 fragment was obtained. The HindIII-Asp718 fragment derived from the PCR fragment and the HindIII-Asp718 fragment derived from pAMoh were ligated to construct a Gαₛ₄ expression plasmid pAMoh-Gs4.

### (2) Construction of a plasmid pAMoh-Gs-q

pAMoh-Gs4 constructed in the above (1) was cleaved with XbaI and Asp718 to obtain a XbaI-Asp718 fragment comprising oriP. In addition, after pAMoh-Gs4 was cleaved with XbaI, it was partially digested with SphI to obtain a XbaI-SphI fragment encoding Gαₛ₄ with C-terminal deletion. Synthetic DNAs having nucleotide sequences represented by SEQ ID NOS: 95 and 96, respectively, were phosphorylated with T4 polynucleotide kinase and then annealed to obtain a double-stranded DNA containing a domain encoding C-terminal five amino acids of Gα_{q}. The double-stranded DNA, the XbaI-Asp718 fragment and the XbaI-SphI fragment derived from pAMoh-Gs4 were ligated to construct pAMoh-Gs-q.

### (3) Construction of a plasmid pAMoh-Gs-i

Synthetic DNAs having nucleotide sequences represented by SEQ ID NOS: 97 and 98, respectively, were phosphorylated with T4 polynucleotide kinase and annealed to obtain a double-stranded DNA comprising a domain encoding C-terminal five amino acids of Gαᵢ. The double-stranded DNA, the XbaI -Asp 718 fragment derived from pAMoh-Gs4 and the XbaI-SphI fragment derived from pAMoh-Gs4 prepared in the above (2) were ligated to construct pAMoh-Gs-i.

### (4) Construction of a plasmid pAMopGs-qMoGs-i

pACREluc was cleaved with ClaI, subjected to a Klenow treatment, and further cleaved with CpoI to obtain a CpoI-ClaI (blunt end) fragment comprising oriP and CRE-firefly luciferase gene. pAMoh-Gs-i was cleaved with BssHII and CpoI to prepare a BssHII-CpoI fragment encoding Gαₛ₋ᵢ. pAGE248 [*J.Biol. Chem.,* 269, 14730 (1994)] was cleaved with XhoI, subjected to a Klenow treatment, and further cleaved with BssHII to obtain a XhoI (blunt end)-BssHII fragment comprising a part of LTR promoter [hereinafter, sometimes abbreviated as Mo promoter] sequence of Moloney murine leukemia virus. The CpoI-ClaI (blunt end) fragment derived from pACREluc, the BssHII-CpoI fragment derived frompAMoh-Gs-i and the XhoI (blunt end)-BssHII fragment derived from pAGE248 were ligated to construct a plasmid pACRElucMoGs-i.

pAMoh-Gs-q was cleaved with BssHII and CpoI to obtain a BssHII-CpoI fragment encoding Gα_{s-q}. The BssHII-CpoI fragment derived from pAMoh-Gs-i and the already-prepared CpoI-ClaI (blunt end) fragment derived from pACREluc and the XhoI (blunt end)-BssHII fragment derived from pAGE248 were ligated to construct a plasmid pACRElucMoGs-q.

pACRElucMoGs-i was cleaved with NaeI and BssHII to obtain a NaeI-BssHII fragment comprising Mo promoter while it was cleaved with CpoI and BssHII to obtain a CpoI-BssHII fragment encoding Gαₛ₋ᵢ. pACRElucMoGs-q was cleaved with NheI and CpoI to obtain a NheI-CpoI fragment comprising CRE-firefly luciferase gene while it was cleaved with NheI and NaeI to obtain a NheI-NaeI fragment encoding Gα_{s-q}. Four fragments comprising the NaeI-BssHII fragment and the CpoI-BssHII fragment derived from pACRElucMoGs-i and the NheI-CpoI fragment and the NheI-NaeI fragment derived from pACRElucMoGs-q were ligated to construct a plasmid pACRElucMoGs-qMoGs-i.

pACRElucMoGs-qMoGs-i plasmid was digested with SalI and NotI and subjected to a Klenow treatment to obtain a SalI (blunt end)-NotI (blunt end) fragment. The fragment were subjected to a self-ligation to construct a plasmid pAMohGs-qMoGs-i where a CRE reporter unit is removed from pACRElucMoGs-qMoGs-i.

pAMohGs-qMoGs-i was cleaved with CpoI, subjected to a Klenow treatment, and further cleaved with AseI to obtain a CpoI (blunt end)-AseI fragment not comprising the hygromycin-resistant gene. pPUR (manufactured by Clontech; GenBank Accession No. U07648) was cleaved with BamHI, subjected to a Klenow treatment, and further cleaved with AseI to obtain a BamHI (blunt end)-AseI fragment comprising the puromycin-resistant gene. The BamHI (blunt end)-AseI fragment derived from pPUR and the already-prepared CpoI (blunt end)-AseI fragment derived from pAMohGs-qMoGs-i were ligated to construct pAMopGs-qMoGs-i where the hygromycin-resistant gene in pAMohGs-qMoGs-i was substituted with the puromycin-resistant gene.

### (5) Preparation of stable transformants where a plasmid pAMopGs-qMoGs-i is integrated in chromosomal DNA of GBC7

After pAMopGs-qMoGs-i was cleaved with SpeI which is a restriction enzyme existing in one place in oriP in pAMopGs-qMoGs-i was dissolved in a TE buffer so as to make 1 µg/µl, it was transfected in an amount of 4 µg per 6 × 10⁶ cells into GBC7 constructed in (4) of Example 19 according to an electroporation method [*Cytotechnology*, 3, 133 (1990)] to give transformants.

The transformants were suspended in 8 ml of RPMI 1640-ITPSG medium and cultured in a CO₂ incubator at 37°C for 24 hours. After the cultivation, blasticidin S (2.0 µg/ml), hygromycin B (300 µg/ml) and puromycin (manufactured by Sigma) (0.2 µg/ml) were added thereto and cultivation was carried out for 7 days more. After the cultivation, the number of living cells of the transformant were determined, the transformants are diluted in an RPMI 1640-ITPSGmedium containing blasticidin S (2.0µg/ml), hygromycin B (300 µg/ml), puromycin (0.2 µg/ml) and KJMGER8 (1 × 10⁶ cells/ml) to make the transformant to be 150 cells/ml, dispensed to a 96-well plate (30 cells/well) and cultured to prepare 93 stable transformants (single clones). Each transformant was subcultured in an RPMI 1640-ITPSG medium containing blasticidin S (2.0 µg/ml), hygromycin B (300 µg/ml) and puromycin (0.2 µg/ml).

### (6) Selection of stable transformants (single clones) having good properties

The 93 clones prepared in the above (5) were plated on a 96-well plate (1 to 2 × 10⁴ cells/well) and pAGal9-AT1 [refer to (3) of the following Example 21] was transfected into each clone using a LipofectAMINE 2000 (manufactured by Gibco) to prepare transformants. Specific method for the gene transfection was carried out in accordance with the directions for LipofectAMINE 2000 and, for each well, 0.3 µg of plasmid and 0.8 µl of LipofectAMINE 2000 were used.

After the transformants were cultured for one day, hygromycin B (300 µg/ml), blasticidin S (2.0 µg/ml), puromycin (0.2 µg/ml) and geneticin (0.5 mg/ml) were added thereto and cultivation was carried out at 37°C for 7 days. To each well was added 17β-estradiol (final concentration 10 nmol/L) and cultivation was carried out for one day more. To each well was added angiotensin II (manufactured by Peptide Laboratories) (final concentration 100 nmol/L) and, after incubating for 6 hours, firefly luciferase activity was measured using the method of (4) of Example 19. At the same time, activity where no stimulation with angiotensin II was done was checked. As a result thereof, 10 clones showing high firefly luciferase activity in response to angiotensin II were selected.

pAGal9-AT1 was transfected into the 10 clones by the above-mentioned electroporation method using 4 µg per 6 × 10⁶ cells to prepare transformants.

The transformants were suspended in 8 ml of an RPMI 1640-ITPSG medium and cultured in a CO₂ incubator at 37°C for 24 hours. After the cultivation, blasticidin S (2.0 µg/ml), hygromycin B (300 µg/ml), puromycin (0.2 µg/ml) and geneticin (500 µg/ml) were added and cultivation was carried out for 14 days more to prepare stable transformants. The transformants were subcultured in an RPMI 1640-ITPSG medium containing blasticidin S (2.0 µg/ml), hygromycin B (300 µg/ml), puromycin (0.2 µg/ml) and geneticin (500 µg/ml).

To each transformant was added 17β-estradiol (final concentration 10 nmol/L) and incubation was carried out for 24 hours and, after that, angiotensin II (final concentration 100 nmol/L) was added, incubation was carried out for 6 hours more and firefly luciferase activity was measured using the above-mentioned method. At the same time, activity where no stimulation with 17β-estradiol was done and activity where no stimulation with angiotensin II was done were measured. GBCC13 which showed a high firefly luciferase activity (85-fold) by reacting with angiotensin II only upon being stimulated with 17β-estradiol was selected as a good cell line.

### (7) Preparation of stable transformants where a plasmid pAMopGs-qMoGs-i is integrated in chromosomal DNA of GBCR2

pAMopGs-qMoGs-i was transfected to GBCR2 using the method mentioned in the above (5) to prepare 94 stable transformants (single clones). Each transformant was subcultured in an RPMI 1640-ITPSG medium containing blasticidin S (2.0 µg/ml), hygromycin B (300 µg/ml) and puromycin (0.2 µg/ml).

### (8) Selection of stable transformants (single clones) having good properties

Selection of clones having good properties from the clones prepared in the above (7) was carried out. The method according to that mentioned in the above (6) was conducted except that activity of *Renilla reniformis* luciferase was measured instead of measurement of firefly luciferase activity. Activity of *Renilla reniformis* luciferase was measured according to the method mentioned in (7) of Example 19.

pAGal9-AT1 was transfected to each of the clones prepared in the above (7) to prepare transformants. Each transformant was treated with 17β-estradiol and stimulated with angiotensin II to measure a *Renilla reniformis* luciferase activity. Simultaneously, an activity in the case without angiotensin II stimulation was measured. Thirteen clones showing high *Renilla reniformis* luciferase activity in response to angiotensin II were selected.

pAGal9-AT1 was transfected to the 13 clones by an electroporation method to prepare transformants. Each transformant was treated with 17β-estradiol and stimulated with angiotensin II to measure a *Renilla reniformis* luciferase activity. At the same time, activity where no stimulation with 17β-estradiol was conducted and activity where no stimulation with angiotensin II was conducted were measured. GBCRC6 which showed a high *Renilla reniformis* luciferase activity (132-fold) in response to angiotensin II only when stimulated with 17β-estradiol was selected as a good cell line.

### [Example 21] Construction and utilization of assay cells for the detection of signals from any GPCR

### (1) Construction and utilization of assay cells for the detection of signals from type 2 vasopressin receptor (V2)

A single-stranded cDNA prepared from mRNA (1 µg; manufactured by Clontech) derived from human kidney was used as a template while, as V2 gene-specific primers, synthetic DNAs having the sequences mentioned by SEQ ID NOS: 99 and 100, respectively, were used whereupon the V2 gene was prepared by a PCR. A HindIII site and a Asp718 site are introduced to the V2 gene-specific primers, respectively.

The resulting V2 gene amplified fragment was cleaved with HindIII and Asp718 to prepare a HindIII-Asp718 fragment. The plasmid pAGal9-d was cleaved with HindIII and Asp718 to prepare a HindIII-Asp718 fragment. The above HindIII-Asp718 fragment derived from the V2 gene amplified fragment and the HindIII-Asp718 fragment derived from pAGal9-d were ligated to construct a V2 inducible expression plasmid pAGal9-V2.

pAGal9-V2 was dissolved in a TE buffer so as to make 1 µg/µl and, after that, it was transfected to GBCRC6 by an electroporation method [*Cytotechology,* 3, 133 (1990)] using 4 µg per 1.6 × 10⁶ cells to give transformants.

The transformants were suspended in 8 ml of an RPMI 1640-ITPSG medium and cultured in a CO₂ incubator at 37°C for 24 hours. After the cultivation, hygromycin B (0.3 mg/ml), blasticidin S (0.2 µg/ml), puromycin (0.2 µg/ml) and geneticin (0.5 mg/ml) were added and cultivation was carried out for 14 days more to prepare stable transformants. The transformants were subcultured in an RPMI 1640-ITPSG medium containing hygromycin B (0.3 mg/ml), blasticidin S (0.2 µg/ml), puromycin (0.2 µg/ml) and geneticin (0.5 mg/ml).

After simulation with 17β-estradiol (final concentration: 10 nmol/L) for 24 hours, vasopressin (Arg8-Vasopressin; manufactured by Peptide Institute) in various concentrations was added and, after 6 hours, activity of *Renilla reniformis* luciferase was measured. The result is shown in Fig. 11.

It is now apparent that, when the present assay system is used, signals from V2 coupled to Gαₛ are able to be detected in a high sensitivity and in a high signal/noise ratio. It is possible to screen agonist or antagonist to V2 using the present assay system.

### (2) Construction and utilization of assay cells for the detection of signals from type 1 corticotropin-releasing hormone receptor (CRHR-1)

A single-stranded cDNA prepared from mRNA (1 µg; manufactured by Clontech) derived from human hypothalamus was used as a template while, as primers specific to the CRHR-1 gene, synthetic DNAs having sequences mentioned by SEQ ID NOS: 101 and 102, respectively, were used whereupon the CRHR-1 gene was prepared by PCR. To the CRHR-1 gene-specific primers, a HindIII site and a NotI site are introduced, respectively.

The resulting CRHR-1 gene amplified fragment was cleaved with HindIII and NotI to obtain a HindIII-NotI fragment. A plasmid pAGal9-d was cleaved with HindIII and NotI to obtain a HindIII-NotI fragment. The above-mentioned HindIII-NotI fragment derived from the CRHR-1 gene amplified fragment and the HindIII-NotI fragment derived from pAGal9-d were ligated to construct pAGal9-CRHR1 which is an inducible expression plasmid of CRHR-1.

pAGal9-CRHR1 was transfected to GBCRC6 according to the methodmentioned in the above (1) to prepare stable transformants. After the transformants were stimulated with 17β-estradiol (final concentration 10 nmol/L) for 24 hours, human corticotropin-releasing hormone (manufactured by Peptide Laboratories) of various concentrations was added thereto and, after 6 hours, activity of *Renilla reniformis* luciferase was measured. The result is shown in Fig. 12.

It has been found that, when the present assay system is used, signals from CRHR-1 coupled to Gαₛ are able to be detected in a high sensitivity and a high signal/noise ratio. Agonist or antagonist of CRHR-1 is able to be screened using the present assay system.

### (3) Construction and utilization of assay cells for the detection of signals from type I angiotensin II receptor (AT1)

A plasmid pAR1.8 [*Nature,* 351, 230 (1991)] was cleaved with HindIII and NotI to obtain a HindIII-NotI fragment comprising the bovine AT1 gene. pAGal9-luc was cleaved with HindIII and NotI to obtain a HindIII-NotI fragment not comprising the firefly luciferase gene. The HindIII-NotI fragment derived from pAR1.8 and the HindIII-NotI fragment derived from pAGal9-luc were ligated to construct pAGal9-AT1.

pAGal9-AT1 was transfected to GBCRC6 according to the methodmentioned in the above (1) to prepare stable transformants. The transformants were stimulated with 17β-estradiol (final concentration 10 nmol/L) for 24 hours, human angiotensin II (manufactured by Bachem) in various concentrations was added and, after 6 hours, activity of *Renilla reniformis* luciferase was measured. The result is shown in Fig. 13.

It is apparent that, when the present assay system is used, signals from AT1 coupled to Gα_{q} or Gαᵢ are able to be detected in a high sensitivity and a high signal/noise ratio. Agonist or antagonist of AT1 is able to be screened using the present assay system.

### (4) Construction and utilization of assay cells for the detection of signals from type 1 bradykinin receptor (B1)

Human chromosomal DNA (40 ng; manufactured by Clontech) was used as a template while, as the B1 gene-specific primers, synthetic DNAs having sequences mentioned in SEQ ID NOS: 103 and 104, respectively, were used to prepare the B1 gene by PCR. To the B1 gene-specific primers, a HindIII site and a Asp718 site were introduced, respectively.

The resulting B1 gene amplified fragment was cleaved with HindIII and Asp718 to obtain a HindIII-Asp718 fragment. The plasmid pAGal9-d was cleaved with HindIII and Asp718I to obtain a HindIII-Asp718 fragment. The HindIII-Asp718 fragment derived from the above B1 gene amplified fragment and the HindIII-Asp718 fragment derived from pAGal9-d were ligated to construct pAGal9-B1 which is an inducible expression plasmid of B1.

pAGal9-B1 was transfected to GBCRC6 according to the methodmentioned in the above (1) toprepare stable transformants. After the transformants were stimulated with 17β-estradiol (final concentration 10 nmol/L) for 24 hours, Des-Arg9-bradykinin (manufactured by Peptide Laboratories) in various concentrations was added and, after 6 hours, activity of *Renilla reniformis* luciferase was measured. The result is shown in Fig. 14.

It is apparent that, when the present assay system is used, signals from B1 coupled to Gα_{q} are able to be detected in a high sensitivity and a high signal/noise ratio. Agonist or antagonist of B1 is able to be screened using the present assay system.

### (5) Construction and utilization of assay cells for the detection of signals from type 5 somatostatin receptor (sst5)

A plasmid pAGal9-sst5 [refer to (2) of Example 23 which will be mentioned later] was transfected to GBCRC6 according to the method mentioned in the above (1) to prepare stable transformants. The transformants were stimulated with 17β-estradiol (final concentration 10 nmol/L) for 24 hours, human somatostatin (manufactured by Peptide Laboratories) in various concentrations was added and, after 6 hours, activity of *Renilla reniformis* luciferase was measured. The result is shown in Fig. 15.

It is apparent that, when the present assay system is used, signals from sst5 coupled to Gαᵢ are able to be detected in a high sensitivity and a high signal/noise ratio. Agonist or antagonist of sst5 is able to be screened using the present assay system.

By the above-mentioned method, good assay cells for the detection of signals from any GPCR coupled to Gαₛ , Gα_{q} or Gαᵢ were able to be constructed. It is possible to screen agonist or antagonist to a specific GPCR using the cells.

### [Example 22] Identification and utilization of a constitutively activated GPCR using a novel host-vector system.

### (1) Construction of inducible expression plasmids of various GPCRs

Various kinds of GPCR genes were prepared by PCR by using single-stranded cDNAs (5 µl) prepared from mRNA (manufactured by Clontech) derived from various human organs or human chromosomal DNA (100 ng; manufactured by Clontech) as templates and by using synthetic DNA having the sequence of SEQ ID NO: mentioned in the following Table 8-1 and Table 8-2 as various GPCR gene-specific primers. With regard to an enzyme, TaKaRa ExTaq (manufactured by Takara Shuzo), Pyrobest DNA Polymerase (manufactured by Takara Shuzo), KOD DNA Polymerase (manufactured by Toyobo), PfuTurbo DNA Polymerase (manufactured by Stratagene), Herculase Enhanced DNA Polymerase (manufactured by Stratagene) or Platinum Pfx DNA Polymerase (manufactured by Gibco) was used. With regard to a buffer for conducting the PCR, a buffer of 10-fold concentration added to the using enzyme was used. In the PCR, a thermal cycler DNA Engine (manufactured by MJ Research) was used and, after treating at 95°C for 5 minutes, a reaction comprising at 94°C for 1 minute, at annealing temperature (55, 60, 62 or 65°C) for 1 minute and at 72°C for 1 minute was carried out for 25 to 35 cycles.

Various kinds of amplified GPCR gene fragments were cleaved with restriction enzymes which cleave the sequence designed on primers used for cloning of each of them (such as HindIII-NotI). Fragments containing a GPCR gene were recovered by an agarose electrophoresis. The cleaved fragments were inserted into restriction enzyme site (such as HindIII-NotI) corresponding to the plasmid pAGal9-nd to construct an inducible expression plasmid. Sequence of the DNA fragment inserted in the plasmid was determined according to the method mentioned in (2) of Example 23 which will be mentioned later to confirm that the aimed GPCR was encoded.

With regard to OGR1, a mutant (called OGR1S221N) where the 221st serine was substituted with asparagine as a result of a PCR error was also isolated. An amino acid sequence of OGR1S221N is shown in SEQ ID NO: 187. A plasmid for expression of the mutant is called pAGal9-OGR1S221N.

### (2) Identification of a constitutively activated GPCR

Inducible expression plasmids for various GPCRs constructed in (1) were used and the constitutive activity of the GPCRs was measured by any of the following methods 1 to 5.

In the following Table 8-1 and Table 8-2, GPCR name, sequence information source for the GPCR gene (accession number of GenBank or International Laid-Open Number), PCR condition, name of GPCR inducible expression plasmid constructed, and constitutive activity value were mentioned only for GPCRs which were found to be constitutively activated GPCRs. The constitutive activity was expressed in an induction ratio of a reporter activity in the case of stimulation with 17β-estradiol.

### [Method 1]

Each of the GPCR inducible expression plasmids constructed hereinabove and pAGal9-nd (control plasmid) was transfected to GBCRC6 according to the method mentioned in (1) of Example 21 to prepare stable transformants. The cells were dispensed in a 96-well plate (about 1 × 10⁴ cells/well) and incubated at 37°C for 1 day and, after that, 17β-estradiol (final concentration 10 nmol/L) was added to each well followed by incubating for 1 day more.

After the incubation, coelenterazineh (Molecular Proves) (final concentration 250 nmol/L) was added to measure the activity of *Renilla reniformis* luciferase. For the activity measurement, a Wallac 1420 ARVOsx Multilabel Counter (manufactured by Wallac Berthold Japan) or a VIM camera (Argus-50/2D Luminometer/MP; manufactured by Hamamatsu Photonics) was used. In addition, the same experiment was carried out without addition of 17β-estradiol and the activity of *Renilla reniformis* luciferase was measured. The value of the activity where 17β-estradiol was added was divided by the activity where no 17β-estradiol was added is named induction ratio.

### [Method 2]

Each of the GPCR inducible expression plasmids constructed hereinabove and pAGal9-nd (control plasmid) was transfected to GBCC13 according to the method mentioned in (1) of Example 21 to give stable transformants. The cells were dispensed in a 96-well plate (about 1 × 10⁴ cells/well) and incubated at 37°C for 1 day, then 17β-estradiol (final concentration 10 nmol/L) was added to each well and incubation was carried out for 1 day more.

After the incubation, Steady Glo Luciferase Assay System (manufactured by Promega) was added and firefly luciferase activity was measured. For the measurement of activity, Auto Lumat LB953 (manufactured by Berthold) or Top Count (Packard) was used. In addition, the same experiment was carried out without addition of 17β-estradiol and activity of firefly luciferase was measured. The value of the activity where 17β-estradiol was added was divided by the activity where no 17β-estradiol was added is named induction ratio.

### [Method 3]

This is a measuring method for constitutive activity of GPCR which is the same as Method 1 except that GBCR2 was used as a host and no puromycin was added in the incubation of the transformants.

### [Method 4]

This is a measuring method for constitutive activity of GPCR which is the same as Method 2 except that GBC7 was used as a host and no puromycin was added in the incubation of the transformants.

### [Method 5]

An inducible expression plasmid constructed hereinabove or pAGal9-nd (control plasmid) was transfected to KJMGER8 according to the method mentioned in (1) of Example 21 together with the reporter plasmid pACREpluc constructed in (1) of Example 19 to give stable transformants.

With regard to a medium for culturing the transformants, a 1640-ITPSG medium to which blasticidin S (0.2 µg/ml), hygromycin B (0.3 mg/ml) and geneticin (0.5 mg/ml) were added was used. The cultured transformants were dispensed in a 96-well plate (about 1 × 10⁴ cells/well) and cultured at 37°C for 1 day and, after that, 17β-estradiol (final concentration 10 nmol/L) was added to each well and cultivation was conducted for 1 day more.

After the cultivation, firefly luciferase activity was measured. The same experiment was conducted without addition of 17β-estradiol and firefly luciferase activity was measured. The value of the activity where 17β-estradiol was added was divided by the activity where no 17β-estradiol was added is named induction ratio.

In each method, the induction ratio of the cells transfected with the control plasmid was about 1. It is judged that the higher the induction ratio of the cells to which a GPCR expression plasmid was transfected, the stronger the constitutive activity of the GPCR.

**Table 8-1**

| GPCR (Information Source for Sequence) | Annealing Temp. | Enzyme | Template (Primer) | Expression Plasmid | Constitutive Activity (Measuring Method) |
|---|---|---|---|---|---|
| GPR 3 (U 18550) | 60°C | KOD | Brain corpus callosum (SEQ ID NOS: 105, 106) | pAGal9-GPR3 | 6.5-fold (Method 5) |
| GPR 4 (U 21051) | 60°C | PfuTurbo | Lung (SEQ ID NOS: 107, 108) | pAGal9-GPR4 | 40-fold (Method 1) |
| GPR 6 (U 18549) | 60°C | ExTaq | Brain (SEQ ID NOS: 109, 110) | pAGal9-GPR6 | 63-fold (Method 1) |
| GPR 12 (U 18548) | 60°C | KOD | Brain corpus callosum (SEQ ID NOS: 111, 112) | pAGal9-GPR12 | 188-fold (Method 3) |
| GPR 25 (U 91939) | 65°C | ExTaq | Human genome (SEQ ID NOS: 113, 114) | pAGal9-GPR25 | 4.8-fold (Method 2) |
| GPR 26 (U 208288) | 55°C | Platinum Pfx | Brain (SEQ ID NOS: 115, 116) | pAGal9-GPR26 | 26-fold (Method 5) |
| GPR 30 (AF 027956) | 65°C | Pyrobest | Human genome (SEQ ID NOS: 117, 118) | pAGal9-GPR30 | 27-fold (Method 1) |
| GPR 35 (NM_005301) | 60°C | PfuTurbo | Human genome (SEQ ID NOS: 119, 120) | pAGal9-GPR35 | 25-fold (Method 1) |
| GPR 52 (AF 096784) | 60°C | PfuTurbo | Caudate nucleus (SEQ ID NOS: 121, 122) | pAGal9-GPR52 | 40-fold (Method 4) |
| GPR 61 (AF 317652) | 65°C | KOD | Human genome (SEQ ID NOS: 123, 124) | pAGal9-GPR61 | 12-fold (Method 1) |
| GPR 62 (AF 317653) | 60°C | Platinum Pfx | Human genome (SEQ ID NOS: 125, 126) | pAGal9-GPR62 | 12-fold (Method 1) |

**Table 8-2**

| GPCR (Information Source for Sequence ) | Annealing Temp. | Enzyme | Template (Primer) | Expression Plasmid | Constitutive Activity (Measuring Method) |
|---|---|---|---|---|---|
| GPR 88 (XM_010608) | 62°C | Platinum Pfx | Human genome (SEQ ID NOS: 127, 128) | pAGal9-GPR 88 | 15-fold (Method 1) |
| GPR 92 (AJ 272207) | 50°C | Herculase | Human genome (SEQ ID NOS: 129, 130) | pAGal9-GPR 92 | 11-fold (Method 1) |
| GPCR 25 (U 95218) | 60°C | Pfu-Turbo | Spleen (SEQ ID NOS: 131, 132) | pAGal9-GPCR 25 | 20-fold (Method 1) |
| RE 2 (AF 091890) | 60°C | Platinum Pfx | Brain (SEQ ID NOS: 133, 134) | pAGal9-RE 2 | 12-fold (Method 1) |
| hRUP4 (WO00/31258) | 50°C | Pyrobest | Kidney (SEQ ID NOS: 135, 136) | pAGal9-hRUP4 | 2.2-fold (Method 1) |
| G2A (NM 013345) | 65°C | Pyrobest | Spleen (SEQ ID NOS: 137, 138) | pAGal9-G2A | 20-fold (Method 1) |
| OGR1 (U 48405) | 60°C | Pfu-Turbo | Brain (SEQ ID NOS: 139, 140) | pAGal9-OGR1 | 3.6-fold (Method 1) |
| OGR1S221N (U 48405) | 60°C | Pfu-Turbo | Brain (SEQ ID NOS: 139, 140) | pAGal9-OGR1S221N | 12.7-fold (Method 1) |
| P2Y12 (XM_010410) | 50°C | Platinum Pfx | Lymph node (SEQ ID NOS: 141, 142) | pAGal9-P2Y12 | 6.0-fold (Method 1) |
| IGS-1 (WO01/091841) | 60°C | KOD | Human genome (SEQ ID NOS: 143, 144) | pAGal9-IGS1 | 85-fold (Method 1) |
| HG52 (AX 147830) | 65°C | Pyrobest | Human genome (SEQ ID NOS: 145, 146) | pAGal9-HG52 | 43-fold (Method 1) |
| RUP12 (AX 148168) | 50°C | Pyrobest | Human genome (SEQ ID NOS: 147, 148) | pAGal9-RUP12 | 7-fold (Method 1) |

As shown above, a constitutive activity of GPCR is able to be measured by any of methods 1 to 5 and it is apparent that the GPCRs prepared here are constitutively activated GPCRs. Although OGR1 itself is a constitutively activated GPCR, the constitutive activity of the mutant OGR1S221N further increased as compared with the constitutive activity of OGR1 itself. The 221st Serine in OGR1 is an amino acid which is the 22nd residue to N-terminal as counted from a proline residue highly conserved in the sixth transmembrane region. When it is applied to the crystal structure of rhodopsin which has been clarif ied recently [*Science,* 289, 739 (2000)], the amino acid at this site is presumed to be an important residue in higher-order structure and activation of GPCR as a whole. With regard to other GPCRs in addition to OGR1, it is also believed that, when the amino acid residue is substituted with other amino acid, a constitutively activated mutant or a mutant where the constitutive activity is enhanced is able to be prepared.

When any substance is added before addition of 17β-estradiol to each of the above-mentioned transformants, it is possible to screen a substance (such as inverse agonist or agonist) which inhibits or increases the constitutive activity of each GPCR.

### [Example 23] Expression cloning of a GPCR gene using a novel host-vector system

### (1) Preparation of a cDNA library derived from human hypothalamus

A kit manufactured by Gibco (superscript Choice System for cDNA Synthesis) was used and double-stranded cDNAs were synthesized from 5.0 µg of poly(A)+RNA derived from human hypothalamus (manufactured by Clontech) using oligo dT as a primer. After adding phosphorylated a SfiI linker (Japanese Published Unexamined Patent Application No. 336963/1993) to both ends of the double-stranded cDNAs, they were subjected to an agarose gel electrophoresis to recover DNA fragments with not less than 1.0 kb.

pAGal9-nd (24 µg) constructed in (3) of Example 18 was dissolved in 590 µl of a buffer (hereinafter, abbreviated as Y-50 buffer) comprising 10 mmol/L Tris-HCl (pH 7.5), 6 mmol/L MgCl₂, 50 mmol/L NaCl and 6 mmol/L 2-mercaptoethanol and, after that, 80 units of SfiI (manufacturedbyTakara Shuzo; with regard to a restriction enzyme, that which is manufactured by Takara Shuzo was used unless otherwise mentioned) was added thereto followed by digestion at 37°C for 16 hours. After the reaction, about 7.0-kb DNA fragments were recovered from the reaction solution by means of an agarose gel electrophoresis.

The double-stranded DNAs (not less than 1.0 kb) to which SfiI linkers were added were dissolved in 250 µl of T4 ligase buffer, then 2 µg of DNA fragments of about 7.0 kb derived from pAGal9-nd and 2000 units of T4DNA ligase were added to the dissolved solution and a ligation reaction was carried out at 16°C for 16 hours. After the reaction, 5 µg of transfer RNA (tRNA) was added to the reaction solution and, after precipitating with ethanol, it was dissolved in 20 µl of a TE buffer.

*Escherichia coli* MC 1061 ("Molecular Cloning", Third Edition) was transformed by an electroporation method [*Nucleic Acids Res.*, 16, 6127 (1988)] using the above dissolved solution to construct a cDNA library (*E. coli*). From the cDNA library, about 3 × 10⁷ transformants (clones) having resistance to ampicillin were obtained.

The cDNA library (*E*. *coli*) was divided into pools each comprising about 100 clones and incubated in a 96-well plate (Multiplate 96FII for incubation of cells; manufactured by Sumitomo Bakelite). A part of the above was taken out, glycerol (final concentration 25%) was added thereto and the mixture was cryopreserved at -80°C.

From the residual culture solution was prepared plasmids using a QIAprep 96 Turbo Miniprep Kit (manufactured by Qiagen). The plasmids were dissolved in a TE buffer so as to make about 0.1 µg/µl.

### (2) Expression cloning of a cDNA encoding a receptor reacting with cortistatin

The plasmids derived from about 100 clones prepared in the above (1) were transfected to GBCRC6 prepared in Example 20 using lipofectAMINE 2000 (manufactured by Gibco), which was done for each pool to the cell. A specific method for the gene transfection was conducted in accordance with the directions for use of LipofectAMINE 2000, and 0.3 µg of plasmid and 0.8 µl of LipofectAMINE 2000 were used per well.

After incubating for one day, each 1/20 amount of the cells were dispensed to a new 96-well plate. The medium was exchanged to an RPMI 1640-ITPSG medium containing hygromycin B (0.3 mg/ml), blasticidin S (0.2 µg/ml), puromycin (0.2 µg/ml) and geneticin (0.5 mg/ml) and incubation was carried out at 37°C for 5 days to prepare stable transformants.

To each well was added 17β-estradiol (final concentration 10 nmol/L) and incubation was carried out for one day more. To each well was added cortistatin (manufactured by Peptide Laboratories) (final concentration 100 nmol/L), incubation was carried out for 6 hours, then coelenterazineh (Molecular Probes) (final concentration 250 nmol/L) was added thereto and activity of *Renilla reniformis* luciferase was measured. For the activity measurement, a Wallac 1420 ARVOsx multilabel counter (manufactured by Wallac Berthold Japan) or a VIM camera (Argus-50/2D luminometer/MP; manufactured by Hamamatsu Photonics) was used.

A pool (100 clones of *E. coli*) corresponding to a well showing high activity was divided for single clone and incubated in a 96-well plate, a part thereof was taken out, glycerol (final concentration 25%) was added thereto and the mixture was cryopreserved at -80°C. APlasmidwaspreparedfromtheresidual culture liquid using a QIAprep 96 Turbo Miniprep Kit (manufactured by Qiagen). The plasmid was dissolved in a TE buffer so as to make about 0.1 µg/µl.

The same assay as above was conducted using the plasmids whereupon a'plasmid giving the reactivity to cortistatin was identified.

Sequences at 5'-side and 3'-side of the cDNA were determined using primers (synthetic DNA having sequences represented by SEQ ID NO: 149 and NO: 150) which are specific to the sequences in pAGal9-nd. Synthetic DNAs specific to the determined sequence were prepared and used as primers and further nucleotide sequence was determined. Such an operation was repeated whereby the entire nucleotide sequence of the cDNA was determined. A DNA sequencer 377 of Perkin-Elmer and a reaction kit (ABI Prism™ BigDye™ Terminator Cycle Sequencing Ready Reaction Kit; Applied Biosystems) were used for the determination of the nucleotide sequences. As a result, it was found that the cDNA contained in the plasmid encodes a type 5 somatostatin receptor (sst5). The plasmid was named pAGal9-sst5.

As mentioned above, it was found that, when the present system is used, a gene having an influence on reactivity to any substance such as cortistatin is able to be obtained. Using cortistatin and GBCRC6 to which pAGal9-sst5 is transfected, it is also possible to screen a substance which inhibits the activation of sst5 by cortistatin.

### (3) Expression cloning of a cDNA encoding a receptor reacting with proadrenomedullin N-20 terminal peptide (PAMP)

PAMP is a peptide comprising 20 amino acids and excised from a precursor protein of adrenomedullin and its receptor has not been clarified yet [*Frontiers in Neuroendocrinology,* 19, 100 (1998)]. Therefore, expression cloning of PAMP receptor gene was attempted.

Screening of a gene affecting on the reactivity to human PAMP-12 was carried out according to the method of the above (2) using human PAMP-12 (human PAMP [9-20]; manufactured by Peptide Laboratories) (final concentration 100 nmol/L) instead of cortistatin (final concentration 100 nmol/L).

The result was that plural plasmids giving the reactivity to PAMP-12 were isolated. When the nucleotide sequences of cDNAs contained in those plasmids were determined, it was found that all of them encode melanocortin receptor type 1 (MC1R). One of the plasmids was named pAGal9-MC1R. The nucleotide sequence was determined by the method mentioned in the above (2).

According to the method mentioned in (1) of Example 21, pAGal9-MC1R was transfected to GBCR2 to prepare stable transformants. The transformants were stimulated with 17β-estradiol (final concentration 10 nmol/L) for 24 hours, then human PAMP-12 or human PAMP (human PAMP [1-20]; manufactured by Peptide Laboratories) in various concentrations was added and, after 6 hours, the activity of *Renilla reniformis* luciferase was measured. The result is shown in Fig. 16.

It was found that MC1R reacts with human PAMP-12 or human PAMP in a very low concentration. Accordingly, it is believed that PAMP-12 or PAMP acts as a ligand for MC1R *in vivo.*

On the basis of the present invention, it has been firstly found that PAMP-12 or PAMP acts as a ligand for MC1R. The present system is also useful for screening of a substance (such as antagonist) which inhibits the activation of MC1R receptor by PAMP-12 or PAMP.

### (4) Expression cloning of cDNAs encoding a constitutively activated GPCR (type 2 metabotropic glutamate receptor; mGlu2), a signal transduction molecule (Gαₛ₁) or transcription factors (DP2 and ID4)

GBCRC6 was suspended in an RPMI 1640-ITPSG medium, dispensed in a 96-well plate (about 1 × 10⁴/well) and incubated at 37°C for one day. After the incubation, the plasmids derived from each pool (about 100 clones) prepared in the above (1) were transfected to the cell for each pool to prepare stable transformants according to the method mentioned in the above (2).

17β-Estradiol (final concentration 10 nmol/L) was added to each well and incubated for one day more and coelenterazine h (final concentration 250 nmol/L) was added to measure the activity of *Renilla reniformis* luciferase. The activity measurement was conducted according to the method mentioned in the above (2).

A pool (*E*. *coli* of 100 clones) corresponding to a well showing a high activity was divided into single clone and incubated in a 96-well plate (Multiplate 96FII for cell incubation; manufactured by Sumitomo Bakelite), a part thereof was taken out, glycerol (final concentration 25%) was added thereto and the mixture was cryopreserved at -80°C. A plasmid was prepared from the residual culture liquid using a QIAprep 96 Turbo Miniprep Kit (manufactured by Qiagen). The plasmid was dissolved in a TE buffer so as to make about 1 µg/µl.

The same assay as above was carried out using the plasmid to isolate 4 kinds of plasmids giving high activity. Nucleotide sequences of cDNAs contained in those plasmids were determined according to the method mentioned in the above (2) and the result was that each of those plasmids contained the gene encoding type 2 metabotoropic glutamate receptor (mGlu2), signal transduction molecule Gαₛ₁, transcription factor DP2, or transcription factor ID4. Each of the plasmids was named pAGal9-mGlu2, pAGal9-Gαs1, pAGal9-dP2 and pAGal9-ID4, respectively.

It has been proved that constitutively activated GPCRs (such as mGlu2), signal transduction molecules (such as Gαₛ₁) and transcription factors (such as DP2 and ID4) are able to be isolated using the present system. The result shows that, when the present system is used, it is possible to isolated molecules which are able to control, either directly or indirectly, gene transcription associated with CRE, in addition to the constitutively activated GPCRs.

It is noted that, when other reporter system is used depending upon the purpose, genes of various molecules which are able to control the activity of the reporter are able to be obtained. For example, when a reporter system for the detection of activation of NFκB is used, genes of various molecules (such as receptors, secreted proteins, signal transduction molecules and transcription factors) participating in activation of NFκB are able to be isolated by the present method.

According to the method mentioned in Example 22, it is possible to screen a substance which inhibits or activates the activity of the above-obtained peptides (such as receptors showing constitutive activity, transcription factors, signal transduction molecules and enzymes). Thus, any substance is added to a transformant where pAGal9-mGlu2 is transfected to GBCRC6 or GBCC13 and, after that, 17β-estradiol is added thereto whereupon it is possible to screen a substance which inhibits or increases the constitutive activity of mGlu2 (such as inverse agonist or agonist).

When any substance is added to a transformant where pAGal9-Gαₛ₁ is transfected to GBCRC6 or GBCC13 and, after that, 17β-estradiol is added, it is possible to screen a substance which inhibits or increases signal transduction in which Gαₛ₁ is participated.

When any substance is added to a transformant where pAGal9-dP2 is transfected to GBCRC6 or GBCC13 and, after that, 17β-estradiol is added, it is possible to screen a substance which inhibits or increases transcription from CRE by way of DP2.

Further, when any substance is added to a transformant where pAGal9-ID4 is transfected to GBCRC6 or GBCC13 and, after that, 17β-estradiol is added, it is possible to screen a substance which inhibits or increases transcription from CRE by way of ID4.

As such, a reporter system using a B-cell line which expresses the EBNA-1 gene and is adapted for serum-free culture is a system with very high applicability and sensitivity, and is a system being very useful for expression cloning, screening of substance, etc.

### [Example 24] Construction of a constitutively activated mutant GPCR using a novel host-vector system

When a library of mutated GPCRs was used instead of a cDNA library in the expression cloning mentioned in Example 23, a constitutively activated mutant GPCR is able to be efficientlyconstructed. It is alsopossible that each specific mutant GPCR is evaluated according to the method mentioned in Example 22 whereby a constitutively activated mutant GPCR is able to be constructed.

### (1) Construction of pAGal9-PGMO334 which is an inducible expression plasmid of PGMO334

pAMo-PGMO334 (Japanese Published Unexamined Patent Application No. 211,885/2001) which is an expression plasmid of PGMO334, which is not a constitutively activated GPCR, was cleavedwith HindIII and NotI to prepare a HindIIT-NotI fragment comprising PGMO334 cDNA. pAGal9-nd was cleaved with HindIII and NotI to prepare a HindIII-NotI fragment of about 1.4 kb. The HindIII-NotI fragment derived from pAMo-PGMO334 and the HindIII-NotI fragment derived from pAGal9-nd were ligated to construct pAGal9-PGMO334 which is an inducible expression plasmid of PGMO334.

### (2) Construction of a mutated GPCR library

Many mutants where random mutations were introduced to the area ranging from the second half of the third transmembrane region to the first half of the second intracellular domain of PGMO334 polypeptide or to the area ranging from the second half of the third intracellular domain to the first half of the sixth transmembrane region thereof were constructed as follows utilizing PCRs.

Synthetic DNAs having nucleotide sequences mentioned in SEQ ID NOS: 151 to 160 were prepared to introduce randommutations to the area ranging from the second half of the third transmembrane region to the first half of the second intracellular domain of PGMO334 polypeptide (from the 132nd amino acid to the 141st amino acid of PGMO334 polypeptide).

Synthetic DNAs having nucleotide sequences mentioned in SEQ ID NOS: 161 to 17 0 were prepared to introduce randommutations to the area ranging from the second half of the third intracellular domain to the first half of the sixth transmembrane region of PGMO334 polypeptide (from the 256th amino acid to the 265th amino acid of PGMO334 polypeptide).

Synthetic DNAs were prepared having nucleotide sequences mentioned in SEQ ID NOS: 171 and 172 as primers corresponding to the vector parts of 5'-side and 3'-side of PGMO334 cDNA in pAGal9-PGMO334.

pAGal9-PGMO334 (1 ng), any one of synthetic DNAs having nucleotide sequences mentioned in SEQ ID NOS: 151 to 160 (20 pmol), a synthetic DNA having a nucleotide sequence mentioned in SEQ ID NO: 172 (20 pmol), 1.6 µl of a 2.5 mmol/l dNTP mixed solution, 1 µl of DMSO, 0.1 µl of a 5 units/µl of Pyrobest DNA Polymerase (manufactured by Takara Shuzo) and 2 µl of 10 × reaction buffer were added and then sterilized water was added thereto whereupon the total volume was made 20 µl. PCR was carried out under the following condition using the prepared solution. Thus, using a thermal cycler DNA Engine (manufactured by MJ Research), treatment at 95°C for 5 minutes was conducted and a reaction comprising at 94°C for 1 minute, at 50°C for 30 seconds and at 72°C for 1 minute was carried out for 25 cycles.

From the reaction solution, amplified DNA fragments (mutated DNA1) were recovered by means of an agarose gel electrophoresis. Each of the DNA fragments is a DNA encoding a polypeptide (a polypeptide of the C-terminal side) to which a random mutation is introduced into any one of the 132nd to the 141st amino acids of PGMO334 polypeptide.

Similarly, a PCR was carried out using pAGal9-PGMO334 as a template and any one of synthetic DNAs having nucleotide sequences mentioned in SEQ ID NOS: 161 to 170 and a synthetic DNA having a nucleotide sequence mentioned in SEQ ID NO: 171 as primers to prepare a DNA (mutated DNA2) encoding a polypeptide (a polypeptide of the N-terminal side) to which a random mutation was introduced to any one of the amino acids from 256th one to 265th one of PGMO334 polypeptide.

A 1/25 amount of the prepared mutated DNA1 and a 1/25 amount of the prepared mutated DNA2, 1.6 µl of a 2.5 mmol/l of dNTP mixed solution, 1 µl of DMSO, 0.1 µl of a 5 units/µl of Pyrobest DNA Polymerase (manufactured by Takara Shuzo) and 2 µl of 10 × reaction buffer were added and then sterilized water was added thereto so as to make the total volume 20 µl. The solution prepared as such was used and subjected to a PCR under the following condition. Thus, using a thermal cycler DNA Engine (manufactured by MJ Research), treatment at 98°C for 5 minutes was conducted and a reaction comprising at 98°C for 15 seconds, at 50°C for 20 seconds and at 72°C for 30 seconds was carried out for 6 cycles.

After addition of a synthetic DNA (20 pmol) having a nucleotide sequence mentioned in SEQ ID NO: 171 and a synthetic DNA (20 pmol) having a nucleotide sequence mentioned in SEQ ID NO: 172 to the reaction solution, using a thermal cycler DNA Engine (manufactured by MJ Research), treatment at 95°C for 2 minutes was conducted and, after that, a reaction comprising at 95°C for 15 seconds, at 60°C for 20 seconds and at 72°C for 1 minute was carried out for 23 cycles.

From the reaction solution, amplified DNA fragments (mutated DNA3) were recovered by means of an agarose gel electrophoresis. The mutated DNA3 was cleaved with HindIII and NotI and inserted between HindIII-NotI of the plasmid pAGal9-nd to construct a mutated PGMO334 library which is able to inducibly express the PGMO334 mutants. Construction of the library was carried out according to the method mentioned in (1) of Example 22. Thus, *Escherichia coli* MC 1061 (Molecular Cloning, third edition) was used as a host to prepare about 1 × 10⁴ transformants having resistance to ampicillin whereupon a mutated PGMO334 library (*E. coli*) was constructed.

The mutated PGMO334 library (*E. coli*) was divided into pools each comprising 10 clones and incubated in a 96-well plate (Multiplate 96FII for incubation of cells; manufactured by Sumitomo Bakelite), a part of it was taken out, glycerol (final concentration 25%) was added thereto and the mixture was cryopreserved at -80°C. Plasmids were prepared from the residual culture liquid using a QIAprep 96 Turbo Miniprep Kit (manufactured by Qiagen). The plasmids were dissolved in a TE buffer so as to make about 0.1 µg/µl.

### (3) Preparation of a constitutively activated mutant GPCR

According to the method mentioned in (2) of Example 23, each of the plasmids derived from 10 clones of the mutated GPCR library prepared in the above (2) was transfected to GBCRC 6 to obtain stable transformants. To each well was added 17β-estradiol (final concentration 10 nmol/L), incubation was carried out for one day more, coelenterazine h (final concentration 250 nmol/L) was added thereto and activity of *Renilla reniformis* luciferase was measured. The activity measurement was conducted by the method mentioned in (2) of Example 23.

A pool (*E. coli* of 10 clones) corresponding to a well showing a high activity was divided into a single clone and incubated in a 96-well plate (Multiplate 96FII for cell incubation; manufactured by Sumitomo Bakelite), a part thereof was taken out, glycerol (final concentration 25%) was added thereto and the mixture was cryopreserved at -80°C. A plasmid was prepared from the residual culture liquid using a QIAprep 96 Turbo Miniprep Kit (manufactured by Qiagen). The plasmid was dissolved in a TE buffer so as to make about 0.1 µg/µl.

The same assay as above was carried out using the plasmid to identify a plasmid giving high activity. After the identification, the nucleotide sequence of cDNA contained in the plasmid was determined according to the method mentioned in (2) of Example 23.

As a result of the above screening, the following 4 kinds of constitutively activated mutants were isolated.
(i) A mutant (called PGMO334E135F) where the 135th glutamic acid of the PGMO334 polypeptide was substituted with phenylalanine: As a result of evaluation by the method mentioned in (2) of Example 22 (mostly utilizing GBCRC6 as a host), an induction ratio of about 3-fold was noted whereby it was confirmed to be a constitutively activated GPCR. The amino acid sequence is shown in SEQ ID NO: 193.
(ii) A mutant (called PGMO334E135Q) where the 135th glutamic acid of PGMO334 polypeptide was substituted with glutamine: As a result of the same evaluation, an induction ratio of about 3-fold was noted whereby it was confirmed to be a constitutively activated GPCR. The amino acid sequence is shown in SEQ ID NO: 194.
(iii) A mutant (called PGMO334E135A) where the 135th glutamic acid of PGMO334 polypeptide was substituted with alanine: As a result of the same evaluation, an induction ratio of about 14-fold was noted whereby it was confirmed to be a constitutively activated GPCR. The amino acid sequence is shown in SEQ ID NO: 195.
(iv) A mutant (called PGMO334D259S) where the 259th aspartic acid of PGMO334 polypeptide was substituted with serine: As a result of the same evaluation, an induction ratio of about 5-fold was noted whereby it was confirmed to be a constitutively activated GPCR. The amino acid sequence is shown in SEQ ID NO: 196. The 259th aspartic acid of PGMO334 is the amino acid which is the 20th residue to the N-terminal counted fromproline residue highly conserved in the sixth transmembrane region and, in many GPCRs, glutamic acid or aspartic acid is conserved. When that is applied to crystal structure of rhodopsin which has been clarified recently [*Science*, 289, 739 (2000)], the side chain of the amino acid in this site faces to aspartic acid or glutamic acid residue in a D/ERY motif mentioned in the following (4) in view of steric structures and it is presumed to be an important residue for higher-order structure and activation of GPCRs as a whole. With regard to GPCRs other than PGMO334, it is believed that a constitutively activated mutant is able to be prepared when the amino acid residue is substituted with other amino acid.

In addition, with regard to molecules (such as transcription factors, signal transduction molecules or enzymes) having an activity of increasing the transcription from the used transcription factor-responsive element (such as CRE and NFκB-responsive element) either directly or indirectly, it is also possible to isolate a constitutively activated mutant by introducing random mutations by the same manner as above.

It is further possible to isolate a mutant showing no constitutive activity any more or a dominant-negative mutant by introduction of random mutations into the above molecule.

### (4) Construction of other constitutively activated mutants

In the intracellular side of the third transmembrane region of GPCR, a highly conserved D/ERY motif is present. It has been suggested that aspartic acid or glutamic acid residue in the motif plays an important role in an interaction with other transmembrane region [*Science,* 289, 739 (2000); *EMBO J*., 12 , 1693 (1993)]. It has been also known that, in some of GPCR (such as V2 vasopressin receptor), a constitutively activated mutant is able to be prepared by substituting the aspartic acid or glutamic acid residue with other amino acid [*FEBS Letter,* 441, 470 (1998)]. Therefore, with regard to many orphan GPCRs, Construction of DNA encoding a mutant where the aspartic acid or glutamic acid residue was converted to alanine as follow was conducted and constitutive activity of the mutant was evaluated.

The aimed mutagenesis to a GPCR gene was carried out as follows using QuickChange Site-Directed Mutagenesis Kit (manufactured by Stratagene) and according to a protocol of the kit.

In the construction of mutants of orphan GPCRs as shown in Table 9, complementary two primers of about 35 bases shown in Table 9 containing the aimed mutation and having the same sequence as surrounding area of the mutation introduction site were synthesized. PCR was carried out using the primers where a GPCR expression plasmid was used as a template. DpnI (a restriction enzyme which selectively cleaves methylated DNA) was added to the reaction solution and the template plasmid was selectively cleaved. The DNA fragment prepared as such contains vector part and a gene to which the aimed mutation is introduced. The resulting DNA fragment was transformed to *Escherichia coli* to give a plasmid containing the gene to which aimed mutation was introduced.

Constitutive activity of the mutant GPCR encoded by the gene contained in the resulting plasmid was measured by the method 1 mentioned in (2) of Example 22.

Mutants of orphan GPCRs where potentiation of constitutive activity was found as a result thereof are shown in Table 9.

**Table 9**

| GPCR Mutant (original GPCR) | Mutated Position | Mutation | Template Plasmid (SEQ ID NO of primer) | Expression Plasmid | Constitutive Activity |
|---|---|---|---|---|---|
| OGR1S221N/D118A (OGR1S221N) | 118 | Asp → Ala | pAgal9-OGR1S221N | pAGal19-OGR1S221N/D118A | 56-fold |
| RE2D124A (RE2) | 124 | Asp → Ala | pAgal9-RE2 (175, 176) | pAGal9-RE2D124A | 32-fold |
| GPR35D113A (GPR35) | 113 | Asp → Ala | pAgal9-GPR35 (177, 178) | pAGal9-GPR35D113A | 97-fold |
| GPCR25D111A (GPCR25) | 111 | Asp → Ala | pAgal9-GPCR25 (179, 180) | pAGal9-GPCR25D111A | 35-fold |
| Mutated position: The position from N-terminal of polypeptide | | | | | |
| Asp → Ala: Substitution of aspartic acid with alanine | | | | | |

As shown in Table 8-1 or Table 8-2, although OGR1S221N, RE2, GPR35 and GPCR25 are inherently constitutively activated GPCRs, it was clarified that their constitutive activity further increases by introducing above mutations. On the other hand, it was also clarified that such a mutation did not always construct a constitutively activated GPCR. Amino acid sequences of OGR1S221N/D118A, RE2D124A, GPR35D113A and GPCR25FD111A are shown in SEQ ID NOS: 189, 190, 191 and 192, respectively. Like OGR1S221N/D118A, it is also believed that a mutant OGR1D118 (amino acid sequence thereof is shown in SEQ ID NO: 188) where the 118th aspartic acid of OGR1 was substituted with alanine similarly shows a constitutive activity.

### [Example 25] Screening of ligands or agonists of orphan GPCRs using a novel host-vector system

### (1) Construction of pAGal9-GPR43 which is an inducible expression plasmid of GPR43

According to the method mentioned in (1) of Example 20, the GPR43 gene was prepared by PCR and the gene was inserted in an expression vector pAGal9-d to construct pAGal9-GPR43 which is an inducible expression plasmid of GPR43. With regard to a template, human chromosomal DNA (100 ng; manufactured by Clontech) was used. With regard to the GPR43 gene-specific primers, synthetic DNA having sequences mentioned in SEQ ID NOS: 181 and 182, respectively, were used. A HindIII site and a NotI site were introduced to the above primers, respectively.

PCR-amplified fragment was cleaved with HindIII and NotI to prepare a HindIII-NotI fragment. A plasmid pAGal9-d was cleaved with HindIII and NotI to prepare a HindIII-NotI fragment. The HindIII-NotI fragment derived from PCR fragment and the HindIII-NotI fragment derived from pAGal9-d were ligated to construct pAGal9-GPR43 which was an inducible expression plasmid of GPR43.

### (2) Screening ligands or agonists of GPR43

According to the method mentioned in (1) of Example 21, pAGal9-GPR43 was transfected to GBCRC6 to prepare stable transformants. After the transformants were stimulated with 17β-estradiol (final concentration 10 nmol/L) for 24 hours, various substances of 100 nmol/L to 100 µmol/L were added thereto and, after 6 hours, activity of *Renilla reniformis* luciferase was measured.

As a result, it was found that 100 µmol/L of acetic acid (CH₃COOH), propionic acid (CH₃CH₂COOH) and acetate (CH₃COONa) acted as agonists for GPR43 (induction ratio of the reporter was 4-fold, 5-fold and 9-fold, respectively). Accordingly, it is possible to screen an antagonist for GPR43 using acetic acid, propionic acid, acetate or propionate as an agonist. Since acetic acid and acetate did not act on assay cells to which other GPCRs were transfected, they were found to have a GPR43-specific agonist activity. As shown in (4) which will be given later, propionic acid also showed an agonist activity to GPR41.

### (3) Construction of pAGal9-GPR41 which is an inducible expression plasmid of GPR41

According to the method mentioned in (1) of Example 20, the GPR41 gene was prepared by PCR and the gene was inserted in an expression vector pAGal9-d to construct pAGal9-GPR41 which is an inducible expression plasmid of GPR41. With regard to a template, human chromosomal DNA (100 ng; manufactured by Clontech) was used. With regard to the GPR41 gene-specific primers, synthetic DNAs having sequences mentioned in SEQ ID NOS: 183 and 184, respectively, were used. To the above primers, a HindIII site and a NotI site were introduced, respectively.

A PCR-amplified fragment was cleaved with HindIII and NotI to prepare a HindIII-NotI fragment. A plasmid pAGal9-d was cleaved with HindIII and NotI to prepare a HindIII-NotI fragment. The HindIII-NotI fragment derived from PCR fragment and the HindIII-NotI fragment derived frompAGal9-dwere ligated to construct pAGal9-GPR41 which was an inducible expression plasmid of GPR41.

### (4) Screening of ligands or agonists of GPR41

According to the method mentioned in (1) of Example 21, pAGal9-GPR41 was transfected to GBCRC6 to prepare stable transformants. After the transformants were stimulated with 17β-estradiol (final concentration 10 nmol/L) for 24 hours, various substances of 100 nmol/L to 1000 nmol/L were added thereto and, after 6 hours, activity of *Renilla reniformis* luciferase was measured.

As a result, it was found that 100 µmol/L of propionic acid (CH₃CH₂COOH) and cyclopropanecarboxylic acid acted as agonists for GPR41 (induction ratio of the reporters was 21-fold and 22-fold, respectively). Accordingly, it is possible to screen an antagonist for GPR41 using propionic acid or cyclopropanecarboxylic acid as an agonist. Since cyclopropanecarboxylic acid did not act on assay cells to which other GPCRs were transfected, that was found to have a GPR41-specific agonist activity. Acetic acid and acetate which showed an agonist activity to GPR43 did not show an agonist activity to GPR41.

### (5) Construction of pAGal9-G10d which is an inducible expression plasmid of G10d

According to the method mentioned in (1) of Example 20, the G10d gene was prepared by PCR and the gene was inserted in an expression vector pAGal9-d to construct pAGal9-G10d which is an inducible expression plasmid of G10d. With regard to a template, single-stranded DNAs derived from human spleen were used. With regard to the G10d gene-specific primers, synthetic DNAs having sequences mentioned in SEQ ID NOS: 185 and 186 were used. To the above primers, a HindIII site and a NotI site were introduced, respectively.

A PCR-amplified fragment was cleaved with HindIII and NotI to prepare a HindIII-NotI fragment. A plasmid pAGal9-d was cleaved with HindIII and NotI to prepare a HindIII-NotI fragment. The HindIII-NotI fragment derived from the PCR fragment and the HindIII-NotI fragment derived from pAGal9-d were ligated to construct pAGal9-G10d which was an inducible expression plasmid of G10d.

### (6) Screening of ligands or agonists of G10d

According to the method mentioned in (1) of Example 21, pAGal9-G10d was transfected to GBCRC6 to prepare stable transformants. After the transformants were stimulated with 17β-estradiol (final concentration 10 nmol/L) for 24 hours, various substances of 100 nmol/L or 1000 nmol/L were added thereto and, after 6 hours, activity of *Renilla reniformis* luciferase was measured. As a result, it was found that 100 µmol/L of α-melanocyte-stimulating hormone (α-MSH) and 100 nmol/L of adrenocorticotropic hormone (ACTH) acted as agonists for G10d (induction ratio of the reporters was 10-fold and 11-fold, respectively). α-MSH or ACTH did not act on GBCRC6 to which control vector (pAGal9-d) was transfected. It is possible to screen an antagonist for G10d using α-MSH or ACTH as an agonist.

### [Example 26] Construction of a constitutively activated mutant of GPR43

By the same method as mentioned in Example 24, a constitutively activated mutant of GPR43 was constructed.

### (1) Construction of a mutated GPR43 library

By the same method as mentioned in (2) of Example 24, there was constructed a mutated GRP43 library in which random mutations were introduced to the area ranging from the second half of the third transmembrane region to the first half of the second intracellular domain of GPR43 polypeptide (from the 103rd amino acid to the 112th amino acid of GPR43 polypeptide), or to the area ranging from the second half of the third intracellular domain to the first half of the sixth transmembrane region thereof (from the 213th amino acid to the 222nd amino acid of GPR43 polypeptide). With regard to a template of the PCR for the introduction of randommutations, pAGal9-GPR43 which is a GPR43 inducible expression plasmid constructed in (1) of Example 25 was used.

### (2) Preparation of a constitutively activated mutant of GPR43

By the same method as mentioned in (3) of Example 24, a constitutively activated mutant of GPR43 was selected using the mutated GPR43 library constructed in the above (1). As a result of evaluation of a mutant (called GPR43R217P), where the 217th arginine of GPR43 polypeptide was substituted with proline by the method mentioned in (2) of Example 22, utilizing GBCRC6 as a host, induction ratio of about 2-fold was noted whereby it was confirmed to be a constitutively activated GPCR. An amino acid sequence of GPR43R217P is shown in SEQ ID NO: 197.

### (3) Construction of a mutant where further mutation was introduced to GPR43R217P polypeptide

By the same method for the introduction of a random mutation as mentioned in (2) of Example 24, there was constructed a DNA encoding a mutant where the 106th glutamic acid residue in a D/ERY motif of GPR43R217P polypeptide was substituted with other amino acids. With regard to a template for the mutagenesis, pAGal9-GPR43R217P which is an inducible expression plasmid of GPR43R217P isolated in the above (2) was used.

Constitutive activity of the mutants encoded by DNAs constructed above was evaluated by the method mentioned in (2) of Example 22 (utilizing GBCRC6 as a host) whereby a mutant in which a constitutive activity further increased was selected. As a result, it was found that a mutant (called GPR43R217PE106D) where the 106th glutamic acid of GPR43R217P polypeptide was substituted with aspartic acid showed induction ratio of about 17-fold and, as compared with GPR43R217P polypeptide, it was found that the constitutive activity further increased. An amino acid sequence of GPR43R217PE106D is shown in SEQ ID NO: 198.

A DNA encoding GPR43R217PE106D is also able to be constructed by a kit for mutagenesis (Quick Change Site-Directed Mutagenesis Kit manufactured by Stratagene) using pAGal9-GPR43 which is an inducible expression plasmid of GPR43 as a template.

### Industrial Applicability

In accordance with the present invention, there are provided various cell lines derived from endocrine cells of mammals, process for the production of active peptides and expression cloning system of active peptide precursor genes using the cell line as a host, method for the screening and for the evaluation of a substance acting on the cells using the cell line, method for the screening and for the preparation of useful genes and useful peptides using the cell lines and a highly-sensitive and simple assay system for GPCR ligands used in the above expression cloning system.

### Free Text of Sequence Listing

SEQ ID NO: 1 - Inventors: Katsutoshi Sasaki, Kazumi Miura and Satoshi Saeki; Inventors: Misako Yoshizawa and Kazuya Kishimoto; Inventors: Hirofumi Kunitomo, Tatsunari Nishi and Masuo Obinata
SEQ ID NO: 1 - Synthetic DNA
SEQ ID NO: 2 - Synthetic DNA
SEQ ID NO: 3 - Synthetic DNA
SEQ ID NO: 4 - Synthetic DNA
SEQ ID NO: 5 - Synthetic DNA
SEQ ID NO: 6 - Synthetic DNA
SEQ ID NO: 7 - Synthetic DNA
SEQ ID NO: 8 - Synthetic DNA
SEQ ID NO: 9 - Synthetic DNA
SEQ ID NO: 10 - Synthetic DNA
SEQ ID NO: 11 - Synthetic DNA
SEQ ID NO: 12 - Synthetic DNA
SEQ ID NO: 13 - Synthetic DNA
SEQ ID NO: 14 - Synthetic DNA
SEQ ID NO: 15 - Synthetic DNA
SEQ ID NO: 16 - Synthetic DNA
SEQ ID NO: 17 - Synthetic DNA
SEQ ID NO: 18 - Synthetic DNA
SEQ ID NO: 19 - Synthetic DNA
SEQ ID NO: 20 - Synthetic DNA
SEQ ID NO: 21 - Synthetic DNA
SEQ ID NO: 22 - Synthetic DNA
SEQ ID NO: 23 - Synthetic DNA
SEQ ID NO: 24 - Synthetic DNA
SEQ ID NO: 25 - Synthetic DNA
SEQ ID NO: 26 - Synthetic DNA
SEQ ID NO: 27 - Synthetic DNA
SEQ ID NO: 28 - Synthetic DNA
SEQ ID NO: 29 - Synthetic DNA
SEQ ID NO: 30 - Synthetic DNA
SEQ ID NO: 31 - Synthetic DNA
SEQ ID NO: 32 - Synthetic DNA
SEQ ID NO: 33 - Synthetic DNA
SEQ ID NO: 34 - Synthetic DNA
SEQ ID NO: 35 - Synthetic DNA
SEQ ID NO: 36 - Synthetic DNA
SEQ ID NO: 37 - Synthetic DNA
SEQ ID NO: 38 - Synthetic DNA
SEQ ID NO: 39 - Synthetic DNA
SEQ ID NO: 40 - Synthetic DNA
SEQ ID NO: 41 - Synthetic DNA
SEQ ID NO: 42 - Synthetic DNA
SEQ ID NO: 43 - Synthetic DNA
SEQ ID NO: 44 - Synthetic DNA
SEQ ID NO: 45 - Synthetic DNA
SEQ ID NO: 46 - Synthetic DNA
SEQ ID NO: 47 - Synthetic DNA
SEQ ID NO: 48 - Synthetic DNA
SEQ ID NO: 49 - Synthetic DNA
SEQ ID NO: 50 - Synthetic DNA
SEQ ID NO: 51 - Synthetic DNA
SEQ ID NO: 52 - Synthetic DNA
SEQ ID NO: 53 - Synthetic DNA
SEQ ID NO: 54 - Synthetic DNA
SEQ ID NO: 55 - Synthetic DNA
SEQ ID NO: 56 - Synthetic DNA
SEQ ID NO: 57 - Synthetic DNA
SEQ ID NO: 58- Synthetic DNA
SEQ ID NO: 59 - Synthetic DNA
SEQ ID NO: 60 - Synthetic DNA
SEQ ID NO: 61 - Synthetic DNA
SEQ ID NO: 62 - Synthetic DNA
SEQ ID NO: 63 - Synthetic DNA
SEQ ID NO: 64 - Synthetic DNA
SEQ ID NO: 65 - Synthetic DNA
SEQ ID NO: 66 - Synthetic DNA
SEQ ID NO: 67 - Synthetic DNA
SEQ ID NO: 68 - Synthetic DNA
SEQ ID NO: 69 - Synthetic DNA
SEQ ID NO: 70 - Synthetic DNA
SEQ ID NO: 71 - Synthetic DNA
SEQ ID NO: 72 - Synthetic DNA
SEQ ID NO: 73 - Synthetic DNA
SEQ ID NO: 74 - Synthetic DNA
SEQ ID NO: 75 - Synthetic DNA
SEQ ID NO: 76 - Synthetic DNA
SEQ ID NO: 77 - Synthetic DNA
SEQ ID NO: 78 - Synthetic DNA
SEQ ID NO: 79 - Synthetic DNA
SEQ ID NO: 80 - Synthetic DNA
SEQ ID NO: 81 - Synthetic DNA
SEQ ID NO: 82 - Synthetic DNA
SEQ ID NO: 83 - Synthetic DNA
SEQ ID NO: 84 - Synthetic DNA
SEQ ID NO: 85 - Synthetic DNA
SEQ ID NO: 86 - Synthetic DNA
SEQ ID NO: 87 - Synthetic DNA
SEQ ID NO: 88 - Synthetic DNA
SEQ ID NO: 89 - Synthetic DNA
SEQ ID NO: 90 - Synthetic DNA
SEQ ID NO: 91 - Synthetic DNA
SEQ ID NO: 92 - Synthetic DNA
SEQ ID NO: 93 - Synthetic DNA
SEQ ID NO: 94 - Synthetic DNA
SEQ ID NO: 95 - Synthetic DNA
SEQ ID NO: 96 - Synthetic DNA
SEQ ID NO: 97 - Synthetic DNA
SEQ ID NO: 98 - Synthetic DNA
SEQ ID NO: 99 - Synthetic DNA
SEQ ID NO: 100 - Synthetic DNA
SEQ ID NO: 101 - Synthetic DNA
SEQ ID NO: 102 - Synthetic DNA
SEQ ID NO: 103 - Synthetic DNA
SEQ ID NO: 104 - Synthetic DNA
SEQ ID NO: 105 - Synthetic DNA
SEQ ID NO: 106 - Synthetic DNA
SEQ ID NO: 107 - Synthetic DNA
SEQ ID NO: 108 - Synthetic DNA
SEQ ID NO: 109 - Synthetic DNA
SEQ ID NO: 110 - Synthetic DNA
SEQ ID NO: 111 - Synthetic DNA
SEQ ID NO: 112 - Synthetic DNA
SEQ ID NO: 113 - Synthetic DNA
SEQ ID NO: 114 - Synthetic DNA
SEQ ID NO: 115 - Synthetic DNA
SEQ ID NO: 116 - Synthetic DNA
SEQ ID NO: 117 - Synthetic DNA
SEQ ID NO: 118 - Synthetic DNA
SEQ ID NO: 119 - Synthetic DNA
SEQ ID NO: 120 - Synthetic DNA
SEQ ID NO: 121 - Synthetic DNA
SEQ ID NO: 122 - Synthetic DNA
SEQ ID NO: 123 - Synthetic DNA
SEQ ID NO: 124 - Synthetic DNA
SEQ ID NO: 125 - Synthetic DNA
SEQ ID NO: 126 - Synthetic DNA
SEQ ID NO: 127 - Synthetic DNA
SEQ ID NO: 128 - Synthetic DNA
SEQ ID NO: 129 - Synthetic DNA
SEQ ID NO: 130 - Synthetic DNA
SEQ ID NO: 131 - Synthetic DNA
SEQ ID NO: 132 - Synthetic DNA
SEQ ID NO: 133 - Synthetic DNA
SEQ ID NO: 134 - Synthetic DNA
SEQ ID NO: 135 - Synthetic DNA
SEQ ID NO: 136 - Synthetic DNA
SEQ ID NO: 137 - Synthetic DNA
SEQ ID NO: 138 - Synthetic DNA
SEQ ID NO: 139 - Synthetic DNA
SEQ ID NO: 140 - Synthetic DNA
SEQ ID NO: 141 - Synthetic DNA
SEQ ID NO: 142 - Synthetic DNA
SEQ ID NO: 143 - Synthetic DNA
SEQ ID NO: 144 - Synthetic DNA
SEQ ID NO: 145 - Synthetic DNA
SEQ ID NO: 146 - Synthetic DNA
SEQ ID NO: 147 - Synthetic DNA
SEQ ID NO: 148 - Synthetic DNA
SEQ ID-NO: 149 - Synthetic DNA
SEQ ID NO: 150 - Synthetic DNA
SEQ ID NO: 151 - Synthetic DNA
SEQ ID NO: 151 - n is a, g, c or t
SEQ ID NO: 152 - Synthetic DNA
SEQ ID NO: 152 - n is a, g, c or t
SEQ ID NO: 153 - Synthetic DNA
SEQ ID NO: 153 - n is a, g, c or t
SEQ ID NO: 154 - Synthetic DNA
SEQ ID NO: 154 - n is a, g, c or t
SEQ ID NO: 155 - Synthetic DNA
SEQ ID NO: 155 - n is a, g, c or t
SEQ ID NO: 156 - Synthetic DNA
SEQ ID NO: 156 - n is a, g, c or t
SEQ ID NO: 157 - Synthetic DNA
SEQ ID NO: 157 - n is a, g, c or t
SEQ ID NO: 158 - Synthetic DNA
SEQ ID NO: 158 - n is a, g, c or t
SEQ ID NO: 159 - Synthetic DNA
SEQ ID NO: 159 - n is a, g, c or t
SEQ ID NO: 160 - Synthetic DNA
SEQ ID NO: 160 - n is a, g, c or t
SEQ ID NO: 161 - Synthetic DNA
SEQ ID NO: 161 - n is a, g, c or t
SEQ ID NO: 162 - Synthetic DNA
SEQ ID NO: 162 - n is a, g, c or t
SEQ ID NO: 163 - Synthetic DNA
SEQ ID NO: 163 - n is a, g, c or t
SEQ ID NO: 164 - Synthetic DNA
SEQ ID NO: 164 - n is a, g, c or t
SEQ ID NO: 165 - Synthetic DNA
SEQ ID NO: 165 - n is a, g, c or t
SEQ ID NO: 166 - Synthetic DNA
SEQ ID NO: 166 - n is a, g, c or t
SEQ ID NO: 167 - Synthetic DNA
SEQ ID NO: 167 - n is a, g, c or t
SEQ ID NO: 168 - Synthetic DNA
SEQ ID NO: 168 - n is a, g, c or t
SEQ ID NO: 169 - Synthetic DNA
SEQ ID NO: 169 - n is a, g, c or t
SEQ ID NO: 170 - Synthetic DNA
SEQ ID NO: 170 - n is a, g, c or t
SEQ ID NO: 171 - Synthetic DNA
SEQ ID NO: 172 - Synthetic DNA
SEQ ID NO: 173 - Synthetic DNA
SEQ ID NO: 174 - Synthetic DNA
SEQ ID NO: 175 - Synthetic DNA
SEQ ID NO: 176 - Synthetic DNA
SEQ ID NO: 177 - Synthetic DNA
SEQ ID NO: 178 - Synthetic DNA
SEQ ID NO: 179 - Synthetic DNA
SEQ ID NO: 180 - Synthetic DNA
SEQ ID NO: 181 - Synthetic DNA
SEQ ID NO: 182 - Synthetic DNA
SEQ ID NO: 183 - Synthetic DNA
SEQ ID NO: 184 - Synthetic DNA
SEQ ID NO: 185 - Synthetic DNA
SEQ ID NO: 186 - Synthetic DNA
SEQ ID NO: 187 - OGR1S221N
SEQ ID NO: 188 - OGR1D118A
SEQ ID NO: 189 - OGR1S221N/D118A
SEQ ID NO: 190 - RE2D124A
SEQ ID NO: 191 - GPR35D113A
SEQ ID NO: 192 - GPCR25D111A
SEQ ID NO: 193 - PGMO334E135F
SEQ ID NO: 194 - PGMO334E135Q
SEQ ID NO: 195 - PGMO334E135A
SEQ ID NO: 196 - PGMO335D259S
SEQ ID NO: 197 - GPR43R217P
SEQ ID NO: 198 - GPR43R217PE106D

## Claims

1. A method of isolating a DNA encoding a peptide capable of acting as an agonist, antagonist or inverse agonist for an aimed receptor, which comprises the following steps (1) to (5) :
(1) transfecting a cDNA or a DNA derived from a chromosome into a cell line derived from an endocrine cell to obtain a transformant;
(2) culturing the transformant of the above (1) to express the transfected DNA and contacting a culture supernatant, a cell extract or a membrane fraction of the transformant or the transformant *per se* with a cell where the aimed receptor is expressed;
(3) detecting a response reaction of the cell on the basis of the receptor;
(4) selecting a transformant where the culture supernatant, the cell extract or the membrane fraction of the transformant or the transformant *per se* shows the aimed activity; and
(5) identifying the DNA transfected in the transformant of the above (4) as a DNA for giving the aimed activity to the transformant.

2. A method of isolating a DNA encoding a peptide acting capable of as an agonist, antagonist or inverse agonist for an aimed receptor, which comprises the following steps (1) to (7):
(1) dividing a cDNA library prepared using an expression vector into pools each having 1 to 10,000 clone(s);
(2) transfecting a mixture of cDNA clones derived from each pool into a cell line derived from an endocrine cell to obtain a transformant;
(3) culturing the transformant of the above (2) for each pool to express the transfected cDNA and then contacting a culture supernatant, a cell extract or a membrane fraction of the transformant or the transformant *per se* for each pool with a cell where the aimed receptor is expressed;
(4) detecting a response reaction of the cell on the basis of the receptor for each pool;
(5) selecting a pool where the culture supernatant, cell extract or membrane fraction of the transformant or the transformant *per se* shows the aimed activity and dividing the selected pool into smaller pools than those in (1);
(6) repeating the operations of (2) to (5) until each pool consists of 1 clone; and
(7) identifying the cDNA which gives the aimed activity to the transformant.

3. The method of isolating a DNA according to claim 1 or 2, wherein the cDNA or the gene derived from a chromosome is a gene encoding an active peptide precursor.

4. The method of isolating a DNA according to claim 1 or 2, wherein the receptor is a G-protein coupled receptor.

5. The method of isolating a DNA according to claim 4, wherein the G-protein coupled receptor is an orphan G-protein coupled receptor.

6. The method of isolating a DNA according to claim 1 or 2, wherein the cell line derived from an endocrine cell is a cell line derived from an endocrine cell where the large T antigen gene of SV40 is expressed.

7. The method of isolating a DNA according to claim 1 or 2, wherein the cell line derived from an endocrine cell is a cell line derived from an endocrine cell where the large T antigen gene of temperature-sensitive mutant strain of SV40 is expressed.

8. The method of isolating a DNA according to claim 1, 2, 6 or 7, wherein the cell line derived from an endocrine cell is a cell line derived from a cell of a non-human transgenic animal.

9. The method of isolating a DNA according to claim 8, wherein the non-human transgenic animal is a transgenic rat.

10. The method of isolating a DNA according to claim 7, wherein the temperature-sensitive mutant strain of SV40 is SV40tsA58.

11. The method of isolating a DNA according to claim 1, 2, 6, 7 or 8, wherein the cell line derived from an endocrine cell is a cell line derived from hypothalamus or Langerhans islets.

12. The method of isolating a DNA according to claim 1, 2, 6, 7 or 8, wherein the cell line derived from an endocrine cell is a cell derived from hypothalamus where at least one gene selected from the group consisting of leptin receptor (Ob-Rb) gene, preproneuromedin U gene, RFamide-related peptide (RFRP) preproprotein gene, preproorexin gene, preproopiomelanocortin gene, preproneuropeptide Y gene, preproneuropeptide FF gene, preprocorticotropin-releasing hormone gene, preprothyrotropin-releasing hormone gene, preproghrelin gene, prepromelanin concentration hormone gene, cocaine- and amphetamine-regulated transcript (CART) gene, type 2 neuromedin U receptor (NMU2R) gene, RFRP receptor gene, type 4 melanocortin receptor (MC4R) gene, type 1 neuropeptide Y receptor (NPY1R) gene, type 5 neuropeptide Y receptor (NPY5R) gene, type 2 neuropeptide FF receptor (NPFF2) gene, type 1 corticotropin-releasing hormone receptor (CRHR-1) gene, type 2 corticotropin-releasing hormone receptor (CRHR-2) gene, ghrelin receptor gene, type 1 melanin concentration hormone receptor (MCHR1) gene, preproagouti-related peptide gene, sulfonylurea receptor gene, ciliary neurotrophic factor (CNTF) receptor gene, type 1 neuromedin U receptor (NMU1R) gene, type 1 orexin receptor (OX1R) gene, type 2 orexin receptor (OX2R) gene, type 1 angiotensin II receptor gene, galanin receptor, glucagon-like peptide-1 (GLP-1) receptor gene and glucagon-like peptide-2 (GLP-2) receptor gene is endogenously expressed.

13. The method of isolating a DNA according to claim 1, 2, 6, 7 or 8, wherein the cell line derived from an endocrine cell is a cell derived from hypothalamus where at least one peptide selected from the group consisting of leptin receptor (Ob-Rb), neuromedin U, RFamide-related peptide (RFRP) protein, orexin, opiomelanocortin, neuropeptide Y, neuropeptide FF, corticotropin-releasing hormone, thyrotropin-releasing hormone, ghrelin, melanin concentration hormone, cocaine- and amphetamine-regulated transcript (CART), type 2 neuromedin U receptor (NMU2R), RFRP receptor, type 4 melanocortin receptor (MC4R), type 1 neuropeptide Y receptor (NPY1R), type 5 neuropeptide Y receptor (NPY5R), type 2 neuropeptide FF receptor (NPFF2), type 1 corticotropin-releasing hormone receptor (CRHR-1), type 2 corticotropin-releasing hormone receptor (CRHR-2), ghrelin receptor, type 1 melanin concentration hormone receptor (MCHR1), agouti-relatedpeptide, sulfonylurea receptor, ciliary neurotrophic factor (CNTF) receptor, type 1 neuromedin U receptor (NMU1R), type 1 orexin receptor (OX1R), type 2 orexin receptor (OX2R), type 1 angiotensin II receptor, galanin receptor, glucagon-like peptide-1 (GLP-1) receptor, glucagon-like peptide-2 (GLP-2) receptor and endorphin is endogenously expressed.

14. The method of isolating a DNA according to claim 1, 2, 6, 7 or 8, wherein the cell of the cell line an endocrine cell is a cell derived from Langerhans islets where at least one gene selected from the group consisting of preproinsulin gene, prepro-glucagon gene, preprosomatostatin gene, prepropancreatic polypeptide gene, prohormone convertase 1 (PC1) gene, prohormone convertase 2 (PC2) gene, glucagon-like peptide-1 (GLP-1) receptor gene, PDX1 (pancreatic-duodenal homeobox 1) gene, Pax 4 gene, Pax 6 gene, neurogenin 3 gene, neuro D gene, Nkx 2.2 gene, Nkx 6.1 gene, glucokinase gene, type 2 glucose transporter gene, beta-cellulin gene, sulfonylurea gene, P2Y₁ receptor gene, glucagon-like peptide-1 (GLP-1) receptor gene, type 1 somatostatin receptor gene, type 2 somatostatin receptor gene, type 3 somatostatin receptor gene, type 4 somatostatin receptor gene, type 5 somatostatin receptor gene, insulin receptor gene, glucose transporter gene and nestin gene is endogenously expressed.

15. The method of isolating a DNA according to claim 1 or 2, wherein the cell in which the receptor is expressed is a B-cell line which is adapted for serum-free culture and in which the EBNA-1 gene of Epstein-Barr virus is expressed, where at least one of the following (1) to (3) is integrated in chromosomal DNA:
(1) DNA construct for expression of a transcription factor necessary for construction of an inducible expression system;
(2) DNA construct where a reporter gene is ligated at the downstream area of promoter having a responsive element of a transcription factor; and
(3) DNA construct for expression of Gα protein or chimeric Gα protein.

16. The method of isolating a DNA according to claim 15, wherein the B-cell line is a Namalwa cell adapted for serum-free culture.

17. The method of isolating a DNA according to claim 16, wherein the Namalwa cell adapted for serum-free culture is Namalwa KJM-1 cell.

18. The method of isolating a DNA according to claim 15, wherein the transcription factor necessary for construction of the inducible expression system is a chimeric protein of a ligand binding domain of estrogen receptor and yeast Gal4p.

19. The method of isolating a DNA according to claim 15, wherein the responsive element of the transcription factor is cAMP responsive element (CRE), TPA responsive element (TRE), NFAT (nuclear factor of activated T cells) responsive element or serum responsive element (SRE).

20. The method of isolating a DNA according to claim 15, wherein the reporter gene is firefly luciferase gene, *Renilla reniformis* luciferase gene, chloramphenicol acetyltransf erase gene, β-galactosidase gene, β-lactamase gene or green fluorescent protein gene.

21. The method of isolating a DNA according to claim 15, wherein the Gα protein is at least one Gα protein selected from the group consisting of Gα₁₆, Gα₁₅, Gα_{q}, Gα₁₁, Gαₛ, Gαᵢ, Gαₒ, Gα_{z}, Gα₁₂, Gα₁₃, Gα_{gust}, Gαₜ and Gα₁₄.

22. The method of isolating a DNA according to claim 15, wherein the chimeric Gα protein is at least one chimeric Gα protein selected from the group consisting of the following (1) to (20):
(1) chimeric Gα protein where C-terminal 5 amino acids of Gαₛ are substituted with C-terminal 5 amino acids of Gα_{q};
(2) chimeric Gα protein where C-terminal 5 amino acids of Gαₛ are substituted with C-terminal 5 amino acids of Gαᵢ;
(3) chimeric Gα protein where C-terminal 5 amino acids of Gαₛ are substituted with C-terminal 5 amino acids of Gαₒ;
(4) chimeric Gα protein where C-terminal 5 amino acids of Gαₛ are substituted with C-terminal 5 amino acids of Gα_{z};
(5) chimeric Gα protein where C-terminal 5 amino acids of Gαₛ are substituted with C-terminal 5 amino acids of Gα₁₂;
(6) chimeric Gα protein where C-terminal 5 amino acids of Gαₛ are substituted with C-terminal 5 amino acids of Gα₁₃;
(7) chimeric Gα protein where C-terminal 5 amino acids of Gαₛ are substituted with C-terminal 5 amino acids of Gα_{Gust};
(8) chimeric Gα protein where C-terminal 5 amino acids of Gαₛ are substituted with C-terminal 5 amino acids of Gαₜ;
(9) chimeric Gα protein where C-terminal 5 amino acids of Gαₛ are substituted with C-terminal 5 amino acids of Gα₁₄;
(10) chimeric Gα protein where C-terminal 5 amino acids of Gαₛ are substituted with C-terminal 5 amino acids of Gα₁₆;
(11) chimeric Gα protein where C-terminal 5 amino acids of Gα_{q} are substituted with C-terminal 5 amino acids of Gαₛ;
(12) chimeric Gα protein where C-terminal 5 amino acids of Gα_{q} are substituted with C-terminal 5 amino acids of Gαᵢ;
(13) chimeric Gα protein where C-terminal 5 amino acids of Gα_{q} are substituted with C-terminal 5 amino acids of Gαₒ;
(14) chimeric Gα protein where C-terminal 5 amino acids of Gα_{q} are substituted with C-terminal 5 amino acids of Gα_{z};
(15) chimeric Gα protein where C-terminal 5 amino acids of Gα_{q} are substituted with C-terminal 5 amino acids of Gα₁₂;
(16) chimeric Gα protein where C-terminal 5 amino acids of Gα_{q} are substituted with C-terminal 5 amino acids of Gα₁₃;
(17) chimeric Gα protein where C-terminal 5 amino acids of Gα_{q} are substituted with C-terminal 5 amino acids of Gα_{Gust};
(18) chimeric Gα protein where C-terminal 5 amino acids of Gα_{q} are substituted with C-terminal 5 amino acids of Gαₜ;
(19) chimeric Gα protein where C-terminal 5 amino acids of Gα_{q} are substituted with C-terminal 5 amino acids of Gα₁₄; and
(20) chimeric Gα protein where C-terminal 5 amino acids of Gα_{q} are substituted with C-terminal 5 amino acids of Gα₁₆.

23. The method of isolating a DNA according to claim 15, wherein the transcription factor necessary for construction of the inducible expression system is a chimeric protein of a ligand binding domain of estrogen receptor and yeast Gal4p, the promoter having a responsive element of the transcription factor is a promoter having a cAMP responsive element (CRE) and the reporter gene is firefly luciferase gene or *Renilla reniformis* luciferase gene.

24. The method of isolating a DNA according to claim 15, wherein the transcription factor necessary for construction of the inducible expression system is the chimeric protein of the ligand binding domain of estrogen receptor and yeast Gal4p, the promoter having a responsive element of the transcription factor is a promoter having a cAMP responsive element (CRE), the reporter gene is firefly luciferase gene or *Renilla reniformis* luciferase gene and the chimeric Gα protein is a chimeric Gα protein where C-terminal 5 amino acids of Gαₛ are substituted with C-terminal 5 amino acids of Gα_{q} or a chimeric Gα protein where C-terminal 5 amino acids of Gαₛ are substituted with C-terminal 5 amino acids of Gαᵢ.

25. The method of isolating a DNA according to claim 1 or 2, wherein the response reaction of the cell is at least one response reaction selected from the group consisting of liberation of arachidonic acid, liberation of acetylcholine, increase of intracellular Ca²⁺, production of intracellular cAMP, decrease of intracellular cAMP, production of intracellular cGMP, production of inositol phosphate, change in cell membrane potential, phosphorylation of intracellular protein, activation of c-fos, change in intracellular pH, cell growth, expression level of reporter gene and expression level of marker gene.

26. The method of isolating a DNA according to claim 1 or 2, wherein the contact to the cell expressing the aimed receptor is a contact by layering of the cell onto a transformant.

27. A cell line derived from hypothalamus where at least one gene selected from the group consisting of leptin receptor (Ob-Rb) gene, preproneuromedin U gene, RFamide-related peptide (RFRP) preproprotein gene, preproorexin gene, preproopiomelanocortin gene, preproneuropeptide, Y gene, preproneuropeptide FF gene, preprocorticotropin-releasing hormone gene, preprothyrotropin-releasing hormone gene, preproghrelin gene, prepromelanin concentration hormone gene, cocaine- and amphetamine-regulated transcript (CART) gene, type 2 neuromedin U receptor (NMU2R) gene, RFRP receptor gene, type 4 melanocortin receptor (MC4R) gene, type 1 neuropeptide Y receptor (NPY1R) gene, type 5 neuropeptide Y receptor (NPY5R) gene, type 2 neuropeptide FF receptor (NPFF2) gene, type 1 corticotropin-releasing hormone receptor (CRHR-1) gene, type 2 corticotropin-releasing hormone receptor (CRHR-2) gene, ghrelin receptor gene, type 1 melanin concentration hormone receptor (MCHR1) gene, preproagouti-related peptide gene, sulfonylurea receptor gene, ciliary neurotrophic factor (CNTF) receptor gene, type 1 neuromedin U receptor (NMU1R) gene, type 1 orexin receptor (OX1R) gene, type 2 orexin receptor (OX2R) gene, type 1 angiotensin II receptor gene, galanin receptor gene, glucagon-like peptide-1 (GLP-1) receptor gene and glucagon-like peptide-2 (GLP-2) receptor gene is endogenously expressed.

28. A cell line derived from hypothalamus where at least one peptide selected from the group consisting of leptin receptor (Ob-Rb), neuromedin U, RFamide-related peptide (RFRP) protein, orexin, opiomelanocortin, neuropeptide Y, neuropeptide FF, corticotropin-releasing hormone, thyrotropin-releasing hormone, ghrelin, melanin concentration hormone, cocaine- and amphetamine-regulated transcript (CART), type 2 neuromedin U receptor (NMU2R), RFRP receptor, type 4 melanocortin receptor (MC4R), type 1 neuropeptide Y receptor (NPY1R), type 5 neuropeptide Y receptor (NPY5R) , type 2 neuropeptide FF receptor (NPFF2), type 1 corticotropin-releasing hormone receptor (CRHR-1), type 2 corticotropin-releasing hormone receptor (CRHR-2), ghrelin receptor, type 1 melanin concentration hormone receptor (MCHR1), agouti-related peptide, sulfonylurea receptor, ciliary neurotrophic factor (CNTF) receptor, type 1 neuromedin U receptor (NMU1R), type 1 orexin receptor (OX1R), type 2 orexin receptor (OX2R), type 1 angiotensin II receptor, galanin receptor, glucagon-like peptide-1 (GLP-1) receptor, glucagon-like peptide-2 (GLP-2) receptor and endorphin is endogenously expressed.

29. The cell line according to claim 27 or 28, wherein the cell line is a cell line in which the large T antigen gene of SV40 is expressed.

30. The cell line according to claim 27 or 28, wherein the cell line is a cell line in which the large T antigen gene of temperature-sensitive mutant strain of SV40 is expressed.

31. The cell line according to any of claims 27 to 30, wherein the cell line is a cell line derived from a non-human transgenic animal cell.

32. An immortalized cell line obtained from Langerhans islets or hypothalamus of a non-human transgenic animal transfected with the large T antigen gene of temperature-sensitive mutant strain of SV40.

33. The cell line according to claim 32, wherein the immortalized cell line obtained from Langerhans islets is a cell line where at least one gene of the genes selected from the group consisting of preproinsulin gene, prepro-glucagon gene, preprosomatostatin gene, prepropancreatic polypeptide gene, prohormone convertase 1 (PC1) gene, prohormone convertase 2 (PC2) gene, glucagon-like peptide-1 (GLP-1) receptor gene, PDX1 (pancreatic-duodenal homeobox 1) gene, Pax 4 gene, Pax 6 gene, neurogenin 3 gene, neuro D gene, Nkx 2.2 gene, Nkx 6.1 gene, glucokinase gene, type 2 glucose transporter gene, beta-cellulin gene, sulfonylurea gene, P2Y₁ receptor gene, glucagon-like peptide-1 (GLP-1) receptor gene, type 1 somatostatin receptor gene, type 2 somatostatin receptor gene, type 3 somatostatin receptor gene, type 4 somatostatin receptor gene, type 5 somatostatin receptor gene, insulin receptor gene, glucose transporter gene and nestin gene is endogenously expressed.

34. The cell line according to claim 30 or 32, wherein the temperature-sensitive mutant strain of SV40 is SV40tsA58.

35. The cell line according to claim 31 or 32, wherein the non-human transgenic animal is a transgenic rat.

36. A process for producing a peptide, which comprises the following steps (1) and (2):
(1) culturing the cell line mentioned in any of claims 27 to 35 to produce and accumulate a peptide which is expressed by the cell endogenously, in the culture; and
(2) recovering the peptide from the culture obtained in the above (1).

37. A process for producing a peptide, which comprises the following steps (1) to (3):
(1) transfecting a DNA encoding an aimed peptide into a host cell to obtain a transformant wherein the cell line mentioned in any of claims 27 to 35 is used as the host cell;
(2) culturing the transformant to produce and accumulate a peptide in the culture; and
(3) recovering the peptide from the culture obtained in the above (2).

38. The process for producing a peptide according to claim 36 or 37, wherein culturing is carried out at the temperature where activity of the large T antigen of the temperature-sensitive mutant strain of SV40 is not suppressed.

39. The process for producing a peptide according to any of claims 36 to 38, which comprises a step for culturing in a serum-free medium, a medium containing not more than 2% of serum or a serum-free medium to which an N-supplement is added.

40. The process for producing a peptide according to any of claims 36 to 39, which comprises a step for culturing in a medium containing 5 to 30 mmol/L of glucose.

41. The process for producing a peptide according to any of claims 36 to 40, which comprises a step for culturing in a medium to which an agonist or antagonist for a receptor, a transporter or a channel expressed in the host cell is added.

42. The process for producing a peptide according to claim 41, wherein the receptor is G-protein coupled receptor, nuclear receptor, growth factor receptor, sulfonylurea receptor, ciliary neurotrophic factor receptor, leptin receptor, cytokine receptor or sulfonylurea receptor.

43. The process for producing a peptide according to claim 41, wherein the transporter is a glucose transporter.

44. The process for producing a peptide according to claim 41, wherein the channel is Ca channel, K channel, Cl channel or Na channel.

45. The process for producing a peptide according to any of claims 36 to 44, which comprises a step for culturing in a medium to which a substance capable of substituting a signal of a receptor expressed in a host cell is added.

46. The process for producing a peptide according to claim 45, wherein the substance capable of substituting the signal of the receptor is at least one substance selected from the group consisting of adenylate cyclase, protein kinase, phosphodiesterase, low-molecular G protein, substance capable of changing intracellular cAMP or intracellular Ca²⁺ content, forskolin, 8-bromo-cyclic AMP (8-Br-cAMP), phorbol 12-myristate 13-acetate (PMA), ionomycin and 3-isobutyl-1-methylxanthine.

47. The process for producing a peptide according to any of claims 36 to 46, which comprises a step for culturing on a dish coated with laminin or gelatin.

48. The process for producing a peptide according to any of claims 36 to 46, wherein culturing is carried out in a medium to which succinylated concanavalin A is added.

49. The process for producing a peptide according to any of claims 36 to 48, wherein culturing is carried out in a medium to which at least one substance selected from the group consisting of activin, glucagon-like peptide-1 (GLP-1), follistatin, glucose, hepatocyte growth factor, epidermal growth factor, nicotinamide, beta-cellulin, parathyroid hormone-related peptide, thyrotropin-releasing hormone, vascular endothelial growth factor, islet neogenesis-associated protein, platelet-derived growth factor, insulin-like growth factor I, fibroblast growth factor, nerve growth factor and Reg protein is added.

50. The process for producing a peptide according to any of claims 36 to 49, wherein a secretagogue is added after an active peptide is produced and accumulated in the culture.

51. The process for producing a peptide according to claim 50, wherein the secretagogue is potassium, glucose, tolbutamide or ATP.

52. The process for producing a peptide according to any of claims 37 to 51, wherein DNA encoding one or more peptide(s) is introduced into a host cell to produce plural peptides.

53. A method of detecting or isolating a peptide capable of acting as an agonist, an antagonist or an inverse agonist for an aimed receptor, which comprises the following steps (1) to (4):
(1) isolating a DNA encoding a peptide capable of acting as an agonist, an antagonist or an inverse agonist for an aimed receptor by the method mentioned in any of claims 1 to 26;
(2) contacting the peptide encoded by the DNA or a partial peptide thereof with a cell where the aimed receptor is expressed;
(3) detecting a response reaction of the cell on the basis of the receptor; and
(4) identifying the peptide or the partial peptide which gives a response reaction in the above (3).

54. A method of detecting or isolating a peptide capable of acting as an agonist, an antagonist or an inverse agonist for an aimed receptor, which comprises the following steps (1) to (4):
(1) preparing a peptide by the method of producing a peptide mentioned in any of claims 36 to 52;
(2) contacting the peptide with a cell where an aimed receptor is expressed;
(3) detecting a response reaction of the cell on the basis of the receptor; and
(4) identifying the peptide which gives a response reaction in the above (3).

55. The method of detecting or isolating a peptide according to claim 53 or 54, wherein the receptor is a G-protein coupled receptor.

56. The method of detecting or isolating a peptide according to claim 55, wherein the G-protein coupled receptor is an orphan G-protein coupled receptor.

57. The method of detecting or isolating a peptide according to claim 53 or 54, wherein the cell where the receptor is expressed is a B-cell line which is adapted for serum-free culture and in which the EBNA-1 gene of Epstein-Barr virus is expressed, where at least one of the following (1) to (3) is integrated into a chromosomal DNA:
(1) DNA construct for expression of transcription factor necessary for construction of an inducible expression system;
(2) DNA construct where a reporter gene is ligated at the downstream area of promoter having a responsive element of a transcription factor; and
(3) DNA construct for expression of Gα protein or a chimeric Gα protein.

58. The method of detecting or isolating a peptide according to claim 57, wherein the B-cell line is a Namalwa cell adapted for serum-free culture.

59. The method of detecting or isolating a peptide according to claim 58, wherein a Namalwa cell adapted for serum-free culture is Namalwa KJM-1 cell.

60. The method of detecting or isolating a peptide according to claim 57, wherein the transcription factor necessary for construction of the inducible expression system is a chimeric protein of a ligand binding domain of estrogen receptor and yeast Gal4p.

61. The method of detecting or isolating a peptide according to claim 57, wherein the responsive element of the transcription factor is cAMP responsive element (CRE), TPA responsive element (TRE), NFAT (nuclear factor of activated T cells) responsive element or serum responsive element (SRE) .

62. The method of detecting or isolating a peptide according to claim 57, wherein the reporter gene is firefly luciferase gene, *Renilla reniformis* luciferase gene, chloramphenicol acetyltransf erase gene, β-galactosidase gene, β-lactamase gene or green fluorescent protein gene.

63. The method of detecting or isolating a peptide according to claim 57, wherein the Gα protein is at least one Gα protein selected from the group consisting of Gα₁₆, Gα₁₅, Gα_{q}, Gα₁₁, Gαₛ , Gαᵢ, Gαₒ, Gα_{z}, Gα₁₂, Gα₁₃, Gα_{gust}, Gαₜ and Gα₁₄.

64. The method of detecting or isolating a peptide according to claim 57, wherein the chimeric Gα protein is at least one chimeric Gα protein selected from the group consisting of the following (1) to (20):
(1) chimeric Gα protein where C-terminal 5 amino acids of Gαₛ are substituted with C-terminal 5 amino acids of Gα_{q};
(2) chimeric Gα protein where C-terminal 5 amino acids of Gαₛ are substituted with C-terminal 5 amino acids of Gαᵢ;
(3) chimeric Gα protein where C-terminal 5 amino acids of Gαₛ are substituted with C-terminal 5 amino acids of Gαₒ;
(4) chimeric Gα protein where C-terminal 5 amino acids of Gαₛ are substituted with C-terminal 5 amino acids of Gα_{z};
(5) chimeric Gα protein where C-terminal 5 amino acids of Gαₛ are substituted with C-terminal 5 amino acids of Gα₁₂;
(6) chimeric Gα protein where C-terminal 5 amino acids of Gαₛ are substituted with C-terminal 5 amino acids of Gα₁₃;
(7) chimeric Gα protein where C-terminal 5 amino acids of Gαₛ are substituted with C-terminal 5 amino acids of Gα_{gust};
(8) chimeric Gα protein where C-terminal 5 amino acids of Gαₛ are substituted with C-terminal 5 amino acids of Gαₜ;
(9) chimeric Gα protein where C-terminal 5 amino acids of Gαₛ are substituted with C-terminal 5 amino acids of Gα₁₄;
(10) chimeric Gα protein where C-terminal 5 amino acids of Gαₛ are substituted with C-terminal 5 amino acids of Gα₁₆;
(11) chimeric Gα protein where C-terminal 5 amino acids of Gα_{q} are substituted with C-terminal 5 amino acids of Gαₛ ;
(12) chimeric Gα protein where C-terminal 5 amino acids of Gα_{q} are substituted with C-terminal 5 amino acids of Gαᵢ;
(13) chimeric Gα protein where C-terminal 5 amino acids of Gα_{q} are substituted with C-terminal 5 amino acids of Gαₒ;
(14) chimeric Gα protein where C-terminal 5 amino acids of Gα_{q} are substituted with C-terminal 5 amino acids of Gα_{z};
(15) chimeric Gα protein where C-terminal 5 amino acids of Gα_{q} are substituted with C-terminal 5 amino acids of Gα₁₂;
(16) chimeric Gα protein where C-terminal 5 amino acids of Gα_{q} are substituted with C-terminal 5 amino acids of Gα₁₃;
(17) chimeric Gα protein where C-terminal 5 amino acids of Gα_{q} are substituted with C-terminal 5 amino acids of Gα_{gust};
(18) chimeric Gα protein where C-terminal 5 amino acids of Gα_{q} are substituted with C-terminal 5 amino acids of Gαₜ;
(19) chimeric Gα protein where C-terminal 5 amino acids of Gα_{q} are substituted with C-terminal 5 amino acids of Gα₁₄; and
(20) chimeric Gα protein where C-terminal 5 amino acids of Gα_{q} are substituted with C-terminal 5 amino acids of Gα₁₆.

65. The method of detecting or isolating a peptide according to claim 57, wherein the transcription factor necessary for construction of the inducible expression system is a chimeric protein of a ligand binding domain of estrogen receptor and yeast Gal4p, the promoter having a responsive element of the transcription factor is a promoter having a cAMP responsive element (CRE) and the reporter gene is firefly luciferase gene or *Renilla reniformis* luciferase gene.

66. The method of detecting or isolating a peptide according to claim 57, wherein the transcription factor necessary for construction of the inducible expression system is a chimeric protein of a ligand binding domain of estrogen receptor and yeast Gal4p, the promoter having a responsive element of the transcription factor is a promoter having a cAMP responsive element (CRE), the reporter gene is firefly luciferase gene or *Renilla reniformis* luciferase gene and the chimeric Gα protein is a chimeric Gα protein where C-terminal 5 amino acids of Gαₛ are substituted with C-terminal 5 amino acids of Gα_{q} or a chimeric Gα protein where C-terminal 5 amino acids of Gαₛ are substituted with C-terminal 5 amino acids of Gαᵢ.

67. The method of detecting or isolating a peptide according to claim 53 or 54, wherein the response reaction of the cell is at least one response reaction selected from the group consisting of release of arachidonic acid, release of acetylcholine, increase of intracellular Ca²⁺, production of intracellular cAMP, decrease of intracellular cAMP, production of intracellular cGMP, production of inositol phosphate, change in cell membrane potential, phosphorylation of intracellular protein, activation of c-fos, change in intracellular pH, cell growth, expression level of reporter gene and expression level of marker gene.

68. A cell line derived from a B-cell line which is adapted for serum-free culture and in which the EBNA-1 gene of Epstein-Barr virus is expressed, where at least one of the following (1) to (3) is integrated into a chromosomal DNA:
(1) DNA construct for expression of a transcription factor necessary for construction of an inducible expression system;
(2) DNA construct where a reporter gene is ligated at the downstream area of a promoter having a responsive element of a transcription factor; and
(3) DNA construct for expression of Gα protein or a chimeric Gα protein.

69. The cell line according to claim 68, wherein the cell line is a Namalwa cell adapted for serum-free culture.

70. The cell line according to claim 69, wherein the Namalwa cell adapted for serum-free culture is Namalwa KJM-1 cell.

71. The cell line according to claim 68, wherein the transcription factor necessary for construction of the inducible expression system is a chimeric protein of a ligand binding domain of estrogen receptor and yeast Ga14p.

72. The cell line according to claim 68, wherein the responsive element of the transcription factor is cAMP responsive element (CRE), TPA responsive element (TRE), NFAT (nuclear factor of activated T cells) responsive element or serum responsive element (SRE).

73. The cell line according to claim 68, wherein the reporter gene is firefly luciferase gene, *Renilla reformis* luciferase gene, chloramphenicol acetyltransferase gene, β-galactosidase gene, β-lactamase gene or green fluorescent protein gene.

74. The cell line according to claim 68, wherein the Gα protein is at least one Gα protein selected from the group consisting of Gα₁₆, Gα₁₅, Gα_{q}, Gα₁₁, Gαₛ, Gαᵢ, Gαₒ, Gα_{z}, Gα₁₂, Gα₁₃, Gα_{gust}, Gαₜ and Gα₁₄.

75. The cell line according to claim 68, wherein the chimeric Gα protein is at least one chimeric Gα protein selected from the group consisting of the following (1) to (20):
(1) chimeric Gα protein where C-terminal 5 amino acids of Gαₛ are substituted with C-terminal 5 amino acids of Gα_{q};
(2) chimeric Gα protein where C-terminal 5 amino acids of Gαₛ are substituted with C-terminal 5 amino acids of Gαᵢ;
(3) chimeric Gα protein where C-terminal 5 amino acids of Gαₛ are substituted with C-terminal 5 amino acids of Gαₒ;
(4) chimeric Gα protein where C-terminal 5 amino acids of Gαₛ are substituted with C-terminal 5 amino acids of Gα_{z};
(5) chimeric Gα protein where C-terminal 5 amino acids of Gαₛ are substituted with C-terminal 5 amino acids of Gα₁₂;
(6) chimeric Gα protein where C-terminal 5 amino acids of Gαₛ are substituted with C-terminal 5 amino acids of Gα₁₃;
(7) chimeric Gα protein where C-terminal 5 amino acids of Gαₛ are substituted with C-terminal 5 amino acids of Gα_{gust};
(8) chimeric Gα protein where C-terminal 5 amino acids of Gαₛ are substituted with C-terminal 5 amino acids of Gαₜ;
(9) chimeric Gα protein where C-terminal 5 amino acids of Gαₛ are substituted with C-terminal 5 amino acids of Gα₁₄;
(10) chimeric Gα protein where C-terminal 5 amino acids of Gαₛ are substituted with C-terminal 5 amino acids of Gα₁₆;
(11) chimeric Gα protein where C-terminal 5 amino acids of Gα_{q} are substituted with C-terminal 5 amino acids of Gαₛ ;
(12) chimeric Gα protein where C-terminal 5 amino acids of Gα_{q} are substituted with C-terminal 5 amino acids of Gαᵢ;
(13) chimeric Gα protein where C-terminal 5 amino acids of Gα_{q} are substituted with C-terminal 5 amino acids of Gαₒ;
(14) chimeric Gα protein where C-terminal 5 amino acids of Gα_{q} are substituted with C-terminal 5 amino acids of Gα_{z};
(15) chimeric Gα protein where C-terminal 5 amino acids of Gα_{q} are substituted with C-terminal 5 amino acids of Gα₁₂;
(16) chimeric Gα protein where C-terminal 5 amino acids of Gα_{q} are substituted with C-terminal 5 amino acids of Gα₁₃;
(17) chimeric Gα protein where C-terminal 5 amino acids of Gα_{q} are substituted with C-terminal 5 amino acids of Gα_{gust};
(18) chimeric Gα protein where C-terminal 5 amino acids of Gα_{q} are substituted with C-terminal 5 amino acids of Gαₜ;
(19) chimeric Gα protein where C-terminal 5 amino acids of Gα_{q} are substituted with C-terminal 5 amino acids of Gα₁₄; and
(20) chimeric Gα protein where C-terminal 5 amino acids of Gα_{q} are substituted with C-terminal 5 amino acids of Gα₁₆.

76. The cell line according to claim 68, wherein the transcription factor necessary for construction of the inducible expression system is a chimeric protein of a ligand binding domain of estrogen receptor and yeast Gal4p, the promoter having a responsive element of the transcription factor is a promoter having a cAMP responsive element (CRE) and the reporter gene is firefly luciferase gene or *Renilla reniformis* luciferase gene.

77. The cell line according to claim 68, wherein the transcription factor necessary for construction of the inducible expression system is a chimeric protein of a ligand binding domain of estrogen receptor and yeast Gal4p, the promoter having a responsive element of the transcription factor is a promoter having a CAMP responsive element (CRE), the reporter gene is firefly luciferase gene or *Renilla reformis* luciferase gene and the chimeric Gα protein is a chimeric Gα protein where C-terminal 5 amino acids of Gαₛ are substituted with C-terminal 5 amino acids of Gα_{q} or a chimeric Gα protein where C-terminal 5 amino acids of Gαₛ are substituted with C-terminal 5 amino acids of Gαᵢ.

78. A method of isolating a DNA encoding a peptide capable of reacting with a substance to be tested, which comprises the following steps (1) to (4):
(1) transfecting a cDNA or a DNA derived from a chromosome into the cell line mentioned in any of claims 68 to 77 to obtain a transformant;
(2) measuring a response reaction of the transformant in the presence of the substance to be tested using the transformant in which the transfected cDNA or chromosomal DNA is expressed;
(3) measuring a response reaction of the transformant in the absence of the substance to be tested using the transformant in which the transfected cDNA or chromosomal DNA is expressed; and
(4) comparing the above response reactions (2) and (3), selecting the transformant showing different response reaction and identifying the DNA transfected in the transformant.

79. A method of isolating a DNA encoding a peptide capable of reacting with a substance to be tested, which comprises the following steps (1) to (7):
(1) dividing a cDNA library prepared using expression vector into pools each having 1 to 10,000 clone(s);
(2) transfecting a mixture of cDNA clones derived from each pool into a cell line mentioned in any of claims 68 to 77 to obtain a transformant;
(3) measuring a response reaction of the transformant in the presence of the substance to be tested for each pool using the transformant in which the transfected cDNA is expressed;
(4) measuring a response reaction of the transformant in the absence of the substance to be tested for each pool using the transformant in which the transfected cDNA is expressed;
(5) comparing the above response reactions of (3) and (4), selecting the pool showing different response reaction and dividing the selected pool into smaller pools than those in (1);
(6) repeating the operations of (2) to (5) until each pool consists of one clone; and
(7) identifying the transformant showing difference response reaction in the presence and the absence of the substance to be tested and identifying the DNA which is transfected in the transformant.

80. The method of isolating a DNA according to claim 78 or 79, wherein the response reaction of the cell is at least one response reaction selected from the group consisting of release of arachidonic acid, release of acetylcholine, increase of intracellular Ca²⁺, production of intracellular cAMP, decrease of intracellular cAMP, production of intracellular cGMP, production of inositol phosphate, change in cell membrane potential, phosphorylation of intracellular protein, activation of c-fos, change in intracellular pH, cell growth, expression level of a reporter gene and expression level of a marker gene.

81. The method of isolating a DNA according to claim 78 or 79, wherein the substance to be tested is a substance which is prepared by culturing the cell line mentioned in any of claims 27 to 35.

82. The method of isolating a DNA according to any of claims 78 to 81, wherein culturing is carried out at the temperature where activity of large T antigen of temperature-sensitive mutant of SV40 is suppressed or disappeared.

83. A method of isolating a DNA encoding a peptide, which comprises the following steps (1) to (5):
(1) selecting a cell line where a DNA construct comprising a reporter gene ligated to downstream area of promoter having a response element of transcription factor is integrated in chromosomal DNA, which is selected from the cell lines mentioned in any of claims 68 to 77, as a host cell;
(2) transfecting a cDNA or a DNA derived from a chromosome into the host cell to obtain a transformant;
(3) measuring the expression level of the reporter gene in the transformant obtained in the above (2) when the transfected cDNA or DNA is expressed;
(4) measuring the expression level of the reporter gene in the host cell or in the transformant obtained in the above (2) when the transfected cDNA or DNA is not expressed; and
(5) comparing the expression levels of the reporter gene in the above (3) and (4), selecting the transformant showing different expression level of the reporter gene, and identifying a DNA transfected in the transformant.

84. A method of isolating a DNA encoding a peptide, which comprises the following steps (1) to (8):
(1) dividing a cDNA library prepared using the inducible expression vector into pools each having 1 to 10,000 clone(s);
(2) selecting a cell line where DNA construct comprising a reporter gene ligated to downstream area of promoter having a response element of transcription factor is integrated in chromosomal DNA, which is selected from the cell lines mentioned in any of claims 68 to 77, as a host cell;
(3)transfecting a mixture of cDNA clones derived from each pool divided in the above (1) into the host cell obtained in the above (2) to obtain a transformant for each pool;
(4) measuring the expression level of reporter gene in the transformant for each pool of the above (3) when the transfected cDNA is expressed;
(5) measuring the expression level of reporter gene in the transformant for each pool of the above (3) when the transfected cDNA is not expressed;
(6) comparing the expression levels of reporter genes in the above (4) and (5) for each pool, selecting the transformant showing higher expression level of reporter gene in (4) and dividing the selected pool into smaller pools than those in (1);
(7) repeating the operations of the above (2) to (6) until each pool consists of one clone; and
(8) identifying a transformant showing higher expression level of reporter gene in case the transfected cDNA is expressed than in case the transfected cDNA is not expressed and identifying the DNA transfected in the transformant.

85. The method of isolating a DNA according to claim 78, 79, 83 or 84, wherein the peptide is a receptor, a transcription factor, a signal transduction molecule or an enzyme.

86. The method of isolating a DNA according to claim 85, wherein the receptor is a G-protein coupled receptor.

87. The method of isolating a DNA according to claim 85, wherein the receptor is a constitutively activated G-protein coupled receptor.

88. The method of isolating a DNA according to claim 86 or 87, wherein the G-protein coupled receptor is an orphan G-protein coupled receptor.

89. The method of isolating a DNA according to claim 78, 79, 83 or 84, wherein the peptide is a transcription factor, a signal transduction molecule or an enzyme, each of which has an activity to increase the activity of promoter having a responsive element of a transcription factor.

90. The method of isolating a DNA according to claim 78, 79, 83 or 84, wherein the cDNA is a cDNA with a random mutation, encoding a mutant G-protein coupled receptor, and the peptide is a constitutively activated G-protein coupled receptor.

91. The method of isolating a DNA according to claim 90, wherein the site into which the random mutation is introduced is from the second-half part of the third transmembrane region to the first-half part of the second intracellular region, or from the second-half part of the third intracellular region to the first-half part of the sixth transmembrane region in the G-protein coupled receptor.

92. The method of isolating a DNA according to claim 90 or 91, wherein the site into which the random mutation is introduced is an amino acid which is the 20th residue or the 22nd residue directed to the N-terminal side from a proline residue existing in the sixth transmembrane region, in which the proline residue is one of conserved amino acid residues in a G-protein coupled receptor, or an amino acid residue corresponding to the proline residue.

93. A constitutively activated mutant G-protein coupled receptor which is isolated by the method mentioned in any of claims 90 to 92.

94. A constitutively activated mutant G-protein coupled receptor having a mutation at an amino acid which is the 20th residue or the 22nd residue directed to the N-terminal side fromaproline residue existing in the sixth transmembrane region, in which the proline residue is one of conserved amino acid residues in a G-protein coupled receptor, or an amino acid residue corresponding to the proline residue.

95. A constitutively activated mutant G-protein coupled receptor selected from the group consisting of a constitutively activated mutant G-protein coupled receptor where the 221st serine from N terminal of OGR1 is substituted with asparagine; a constitutively activated mutant G-protein coupled receptor where the 118th aspartic acid from N terminal of OGR1 is substituted with alanine; a constitutively activated mutant G-protein coupled receptor where the 118th aspartic acid from N terminal of OGR1 is substituted with alanine and serine which is the 221st one is substitutedwith asparagine; a constitutively activated mutant G-protein coupled receptor where the 124th aspartic acid fromN terminal of RE2 is substituted with alanine; a constitutively activated mutant G-protein coupled receptor where the 113th aspartic acid from N terminal of GPR35 is substituted with alanine; a constitutively activated mutant G-protein coupled receptor where the 111th aspartic acid from N terminal of GPCR25 is substituted with alanine; a constitutively activated mutant G-protein coupled receptor where the 135th glutamic acid from N terminal of PGMO334 is substituted with phenylalanine, glutamine or alanine; a constitutively activated mutant G-protein coupled receptor where the 259th aspartic acid from N terminal of PGMO334 is substituted with serine; a constitutively activated mutant G-protein coupled receptor where the 217th arginine from N-terminal of GPR43 is substituted with proline; and a constitutively activated mutant G-protein coupled receptor where the 217th arginine and the 106th glutamic acid from N-terminal of GPR43 is substituted with proline and aspartic acid, respectively.

96. A method of screening or isolating an antagonist for MC1R using a type 1 melanocortin receptor (MC1R) and proadrenomedullin N-20 terminal peptide (PAMP) or a peptide consisting of the 9th to 20th amino acid residues from N-terminal of PAMP.

97. A method of screening or isolating an antagonist for GPR43 using an orphan G-protein coupled receptor GPR43 and acetic acid, propionic acid, acetate or propionate.

98. A method of screening or isolating an antagonist for GPR41 using an orphan G-protein coupled receptor GPR41 and cyclopropanecarboxylic acid, propionic acid, cyclopropanecarboxylate or propionate.

99. A method of screening or isolating an antagonist for G10d using an orphan G-protein coupled receptor G10d and an α-melanocyte stimulating hormone or adrenocorticotropic hormone.

100. A method of isolating an agonist for a G-protein coupled receptor, which comprises the following steps (1) to (4):
(1) transfecting a DNA encoding a G-protein coupled receptor into the cell line mentioned in any of claims 68 to 77 to obtain a transformant;
(2) measuring a response reaction of the transformant in the presence of a substance to be tested using the transformant in which the transfected DNA is expressed;
(3) measuring a response reaction of the transformant in the absence of a substance to be tested using the transformant in which the transfected DNA is expressed; and
(4) comparing the response reactions of the above (2) and (3) and isolating the substance to be tested which induces the changes in the response reaction as an agonist.

101. A method of isolating an antagonist for a G-protein coupled receptor, which comprises the following steps (1) to (4):
(1) transfecting a DNA encoding a G-protein coupled receptor into the cell line mentioned in any of claims 68 to 77 to obtain a transformant;
(2) measuring a response reaction of the transformant in the presence of an agonist for the G-protein coupled receptor using the transformant in which the transf ected DNA is expressed;
(3) measuring a response reaction of the transformant in the presence of both an agonist for the G-protein coupled receptor and a substance to be tested using the transformant in which the transfected DNA is expressed; and
(4) isolating the substance to be tested which disappears the response reaction on the basis of the agonist of the transformant as an antagonist.

102. The method of isolating an agonist or an antagonist for a G-protein coupled receptor according to claim 100 or 101, wherein the response reaction of the cell is at least one response reaction selected from the group consisting of release of arachidonic acid, release of acetylcholine, increase of intracellular Ca²⁺, production of intracellular cAMP, decrease of intracellular cAMP, production of intracellular cGMP, production of inositol phosphate, change in cell membrane potential, phosphorylation of intracellular protein, activation of c-fos, change in intracellular pH, cell growth, expression level of reporter gene and expression level of marker gene.

103. Amethodof isolating an antagonist or inverse agonist for a G-protein coupled receptor, which comprises the following steps (1) to (5):
(1) selecting a cell line where a DNA construct comprising a reporter gene ligated to downstream area of promoter having a response element of transcription factor is integrated in chromosomal DNA, which is selected from the cell lines mentioned in any of claims 68 to 77, as a host cell;
(2) transfecting a DNA encoding a constitutively activated G-protein coupled receptor into the host cell to obtain a transformant;
(3) measuring the expression level of a reporter gene in the transformant in which the transfected DNA is expressed in the absence of a substance to be tested;
(4) measuring the expression level of a reporter gene in the transformant in which the transfected DNA is expressed in the presence of a substance to be tested; and
(5) comparing the expression levels of the reporter gene of the above (3) and (4) and isolating a substance to be tested which decrease the expression level of the reporter gene as an antagonist or an inverse agonist for the G-protein coupled receptor of (2).

104. A method of isolating an activator or inhibitor for a peptide selected from the group consisting of transcription factors, signal transduction molecules and enzymes, which comprises the following steps (1) to (4):
(1) transfecting a DNA encoding a peptide selected from the group consisting of transcription factors, signal transduction molecules and enzymes into the cell line mentioned in any of claims 68 to 77 to obtain a transformant;
(2) measuring a response reaction of the transformant in the absence of a substance to be tested using the transformant in which the transfected DNA is expressed;
(3) measuring a response reaction of the transformant in the presence of a substance to be tested using the transformant in which the transfected DNA is expressed; and
(4) comparing the response reactions of the above (2) and (3) and isolating the substance to be tested which changes the response reaction as an activator or an inhibitor for the peptide.

105. The method of isolating an activator or inhibitor according to claim 104, wherein the response reaction of the cell is at least one response reaction selected from the group consisting of release of arachidonic acid, release of acetylcholine, increase of intracellular Ca²⁺, production of intracellular cAMP, decrease of intracellular cAMP, production of intracellular cGMP, production of inositol phosphate, change in cell membrane potential, phosphorylation of intracellular protein, activation of c-fos, change in intracellular pH, cell growth, expression level of reporter gene and expression level of marker gene.

106. A host-vector system which is **characterized in that** a cell line mentioned in any of claims 68 to 77 is used as a host cell and an expression vector having a promoter and oriP of Epstein-Barr virus is used as a vector.

107. The host-vector system according to claim 106, wherein the promoter is a Gal4p-responsible inducible expression promoter.

108. The host-vector system according to claim 106 or 107, wherein the expression vector is pAMO, pAMO-nd, pAMO-d, pAGal9-nd or pAGal9-d.
